(19) **Europäisches Patentamt / European Patent Office / Office européen des brevets**

(11) **EP 2 661 626 B1**

(12) # EUROPEAN PATENT SPECIFICATION

(45) Date of publication and mention of the grant of the patent:
**01.06.2016 Bulletin 2016/22**

(21) Application number: **12732048.9**

(22) Date of filing: **08.01.2012**

(51) Int Cl.:
**G01N 33/53** *(2006.01)*    **G01N 33/68** *(2006.01)*

(86) International application number:
**PCT/US2012/020572**

(87) International publication number:
**WO 2012/094658 (12.07.2012 Gazette 2012/28)**

(54) **METHODS AND COMPOSITIONS FOR DIAGNOSIS AND PROGNOSIS OF RENAL INJURY AND RENAL FAILURE**

VERFAHREN UND ZUSAMMENSETZUNGEN ZUR DIAGNOSE UND PROGNOSE VON NIERENVERLETZUNGEN UND NIERENINSUFFIZIENZ

PROCÉDÉS ET COMPOSITIONS POUR DIAGNOSTIQUER ET PRONOSTIQUER UNE LÉSION RÉNALE ET UNE INSUFFISANCE RÉNALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.01.2011   US 201161430989 P**
**08.01.2011   US 201161430986 P**
**08.01.2011   US 201161430977 P**
**08.01.2011   US 201161430979 P**
**08.01.2011   US 201161430980 P**
**08.01.2011   US 201161430982 P**
**08.01.2011   US 201161430987 P**
**08.01.2011   US 201161430981 P**
**08.01.2011   US 201161430984 P**
**08.01.2011   US 201161430983 P**
**08.01.2011   US 201161430988 P**
**08.01.2011   US 201161430978 P**
**08.01.2011   US 201161430985 P**

(43) Date of publication of application:
**13.11.2013  Bulletin 2013/46**

(73) Proprietor: **Astute Medical, Inc.**
**San Diego, CA 92121 (US)**

(72) Inventors:
• **ANDERBERG, Joseph**
**Encinitas, CA 92024 (US)**
• **GRAY, Jeff**
**Solana Beach, CA 92075 (US)**
• **MCPHERSON, Paul**
**Encinitas, CA 92024 (US)**
• **NAKAMURA, Kevin**
**Cardiff By The Sea, CA 92007 (US)**
• **KAMPF, James, Patrick**
**San Diego, CA 92130 (US)**

(74) Representative: **Schiweck, Weinzierl & Koch**
**European Patent Attorneys**
**Landsberger Straße 98**
**80339 München (DE)**

(56) References cited:
**WO-A1-2010/059996      WO-A1-2010/091236**
**US-A1- 2009 022 730      US-A1- 2009 197 287**
**US-A1- 2010 022 627**

• **S. STEIN ET AL: "Serum Levels of the Adipokine FGF21 Depend on Renal Function", DIABETES CARE, vol. 32, no. 1, 1 January 2009 (2009-01-01), pages 126-128, XP055098519, ISSN: 0149-5992, DOI: 10.2337/dc08-1054**
• **HAN S H ET AL: "Serum fibroblast growth factor-21 concentration is associated with residual renal function and insulin resistance in end-stage renal disease patients receiving long-term peritoneal dialysis", METABOLISM, CLINICAL AND EXPERIMENTAL, W.B. SAUNDERS CO., PHILADELPHIA, PA, US, vol. 59, no. 11, 1 November 2010 (2010-11-01), pages 1656-1662, XP027421750, ISSN: 0026-0495, DOI: 10.1016/J.METABOL.2010.03.018 [retrieved on 2010-04-27]**

**(Cont. next page)**

- YUE DU ET AL: "Urinary biomarkers to detect acute kidney injury in the pediatric emergency center", PEDIATRIC NEPHROLOGY ; JOURNAL OF THE INTERNATIONAL PEDIATRIC NEPHROLOGY ASSOCIATION, SPRINGER, BERLIN, DE, vol. 26, no. 2, 27 October 2010 (2010-10-27), pages 267-274, XP019856153, ISSN: 1432-198X, DOI: 10.1007/S00467-010-1673-0
- COCA STEVEN G ET AL: "Urinary biomarkers for acute kidney injury: perspectives on translation", CLINICAL JOURNAL OF THE AMERICAN SOCIETY OF NEPHROLOGY : CJASN, AMERICAN SOCIETY OF NEPHROLOGY, US, vol. 3, no. 2, 1 March 2008 (2008-03-01), pages 481-490, XP009108667, ISSN: 1555-905X, DOI: 10.2215/CJN.03520807
- Publishing Blackwell ET AL: "Publishing Ltd Protein biomarkers associated with acute renal failure and chronic kidney disease", European Journal of Clinical Investigation Journal Compilation Blackwell Eur J Clin Invest, 1 January 2006 (2006-01-01), pages 753-763, XP55098668, Retrieved from the Internet: URL:http://onlinelibrary.wiley.com/store/10.1111/j.1365-2362.2006.01729.x/asset/j.1365-2362.2006.01729.x.pdf?v=1&t=hqxw8hun&s=f312bbc9e40495d2fe10c345fb2690a9a4e077ed [retrieved on 2014-01-27]
- LIANG X L ET AL: "Beyond Early Diagnosis: Prognostic Biomarkers for Monitoring Acute Kidney Injury", HONG KONG JOURNAL OF NEPHROLOGY, XX, HG, vol. 12, no. 2, 1 October 2010 (2010-10-01), pages 45-49, XP027547649, ISSN: 1561-5413 [retrieved on 2010-10-01]
- WINCHESTER ET AL.: 'Sorbents in Acute Renal Failure and End-Stage Renal Disease: Middle Molecule and Cytokine Removal.' BLOOD PURIF vol. 22, 2004, pages 73 - 77, XP055092301
- SCHMALDIENST ET AL.: 'Angiogenin: a novel Inhibitor of neutrophil lactoterrin release during extracorporeal circulation.' KIDNEY BLOOD PRESS RES. vol. 26, no. 2, 2003, pages 107 - 12
- ANILKUMAR ET AL.: 'Trimeric assembly of the C-terminal region of Thrombospondin-1 or Thrombospondin-2 is necessary for cell spreading and fascin spike organisation.' J CELL SCI. vol. 115, no. 11, 2002, pages 2357 - 2366, XP055092304
- ARRIBAS ET AL.: 'ADAM17 as a Therapeutic Target in Multiple Diseases' CURR PHARM DES. vol. 15, no. 20, 2009, pages 2319 - 2335, XP009145784
- HUGO ET AL.: 'Thrombospondin in Renal Disease' NEPHRON EXP NEPHROL vol. 111, 2009, pages E61 - E66, XP055092308
- OHNO ET AL.: 'Prognostic significance of tenascin-C expression in clear cell renal cell carcinoma.' ONCOL REP. vol. 20, no. 3, 2008, pages 511 - 516, XP055092309
- NGUYEN ET AL.: 'Heparin-Binding EGF-Like Growth Factor Is Up-Regulated in the Obstructed Kidney in a Cell- and Region-Specific Manner and Acts to Inhibit Apoptosis.' AM J PATHOL. vol. 156, no. 3, 2000, pages 889 - 898, XP055092313

**Description**

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222, and which is hereby incorporated by reference in their entirety:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |
| **Intrinsic Renal** | |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Acute tubulointerstitial | Drug reaction (eg, $\beta$-lactams, NSAIDs, sulfonamides, |

(continued)

| Type | Risk Factors |
|---|---|
| nephritis | ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| **Postrenal** | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005, As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004, investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, proposes the following classifications for stratifying AKI patients:

"Risk": serum creatinine increased 1.5 fold from baseline OR urine production of <0.5 ml/kg body weight/hr for 6 hours;
"Injury": serum creatinine increased 2.0 fold from baseline OR urine production <0.5 ml/kg/hr for 12 h;
"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.
"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, the RIFLE criteria provide a uniform definition of AKI which has been validated in numerous studies.
More recently, Mehta et al., Crit. Care 11:R31 (doi:10.1186.cc5713), 2007, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;
"Stage II": increase in serum creatinine to more than 200% (> 2-fold) from baseline OR urine output less than 0.5 mL/kg per hour for more than 12 hours;
"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197, uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.
Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI. WO2010059996 discloses a method for the prognosis of acute renal failure on the basis of a panel of biomarkers, which however lacks FGF21.
These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1 (XVIII) chain (each referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The invention is defined by the appended claims.

The kidney injury markers of the present invention may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.,* hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present disclosure relates to methods for evaluating renal status in a subject. These methods comprise performing an assay method that is configured to detect one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1(XVIII) chain is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc.,

and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma. In the case of those kidney injury markers which are membrane proteins as described hereinafter, preferred assays detect soluble forms thereof.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score, risk scores of Thakar et al. (J. Am. Soc. Nephrol. 16: 162-68, 2005), Mehran et al. (J. Am. Coll. Cardiol. 44: 1393-99, 2004), Wijeysundera et al. (JAMA 297: 1801-9, 2007), Goldstein and Chawla (Clin. J. Am. Soc. Nephrol. 5: 943-49, 2010), or Chawla et al. (Kidney Intl. 68: 2274-80, 2005)), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, etc. In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more biomarkers selected from the group consisting of Proheparin-binding EGF-like growth factor, Tenascin C, Angiopoietin-related protein 4, Fibroblast growth factor 19, Fibroblast growth factor 21, Heparin-binding growth factor 1, Angiopoietin-related protein 6, Proepiregulin, Probetacellulin, Amphiregulin, Angiogenin, Thrombospondin-2, and Collagen alpha-1 (XVIII) chain or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc*. "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

As used herein, the term "Proheparin-binding EGF-like growth factor" refers to one or more polypeptides present in a biological sample that are derived from the Proheparin-binding EGF-like growth factor precursor (Swiss-Prot Q99075 (SEQ ID NO: 1)):

```
        10         20         30         40         50         60
    MKLLPSVVLK LFLAAVLSAL VTGESLERLR RGLAAGTSNP DPPTVSTDQL LPLGGGRDRK

        70         80         90        100        110        120
    VRDLQEADLD LLRVTLSSKP QALATPNKEE HGKRKKKGKG LGKKRDPCLR KYKDFCIHGE

       130        140        150        160        170        180
    CKYVKELRAP SCICHPGYHG ERCHGLSLPV ENRLYTYDHT TILAVVAVVL SSVCLLVIVG

       190        200
    LLMFRYHRRG GYDVENEEKV KLGMTNSH
```

Most preferably, the Proheparin-binding EGF-like growth factor assay detects one or more soluble forms of Proheparin-binding EGF-like growth factor. Proheparin-binding EGF-like growth factor is a type I membrane protein having a large extracellular domain, most or all of which is present in soluble forms of Proheparin-binding EGF-like growth factor generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Proheparin-binding EGF-like growth factor:

| Residues | Length | Domain ID |
|---|---|---|
| 1-19 | 19 | Signal peptide |
| 20-208 | 189 | Proheparin-binding EGF-like growth factor |
| 63-148 | 86 | Heparin-binding EGF-like growth factor |
| 20-62 | 43 | Propeptide (alternative endings at 72, 73, 76, and 81) |
| 149-208 | 60 | Propeptide |
| 20-160 | 141 | Extracellular |
| 161-184 | 24 | Transmembrane |
| 185-208 | 24 | Cytoplasmic |

As used herein, the term "Tenascin" refers to one or more polypeptides present in a biological sample that are derived from the Tenascin precursor (Swiss-Prot P24821 (SEQ ID NO: 2))

```
              10         20         30         40         50         60
         MGAMTQLLAG VFLAFLALAT EGGVLKKVIR HKRQSGVNAT LPEENQPVVF NHVYNIKLPV

              70         80         90        100        110        120
         GSQCSVDLES ASGEKDLAPP SEPSESFQEH TVDGENQIVF THRINIPRRA CGCAAAPDVK

             130        140        150        160        170        180
         ELLSRLEELE NLVSSLREQC TAGAGCCLQP ATGRLDTRPF CSGRGNFSTE GCGCVCEPGW

             190        200        210        220        230        240
         KGPNCSEPEC PGNCHLRGRC IDGQCICDDG FTGEDCSQLA CPSDCNDQGK CVNGVCICFE

             250        260        270        280        290        300
         GYAGADCSRE ICPVPCSEEH GTCVDGLCVC HDGFAGDDCN KPLCLNNCYN RGRCVENECV

             310        320        330        340        350        360
         CDEGFTGEDC SELICPNDCF DRGRCINGTC YCEEGFTGED CGKPTCPHAC HTQGRCEEGQ

             370        380        390        400        410        420
         CVCDEGFAGV DCSEKRCPAD CHNRGRCVDG RCECDDGFTG ADCGELKCPN GCSGHGRCVN

             430        440        450        460        470        480
         GQCVCDEGYT GEDCSQLRCP NDCHSRGRCV EGKCVCEQGF KGYDCSDMSC PNDCHQHGRC

             490        500        510        520        530        540
         VNGMCVCDDG YTGEDCRDRQ CPRDCSNRGL CVDGQCVCED GFTGPDCAEL SCPNDCHGQG
```

```
          550        560        570        580        590        600
   RCVNGQCVCH EGFMGKDCKE QRCPSDCHGQ GRCVDGQCIC HEGFTGLDCG QHSCPSDCNN

          610        620        630        640        650        660
   LGQCVSGRCI CNEGYSGEDC SEVSPPKDLV VTEVTEETVN LAWDNEMRVT EYLVVYTPTH

          670        680        690        700        710        720
   EGGLEMQFRV PGDQTSTIIQ ELEPGVEYFI RVFAILENKK SIPVSARVAT YLPAPEGLKF

          730        740        750        760        770        780
   KSIKETSVEV EWDPLDIAFE TWEIIFRNMN KEDEGEITKS LRRPETSYRQ TGLAPGQEYE

          790        800        810        820        830        840
   ISLHIVKNNT RGPGLKRVTT TRLDAPSQIE VKDVTDTTAL ITWFKPLAEI DGIELTYGIK

          850        860        870        880        890        900
   DVPGDRTTID LTEDENQYSI GNLKPDTEYE VSLISRRGDM SSNPAKETFT TGLDAPRNLR

          910        920        930        940        950        960
   RVSQTDNSIT LEWRNGKAAI DSYRIKYAPI SGGDHAEVDV PKSQQATTKT TLTGLRPGTE

          970        980        990       1000       1010       1020
   YGIGVSAVKE DKESNPATIN AATELDTPKD LQVSETAETS LTLLWKTPLA KFDRYRLNYS

         1030       1040       1050       1060       1070       1080
   LPTGQWVGVQ LPRNTTSYVL RGLEPGQEYN VLLTAEKGRH KSKPARVKAS TEQAPELENL

         1090       1100       1110       1120       1130       1140
   TVTEVGWDGL RLNWTAADQA YEHFIIQVQE ANKVEAARNL TVPGSLRAVD IPGLKAATPY

         1150       1160       1170       1180       1190       1200
   TVSIYGVIQG YRTPVLSAEA STGETPNLGE VVVAEVGWDA LKLNWTAPEG AYEYFFIQVQ

         1210       1220       1230       1240       1250       1260
   EADTVEAAQN LTVPGGLRST DLPGLKAATH YTITIRGVTQ DFSTTPLSVE VLTEEVPDMG

         1270       1280       1290       1300       1310       1320
   NLTVTEVSWD ALRLNWTTPD GTYDQFTIQV QEADQVEEAH NLTVPGSLRS MEIPGLRAGT

         1330       1340       1350       1360       1370       1380
   PYTVTLHGEV RGHSTRPLAV EVVTEDLPQL GDLAVSEVGW DGLRLNWTAA DNAYEHFVIQ

         1390       1400       1410       1420       1430       1440
   VQEVNKVEAA QNLTLPGSLR AVDIPGLEAA TPYRVSIYGV IRGYRTPVLS AEASTAKEPE

         1450       1460       1470       1480       1490       1500
   IGNLNVSDIT PESFNLSWMA TDGIFETFTI EIIDSNRLLE TVEYNISGAE RTAHISGLPP

         1510       1520       1530       1540       1550       1560
   STDFIVYLSG LAPSIRTKTI SATATTEALP LLENLTISDI NPYGFTVSWM ASENAFDSFL

         1570       1580       1590       1600       1610       1620
   VTVVDSGKLL DPQEFTLSGT QRKLELRGLI TGIGYEVMVS GFTQGHQTKP LRAEIVTEAE

         1630       1640       1650       1660       1670       1680
   PEVDNLLVSD ATPDGFRLSW TADEGVFDNF VLKIRDTKKQ SEPLEITLLA PERTRDITGL
```

14

```
       1690       1700       1710       1720       1730       1740
  REATEYEIEL YGISKGRRSQ TVSAIATTAM GSPKEVIFSD ITENSATVSW RAPTAQVESF

       1750       1760       1770       1780       1790       1800
  RITYVPITGG TPSMVTVDGT KTQTRLVKLI PGVEYLVSII AMKGFEESEP VSGSFTTALD

       1810       1820       1830       1840       1850       1860
  GPSGLVTANI TDSEALARWQ PAIATVDSYV ISYTGEKVPE ITRTVSGNTV EYALTDLEPA

       1870       1880       1890       1900       1910       1920
  TEYTLRIFAE KGPQKSSTIT AKFTTDLDSP RDLTATEVQS ETALLTWRPP RASVTGYLLV

       1930       1940       1950       1960       1970       1980
  YESVDGTVKE VIVGPDTTSY SLADLSPSTH YTAKIQALNG PLRSNMIQTI FTTIGLLYPF

       1990       2000       2010       2020       2030       2040
  PKDCSQAMLN GDTTSGLYTI YLNGDKAEAL EVFCDMTSDG GGWIVFLRRK NGRENFYQNW

       2050       2060       2070       2080       2090       2100
  KAYAAGFGDR REEFWLGLDN LNKITAQGQY ELRVDLRDHG ETAFAVYDKF SVGDAKTRYK

       2110       2120       2130       2140       2150       2160
  LKVEGYSGTA GDSMAYHNGR SFSTFDKDTD SAITNCALSY KGAFWYRNCH RVNLMGRYGD

       2170       2180       2190       2200
  NNHSQGVNWF HWKGHEHSIQ FAEMKLRPSN FRNLEGRRKR A
```

The following domains have been identified in Tenascin:

| Residues | Length | Domain ID |
| --- | --- | --- |
| 1-22 | 22 | Signal peptide |
| 23-2201 | 2179 | Tenascin |
| 1072-1435 | | Missing in isoform 2 |
| 1527-1617 | | Missing in isoform 2 |
| 1072-1435 | | Missing in isoform 3 |
| 1527-1617 | | Missing in isoform 4 |
| 1072-1617 | | Missing in isoform 5 |
| 1072-1708 | | Missing in isoform 6 |

As used herein, the term "Angiopoietin-related protein 4" refers to one or more polypeptides present in a biological sample that are derived from the Angiopoietin-related protein 4 precursor (Swiss-Prot Q9BY76 (SEQ ID NO: 3))

```
            10         20         30         40         50         60
     MSGAPTAGAA LMLCAATAVL LSAQGGPVQS KSPRFASWDE MNVLAHGLLQ LGQGLREHAE

            70         80         90        100        110        120
     RTRSQLSALE RRLSACGSAC QGTEGSTDLP LAPESRVDPE VLHSLQTQLK AQNSRIQQLF

           130        140        150        160        170        180
     HKVAQQQRHL EKQHLRIQHL QSQFGLLDHK HLDHEVAKPA RRKRLPEMAQ PVDPAHNVSR

           190        200        210        220        230        240
     LHRLPRDCQE LFQVGERQSG LFEIQPQGSP PFLVNCKMTS DGGWTVIQRR HDGSVDFNRP

           250        260        270        280        290        300
     WEAYKAGFGD PHGEFWLGLE KVHSITGDRN SRLAVQLRDW DGNAELLQFS VHLGGEDTAY

           310        320        330        340        350        360
     SLQLTAPVAG QLGATTVPPS GLSVPFSTWD QDHDLRRDKN CAKSLSGGWW FGTCSHSNLN

           370        380        390        400
     GQYFRSIPQQ RQKLKKGIFW KTWRGRYYPL QATTMLIQPM AAEAAS
```

The following domains have been identified in Angiopoietin-related protein 4:

| Residues | Length | Domain ID |
|----------|--------|-----------|
| 1-25 | 25 | Signal peptide |
| 26-406 | 381 | Angiopoietin-related protein 4 |

As used herein, the term "Fibroblast growth factor 19" refers to one or more polypeptides present in a biological sample that are derived from the Fibroblast growth factor 19 precursor (Swiss-Prot 095750 (SEQ ID NO: 4))

```
            10         20         30         40         50         60
     MRSGCVVVHV WILAGLWLAV AGRPLAFSDA GPHVHYGWGD PIRLRHLYTS GPHGLSSCFL

            70         80         90        100        110        120
     RIRADGVVDC ARGQSAHSLL EIKAVALRTV AIKGVHSVRY LCMGADGKMQ GLLQYSEEDC

           130        140        150        160        170        180
     AFEEEIRPDG YNVYRSEKHR LPVSLSSAKQ RQLYKNRGFL PLSHFLPMLP MVPEEPEDLR

           190        200        210
     GHLESDMFSS PLETDSMDPF GLVTGLEAVR SPSFEK
```

The following domains have been identified in Fibroblast growth factor 19:

| Residues | Length | Domain ID |
|----------|--------|-----------|
| 1-24 | 24 | Signal peptide |
| 25-216 | 192 | Fibroblast growth factor 19 |

As used herein, the term "Fibroblast growth factor 21" refers to one or more polypeptides present in a biological sample that are derived from the Fibroblast growth factor 21 precursor (Swiss-Prot Q9NSA1 (SEQ ID NO: 5))

```
           10          20          30          40          50          60
MDSDETGFEH SGLWVSVLAG LLLGACQAHP IPDSSPLLQF GGQVRQRYLY TDDAQQTEAH

           70          80          90         100         110         120
LEIREDGTVG GAADQSPESL LQLKALKPGV IQILGVKTSR FLCQRPDGAL YGSLHFDPEA

          130         140         150         160         170         180
CSFRELLLED GYNVYQSEAH GLPLHLPGNK SPHRDPAPRG PARFLPLPGL PPALPEPPGI

          190         200
LAPQPPDVGS SDPLSMVGPS QGRSPSYAS
```

The following domains have been identified in Fibroblast growth factor 21:

| Residues | Length | Domain ID |
|---|---|---|
| 1-28 | 28 | Signal peptide |
| 29-209 | 181 | Fibroblast growth factor 21 |

As used herein, the term "Heparin-binding growth factor 1" refers to one or more polypeptides present in a biological sample that are derived from the Heparin-binding growth factor 1 precursor (Swiss-Prot P05230 (SEQ ID NO: 6))

```
           10          20          30          40          50          60
MAEGEITTFT ALTEKFNLPP GNYKKPKLLY CSNGGHFLRI LPDGTVDGTR DRSDQHIQLQ

           70          80          90         100         110         120
LSAESVGEVY IKSTETGQYL AMDTDGLLYG SQTPNEECLF LERLEENHYN TYISKKHAEK

          130         140         150
NWFVGLKKNG SCKRGPRTHY GQKAILFLPL PVSSD
```

The following domains have been identified in Heparin-binding growth factor 1:

| Residues | Length | Domain ID |
|---|---|---|
| 1-15 | 15 | Signal peptide |
| 16-155 | 140 | Heparin-binding growth factor 1 |
| 57-60 | | IQLQ → TDTK in isoform 2 |
| 61-155 | | Missing in isoform 2 |

As used herein, the term "Angiopoietin-related protein 6" refers to one or more polypeptides present in a biological sample that are derived from the Angiopoietin-related protein 6 precursor (Swiss-Prot Q8NI99 (SEQ ID NO: 7))

```
          10           20           30           40           50           60
MGKPWLRALQ  LLLLLGASWA  RAGAPRCTYT  FVLPPQKFTG  AVCWSGPAST  RATPEAANAS

          70           80           90          100          110          120
ELAALRMRVG  RHEELLRELQ  RLAAADGAVA  GEVRALRKES  RGLSARLGQL  RAQLQHEAGP

         130          140          150          160          170          180
GAGPGADLGA  EPAAALALLG  ERVLNASAEA  QRAAARFHQL  DVKFRELAQL  VTQQSSLIAR

         190          200          210          220          230          240
LERLCPGGAG  GQQQVLPPPP  LVPVVPVRLV  GSTSDTSRML  DPAPEPQRDQ  TQRQQEPMAS

         250          260          270          280          290          300
PMPAGHPAVP  TKPVGPWQDC  AEARQAGHEQ  SGVYELRVGR  HVVSVWCEQQ  LEGGGWTVIQ

         310          320          330          340          350          360
RRQDGSVNFF  TTWQHYKAGF  GRPDGEYWLG  LEPVYQLTSR  GDHELLVLLE  DWGGRGARAH

         370          380          390          400          410          420
YDGFSLEPES  DHYRLRLGQY  HGDAGDSLSW  HNDKPFSTVD  RDRDSYSGNC  ALYQRGGWWY

         430          440          450          460          470
HACAHSNLNG  VWHHGGHYRS  RYQDGVYWAE  FRGGAYSLRK  AAMLIRPLKL
```

The following domains have been identified in Angiopoietin-related protein 6:

| Residues | Length | Domain ID |
| --- | --- | --- |
| 1-20 | 20 | Signal peptide |
| 21-470 | 450 | Angiopoietin-related protein 6 |

As used herein, the term "Proepiregulin" refers to one or more polypeptides present in a biological sample that are derived from the Proepiregulin precursor (Swiss-Prot 014944 (SEQ ID NO: 8)):

```
          10           20           30           40           50           60
MTAGRRMEML  CAGRVPALLL  CLGFHLLQAV  LSTTVIPSCI  PGESSDNCTA  LVQTEDNPRV

          70           80           90          100          110          120
AQVSITKCSS  DMNGYCLHGQ  CIYLVDMSQN  YCRCEVGYTG  VRCEHFFLTV  HQPLSKEYVA

         130          140          150          160
LTVILIILFL  ITVVGSTYYF  CRWYRNRKSK  EPKKEYERVT  SGDPELPQV
```

Most preferably, the Proepiregulin assay detects one or more soluble forms of Proepiregulin. Proepiregulin is a type I membrane protein having a large extracellular domain, most or all of which is present in soluble forms of Proepiregulin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). Preferred assays detect Proepiregulin. The following domains have been identified in Proepiregulin:

| Residues | Length | Domain ID |
| --- | --- | --- |
| 1-29 | 29 | Signal peptide |
| 30-169 | 140 | Proepiregulin |
| 60-108 | 49 | Proepiregulin |
| 109-169 | 61 | Propeptide |
| 60-119 | 60 | Extracellular |
| 120-140 | 21 | Transmembrane |
| 141-169 | 61 | Cytoplasmic |

As used herein, the term "Probetacellulin" refers to one or more polypeptides present in a biological sample that are derived from the Probetacellulin precursor (Swiss-Prot P35070 (SEQ ID NO: 9)):

```
          10         20         30         40         50         60
MDRAARCSGA SSLPLLLALA LGLVILHCVV ADGNSTRSPE TNGLLCGDPE ENCAATTTQS

          70         80         90        100        110        120
KRKGHFSRCP KQYKHYCIKG RCRFVVAEQT PSCVCDEGYI GARCERVDLF YLRGDRGQIL

         130        140        150        160        170
VICLIAVMVV FIILVIGVCT CCHPLRKRRK RKKKEEEMET LGKDITPINE DIEETNIA
```

Most preferably, the Probetacellulin assay detects one or more soluble forms of Probetacellulin. Probetacellulin is a type I membrane protein having a large extracellular domain, most or all of which is present in soluble forms of Probetacellulin generated either through alternative splicing event which deletes all or a portion of the transmembrane domain, or by proteolysis of the membrane-bound form. In the case of an immunoassay, one or more antibodies that bind to epitopes within this extracellular domain may be used to detect these soluble form(s). The following domains have been identified in Probetacellulin:

| Residues | Length | Domain ID |
|---|---|---|
| 1-31 | 31 | Signal peptide |
| 32-178 | 147 | Probetacellulin |
| 32-111 | 80 | Betacellulin |
| 112-178 | 67 | Propeptide |
| 32-118 | 87 | Extracellular |
| 119-139 | 21 | Transmembrane |
| 140-178 | 39 | Cytoplasmic |

As used herein, the term "Amphiregulin" refers to one or more polypeptides present in a biological sample that are derived from the Amphiregulin precursor (Swiss-Prot P15514 (SEQ ID NO: 10)):

```
          10         20         30         40         50         60
MRAPLLPPAP VVLSLLILGS GHYAAGLDLN DTYSGKREPF SGDHSADGFE VTSRSEMSSG

          70         80         90        100        110        120
SEISPVSEMP SSSEPSSGAD YDYSEEYDNE PQIPGYIVDD SVRVEQVVKP PQNKTESENT

         130        140        150        160        170        180
SDKPKRKKKG GKNGKNRRNR KKKNPCNAEF QNFCIHGECK YIEHLEAVTC KCQQEYFGER

         190        200        210        220        230        240
CGEKSMKTHS MIDSSLSKIA LAAIAAFMSA VILTAVAVIT VQLRRQYVRK YEGEAEERKK

         250
LRQENGNVHA IA
```

The following domains have been identified in Amphiregulin:

| Residues | Length | Domain ID |
|---|---|---|
| 1-19 | 19 | Signal peptide |
| 20-100 | 81 | Propeptide |
| 101-184 | 84 | Amphiregulin |
| 185-252 | 68 | Propeptide |
| 199-221 | 23 | Transmembrane |

As used herein, the term "Angiogenin" refers to one or more polypeptides present in a biological sample that are derived from the Angiogenin precursor (Swiss-Prot P03950 (SEQ ID NO: 11))

```
         10         20         30         40         50         60
MVMGLGVLLL VFVLGLGLTP PTLAQDNSRY THFLTQHYDA KPQGRDDRYC ESIMRRRGLT

         70         80         90        100        110        120
SPCKDINTFI HGNKRSIKAI CENKNGNPHR ENLRISKSSF QVTTCKLHGG SPWPPCQYRA

        130        140
TAGFRNVVVA CENGLPVHLD QSIFRRP
```

The following domains have been identified in Angiogenin:

| Residues | Length | Domain ID |
| --- | --- | --- |
| 1-24 | 24 | Signal peptide |
| 25-147 | 123 | Angiogenin |

As used herein, the term "Thrombospondin-2" refers to one or more polypeptides present in a biological sample that are derived from the Thrombospondin-2 precursor (Swiss-Prot P35442 (SEQ ID NO: 12))

```
         10         20         30         40         50         60
MVWRLVLLAL WVWPSTQAGH QDKDTTFDLF SISNINRKTI GAKQFRGPDP GVPAYRFVRF

         70         80         90        100        110        120
DYIPPVNADD LSKITKIMRQ KEGFFLTAQL KQDGKSRGTL LALEGPGLSQ RQFEIVSNGP

        130        140        150        160        170        180
ADTLDLTYWI DGTRHVVSLE DVGLADSQWK NVTVQVAGET YSLHVGCDLI DSFALDEPFY

        190        200        210        220        230        240
EHLQAEKSRM YVAKGSARES HFRGLLQNVH LVFENSVEDI LSKKGCQQGQ GAEINAISEN

        250        260        270        280        290        300
TETLRLGPHV TTEYVGPSSE RRPEVCERSC EELGNMVQEL SGLHVLVNQL SENLKRVSND

        310        320        330        340        350        360
NQFLWELIGG PPKTRNMSAC WQDGRFFAEN ETWVVDSCTT CTCKKFKTIC HQITCPPATC

        370        380        390        400        410        420
ASPSFVEGEC CPSCLHSVDG EEGWSPWAEW TQCSVTCGSG TQQRGRSCDV TSNTCLGPSI

        430        440        450        460        470        480
QTRACSLSKC DTRIRQDGGW SHWSPWSSCS VTCGVGNITR IRLCNSPVPQ MGGKNCKGSG
```

```
            490        500        510        520        530        540
     RETKACQGAP CPIDGRWSPW SPWSACTVTC AGGIRERTRV CNSPEPQYGG KACVGDVQER

            550    .   560        570        580        590        600
     QMCNKRSCPV DGCLSNPCFP GAQCSSFPDG SWSCGSCPVG FLGNGTHCED LDECALVPDI

            610        620        630        640        650        660
     CFSTSKVPRC VNTQPGFHCL PCPPRYRGNQ PVGVGLEAAK TEKQVCEPEN PCKDKTHNCH

            670        680        690        700        710        720
     KHAECIYLGH FSDPMYKCEC QTGYAGDGLI CGEDSDLDGW PNLNLVCATN ATYHCIKDNC

            730        740        750        760        770        780
     PHLPNSGQED FDKDGIGDAC DDDDDNDGVT DEKDNCQLLF NPRQADYDKD EVGDRCDNCP

            790        800        810        820        830        840
     YVHNPAQIDT DNNGEGDACS VDIDGDDVFN ERDNCPYVYN TDQRDTDGDG VGDHCDNCPL

            850        860        870        880        890        900
     VHNPDQTDVD NDLVGDQCDN NEDIDDDGHQ NNQDNCPYIS NANQADHDRD GQGDACDPDD

            910        920        930        940        950        960
     DNDGVPDDRD NCRLVFNPDQ EDLDGDGRGD ICKDDFDNDN IPDIDDVCPE NNAISETDFR

            970        980        990       1000       1010       1020
     NFQMVPLDPK GTTQIDPNWV IRHQGKELVQ TANSDPGIAV GFDEFGSVDF SGTFYVNTDR

           1030       1040       1050       1060       1070       1080
     DDDYAGFVFG YQSSSRFYVV MWKQVTQTYW EDQPTRAYGY SGVSLKVVNS TTGTGEHLRN

           1090       1100       1110       1120       1130       1140
     ALWHTGNTPG QVRTLWHDPR NIGWKDYTAY RWHLTHRPKT GYIRVLVHEG KQVMADSGPI

           1150       1160       1170
     YDQTYAGGRL GLFVFSQEMV YFSDLKYECR DI
```

The following domains have been identified in Thrombospondin-2:

| Residues | Length | Domain ID |
|---|---|---|
| 1-18 | 18 | Signal peptide |
| 19-1172 | 1154 | Thrombospondin-2 |

As used herein, the term "Collagen alpha-1(XVIII) chain" refers to one or more polypeptides present in a biological sample that are derived from the Collagen alpha-1(XVIII) chain precursor (Swiss-Prot P39060 (SEQ ID NO: 13))

```
            10         20         30         40         50         60
     MAPYPCGCHI LLLLFCCLAA ARANLLNLNW LWFNNEDTSH AATTIPEPQG PLPVQPTADT

            70         80         90        100        110        120
```

```
TTHVTPRNGS TEPATAPGSP EPPSELLEDG QDTPTSAESP DAPEENIAGV GAEILNVAKG
           130        140        150        160        170        180
IRSFVQLWND TVPTESLARA ETLVLETPVG PLALAGPSST PQENGTTLWP SRGIPSSPGA
           190        200        210        220        230        240
HTTEAGTLPA PTPSPPSLGR PWAPLTGPSV PPPSSGRASL SSLLGGAPPW GSLQDPDSQG
           250        260        270        280        290        300
LSPAAAAPSQ QLQRPDVRLR TPLLHPLVMG SLGKHAAPSA FSSGLPGALS QVAVTTLTRD
           310        320        330        340        350        360
SGAWVSHVAN SVGPGLANNS ALLGADPEAP AGRCLPLPPS LPVCGHLGIS RFWLPNHLHH
           370        380        390        400        410        420
ESGEQVRAGA RAWGGLLQTH CHPFLAWFFC LLLVPPCGSV PPPAPPPCCQ FCEALQDACW
           430        440        450        460        470        480
SRLGGGRLPV ACASLPTQED GYCVLIGPAA ERISEEVGLL QLLGDPPPQQ VTQTDDPDVG
           490        500        510        520        530        540
LAYVFGPDAN SGQVARYHFP SLFFRDFSLL FHIRPATEGP GVLFAITDSA QAMVLLGVKL
           550        560        570        580        590        600
SGVQDGHQDI SLLYTEPGAG QTHTAASFRL PAFVGQWTHL ALSVAGGFVA LYVDCEEFQR
           610        620        630        640        650        660
MPLARSSRGL ELEPGAGLFV AQAGGADPDK FQGVIAELKV RRDPQVSPMH CLDEEGDDSD
           670        680        690        700        710        720
GASGDSGSGL GDARELLREE TGAALKPRLP APPPVTTPPL AGGSSTEDSR SEEVEEQTTV
           730        740        750        760        770        780
ASLGAQTLPG SDSVSTWDGS VRTPGGRVKE GGLKGQKGEP GVPGPPGRAG PPGSPCLPGP
           790        800        810        820        830        840
PGLPCPVSPL GPAGPALQTV PGPQGPPGPP GRDGTPGRDG EPGDPGEDGK PGDTGPQGFP
           850        860        870        880        890        900
GTPGDVGPKG DKGDPGVGER GPPGPQGPPG PPGPSFRHDK LTFIDMEGSG FGGDLEALRG
           910        920        930        940        950        960
PRGFPGPPGP PGVPGLPGEP GRFGVNSSDV PGPAGLPGVP GREGPPGFPG LPGPPGPPGR
           970        980        990        1000       1010       1020
EGPPGRTGQK GSLGEAGAPG HKGSKGAPGP AGARGESGLA GAPGPAGPPG PPGPPGPPGP
           1030       1040       1050       1060       1070       1080
GLPAGFDDME GSGGPFWSTA RSADGPQGPP GLPGLKGDPG VPGLPGAKGE VGADGVPGFP
           1090       1100       1110       1120       1130       1140
GLPGREGIAG PQGPKGDRGS RGEKGDPGKD GVGQPGLPGP PGPPGPVVYV SEQDGSVLSV
           1150       1160       1170       1180       1190       1200
PGPEGRPGFA GFPGPAGPKG NLGSKGERGS PGPKGEKGEP GSIFSPDGGA LGPAQKGAKG
```

22

```
        1210        1220        1230        1240        1250        1260
     EPGFRGPPGP  YGRPGYKGEI  GFPGRPGRPG  MNGLKGEKGE  PGDASLGFGM  RGMPGPPGPP

        1270        1280        1290        1300        1310        1320
     GPPGPPGTPV  YDSNVFAESS  RPGPPGLPGN  QGPPGPKGAK  GEVGPPGPPG  QFPFDFLQLE

        1330        1340        1350        1360        1370        1380
     AEMKGEKGDR  GDAGQKGERG  EPGGGGFFGS  SLPGPPGPPG  PPGPRGYPGI  PGPKGESIRG

        1390        1400        1410        1420        1430        1440
     QPGPPGPQGP  PGIGYEGRQG  PPGPPGPPGP  PSFPGPHRQT  ISVPGPPGPP  GPPGPPGTMG

        1450        1460        1470        1480        1490        1500
     ASSGVRLWAT  RQAMLGQVHE  VPEGWLIFVA  EQEELYVRVQ  NGFRKVQLEA  RTPLPRGTDN

        1510        1520        1530        1540        1550        1560
     EVAALQPPVV  QLHDSNPYPR  REHPHPTARP  WRADDILASP  PRLPEPQPYP  GAPHHSSYVH

        1570        1580        1590        1600        1610        1620
     LRPARPTSPP  AHSHRDFQPV  LHLVALNSPL  SGGMRGIRGA  DFQCFQQARA  VGLAGTFRAF

        1630        1640        1650        1660        1670        1680
     LSSRLQDLYS  IVRRADRAAV  PIVNLKDELL  FPSWEALFSG  SEGPLKPGAR  IFSFDGKDVL

        1690        1700        1710        1720        1730        1740
     RHPTWPQKSV  WHGSDPNGRR  LTESYCETWR  TEAPSATGQA  SSLLGGRLLG  QSAASCHHAY

        1750
     IVLCIENSFM  TASK
```

The following domains have been identified in Collagen alpha-1(XVIII) chain. Preferred assays detect Endostatin:

| Residues | Length | Domain ID |
|---|---|---|
| 1-23 | 23 | Signal peptide |
| 24-1754 | 1731 | Collagen alpha-1(XVIII) chain |
| 1572-1754 | 183 | Endostatin |
| 216-450 | | Missing in isoform 2 |
| 1-415 | | Missing in isoform 3 |
| 416-450 | | QDACWSRLGGGRLPVACASLPTQEDGYCVLIGPAA (SEQ ID NO: 14) → MAPRCPWPWPRRRRLLDVLAPLVLLLGVRAASAEP (SEQ ID NO: 15) in isoform 3 |

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

In this regard, the skilled artisan will understand that the signals obtained from an immunoassay are a direct result of complexes formed between one or more antibodies and the target biomolecule (*i.e.,* the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.*) and nucleic acid measurements (mRNA quatitation). This list is not meant to be limiting.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and *The Immunoassay Handbook,* David Wild, ed. Stockton Press, New York, 1994, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, *etc.*) as well as molecules that may be indirectly detected by production of a detectable reaction product (e.g., enzymes such as horseradish peroxidase, alkaline phosphatase, *etc.*) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHI domains; (ii) a F(ab')2 fragment, a bivalent fragment comprising two Fab fragments linked by a disulfide bridge at the hinge region; (iii) a Fd fragment consisting of the VH and CHI domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ M$^{-1}$, and preferably between about $10^8$ M$^{-1}$ to about $10^9$ M$^{-1}$, about $10^9$ M$^{-1}$ to about $10^{10}$ M$^{-1}$, or about $10^{10}$ M$^{-1}$ to about $10^{12}$ M$^{-1}$.

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g*, Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See*, *e.g.,* U.S. Patent No. 6,057,098, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection therory developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(1-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1 alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfall protein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61 ); RT1.B-1 (alpha) chain of the integral membrane protein (Q5Y7A8); Soluble tumor necrosis factor receptor superfamily member 1A (sTNFR-1, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 $m^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration ($U_{Cr}$), urine flow rate (V), and creatinine's plasma concentration ($P_{Cr}$) are known. Since the product of urine concentration and urine flow rate yields creatinine's excretion rate, creatinine clearance is also said to be its excretion rate ($U_{Cr} \times V$) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times 24\text{-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria
males and females 18 years of age or older;
undergoing a radiographic / angiographic procedure (such as a CT scan or coronary intervention) involving the intravascular administration of contrast media;
expected to be hospitalized for at least 48 hours after contrast administration.
able and willing to provide written informed consent for study participation and to comply with all study procedures.

Exclusion Criteria
renal transplant recipients;
acutely worsening renal function prior to the contrast procedure;
already receiving dialysis (either acute or chronic) or in imminent need of dialysis at enrollment;
expected to undergo a major surgical procedure (such as involving cardiopulmonary bypass) or an additional imaging procedure with contrast media with significant risk for further renal insult within the 48 hrs following contrast administration;
participation in an interventional clinical study with an experimental therapy within the previous 30 days;
known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 cumin/1.73 $m^2$ = 2 points, 20-40 mL/min/1.73 $m^2$ = 4 points, < 20 mL/min/1.73 $m^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria
males and females 18 years of age or older;
undergoing cardiovascular surgery;
Toronto/Ottawa Predictive Risk Index for Renal Replacement risk score of at least 2 (Wijeysundera et al., JAMA 297: 1801-9, 2007); and
able and willing to provide written informed consent for study participation and to comply with all study procedures.

Exclusion Criteria
known pregnancy;
previous renal transplantation;
acutely worsening renal function prior to enrollment (e.g., any category of RIFLE criteria);
already receiving dialysis (either acute or chronic) or in imminent need of dialysis at enrollment;
currently enrolled in another clinical study or expected to be enrolled in another clinical study within 7 days of cardiac surgery that involves drug infusion or a therapeutic intervention for AKI;
known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 1900 adults expected to be in the ICU for at least 48 hours will be enrolled. To be enrolled in the study, each patient must meet all of the following inclusion criteria and none of the following exclusion criteria:

Inclusion Criteria
males and females 18 years of age or older;
Study population 1: approximately 300 patients that have at least one of:

shock (SBP < 90 mmHg and/or need for vasopressor support to maintain MAP > 60 mmHg and/or documented drop in SBP of at least 40 mmHg); and
sepsis;

Study population 2: approximately 300 patients that have at least one of:

IV antibiotics ordered in computerized physician order entry (CPOE) within 24 hours of enrollment;
contrast media exposure within 24 hours of enrollment;
increased Intra-Abdominal Pressure with acute decompensated heart failure; and severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment;

Study population 4: approximately 1000 patients that are 21 years of age or older, within 24 hours of being admitted into the ICU, expected to have an indwelling urinary catheter for at least 48 hours after enrollment, and have at least one of the following acute conditions within 24 hours prior to enrollment:

(i) respiratory SOFA score of $\geq 2$ (PaO2/FiO2 <300), (ii) cardiovascular SOFA score of $\geq 1$ (MAP < 70 mm Hg and/or any vasopressor required).

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets any of the following:

(i) active bleeding with an anticipated need for > 4 units PRBC in a day;

(ii) hemoglobin < 7 g/dL;

(iii) any other condition that in the physician's opinion would contraindicate drawing serial blood samples for clinical study purposes;

meets only the SBP < 90 mmHg inclusion criterion set forth above, and does not have shock in the attending physician's or principal investigator's opinion;

After obtaining informed consent, an EDTA anti-coagulated blood sample (10 mL) and a urine sample (25-50 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), 36 ($\pm$ 2), 48 ($\pm$ 2), 60 ($\pm$ 2), 72 ($\pm$ 2), and 84 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Units for the concentrations reported in the following data tables are as follows: Proheparin-binding EGF-like growth factor - pg/mL, Tenascin C - pg/mL, Angiopoietin-related protein 4 - ng/mL, Fibroblast growth factor 19 - ng/mL, Fibroblast growth factor 21 - ng/mL, Heparin-binding growth factor 1 - pg/mL, Angiopoietin-related protein 6 - ng/mL, Proepiregulin - pg/mL, Probetacellulin - pg/mL, Amphiregulin - pg/mL, Angiogenin - pg/mL, Thrombospondin-2 - pg/mL, and Collagen alpha-1 (XVIII) chain (endostatin domain) - pg/mL,. In the case of those kidney injury markers which are membrane proteins as described herein, the assays used in these examples detect soluble forms thereof.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff') concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

Fig. 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4070 | 8980 | 4070 | 7030 | 4070 | 5170 |
| Average | 7410 | 12000 | 7410 | 9920 | 7410 | 7950 |
| Stdev | 7830 | 9230 | 7830 | 8050 | 7830 | 7320 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 2.1E-10 | | 0.0014 | | 0.65 |
| Min | 55.7 | 132 | 55.7 | 97.6 | 55.7 | 650 |
| Max | 30600 | 30600 | 30600 | 30600 | 30600 | 24000 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5850 | 7630 | 5850 | 9740 | 5850 | 7540 |
| Average | 9700 | 11300 | 9700 | 11000 | 9700 | 10100 |
| Stdev | 8780 | 9140 | 8780 | 8280 | 8780 | 8050 |
| p(t-test) | | 0.19 | | 0.30 | | 0.76 |
| Min | 0.00873 | 677 | 0.00873 | 97.6 | 0.00873 | 426 |
| Max | 30600 | 30600 | 30600 | 30600 | 30600 | 30600 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4280 | 11300 | 4280 | 6950 | 4280 | 4040 |
| Average | 8070 | 12700 | 8070 | 9800 | 8070 | 8380 |
| Stdev | 8270 | 9210 | 8270 | 7750 | 8270 | 7700 |
| p(t-test) | | 6.8E-10 | | 0.033 | | 0.81 |
| Min | 55.7 | 132 | 55.7 | 311 | 55.7 | 650 |
| Max | 30600 | 30600 | 30600 | 30600 | 30600 | 22100 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.57 | 0.66 | 0.61 | 0.55 | 0.59 | 0.55 | 0.54 | 0.52 |
| SE | 0.024 | 0.040 | 0.025 | 0.029 | 0.042 | 0.029 | 0.046 | 0.050 | 0.046 |
| p | 1.7E-10 | 0.10 | 3.1E-10 | 2.5E-4 | 0.25 | 0.0013 | 0.32 | 0.39 | 0.60 |
| nCohort 1 | 430 | 1121 | 506 | 430 | 1121 | 506 | 430 | 1121 | 506 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 195 | 58 | 182 | 132 | 51 | 129 | 46 | 36 | 43 |
| Cutoff 1 | 4060 | 4060 | 4350 | 3490 | 4300 | 3700 | 2680 | 4330 | 2620 |
| Sens 1 | 70% | 71% | 70% | 70% | 71% | 71% | 72% | 72% | 72% |
| Spec 1 | 50% | 39% | 51% | 44% | 41% | 43% | 36% | 41% | 34% |
| Cutoff 2 | 2910 | 3420 | 3350 | 2480 | 2230 | 2770 | 2090 | 2850 | 1880 |
| Sens 2 | 80% | 81% | 80% | 80% | 80% | 81% | 80% | 81% | 81% |
| Spec 2 | 38% | 33% | 40% | 35% | 23% | 35% | 30% | 28% | 26% |
| Cutoff 3 | 1680 | 1810 | 1820 | 1390 | 1440 | 1840 | 1510 | 1680 | 1510 |
| Sens 3 | 90% | 91% | 90% | 90% | 90% | 91% | 91% | 92% | 91% |
| Spec 3 | 23% | 18% | 24% | 18% | 14% | 25% | 19% | 16% | 19% |
| Cutoff 4 | 8010 | 14500 | 9520 | 8010 | 14500 | 9520 | 8010 | 14500 | 9520 |
| Sens 4 | 53% | 36% | 53% | 45% | 39% | 41% | 37% | 25% | 33% |
| Spec 4 | 70% | 70% | 70% | 70% | 70% | 70% | 70% | 70% | 70% |
| Cutoff 5 | 14000 | 19700 | 16100 | 14000 | 19700 | 16100 | 14000 | 19700 | 16100 |
| Sens 5 | 42% | 29% | 42% | 35% | 16% | 28% | 24% | 22% | 26% |
| Spec 5 | 80% | 80% | 80% | 80% | 80% | 80% | 80% | 80% | 80% |
| Cutoff 6 | 20400 | 22700 | 20400 | 20400 | 22700 | 20400 | 20400 | 22700 | 20400 |
| Sens 6 | 16% | 12% | 18% | 8% | 4% | 7% | 4% | 8% | 2% |
| Spec 6 | 92% | 90% | 91% | 92% | 90% | 91% | 92% | 90% | 91% |
| OR Quart 2 p Value | 1.8 | 1.8 | 1.8 | 1.7 | 0.72 | 2.3 | 2.3 | 1.2 | 2.3 |
| 95% CI of OR Quart2 | 0.031 | 0.16 | 0.048 | 0.096 | 0.49 | 0.011 | 0.080 | 0.78 | 0.082 |
| | 1.1 | 0.79 | 1.0 | 0.91 | 0.29 | 1.2 | 0.91 | 0.39 | 0.90 |
| | 3.1 | 4.2 | 3.1 | 3.1 | 1.8 | 4.2 | 5.9 | 3.5 | 5.8 |
| OR Quart 3 p Value | 2.2 | 1.8 | 2.5 | 1.9 | 1.5 | 2.4 | 1.8 | 2.4 | 1.0 |
| 95% CI of OR Quart3 | 0.0041 | 0.16 | 0.0011 | 0.036 | 0.33 | 0.0054 | 0.24 | 0.077 | 1.0 |
| | 1.3 | 0.79 | 1.4 | 1.0 | 0.68 | 1.3 | 0.68 | 0.91 | 0.34 |
| | 3.7 | 4.2 | 4.4 | 3.5 | 3.2 | 4.5 | 4.7 | 6.3 | 2.9 |
| OR Quart | 4.5 | 1.9 | 4.9 | 3.0 | 1.5 | 3.0 | 1.8 | 1.5 | 2.1 |
| 4 | 1.5E-8 | 0.12 | 5.2E-9 | 2.6E-4 | 0.33 | 5.0E-4 | 0.24 | 0.44 | 0.12 |
| p Value | 2.7 | 0.85 | 2.9 | 1.7 | 0.68 | 1.6 | 0.68 | 0.53 | 0.82 |
| 95% CI of OR Quart4 | 7.5 | 4.4 | 8.3 | 5.4 | 3.2 | 5.5 | 4.7 | 4.3 | 5.4 |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.7 | 11.6 | 11.7 | 11.8 | 11.7 | 11.5 |
| Average | 38.9 | 38.7 | 38.9 | 65.8 | 38.9 | 55.3 |
| Stdev | 79.2 | 92.2 | 79.2 | 172 | 79.2 | 122 |
| p(t-test) | | 0.98 | | 0.025 | | 0.37 |
| Min | 0.000949 | 0.000466 | 0.000949 | 0.000981 | 0.000949 | 0.633 |
| Max | 538 | 789 | 538 | 1370 | 538 | 453 |
| n (Samp) | 363 | 168 | 363 | 103 | 363 | 22 |
| n (Patient) | 151 | 168 | 151 | 103 | 151 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.4 | 18.6 | 12.4 | 17.2 | 12.4 | 9.37 |
| Average | 44.1 | 50.5 | 44.1 | 79.9 | 44.1 | 63.1 |
| Stdev | 108 | 70.1 | 108 | 145 | 108 | 135 |
| p(t-test) | | 0.68 | | 0.046 | | 0.39 |
| Min | 0.000734 | 0.577 | 0.000734 | 1.82 | 0.000734 | 2.38 |
| Max | 1370 | 276 | 1370 | 645 | 1370 | 453 |
| n (Samp) | 960 | 49 | 960 | 39 | 960 | 25 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.3 | 11.2 | 12.3 | 12.0 | 12.3 | 11.5 |
| Average | 45.8 | 40.8 | 45.8 | 56.2 | 45.8 | 35.1 |
| Stdev | 91.7 | 100 | 91.7 | 164 | 91.7 | 78.1 |
| p(t-test) | | 0.57 | | 0.38 | | 0.58 |
| Min | 0.000466 | 0.000466 | 0.000466 | 0.000981 | 0.000466 | 0.633 |
| Max | 624 | 789 | 624 | 1370 | 624 | 388 |
| n (Samp) | 444 | 155 | 444 | 104 | 444 | 24 |
| n (Patient) | 172 | 155 | 172 | 104 | 172 | 24 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.58 | 0.48 | 0.50 | 0.58 | 0.47 | 0.47 | 0.46 | 0.46 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.027 | 0.044 | 0.027 | 0.032 | 0.049 | 0.032 | 0.065 | 0.060 | 0.062 |
| p | 0.66 | 0.076 | 0.53 | 0.97 | 0.11 | 0.32 | 0.59 | 0.53 | 0.49 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 | 7.08 | 10.1 | 6.96 | 5.73 | 10.2 | 5.39 | 5.10 | 7.26 | 5.20 |
| Sens 1 | 70% | 71% | 70% | 71% | 72% | 70% | 73% | 72% | 71% |
| Spec 1 | 32% | 42% | 29% | 26% | 42% | 23% | 25% | 30% | 23% |
| Cutoff 2 | 4.53 | 5.80 | 4.61 | 4.21 | 7.47 | 3.76 | 2.96 | 5.34 | 2.60 |
| Sens 2 | 80% | 82% | 80% | 81% | 82% | 81% | 82% | 80% | 83% |
| Spec 2 | 23% | 23% | 20% | 20% | 31% | 15% | 13% | 21% | 9% |
| Cutoff 3 | 2.96 | 3.15 | 3.08 | 2.53 | 3.85 | 2.52 | 1.52 | 4.48 | 1.52 |
| Sens 3 | 90% | 92% | 90% | 90% | 92% | 90% | 91% | 92% | 92% |
| Spec 3 | 13% | 10% | 11% | 10% | 14% | 9% | 5% | 18% | 4% |
| Cutoff 4 | 22.2 | 24.2 | 24.5 | 22.2 | 24.2 | 24.5 | 22.2 | 24.2 | 24.5 |
| Sens 4 | 32% | 45% | 26% | 33% | 31% | 27% | 27% | 16% | 33% |
| Spec 4 | 70% | 70% | 70% | 70% | 70% | 70% | 70% | 70% | 70% |
| Cutoff 5 | 36.8 | 42.2 | 44.4 | 36.8 | 42.2 | 44.4 | 36.8 | 42.2 | 44.4 |
| Sens 5 | 21% | 31% | 20% | 22% | 28% | 17% | 18% | 16% | 17% |
| Spec 5 | 80% | 80% | 80% | 80% | 80% | 80% | 80% | 80% | 80% |
| Cutoff 6 | 96.1 | 96.1 | 107 | 96.1 | 96.1 | 107 | 96.1 | 96.1 | 107 |
| Sens 6 | 8% | 14% | 7% | 14% | 21% | 11% | 14% | 16% | 4% |
| Spec 6 | 90% | 90% | 90% | 90% | 90% | 90% | 90% | 90% | 90% |
| OR Quart 2 p Value 95% CI of OR Quart2 | 1.3 | 0.90 | 1.2 | 0.85 | 1.5 | 1.2 | 1.0 | 1.5 | 0.24 |
| | 0.31 | 0.82 | 0.51 | 0.61 | 0.44 | 0.64 | 0.99 | 0.52 | 0.073 |
| | 0.78 | 0.36 | 0.71 | 0.46 | 0.53 | 0.63 | 0.28 | 0.42 | 0.049 |
| | 2.2 | 2.2 | 2.0 | 1.6 | 4.3 | 2.1 | 3.6 | 5.4 | 1.1 |
| OR Quart 3 p Value 95% CI of OR Quart3 | 1.0 | 1.1 | 1.3 | 0.86 | 2.0 | 0.85 | 1.2 | 2.0 | 0.61 |
| | 0.92 | 0.82 | 0.29 | 0.63 | 0.16 | 0.62 | 0.74 | 0.25 | 0.40 |
| | 0.60 | 0.46 | 0.79 | 0.46 | 0.75 | 0.45 | 0.36 | 0.61 | 0.19 |
| | 1.7 | 2.6 | 2.2 | 1.6 | 5.5 | 1.6 | 4.2 | 6.9 | 1.9 |
| OR Quart 4 p Value 95% CI of OR Quart4 | 1.3 | 2.0 | 1.1 | 1.0 | 2.0 | 1.4 | 1.2 | 1.8 | 1.1 |
| | 0.38 | 0.092 | 0.76 | 0.91 | 0.16 | 0.23 | 0.74 | 0.36 | 0.80 |
| | 0.75 | 0.89 | 0.64 | 0.57 | 0.75 | 0.79 | 0.36 | 0.51 | 0.42 |
| | 2.1 | 4.3 | 1.8 | 1.9 | 5.5 | 2.6 | 4.2 | 6.2 | 3.1 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.105 | 0.0450 | 0.105 | 0.109 | 0.105 | 0.000350 |
| Average | 1.04 | 1.27 | 1.04 | 1.52 | 1.04 | 0.471 |
| Stdev | 2.57 | 7.27 | 2.57 | 3.99 | 2.57 | 1.02 |
| p(t-test) | | 0.60 | | 0.15 | | 0.33 |
| Min | 1.00E-4 | 1.00E-4 | 1.00E-4 | 0.000110 | 1.00E-4 | 0.000110 |
| Max | 25.9 | 89.0 | 25.9 | 22.4 | 25.9 | 4.24 |
| n (Samp) | 333 | 156 | 333 | 97 | 333 | 20 |
| n (Patient) | 141 | 156 | 141 | 97 | 141 | 20 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0677 | 0.214 | 0.0677 | 0.0456 | 0.0677 | 0.000364 |
| Average | 1.10 | 0.704 | 1.10 | 3.20 | 1.10 | 0.501 |
| Stdev | 3.58 | 1.44 | 3.58 | 14.6 | 3.58 | 1.05 |
| p(t-test) | | 0.45 | | 0.0063 | | 0.42 |
| Min | 1.00E-4 | 1.00E-4 | 1.00E-4 | 0.000120 | 1.00E-4 | 1.00E-4 |
| Max | 70.5 | 8.76 | 70.5 | 89.0 | 70.5 | 4.24 |
| n (Samp) | 864 | 47 | 864 | 37 | 864 | 23 |
| n (Patient) | 292 | 47 | 292 | 37 | 292 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.151 | 0.0456 | 0.151 | 0.0635 | 0.151 | 0.000566 |
| Average | 1.06 | 1.38 | 1.06 | 1.64 | 1.06 | 0.329 |
| Stdev | 2.44 | 7.63 | 2.44 | 4.46 | 2.44 | 0.615 |
| p(t-test) | | 0.45 | | 0.081 | | 0.17 |
| Min | 1.00E-4 | 1.00E-4 | 1.00E-4 | 0.000110 | 1.00E-4 | 0.000110 |
| Max | 25.9 | 89.0 | 25.9 | 22.5 | 25.9 | 2.35 |
| n (Samp) | 414 | 143 | 414 | 97 | 414 | 21 |
| n (Patient) | 162 | 143 | 162 | 97 | 162 | 21 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.46 | 0.55 | 0.45 | 0.49 | 0.51 | 0.48 | 0.37 | 0.41 | 0.37 |
| SE | 0.028 | 0.044 | 0.028 | 0.033 | 0.049 | 0.033 | 0.069 | 0.063 | 0.067 |
| P | 0.18 | 0.25 | 0.088 | 0.69 | 0.81 | 0.52 | 0.061 | 0.16 | 0.047 |
| nCohort 1 | 333 | 864 | 414 | 333 | 864 | 414 | 333 | 864 | 414 |
| nCohort 2 | 156 | 47 | 143 | 97 | 37 | 97 | 20 | 23 | 21 |
| Cutoff 1 | 0.000336 | 0.00640 | 0.000278 | 0.000336 | 0.000336 | 0.000336 | 0.000206 | 0.000244 | 0.000206 |
| Sens 1 | 72% | 70% | 74% | 73% | 70% | 73% | 75% | 78% | 76% |
| Spec 1 | 23% | 45% | 23% | 23% | 30% | 24% | 11% | 18% | 12% |
| Cutoff 2 | 0.000249 | 0.000469 | 0.000249 | 0.000249 | 0.000249 | 0.000249 | 0.000203 | 0.000213 | 0.000203 |
| Sens 2 | 83% | 81% | 80% | 81% | 81% | 84% | 80% | 83% | 81% |
| Spec 2 | 16% | 33% | 17% | 16% | 25% | 17% | 9% | 17% | 10% |
| Cutoff 3 | 0.000203 | 0.000244 | 0.000123 | 0.000123 | 0.000157 | 0.000203 | 0.000122 | 0.000122 | 0.000122 |
| Sens 3 | 90% | 91% | 94% | 93% | 92% | 91% | 90% | 96% | 90% |
| Spec 3 | 9% | 18% | 7% | 6% | 11% | 10% | 6% | 6% | 7% |
| Cutoff 4 | 0.610 | 0.548 | 0.708 | 0.610 | 0.548 | 0.708 | 0.610 | 0.548 | 0.708 |
| Sens 4 | 27% | 38% | 24% | 32% | 32% | 28% | 30% | 22% | 19% |
| Spec 4 | 70% | 70% | 70% | 70% | 70% | 70% | 70% | 70% | 70% |
| Cutoff 5 | 1.28 | 1.17 | 1.54 | 1.28 | 1.17 | 1.54 | 1.28 | 1.17 | 1.54 |
| Sens 5 | 15% | 17% | 13% | 20% | 24% | 15% | 10% | 17% | 5% |
| Spec 5 | 80% | 80% | 80% | 80% | 80% | 80% | 80% | 80% | 80% |
| Cutoff 6 | 2.70 | 2.51 | 2:99 | 2.70 | 2.51 | 2.99 | 2.70 | 2.51 | 2.99 |
| Sens 6 | 6% | 4% | 6% | 9% | 14% | 10% | 5% | 4% | 0% |
| Spec 6 | 90% | 90% | 90% | 90% | 90% | 90% | 90% | 90% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 | 1.1 | 1.4 | 1.3 | 1.3 | 1.9 | 1.3 | 1.4 | 0.59 | 2.0 |
| p Value | 0.86 | 0.49 | 0.31 | 0.40 | 0.18 | 0.41 | 0.69 | 0.48 | 0.42 |
| 95% CI or OR Quart2 | 0.61 | 0.55 | 0.77 | 0.70 | 0.75 | 0.69 | 0.30 | 0.14 | 0.37 |
| | 1.8 | 3.5 | 2.3 | 2.5 | 4.9 | 2.5 | 6.3 | 2.5 | 11 |
| OR Quart 3 | 1.3 | 2.1 | 1.3 | 0.94 | 0.85 | 1.4 | 1.0 | 1.6 | 3.7 |
| p Value | 0.31 | 0.10 | 0.31 | 0.86 | 0.78 | 0.33 | 0.99 | 0.40 | 0.11 |
| 95% CI of OR Quart3 | 0.77 | 0.87 | 0.77 | 0.48 | 0.28 | 0.72 | 0.20 | 0.52 | 0.75 |
| | 2.3 | 4.9 | 2.3 | 1.8 | 2.6 | 2.6 | 5.2 | 5.0 | 18 |
| OR Quart 4 | 1.4 | 1.5 | 1.7 | 1.3 | 1.6 | 1.4 | 3.7 | 1.4 | 4.3 |
| p Value | 0.25 | 0.37 | 0.070 | 0.40 | 0.34 | 0.32 | 0.054 | 0.55 | 0.070 |
| 95% CI of OR Quart4 | 0.80 | 0.61 | 0.96 | 0.70 | 0.61 | 0.73 | 0.98 | 0.44 | 0.89 |
| | 2.3 | 3.8 | 2.9 | 2.5 | 4.2 | 2.6 | 14 | 4.5 | 21 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20.6 | 27.6 | 20.6 | 32.6 | 20.6 | 36.8 |
| Average | 46.4 | 39.6 | 46.4 | 101 | 46.4 | 103 |
| Stdev | 99.6 | 48.0 | 99.6 | 289 | 99.6 | 271 |
| p(t-test) | | 0.45 | | 0.0066 | | 0.017 |
| Min | 0.00401 | 0.00389 | 0.00401 | 0.00401 | 0.00401 | 3.84 |
| Max | 1310 | 300 | 1310 | 2190 | 1310 | 11270 |
| n (Samp) | 268 | 138 | 268 | 104 | 268 | 35 |
| n (Patient) | 148 | 138 | 148 | 104 | 148 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.5 | 31.6 | 23.5 | 46.4 | 23.5 | 40.2 |
| Average | 60.4 | 54.7 | 60.4 | 109 | 60.4 | 40.4 |
| Stdev | 177 | 65.1 | 177 | 172 | 177 | 28.4 |
| p(t-test) | | 0.84 | | 0.10 | | 0.59 |
| Min | 0.00389 | 6.31 | 0.00389 | 6.70 | 0.00389 | 4.90 |
| Max | 2190 | 289 | 2190 | 987 | 2190 | 124 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.9 | 29.8 | 23.9 | 37.2 | 23.9 | 39.6 |
| Average | 43.7 | 42.7 | 43.7 | 108 | 43.7 | 134 |
| Stdev | 64.1 | 49.9 | 64.1 | 320 | 64.1 | 289 |
| p(t-test) | | 0.87 | | 8.3E-4 | | 6.8E-6 |
| Min | 0.00131 | 0.00389 | 0.00131 | 0.00401 | 0.00131 | 3.84 |
| Max | 480 | 300 | 480 | 2190 | 480 | 1270 |
| n (Samp) | 313 | 127 | 313 | 102 | 313 | 32 |
| n (Patient) | 152 | 127 | 152 | 102 | 152 | 32 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.58 | 0.54 | 0.60 | 0.68 | 0.60 | 0.57 | 0.59 | 0.60 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.030 | 0.050 | 0.031 | 0.034 | 0.050 | 0.033 | 0.053 | 0.063 | 0.055 |
| P | 0.24 | 0.12 | 0.19 | 0.0031 | 3.0E-4 | 0.0032 | 0.18 | 0.17 | 0.069 |
| nCohort 1 | 268 | 663 | 313 | 268 | 663 | 313 | 268 | 663 | 313 |
| nCohort 2 | 138 | 38 | 127 | 104 | 37 | 102 | 35 | 23 | 32 |
| Cutoff 1 | 16.0 | 16.8 | 16.3 | 18.3 | 24.0 | 20.9 | 16.3 | 22.1 | 18.5 |
| Sens 1 | 70% | 71% | 70% | 70% | 70% | 71% | 71% | 74% | 72% |
| Spec 1 | 40% | 38% | 37% | 45% | 51% | 46% | 40% | 48% | 41% |
| Cutoff 2 | 11.2 | 12.1 | 13.2 | 14.2 | 16.8 | 15.1 | 9.15 | 9.15 | 12.7 |
| Sens 2 | 80% | 82% | 80% | 81% | 81% | 80% | 83% | 83% | 81% |
| Spec 2 | 26% | 26% | 29% | 35% | 38% | 35% | 19% | 18% | 27% |
| Cutoff 3 | 7.44 | 9.02 | 6.50 | 8.80 | 14.5 | 9.55 | 5.56 | 6.50 | 5.56 |
| Sens 3 | 91% | 92% | 91% | 90% | 92% | 90% | 91% | 91% | 91% |
| Spec 3 | 15% | 18% | 13% | 18% | 32% | 20% | 11% | 13% | 11% |
| Cutoff 4 | 41.2 | 43.2 | 43.8 | 41.2 | 43.2 | 43.8 | 41.2 | 43.2 | 43.8 |
| Sens 4 | 28% | 37% | 31% | 43% | 57% | 43% | 43% | 48% | 44% |
| Spec 4 | 70% | 70% | 70% | 70% | 70% | 70% | 70% | 70% | 70% |
| Cutoff 5 | 54.4 | 54.7 | 56.0 | 54.4 | 54.7 | 56.0 | 54.4 | 54.7 | 56.0 |
| Sens 5 | 18% | 29% | 20% | 29% | 46% | 27% | 20% | 22% | 25% |
| Spec 5 | 80% | 80% | 81% | 80% | 80% | 81% | 80% | 80% | 81% |
| Cutoff 6 | 97.1 | 92.1 | 92.1 | 97.1 | 92.1 | 92.1 | 97.1 | 92.1 | 92.1 |
| Sens 6 | 7% | 13% | 8% | 14% | 35% | 14% | 11% | 4% | 19% |
| Spec 6 | 90% | 90% | 90% | 90% | 90% | 90% | 90% | 90% | 90% |
| OR Quart 2 p Value 95% CI of OR Quart2 | 1.2 | 1.3 | 1.5 | 1.7 | 5.8 | 1.4 | 0.47 | 0.39 | 0.65 |
| | 0.57 | 0.61 | 0.22 | 0.12 | 0.024 | 0.31 | 0.23 | 0.27 | 0.52 |
| | 0.65 | 0.47 | 0.80 | 0.87 | 1.3 | 0.72 | 0.13 | 0.075 | 0.18 |
| | 2.2 | 3.6 | 2.7 | 3.5 | 27 | 2.9 | 1.6 | 2.0 | 2.4 |
| OR Quart 3 p Value 95% CI of OR Quart3 | 2.1 | 1.5 | 2.0 | 1.9 | 3.1 | 1.8 | 1.7 | 1.6 | 2.2 |
| | 0.013 | 0.46 | 0.026 | 0.062 | 0.17 | 0.099 | 0.26 | 0.40 | 0.14 |
| | 1.2 | 0.54 | 1.1 | 0.97 | 0.61 | 0.90 | 0.67 | 0.52 | 0.77 |
| | 3.8 | 3.9 | 3.6 | 3.8 | 15 | 3.5 | 4.4 | 5.1 | 6.1 |
| OR Quart 4 p. Value 95% CI of OR Quart4 | 1.2 | 1.8 | 1.5 | 2.5 | 9.9 | 2.6 | 1.3 | 1.6 | 1.7 |
| | 0.47 | 0.25 | 0.22 | 0.0091 | 0.0023 | 0.0052 | 0.64 | 0.41 | 0.31 |
| | 0.68 | 0.67 | 0.80 | 1.3 | 2.3 | 1.3 | 0.47 | 0.52 | 0.60 |
| | 2.3 | 4.6 | 2.7 | 4.8 | 43 | 5.0 | 3.4 | 5.1 | 5.0 |

# EP 2 661 626 B1

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.752 | 0.909 | 0.752 | 1.07 | 0.752 | 0.909 |
| Average | 1.30 | 1.47 | 1.30 | 1.57 | 1.30 | 1.08 |
| Stdev | 2.01 | 1.98 | 2.01 | 2.12 | 2.01 | 1.06 |
| p(t-test) | | 0.42 | | 0.24 | | 0.53 |
| Min | 0.00179 | 0.00179 | 0.00179 | 0.00179 | 0.00179 | 0.00230 |
| Max | 23.1 | 14.9 | 23.1 | 11.6 | 23.1 | 4.42 |
| n (Samp) | 268 | 138 | 268 | 104 | 268 | 35 |
| n (Patient) | 148 | 138 | 148 | 104 | 148 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 1.65 | 0.761 | 1.62 | 0.761 | 1.70 |
| Average | 1.41 | 1.72 | 1.41 | 1.75 | 1.41 | 1.43 |
| Stdev | 2.42 | 1.77 | 2.42 | 1.92 | 2.42 | 1.33 |
| p(t-test) | | 0.44 | | 0.39 | | 0.97 |
| Min | 0.00179 | 0.00179 | 0.00179 | 0.00246 | 0.00179 | 0.00230 |
| Max | 28.3 | 6.72 | 28.3 | 8.64 | 28.3 | 4.42 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.354 | 1.11 | 0.354 | 1.40 | 0.354 | 1.11 |
| Average | 1.15 | 1.55 | 1.15 | 1.66 | 1.15 | 1.19 |
| Stdev | 1.55 | 1.99 | 1.55 | 2.03 | 1.55 | 0.989 |
| p(t-test) | | 0.022 | | 0.0078 | | 0.87 |
| Min | 0.00179 | 0.00179 | 0.00179 | 0.00179 | 0.00179 | 0.00240 |
| Max | 8.77 | 14.9 | 8.77 | 11.6 | 8.77 | 3.36 |
| n (Samp) | 313 | 127 | 313 | 102 | 313 | 32 |
| n (Patient) | 152 | 127 | 152 | 102 | 152 | 32 |

| | 48r prior to AKI stage | | | 24hr prior to AKI stage | | | 0hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.54 | 0.58 | 0.52 | 0.58 | 0.57 | 0.53 | 0.59 | 0.58 |
| SE | 0.052 | 0.063 | 0.055 | 0.034 | 0.050 | 0.033 | 0.030 | 0.050 | 0.031 |
| p | 0.93 | 0.47 | 0.12 | 0.61 | 0.11 | 0.051 | 0.31 | 0.064 | 0.012 |
| nCohort 1 | 268 | 663 | 313 | 268 | 663 | 313 | 268 | 663 | 313 |
| nCohort 2 | 35 | 23 | 32 | 104 | 37 | 102 | 138 | 38 | 127 |
| Cutoff 1 | 0.0435 | 0.0209 | 0.208 | 0.0305 | 0.354 | 0.0407 | 0.0522 | 0.108 | 0.0742 |
| Sens 1 | 77% | 74% | 72% | 71% | 70% | 71% | 71% | 71% | 72% |
| Spec 1 | 31% | 25% | 48% | 24% | 45% | 32% | 32% | 38% | 40% |
| Cutoff 2 | 0.0209 | 0.00342 | 0.0742 | 0.00342 | 0.0209 | 0.00342 | 0.00352 | 0.0378 | 0.00352 |
| Sens 2 | 80% | 83% | 81% | 83% | 81% | 81% | 81% | 84% | 81% |
| Spec 2 | 24% | 17% | 40% | 16% | 25% | 20% | 19% | 29% | 24% |
| Cutoff 3 | 0.00246 | 0.00230 | 0.00332 | 0.00230 | 0.00342 | 0.00230 | 0.00246 | 0.00342 | 0.00246 |
| Sens 3 | 91% | 96% | 91% | 93% | 92% | 92% | 92% | 92% | 93% |
| Spec 3 | 10% | 6% | 15% | 6% | 17% | 6% | 10% | 17% | 13% |
| Cutoff 4 | 1.74 | 1.76 | 1.58 | 1.74 | 1.76 | 1.58 | 1.74 | 1.76 | 1.58 |
| Sens 4 | 29% | 35% | 38% | 33% | 41% | 40% | 39% | 47% | 43% |
| Spec 4 | 72% | 70% | 70% | 72% | 70% | 70% | 72% | 70% | 70% |
| Cutoff 5 | 2.17 | 2.35 | 2.11 | 2.17 | 2.35 | 2.11 | 2.17 | 2.35 | 2.11 |
| Sens 5 | 11% | 30% | 22% | 20% | 22% | 29% | 27% | 29% | 31% |
| Spec 5 | 81% | 80% | 80% | 81% | 80% | 80% | 81% | 80% | 80% |
| Cutoff 6 | 3.12 | 3.36 | 3.12 | 3.12 | 3.36 | 3.12 | 3.12 | 3.36 | 3.12 |
| Sens 6 | 3% | 4% | 3% | 16% | 14% | 19% | 14% | 16% | 14% |
| Spec 6 | 90% | 91% | 90% | 90% | 91% | 90% | 90% | 91% | 90% |

(continued)

| | 0hr prior to AKI stage | | | 24hr prior to AKI stage | | | 48r prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 1.1 | 2.3 | 1.0 | 0.47 | 0.61 | 0.57 | 0.99 | 0.79 | 1.5 |
| | 0.69 | 0.13 | 0.88 | 0.030 | 0.40 | 0.11 | 0.98 | 0.73 | 0.52 |
| | 0.63 | 0.78 | 0.57 | 0.24 | 0.20 | 0.28 | 0.35 | 0.21 | 0.42 |
| | 2.0 | 6.7 | 1.9 | 0.93 | 1.9 | 1.1 | 2.8 | 3.0 | 5.7 |
| OR Quart 3 | 0.87 | 1.6 | 1.1 | 0.90 | 1.7 | 1.0 | 1.4 | 1.4 | 3.7 |
| | 0.65 | 0.40 | 0.76 | 0.75 | 0.26 | 0.90 | 0.48 | 0.56 | 0.029 |
| p Value 95% CI of OR Quart3 | 0.48 | 0.52 | 0.60 | 0.49 | 0.68 | 0.55 | 0.54 | 0.44 | 1.1 |
| | 1.6 | 5.1 | 2.0 | 1.7 | 4.1 | 2.0 | 3.7 | 4.6 | 12 |
| OR Quart 4 p Value 95% CI of OR Quart4 | 1.5 | 2.9 | 2.0 | 0.95 | 1.4 | 1.6 | 0.99 | 1.4 | 2.4 |
| | 0.16 | 0.043 | 0.021 | 0.87 | 0.48 | 0.14 | 0.98 | 0.57 | 0.17 |
| | 0.85 | 1.0 | 1.1 | 0.51 | 0.55 | 0.86 | 0.35 | 0.44 | 0.70 |
| | 2.7 | 8.3 | 3.5 | 1.8 | 3.6 | 2.9 | 2.8 | 4.5 | 8.0 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5040 | 6160 | 5040 | 5290 | 5040 | 4110 |
| Average | 18600 | 14200 | 18600 | 14600 | 18600 | 12700 |
| Stdev | 34200 | 23500 | 34200 | 22100 | 34200 | 23300 |
| p(t-test) | | 0.11 | | 0.21 | | 0.26 |
| Min | 323 | 300 | 323 | 281 | 323 | 485 |
| Max | 238000 | 173000 | 238000 | 133000 | 238000 | 132000 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5580 | 8680 | 5580 | 6800 | 5580 | 4300 |
| Average | 16400 | 28200 | 16400 | 19500 | 16400 | 18400 |
| Stdev | 28100 | 47400 | 28100 | 30200 | 28100 | 35800 |
| p(t-test) | | 0.0028 | | 0.43 | | 0.68 |
| Min | 0.0130 | 300 | 0.0130 | 281 | 0.0130 | 876 |
| Max | 238000 | 190000 | 238000 | 133000 | 238000 | 171000 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5490 | 6080 | 5490 | 5320 | 5490 | 4170 |
| Average | 19900 | 14100 | 19900 | 13200 | 19900 | 15700 |
| Stdev | 34400 | 24200 | 34400 | 17500 | 34400 | 29600 |
| p(t-test) | | 0.036 | | 0.033 | | 0.44 |
| Min | 323 | 389 | 323 | 531 | 323 | 485 |
| Max | 238000 | 180000 | 238000 | 84400 | 238000 | 173000 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.57 | 0.52 | 0.51 | 0.53 | 0.51 | 0.46 | 0.47 | 0.48 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.025 | 0.040 | 0.025 | 0.029 | 0.042 | 0.029 | 0.046 | 0.050 | 0.046 |
| p | 0.19 | 0.070 | 0.32 | 0.66 | 0.55 | 0.82 | 0.33 | 0.53 | 0.65 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 | 3560 | 4110 | 3670 | 3370 | 3090 | 3480 | 2670 | 3160 | 2970 |
| Sens 1 | 70% | 71% | 70% | 70% | 71% | 71% | 72% | 72% | 72% |
| Spec 1 | 37% | 39% | 37% | 36% | 28% | 36% | 28% | 29% | 31% |
| Cutoff 2 | 2780 | 2380 | 3060 | 2420 | 2420 | 2740 | 1870 | 2400 | 2440 |
| Sens 2 | 80% | 81% | 80% | 80% | 80% | 81% | 80% | 81% | 81% |
| Spec 2 | 30% | 19% | 32% | 25% | 20% | 30% | 17% | 20% | 25% |
| Cutoff 3 | 1810 | 1670 | 2040 | 1760 | 1150 | 1880 | 904 | 1340 | 1290 |
| Sens 3 | 90% | 91% | 90% | 90% | 90% | 91% | 91% | 92% | 91% |
| Spec 3 | 16% | 12% | 19% | 16% | 6% | 17% | 6% | 8% | 9% |
| Cutoff 4 | 11600 | 11400 | 13400 | 11600 | 11400 | 13400 | 11600 | 11400 | 13400 |
| Sens 4 | 29% | 41% | 25% | 29% | 33% | 28% | 22% | 22% | 26% |
| Spec 4 | 70% | 70% | 70% | 70% | 70% | 70% | 70% | 70% | 70% |
| Cutoff 5 | 24300 | 21900 | 28700 | 24300 | 21900 | 28700 | 24300 | 21900 | 28700 |
| Sens 5 | 14% | 29% | 12% | 20% | 24% | 14% | 15% | 22% | 16% |
| Spec 5 | 80% | 80% | 80% | 80% | 80% | 80% | 80% | 80% | 80% |
| Cutoff 6 | 51700 | 44600 | 58300 | 51700 | 44600 | 58300 | 51700 | 44600 | 58300 |
| Sens 6 | 6% | 17% | 4% | 10% | 14% | 5% | 7% | 11% | 7% |
| Spec 6 | 90% | 90% | 90% | 90% | 90% | 90% | 90% | 90% | 90% |
| OR Quart 2 p Value 95% CI or OR Quart2 | 1.8 | 0.91 | 2.8 | 1.3 | 0.68 | 1.9 | 1.2 | 1.0 | 1.1 |
| | 0.025 | 0.83 | 9.4E-5 | 0.41 | 0.39 | 0.021 | 0.64 | 0.99 | 0.80 |
| | 1.1 | 0.40 | 1.7 | 0.72 | 0.29 | 1.1 | 0.50 | 0.37 | 0.44 |
| | 2.9 | 2.1 | 4.7 | 2.2 | 1.6 | 3.4 | 3.1 | 2.7 | 2.9 |
| OR Quart 3 p Value 95% CI of OR Quart3 | 2.0 | 1.3 | 2.7 | 1.2 | 1.0 | 1.7 | 1.8 | 1.4 | 1.8 |
| | 0.0050 | 0.45 | 1.4E-4 | 0.47 | 1.0 | 0.070 | 0.20 | 0.48 | 0.20 |
| | 1.2 | 0.63 | 1.6 | 0.70 | 0.46 | 0.96 | 0.74 | 0.55 | 0.74 |
| | 3.3 | 2.9 | 4.6 | 2.2 | 2.2 | 3.0 | 4.2 | 3.5 | 4.2 |
| OR Quart 4 p Value | 1.4 | 1.6 | 1.5 | 1.2 | 1.2 | 1.1 | 1.2 | 1.1 | 1.0 |
| | 0.21 | 0.20 | 0.13 | 0.49 | 0.57 | 0.66 | 0.64 | 0.80 | 0.99 |
| | 0.83 | 0.77 | 0.88 | 0.69 | 0.59 | 0.63 | 0.50 | 0.43 | 0.39 |
| 95% CI of OR Quart4 | 2.3 | 3.4 | 2.6 | 2.1 | 2.6 | 2.1 | 3.1 | 3.0 | 2.6 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.35 | 3.36 | 3.35 | 3.79 | 3.35 | 3.13 |
| Average | 4.65 | 5.50 | 4.65 | 6.97 | 4.65 | 6.32 |
| Stdev | 5.33 | 5.69 | 5.33 | 9.72 | 5.33 | 9.52 |
| p(t-test) | | 0.14 | | 0.0041 | | 0.13 |
| Min | 0.0801 | 0.0298 | 0.0801 | 0.0298 | 0.0801 | 0.376 |
| Max | 46.9 | 33.3 | 46.9 | 81.9 | 46.9 | 43.0 |
| n (Samp) | 264 | 133 | 264 | 99 | 264 | 33 |
| n (Patient) | 147 | 133 | 147 | 99 | 147 | 33 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.54 | 3.07 | 3.54 | 3.69 | 3.54 | 2.94 |
| Average | 6.54 | 4.01 | 6.54 | 5.23 | 6.54 | 5.23 |
| Stdev | 13.5 | 3.62 | 13.5 | 4.09 | 13.5 | 8.08 |
| p(t-test) | | 0.25 | | 0.56 | | 0.65 |
| Min | 0.000104 | 0.0298 | 0.000104 | 0.0298 | 0.000104 | 0.434 |
| Max | 279 | 16.5 | 279 | 15.8 | 279 | 37.5 |
| n (Samp) | 644 | 38 | 644 | 36 | 644 | 22 |
| n (Patient) | 285 | 38 | 285 | 36 | 285 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.36 | 3.82 | 3.36 | 3.88 | 3.36 | 3.13 |
| Average | 4.92 | 5.83 | 4.92 | 7.66 | 4.92 | 7.18 |
| Stdev | 6.15 | 5.78 | 6.15 | 11.2 | 6.15 | 9.67 |
| p(t-test) | | 0.16 | | 0.0024 | | 0.067 |
| Min | 0.000201 | 0.256 | 0.000201 | 0.235 | 0.000201 | 0.376 |
| Max | 49.4 | 33.3 | 49.4 | 81.9 | 49.4 | 43.0 |
| n (Samp) | 308 | 121 | 308 | 94 | 308 | 31 |
| n (Patient) | 151 | 121 | 151 | 94 | 151 | 31 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.44 | 0.57 | 0.57 | 0.53 | 0.57 | 0.51 | 0.44 | 0.52 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.031 | 0.049 | 0.031 | 0.034 | 0.050 | 0.034 | 0.054 | 0.064 | 0.055 |
| p | 0.12 | 0.22 | 0.021 | 0.037 | 0.58 | 0.036 | 0.87 | 0.35 | 0.66 |
| nCohort 1 | 264 | 644 | 308 | 264 | 644 | 308 | 264 | 644 | 308 |
| nCohort 2 | 133 | 38 | 121 | 99 | 36 | 94 | 33 | 22 | 31 |
| Cutoff 1 | 1.95 | 1.66 | 2.23 | 2.10 | 2.43 | 2.12 | 1.02 | 1.56 | 1.21 |
| Sens 1 | 71% | 71% | 70% | 71% | 72% | 70% | 73% | 73% | 71% |
| Spec 1 | 33% | 25% | 34% | 35% | 37% | 33% | 18% | 24% | 20% |
| Cutoff 2 | 1.66 | 1.16 | 1.87 | 1.48 | 1.68 | 1.42 | 0.935 | 1.06 | 0.990 |
| Sens 2 | 80% | 82% | 80% | 81% | 81% | 81% | 82% | 82% | 81% |
| Spec 2 | 28% | 17% | 31% | 25% | 26% | 24% | 16% | 16% | 16% |
| Cutoff 3 | 1.06 | 0.477 | 1.36 | 0.733 | 0.899 | 0.733 | 0.717 | 0.795 | 0.795 |
| Sens 3 | 90% | 92% | 90% | 91% | 92% | 90% | 91% | 91% | 90% |
| Spec 3 | 19% | 6% | 22% | 12% | 12% | 12% | 11% | 11% | 13% |
| Cutoff 4 | 5.15 | 6.29 | 5.26 | 5.15 | 6.29 | 5.26 | 5.15 | 6.29 | 5.26 |
| Sens 4 | 38% | 21% | 40% | 44% | 36% | 45% | 36% | 14% | 42% |
| Spec 4 | 70% | 70% | 70% | 70% | 70% | 70% | 70% | 70% | 70% |
| Cutoff 5 | 6.60 | 9.07 | 6.57 | 6.60 | 9.07 | 6.57 | 6.60 | 9.07 | 6.57 |
| Sens 5 | 30% | 11% | 31% | 38% | 19% | 40% | 27% | 14% | 35% |
| Spec 5 | 80% | 80% | 80% | 80% | 80% | 80% | 80% | 80% | 80% |
| Cutoff 6 | 10.5 | 13.6 | 10.6 | 10.5 | 13.6 | 10.6 | 10.5 | 13.6 | 10.6 |
| Sens 6 | 14% | 3% | 16% | 21% | 6% | 22% | 18% | 9% | 23% |
| Spec 6 | 90% | 90% | 90% | 90% | 90% | 90% | 90% | 90% | 90% |
| OR Quart 2 p Value 95% CI of OR Quart2 | 1.9 | 1.2 | 2.4 | 1.1 | 1.5 | 0.99 | 0.51 | 2.4 | 0.56 |
| | 0.036 | 0.78 | 0.0063 | 0.77 | 0.46 | 0.97 | 0.20 | 0.21 | 0.29 |
| | 1.0 | 0.41 | 1.3 | 0.57 | 0.54 | 0.50 | 0.18 | 0.61 | 0.19 |
| | 3.5 | 3.3 | 4.5 | 2.2 | 3.9 | 1.9 | 1.4 | 9.5 | 1.6 |
| OR Quart 3 p Value 95% CI of OR Quart3 | 0.95 | 1.8 | 1.3 | 0.61 | 1.6 | 0.61 | 0.51 | 1.7 | 0.27 |
| | 0.87 | 0.24 | 0.50 | 0.19 | 0.34 | 0.20 | 0.20 | 0.48 | 0.054 |
| | 0.50 | 0.68 | 0.65 | 0.29 | 0.61 | 0.29 | 0.18 | 0.40 | 0.072 |
| | 1.8 | 4.6 | 2.4 | 1.3 | 4.3 | 1.3 | 1.4 | 7.2 | 1.0 |
| OR Quart 4 p Value 95% CI of OR Quart4 | 2.0 | 1.6 | 2.5 | 2.2 | 1.1 | 2.3 | 0.88 | 2.4 | 1.2 |
| | 0.020 | 0.33 | 0.0048 | 0.014 | 0.79 | 0.010 | 0.79 | 0.21 | 0.67 |
| | 1.1 | 0.61 | 1.3 | 1.2 | 0.41 | 1.2 | 0.35 | 0.61 | 0.49 |
| | 3.7 | 4.3 | 4.6 | 4.2 | 3.2 | 4.2 | 2.2 | 9.5 | 3.0 |

**Heparin-binding growth factor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 876 | 759 | 876 | 657 | 876 | 572 |
| Average | 1250 | 1120 | 1250 | 1250 | 1250 | 915 |
| Stdev | 1560 | 1350 | 1560 | 1570 | 1560 | 972 |
| p(t-test) | | 0.30 | | 0.98 | | 0.15 |
| Min | 0.609 | 12.9 | 0.609 | 8.16 | 0.609 | 0.779 |
| Max | 16700 | 13500 | 16700 | 7750 | 16700 | 3960 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 843 | 688 | 843 | 882 | 843 | 548 |
| Average | 1240 | 908 | 1240 | 917 | 1240 | 1000 |
| Stdev | 1480 | 889 | 1480 | 749 | 1480 | 1120 |
| p(t-test) | | 0.090 | | 0.12 | | 0.34 |
| Min | 0.00328 | 1.77 | 0.00328 | 29.5 | 0.00328 | 13.3 |
| Max | 16700 | 4350 | 16700 | 2920 | 16700 | 3960 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 858 | 772 | 858 | 658 | 858 | 595 |
| Average | 1200 | 1170 | 1200 | 1270 | 1200 | 927 |
| Stdev | 1480 | 1490 | 1480 | 1580 | 1480 | 973 |
| p(t-test) | | 0.84 | | 0.65 | | 0.24 |
| Min | 0.609 | 12.9 | 0.609 | 8.16 | 0.609 | 0.779 |
| Max | 16700 | 13500 | 16700 | 7750 | 16700 | 3960 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.43 | 0.49 | 0.47 | 0.45 | 0.49 | 0.42 | 0.42 | 0.43 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.025 | 0.040 | 0.025 | 0.029 | 0.042 | 0.029 | 0.046 | 0.050 | 0.047 |
| p | 0.52 | 0.086 | 0.84 | 0.33 | 0.26 | 0.60 | 0.082 | 0.13 | 0.16 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 | 477 | 358 | 466 | 318 | 262 | 360 | 359 | 262 | 359 |
| Sens 1 | 70% | 71% | 70% | 70% | 71% | 71% | 72% | 72% | 72% |
| Spec 1 | 31% | 24% | 31% | 23% | 18% | 26% | 25% | 18% | 26% |
| Cutoff 2 | 358 | 231 | 289 | 231 | 113 | 260 | 202 | 167 | 211 |
| Sens 2 | 80% | 81% | 80% | 80% | 80% | 81% | 80% | 81% | 81% |
| Spec 2 | 25% | 15% | 21% | 17% | 8% | 20% | 16% | 12% | 16% |
| Cutoff 3 | 170 | 122 | 159 | 103 | 87.4 | 121 | 41.6 | 54.1 | 51.2 |
| Sens 3 | 90% | 91% | 90% | 90% | 90% | 91% | 91% | 92% | 91% |
| Spec 3 | 13% | 9% | 13% | 10% | 7% | 10% | 5% | 4% | 6% |
| Cutoff 4 | 1440 | 1420 | 1400 | 1440 | 1420 | 1400 | 1440 | 1420 | 1400 |
| Sens 4 | 25% | 21% | 26% | 30% | 27% | 29% | 22% | 31% | 21% |
| Spec 4 | 70% | 70% | 70% | 70% | 70% | 70% | 70% | 70% | 70% |
| Cutoff 5 | 1750 | 1800 | 1720 | 1750 | 1800 | 1720 | 1750 | 1800 | 1720 |
| Sens 5 | 15% | 12% | 16% | 23% | 10% | 24% | 17% | 19% | 14% |
| Spec 5 | 80% | 80% | 80% | 80% | 80% | 80% | 80% | 80% | 80% |
| Cutoff 6 | 2430 | 2550 | 2410 | 2430 | 2550 | 2410 | 2430 | 2550 | 2410 |
| Sens 6 | 9% | 9% | 10% | 11% | 4% | 12% | 9% | 11% | 9% |
| Spec 6 | 90% | 90% | 90% | 90% | 90% | 90% | 90% | 90% | 90% |
| OR Quart 2 p Value 95% CI ot OR Quart2 | 0.98 | 1.4 | 1.0 | 0.66 | 1.5 | 0.74 | 0.88 | 0.44 | 1.6 |
| | 0.93 | 0.41 | 1.0 | 0.16 | 0.33 | 0.31 | 0.80 | 0.17 | 0.33 |
| | 0.60 | 0.62 | 0.61 | 0.37 | 0.68 | 0.42 | 0.33 | 0.13 | 0.61 |
| | 1.6 | 3.3 | 1.6 | 1.2 | 3.2 | 1.3 | 2.4 | 1.4 | 4.3 |
| OR Quart 3 p Value 95% CI of OR Quart3 | 1.9 | 1.6 | 1.6 | 0.89 | 0.63 | 1.2 | 1.9 | 1.2 | 2.0 |
| | 0.0085 | 0.23 | 0.058 | 0.67 | 0.34 | 0.42 | 0.14 | 0.64 | 0.16 |
| | 1.2 | 0.73 | 0.98 | 0.51 | 0.24 | 0.73 | 0.80 | 0.50 | 0.76 |
| | 3.0 | 3.7 | 2.5 | 1.5 | 1.6 | 2.1 | 4.5 | 3.0 | 5.1 |
| OR Quart 4 p Value 95% CI of OR Quart4 | 0.95 | 1.9 | 0.94 | 1.2 | 1.6 | 1.1 | 1.5 | 1.4 | 1.8 |
| | 0.83 | 0.12 | 0.80 | 0.48 | 0.25 | 0.76 | 0.37 | 0.50 | 0.23 |
| | 0.58 | 0.84 | 0.57 | 0.71 | 0.73 | 0.63 | 0.62 | 0.56 | 0.69 |
| | 1.6 | 4.1 | 1.5 | 2.1 | 3.4 | 1.9 | 3.7 | 3.3 | 4.7 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 2.68E-5 | 2.96E-5 | 3.44E-5 | 2.96E-5 | 2.66E-5 |
| Average | 0.00439 | 0.00352 | 0.00439 | 0.00561 | 0.00439 | 0.00939 |
| Stdev | 0.0252 | 0.0302 | 0.0252 | 0.0463 | 0.0252 | 0.0411 |
| p(t-test) | | 0.73 | | 0.73 | | 0.39 |
| Min | 5.62E-6 | 5.62E-6 | 5.62E-6 | 5.62E-6 | 5.62E-6 | 1.34E-5 |
| Max | 0.260 | 0.388 | 0.260 | 0.470 | 0.260 | 0.193 |
| n (Samp) | 363 | 168 | 363 | 103 | 363 | 22 |
| n (Patient) | 151 | 168 | 151 | 103 | 151 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 2.68E-5 | 2.83E-5 | 2.63E-5 |
| Average | 0.00440 | 0.00168 | 0.00440 | 0.0109 | 0.00440 | 0.000380 |
| Stdev | 0.0283 | 0.00444 | 0.0283 | 0.0620 | 0.0283 | 0.00166 |
| p(t-test) | | 0.50 | | 0.19 | | 0.48 |
| Min | 5.62E-6 | 5.62E-6 | 5.62E-6 | 5.62E-6 | 5.62E-6 | 5.62E-6 |
| Max | 0.470 | 0.0164 | 0.470 | 0.388 | 0.470 | 0.00836 |
| n (Samp) | 960 | 49 | 960 | 39 | 960 | 25 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.68E-5 | 2.83E-5 | 4.13E-5 | 2.83E-5 | 3.20E-5 |
| Average | 0.00568 | 0.00415 | 0.00568 | 0.00569 | 0.00568 | 0.0300 |
| Stdev | 0.0323 | 0.0320 | 0.0323 | 0.0461 | 0.0323 | 0.106 |
| p(t-test) | | 0.61 | | 1.00 | | 0.0033 |
| Min | 5.62E-6 | 5.62E-6 | 5.62E-6 | 5.62E-6 | 5.62E-6 | 1.34E-5 |
| Max | 0.359 | 0.388 | 0.359 | 0.470 | 0.359 | 0.492 |
| n (Samp) | 444 | 155 | 444 | 104 | 444 | 24 |
| n (Patient) | 172 | 155 | 172 | 104 | 172 | 24 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.51 | 0.48 | 0.53 | 0.50 | 0.56 | 0.45 | 0.45 | 0.55 |
| SE | 0.027 | 0.042 | 0.027 | 0.033 | 0.047 | 0.032 | 0.065 | 0.060 | 0.062 |
| p | 0.20 | 0.87 | 0.49 | 0.39 | 0.92 | 0.048 | 0.47 | 0.40 | 0.39 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 | 2.17E-5 | 1.92E-5 | 2.17E-5 | 2.46E-5 | 1.92E-5 | 2.46E-5 | 1.88E-5 | 2.17E-5 | 2.17E-5 |
| Sens 1 | 71% | 76% | 72% | 72% | 74% | 71% | 77% | 76% | 71% |
| Spec 1 | 25% | 25% | 27% | 33% | 25% | 35% | 19% | 28% | 27% |
| Cutoff 2 | 1.82E-5 | 1.82E-5 | 1.82E-5 | 1.92E-5 | 1.82E-5 | 1.92E-5 | 1.82E-5 | 1.88E-5 | 1.88E-5 |
| Sens 2 | 86% | 86% | 85% | 81% | 87% | 81% | 86% | 80% | 83% |
| Spec 2 | 16% | 18% | 16% | 23% | 18% | 23% | 16% | 22% | 18% |
| Cutoff 3 | 1.34E-5 | 7.53E-6 | 1.34E-5 | 1.82E-5 | 0 | 1.82E-5 | 1.34E-5 | 5.62E-6 | 1.34E-5 |
| Sens 3 | 93% | 94% | 93% | 91% | 100% | 92% | 91% | 92% | 92% |
| Spec 3 | 10% | 5% | 10% | 16% | 0% | 16% | 10% | 3% | 10% |
| Cutoff 4 | 8.63E-5 | 4.39E-5 | 4.39E-5 | 8.63E-5 | 4.39E-5 | 4.39E-5 | 8.63E-5 | 4.39E-5 | 4.39E-5 |
| Sens 4 | 24% | 31% | 26% | 35% | 36% | 43% | 27% | 20% | 42% |
| Spec 4 | 71% | 72% | 70% | 71% | 72% | 70% | 71% | 72% | 70% |
| Cutoff 5 | 0.000168 | 0.000114 | 0.000114 | 0.000168 | 0.000114 | 0.000114 | 0.000168 | 0.000114 | 0.000114 |
| Sens 5 | 11% | 18% | 17% | 19% | 31% | 27% | 18% | 16% | 33% |
| Spec 5 | 81% | 81% | 80% | 81% | 81% | 80% | 81% | 81% | 80% |
| Cutoff 6 | 0.00442 | 0.00364 | 0.00364 | 0.00442 | 0.00364 | 0.00364 | 0.00442 | 0.00364 | 0.00364 |
| Sens 6 | 8% | 14% | 8% | 8% | 10% | 10% | 9% | 4% | 25% |
| Spec 6 | 90% | 91% | 90% | 90% | 91% | 90% | 90% | 91% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 0.80 | 1.2 | 0.90 | 0.89 | 1.00 | 0.76 | 0.49 | 0.80 | 0.56 |
| | 0.42 | 0.69 | 0.68 | 0.72 | 0.99 | 0.41 | 0.32 | 0.74 | 0.36 |
| | 0.47 | 0.53 | 0.53 | 0.47 | 0.41 | 0.39 | 0.12 | 0.21 | 0.16 |
| | 1.4 | 2.6 | 1.5 | 1.7 | 2.4 | 1.5 | 2.0 | 3.0 | 2.0 |
| OR Quart 3 p Value 95% CI of OR Quart3 | 1.3 | 1.0 | 1.2 | 1.2 | 0.49 | 1.5 | 0.83 | 2.1 | 0.56 |
| | 0.36 | 1.0 | 0.43 | 0.64 | 0.20 | 0.18 | 0.77 | 0.20 | 0.36 |
| | 0.76 | 0.44 | 0.73 | 0.63 | 0.16 | 0.83 | 0.25 | 0.69 | 0.16 |
| | 2.1 | 2.3 | 2.0 | 2.1 | 1.4 | 2.7 | 2.8 | 6.1 | 2.0 |
| OR Quart 4 p Value 95% CI of OR Quart4 | 1.1 | 0.91 | 1.2 | 1.1 | 1.4 | 1.2 | 1.4 | 1.2 | 1.3 |
| | 0.66 | 0.82 | 0.57 | 0.78 | 0.41 | 0.54 | 0.57 | 0.76 | 0.61 |
| | 0.67 | 0.39 | 0.69 | 0.59 | 0.62 | 0.66 | 0.46 | 0.36 | 0.47 |
| | 1.9 | 2.1 | 1.9 | 2.0 | 3.3 | 2.2 | 4.1 | 4.0 | 3.6 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0153 | 0.0125 | 0.0153 | 0.0150 | 0.0153 | 0.00875 |
| Average | 0.113 | 0.157 | 0.113 | 0.196 | 0.113 | 0.234 |
| Stdev | 0.326 | 0.507 | 0.326 | 0.597 | 0.326 | 0.658 |
| p(t-test) | | 0.23 | | 0.064 | | 0.12 |
| Min | 1.14E-9 | 1.40E-9 | 1.14E-9 | 1.14E-9 | 1.14E-9 | 1.40E-9 |
| Max | 3.07 | 3.58 | 3.07 | 4.47 | 3.07 | 2.56 |
| n (Samp) | 363 | 168 | 363 | 103 | 363 | 22 |
| n (Patient) | 151 | 168 | 151 | 103 | 151 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0141 | 0.0212 | 0.0141 | 0.0165 | 0.0141 | 0.00900 |
| Average | 0.160 | 0.225 | 0.160 | 0.237 | 0.160 | 0.218 |
| Stdev | 0.532 | 0.660 | 0.532 | 0.626 | 0.532 | 0.618 |
| p(t-test) | | 0.41 | | 0.38 | | 0.59 |
| Min | 1.14E-9 | 4.16E-6 | 1.14E-9 | 0.000163 | 1.14E-9 | 6.78E-7 |
| Max | 8.92 | 3.42 | 8.92 | 3.58 | 8.92 | 2.56 |
| n (Samp) | 960 | 49 | 960 | 39 | 960 | 25 |
| n (Patient) | 317 | 49 | 317 | 39 | 317 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0157 | 0.0112 | 0.0157 | 0.0152 | 0.0157 | 0.00954 |
| Average | 0.132 | 0.159 | 0.132 | 0.183 | 0.132 | 0.271 |
| Stdev | 0.359 | 0.491 | 0.359 | 0.594 | 0.359 | 0.778 |
| p(t-test) | | 0.46 | | 0.26 | | 0.090 |
| Min | 1.14E-9 | 1.40E-9 | 1.14E-9 | 1.14E-9 | 1.14E-9 | 1.40E-9 |
| Max | 3.07 | 3.58 | 3.07 | 4.47 | 3.07 | 2.96 |
| n (Samp) | 444 | 155 | 444 | 104 | 444 | 24 |
| n (Patient) | 172 | 155 | 172 | 104 | 172 | 24 |

|  | 0hr prior to AKI stage | | | 24hr prior to AKI stage | | | 48hr prior to AKI stage | | |
| --- | --- | --- | --- | --- | --- | --- | --- | --- | --- |
|  | sCr or UO | sCr only | UO only | sCr or UO | sCr only | UO only | sCr or UO | sCr only | UO only |
| AUC | 0.47 | 0.55 | 0.45 | 0.49 | 0.56 | 0.47 | 0.40 | 0.44 | 0.43 |
| SE | 0.027 | 0.043 | 0.027 | 0.032 | 0.049 | 0.032 | 0.066 | 0.060 | 0.062 |
| p | 0.33 | 0.26 | 0.083 | 0.76 | 0.18 | 0.32 | 0.11 | 0.30 | 0.23 |
| nCohort 1 | 363 | 960 | 444 | 363 | 960 | 444 | 363 | 960 | 444 |
| nCohort 2 | 168 | 49 | 155 | 103 | 39 | 104 | 22 | 25 | 24 |
| Cutoff 1 | 0.00513 | 0.00681 | 0.00479 | 0.00401 | 0.00811 | 0.00397 | 0.00605 | 0.00494 | 0.00605 |
| Sens 1 | 70% | 71% | 70% | 71% | 72% | 70% | 73% | 72% | 71% |
| Spec 1 | 26% | 32% | 23% | 22% | 35% | 21% | 29% | 25% | 28% |
| Cutoff 2 | 0.00273 | 0.00498 | 0.00273 | 0.00198 | 0.00544 | 0.00198 | 0.00115 | 0.00372 | 0.00115 |
| Sens 2 | 80% | 82% | 80% | 81% | 82% | 81% | 82% | 80% | 83% |
| Spec 2 | 17% | 25% | 15% | 12% | 27% | 11% | 8% | 20% | 8% |
| Cutoff 3 | 0.00105 | 0.00219 | 0.00105 | 0.000652 | 0.00108 | 0.000685 | 8.37E-7 | 0.000661 | 8.37E-7 |
| Sens 3 | 90% | 92% | 90% | 90% | 92% | 90% | 91% | 92% | 92% |
| Spec 3 | 8% | 13% | 7% | 7% | 7% | 6% | 2% | 6% | 2% |
| Cutoff 4 | 0.0369 | 0.0340 | 0.0438 | 0.0369 | 0.0340 | 0.0438 | 0.0369 | 0.0340 | 0.0438 |
| Sens 4 | 27% | 37% | 25% | 34% | 44% | 28% | 18% | 24% | 25% |
| Spec 4 | 70% | 70% | 70% | 70% | 70% | 70% | 70% | 70% | 70% |
| Cutoff 5 | 0.0727 | 0.0744 | 0.110 | 0.0727 | 0.0744 | 0.110 | 0.0727 | 0.0744 | 0.110 |
| Sens 5 | 18% | 24% | 17% | 25% | 31% | 18% | 18% | 20% | 17% |
| Spec 5 | 80% | 80% | 80% | 80% | 80% | 80% | 80% | 80% | 80% |
| Cutoff 6 | 0.291 | 0.361 | 0.347 | 0.291 | 0.361 | 0.347 | 0.291 | 0.361 | 0.347 |
| Sens 6 | 12% | 12% | 10% | 16% | 15% | 12% | 14% | 12% | 12% |
| Spec 6 | 90% | 90% | 90% | 90% | 90% | 90% | 90% | 90% | 90% |

EP 2 661 626 B1

56

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 | 0.83 | 1.1 | 0.93 | 0.71 | 1.4 | 0.95 | 0.24 | 0.60 | 0.32 |
| p Value | 0.50 | 0.82 | 0.78 | 0.28 | 0.47 | 0.88 | 0.21 | 0.48 | 0.17 |
| 95% CI of OR Quart2 | 0.49 | 0.46 | 0.54 | 0.38 | 0.54 | 0.52 | 0.027 | 0.14 | 0.064 |
| | 1.4 | 2.6 | 1.6 | 1.3 | 3.8 | 1.8 | 2.2 | 2.5 | 1.6 |
| OR Quart 3 | 1.2 | 1.3 | 1.2 | 0.59 | 1.00 | 0.65 | 3.0 | 2.1 | 1.5 |
| p Value | 0.51 | 0.52 | 0.59 | 0.11 | 0.99 | 0.19 | 0.068 | 0.20 | 0.43 |
| 95% CI of OR Quart3 | 0.71 | 0.57 | 0.68 | 0.31 | 0.34 | 0.34 | 0.92 | 0.69 | 0.53 |
| | 2.0 | 3.1 | 2.0 | 1.1 | 2.9 | 1.2 | 9.8 | 6.1 | 4.5 |
| OR Quart 4 | 1.2 | 1.5 | 1.6 | 1.2 | 2.2 | 1.5 | 1.6 | 1.4 | 1.2 |
| p Value | 0.57 | 0.31 | 0.087 | 0.53 | 0.090 | 0.19 | 0.51 | 0.56 | 0.78 |
| 95% Clot OR Quart4 | 0.69 | 0.67 | 0.94 | 0.67 | 0.88 | 0.82 | 0.42 | 0.44 | 0.38 |
| | 1.9 | 3.5 | 2.6 | 2.2 | 5.5 | 2.6 | 5.7 | 4.5 | 3.6 |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 134 | 130 | 134 | 159 | 134 | 141 |
| Average | 140 | 141 | 140 | 160 | 140 | 159 |
| Stdev | 52.5 | 60.1 | 52.5 | 71.7 | 52.5 | 64.0 |
| p(t-test) | | 0.81 | | 0.0056 | | 0.061 |
| Min | 31.0 | 40.9 | 31.0 | 52.1 | 31.0 | 67.2 |
| Max | 311 | 360 | 311 | 444 | 311 | 312 |
| n (Samp) | 250 | 131 | 250 | 95 | 250 | 32 |
| n (Patient) | 145 | 131 | 145 | 95 | 145 | 32 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 132 | 126 | 132 | 164 | 132 | 137 |
| Average | 142 | 137 | 142 | 155 | 142 | 142 |
| Stdev | 60.2 | 51.4 | 60.2 | 57.1 | 60.2 | 53.3 |
| p(t-test) | | 0.67 | | 0.19 | | 1.00 |
| Min | 31.0 | 47.9 | 31.0 | 55.2 | 31.0 | 64.6 |
| Max | 444 | 249 | 444 | 287 | 444 | 241 |
| n (Samp) | 619 | 37 | 619 | 35 | 619 | 21 |
| n (Patient) | 284 | 37 | 284 | 35 | 284 | 21 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 130 | 140 | 130 | 162 | 130 | 162 |
| Average | 136 | 146 | 136 | 162 | 136 | 168 |
| Stdev | 51.8 | 62.2 | 51.8 | 72.8 | 51.8 | 65.3 |
| p(t-test) | | 0.11 | | 2.7E-4 | | 0.0028 |
| Min | 31.0 | 40.9 | 31.0 | 52.1 | 31.0 | 67.2 |
| Max | 311 | 360 | 311 | 444 | 311 | 312 |
| n (Samp) | 291 | 119 | 291 | 91 | 291 | 29 |
| n (Patient) | 149 | 119 | 149 | 91 | 149 | 29 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.49 | 0.49 | 0.53 | 0.57 | 0.58 | 0.60 | 0.58 | 0.51 | 0.64 |
| SE | 0.031 | 0.049 | 0.032 | 0.035 | 0.052 | 0.035 | 0.056 | 0.065 | 0.058 |
| p | 0.75 | 0.81 | 0.29 | 0.048 | 0.13 | 0.0065 | 0.17 | 0.84 | 0.015 |
| nCohort 1 | 250 | 619 | 291 | 250 | 619 | 291 | 250 | 619 | 291 |
| nCohort 2 | 131 | 37 | 119 | 95 | 35 | 91 | 32 | 21 | 29 |
| Cutoff 1 | 105 | 109 | 105 | 107 | 116 | 111 | 122 | 99.6 | 131 |
| Sens 1 | 70% | 70% | 71% | 71% | 71% | 70% | 72% | 71% | 72% |
| Spec 1 | 28% | 35% | 33% | 30% | 39% | 36% | 40% | 29% | 52% |
| Cutoff 2 | 86.4 | 95.7 | 85.9 | 88.0 | 99.6 | 87.8 | 97.1 | 97.5 | 90.2 |
| Sens 2 | 80% | 81% | 81% | 80% | 80% | 80% | 81% | 81% | 83% |
| Spec 2 | 16% | 26% | 17% | 18% | 29% | 19% | 23% | 28% | 21% |
| Cutoff 3 | 76.2 | 76.7 | 76.5 | 74.7 | 80.9 | 74.0 | 88.0 | 76.2 | 77.4 |
| Sens 3 | 90% | 92% | 91% | 91% | 91% | 90% | 91% | 90% | 93% |
| Spec 3 | 9% | 12% | 10% | 8% | 15% | 8% | 18% | 11% | 11% |
| Cutoff 4 | 162 | 166 | 161 | 162 | 166 | 161 | 162 | 166 | 161 |
| Sens 4 | 32% | 24% | 36% | 48% | 49% | 51% | 41% | 38% | 52% |
| Spec 4 | 70% | 70% | 70% | 70% | 70% | 70% | 70% | 70% | 70% |
| Cutoff 5 | 183 | 190 | 180 | 183 | 190 | 180 | 183 | 190 | 180 |
| Sens 5 | 21% | 16% | 25% | 33% | 29% | 35% | 31% | 24% | 45% |
| Spec 5 | 80% | 80% | 80% | 80% | 80% | 80% | 80% | 80% | 80% |
| Cutoff 6 | 203 | 225 | 203 | 203 | 225 | 203 | 203 | 225 | 203 |
| Sens 6 | 14% | 11% | 16% | 24% | 9% | 26% | 25% | 5% | 31% |
| Spec 6 | 90% | 90% | 90% | 90% | 90% | 90% | 90% | 90% | 90% |
| OR Quart 2 p Value 95% CI of OR Quart2 | 0.66 | 1.3 | 0.77 | 0.45 | 0.99 | 0.60 | 0.59 | 1.5 | 0.48 |
| | 0.18 | 0.61 | 0.41 | 0.032 | 0.99 | 0.18 | 0.37 | 0.52 | 0.31 |
| | 0.36 | 0.47 | 0.42 | 0.22 | 0.34 | 0.29 | 0.18 | 0.42 | 0.12 |
| | 1.2 | 3.6 | 1.4 | 0.94 | 2.9 | 1.3 | 1.9 | 5.5 | 2.0 |
| OR Quart 3 p Value 95% CI of OR Quart3 | 0.85 | 2.1 | 0.70 | 0.84 | 1.2 | 1.0 | 0.73 | 1.3 | 1.0 |
| | 0.58 | 0.12 | 0.27 | 0.61 | 0.79 | 1.0 | 0.57 | 0.74 | 1.0 |
| | 0.47 | 0.82 | 0.38 | 0.43 | 0.41 | 0.50 | 0.24 | 0.33 | 0.31 |
| | 1.5 | 5.3 | 1.3 | 1.6 | 3.2 | 2.0 | 2.2 | 4.8 | 3.2 |
| OR Quart 4 p Value | 1.0 | 1.0 | 1.3 | 1.3 | 1.9 | 2.0 | 1.7 | 1.5 | 2.6 |
| | 0.96 | 1.0 | 0.33 | 0.36 | 0.18 | 0.031 | 0.25 | 0.52 | 0.063 |
| | 0.57 | 0.34 | 0.75 | 0.71 | 0.75 | 1.1 | 0.67 | 0.42 | 0.95 |
| 95% CI of OR Quart4 | 1.8 | 2.9 | 2.4 | 2.5 | 4.9 | 3.8 | 4.5 | 5.5 | 7.2 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1150 | 1450 | 1150 | 1130 | 1150 | 927 |
| Average | 1960 | 2200 | 1960 | 2870 | 1960 | 1940 |
| Stdev | 2780 | 2360 | 780 | 7500 | 2780 | 3240 |
| p(t-test) | | 0.29 | | 0.035 | | 0.97 |
| Min | 33.0 | 47.5 | 33.0 | 23.5 | 33.0 | 105 |
| Max | 33000 | 21300 | 33000 | 68100 | 33000 | 19700 |
| n (Samp) | 430 | 195 | 430 | 132 | 430 | 46 |
| n (Patient) | 203 | 195 | 203 | 132 | 203 | 46 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1380 | 1460 | 1380 | 1250 | 1380 | 906 |
| Average | 2390 | 2200 | 2390 | 1670 | 2390 | 1470 |
| Stdev | 3860 | 2030 | 3860 | 1630 | 3860 | 1630 |
| p(t-test) | | 0.70 | | 0.18 | | 0.16 |
| Min | 0.0376 | 47.5 | 0.0376 | 23.5 | 0.0376 | 160 |
| Max | 68100 | 8500 | 68100 | 8210 | 68100 | 7210 |
| n (Samp) | 1121 | 58 | 1121 | 51 | 1121 | 36 |
| n (Patient) | 395 | 58 | 395 | 51 | 395 | 36 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1250 | 1470 | 1250 | 1170 | 1250 | 1080 |
| Average | 2040 | 2280 | 2040 | 3010 | 2040 | 2270 |
| Stdev | 2810 | 2400 | 2810 | 7590 | 2810 | 3520 |
| p(t-test) | | 0.29 | | 0.021 | | 0.61 |
| Min | 13.4 | 122 | 13.4 | 99.8 | 13.4 | 105 |
| Max | 33000 | 21300 | 33000 | 68100 | 33000 | 19700 |
| n (Samp) | 506 | 182 | 506 | 129 | 506 | 43 |
| n (Patient) | 215 | 182 | 215 | 129 | 215 | 43 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.53 | 0.56 | 0.51 | 0.46 | 0.51 | 0.46 | 0.40 | 0.48 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.025 | 0.039 | 0.025 | 0.029 | 0.042 | 0.029 | 0.046 | 0.051 | 0.046 |
| p | 0.015 | 0.47 | 0.017 | 0.81 | 0.35 | 0.83 | 0.40 | 0.041 | 0.67 |
| nCohort 1 | 430 | 1121 | 506 | 430 | 1121 | 506 | 430 | 1121 | 506 |
| nCohort 2 | 195 | 58 | 182 | 132 | 51 | 129 | 46 | 36 | 43 |
| Cutoff 1 | 843 | 999 | 891 | 655 | 914 | 671 | 525 | 589 | 575 |
| Sens 1 | 70% | 71% | 70% | 70% | 71% | 71% | 72% | 72% | 72% |
| Spec 1 | 37% | 38% | 37% | 29% | 35% | 27% | 23% | 23% | 22% |
| Cutoff 2 | 557 | 546 | 619 | 489 | 435 | 502 | 450 | 409 | 450 |
| Sens 2 | 80% | 81% | 80% | 80% | 80% | 81% | 80% | 81% | 81% |
| Spec 2 | 23% | 21% | 25% | 21% | 16% | 20% | 20% | 16% | 19% |
| Cutoff 3 | 314 | 217 | 348 | 362 | 171 | 401 | 275 | 272 | 277 |
| Sens 3 | 90% | 91% | 90% | 90% | 92% | 91% | 91% | 92% | 91% |
| Spec 3 | 13% | 6% | 14% | 16% | 5% | 18% | 12% | 8% | 11% |
| Cutoff 4 | 1870 | 2320 | 2010 | 1870 | 2320 | 2010 | 1870 | 2320 | 2010 |
| Sens 4 | 42% | 33% | 40% | 34% | 16% | 33% | 28% | 17% | 33% |
| Spec 4 | 70% | 70% | 70% | 70% | 70% | 70% | 70% | 70% | 70% |
| Cutoff 5 | 2630 | 3200 | 2780 | 2630 | 3200 | 2780 | 2630 | 3200 | 2780 |
| Sens 5 | 28% | 24% | 29% | 23% | 14% | 24% | 17% | 14% | 19% |
| Spec 5 | 80% | 80% | 80% | 80% | 80% | 80% | 80% | 80% | 80% |
| Cutoff 6 | 4290 | 5100 | 4310 | 4290 | 5100 | 4310 | 4290 | 5100 | 4310 |
| Sens 6 | 15% | 9% | 15% | 12% | 6% | 15% | 9% | 6% | 12% |
| Spec 6 | 90% | 90% | 90% | 90% | 90% | 90% | 90% | 90% | 90% |
| OR Quart 2 p Value 95% CI ot OR Quart2 | 0.94 | 1.1 | 1.2 | 0.85 | 1.8 | 1.1 | 0.89 | 0.83 | 0.62 |
| | 0.80 | 0.84 | 0.38 | 0.55 | 0.20 | 0.70 | 0.81 | 0.77 | 0.34 |
| | 0.57 | 0.49 | 0.76 | 0.49 | 0.74 | 0.65 | 0.35 | 0.25 | 0.23 |
| | 1.5 | 2.4 | 2.1 | 1.5 | 4.3 | 1.9 | 2.3 | 2.8 | 1.7 |
| OR Quart 3 p Value 95% CI of OR Quart3 | 1.2 | 1.3 | 1.1 | 0.85 | 2.3 | 0.84 | 1.2 | 2.1 | 1.1 |
| | 0.39 | 0.45 | 0.70 | 0.57 | 0.051 | 0.55 | 0.65 | 0.16 | 0.81 |
| | 0.76 | 0.63 | 0.67 | 0.49 | 1.00 | 0.48 | 0.51 | 0.76 | 0.47 |
| | 2.0 | 2.9 | 1.8 | 1.5 | 5.5 | 1.5 | 2.9 | 5.5 | 2.6 |
| OR Quart 4 p Value 95% CI of OR Quart4 | 1.7 | 1.4 | 1.8 | 0.99 | 1.4 | 1.1 | 1.6 | 2.2 | 1.2 |
| | 0.035 | 0.35 | 0.017 | 0.97 | 0.49 | 0.80 | 0.29 | 0.11 | 0.66 |
| | 1.0 | 0.67 | 1.1 | 0.58 | 0.55 | 0.62 | 0.68 | 0.84 | 0.52 |
| | 2.7 | 3.1 | 2.9 | 1.7 | 3.5 | 1.8 | 3.7 | 5.9 | 2.8 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.36 | 10.6 | 9.36 | 10.9 | 9.36 | 16.5 |
| Average | 14.9 | 17.8 | 14.9 | 99.7 | 14.9 | 24.7 |
| Stdev | 34.0 | 22.7 | 34.0 | 743 | 34.0 | 53.5 |
| p(t-test) | | 0.37 | | 0.063 | | 0.14 |
| Min | 0.00398 | 0.00398 | 0.00398 | 0.00398 | 0.00398 | 0.0184 |
| Max | 470 | 134 | 470 | 7540 | 470 | 317 |
| n (Samp) | 268 | 138 | 268 | 104 | 268 | 35 |
| n (Patient) | 148 | 138 | 148 | 104 | 148 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.7 | 6.88 | 10.7 | 11.2 | 10.7 | 4.09 |
| Average | 29.4 | 11.0 | 29.4 | 40.6 | 29.4 | 11.8 |
| Stdev | 295 | 12.7 | 295 | 154 | 295 | 13.6 |
| p(t-test) | | 0.70 | | 0.82 | | 0.77 |
| Min | 0.00398 | 0.0132 | 0.00398 | 0.00398 | 0.00398 | 0.0184 |
| Max | 7540 | 50.9 | 7540 | 945 | 7540 | 44.4 |
| n (Samp) | 663 | 38 | 663 | 37 | 663 | 23 |
| n (Patient) | 288 | 38 | 288 | 37 | 288 | 23 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 9.36 | 10.8 | 9.36 | 9.71 | 9.36 | 16.8 |
| Average | 17.9 | 19.0 | 17.9 | 93.1 | 17.9 | 31.5 |
| Stdev | 61.5 | 24.0 | 61.5 | 749 | 61.5 | 60.0 |
| p(t-test) | | 0.85 | | 0.079 | | 0.24 |
| Min | 0.00398 | 0.00398 | 0.00398 | 0.00398 | 0.00398 | 0.0184 |
| Max | 945 | 134 | 945 | 7540 | 945 | 317 |
| n (Samp) | 313 | 127 | 313 | 101 | 313 | 32 |
| n (Patient) | 152 | 127 | 152 | 101 | 152 | 32 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.47 | 0.55 | 0.56 | 0.55 | 0.54 | 0.62 | 0.47 | 0.62 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.030 | 0.049 | 0.031 | 0.034 | 0.050 | 0.033 | 0.053 | 0.062 | 0.055 |
| p | 0.13 | 0.49 | 0.14 | 0.068 | 0.36 | 0.19 | 0.027 | 0.67 | 0.032 |
| nCohort 1 | 268 | 663 | 313 | 268 | 663 | 313 | 268 | 663 | 313 |
| nCohort 2 | 138 | 38 | 127 | 104 | 37 | 101 | 35 | 23 | 32 |
| Cutoff 1 | 0.533 | 0.338 | 0.338 | 1.39 | 0.338 | 0.338 | 0.452 | 0.178 | 3.21 |
| Sens 1 | 70% | 71% | 73% | 70% | 76% | 71% | 71% | 78% | 72% |
| Spec 1 | 32% | 28% | 29% | 35% | 28% | 29% | 31% | 26% | 37% |
| Cutoff 2 | 0.0840 | 0.117 | 0.0748 | 0.103 | 0.315 | 0.0840 | 0.338 | 0.167 | 0.315 |
| Sens 2 | 80% | 82% | 80% | 83% | 84% | 80% | 80% | 83% | 84% |
| Spec 2 | 22% | 23% | 19% | 24% | 26% | 22% | 29% | 24% | 28% |
| Cutoff 3 | 0.0184 | 0.0187 | 0.0179 | 0.0187 | 0.177 | 0.0187 | 0.103 | 0.0179 | 0.0840 |
| Sens 3 | 91% | 97% | 91% | 91% | 92% | 91% | 91% | 100% | 91% |
| Spec 3 | 7% | 11% | 7% | 9% | 24% | 10% | 24% | 9% | 22% |
| Cutoff 4 | 15.6 | 18.2 | 16.5 | 15.6 | 18.2 | 16.5 | 15.6 | 18.2 | 16.5 |
| Sens 4 | 40% | 21% | 39% | 42% | 46% | 42% | 51% | 35% | 50% |
| Spec 4 | 70% | 70% | 71% | 70% | 70% | 71% | 70% | 70% | 71% |
| Cutoff 5 | 20.7 | 25.9 | 21.9 | 20.7 | 25.9 | 21.9 | 20.7 | 25.9 | 21.9 |
| Sens 5 | 31% | 11% | 32% | 31% | 27% | 30% | 34% | 17% | 28% |
| Spec 5 | 80% | 80% | 80% | 80% | 80% | 80% | 80% | 80% | 80% |
| Cutoff 6 | 30.7 | 39.8 | 31.2 | 30.7 | 39.8 | 31.2 | 30.7 | 39.8 | 31.2 |
| Sens 6 | 22% | 5% | 24% | 18% | 11% | 21% | 14% | 4% | 22% |
| Spec 6 | 90% | 90% | 90% | 90% | 90% | 90% | 90% | 90% | 90% |
| OR Quart 2 p Value 95% CI of OR Quart2 | 0.86 | 1.2 | 1.0 | 1.7 | 2.7 | 1.2 | 3.2 | 0.83 | 1.2 |
| | 0.62 | 0.78 | 1.0 | 0.100 | 0.062 | 0.65 | 0.089 | 0.77 | 0.76 |
| | 0.47 | 0.41 | 0.55 | 0.90 | 0.95 | 0.61 | 0.84 | 0.25 | 0.36 |
| | 1.6 | 3.3 | 1.8 | 3.4 | 7.8 | 2.2 | 12 | 2.8 | 4.1 |
| OR Quart 3 p Value 95% CI ot OR Quart3 | 0.79 | 1.9 | 0.95 | 0.76 | 1.4 | 0.74 | 2.8 | 1.2 | 1.9 |
| | 0.44 | 0.17 | 0.88 | 0.46 | 0.56 | 0.38 | 0.14 | 0.78 | 0.27 |
| | 0.43 | 0.75 | 0.52 | 0.37 | 0.44 | 0.37 | 0.72 | 0.39 | 0.61 |
| | 1.4 | 5.0 | 1.7 | 1.6 | 4.6 | 1.5 | 11 | 3.6 | 5.9 |
| OR Quart 4 p Value 95% CI of OR Quart4 | 1.8 | 1.5 | 1.7 | 2.5 | 2.5 | 1.7 | 5.9 | 0.83 | 2.6 |
| | 0.052 | 0.45 | 0.084 | 0.0049 | 0.091 | 0.097 | 0.0068 | 0.77 | 0.087 |
| | 1.00 | 0.54 | 0.94 | 1.3 | 0.86 | 0.91 | 1.6 | 0.25 | 0.87 |
| | 3.1 | 3.9 | 2.9 | 4.8 | 7.3 | 3.1 | 21 | 2.8 | 7.7 |

Fig. 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5340 | 10900 | 5340 | 9120 | 5340 | 5200 |
| Average | 8920 | 12500 | 8920 | 11000 | 8920 | 9060 |
| Stdev | 8290 | 8910 | 8290 | 8520 | 8290 | 8710 |
| p(t-test) | | 6.5E-5 | | 0.018 | | 0.90 |
| Min | 0.00873 | 75.9 | 0.00873 | 303 | 0.00873 | 54.6 |
| Max | 30600 | 30600 | 30600 | 30600 | 30600 | 30600 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6050 | 12100 | 6050 | 14400 | 6050 | 11200 |
| Average | 9720 | 12000 | 9720 | 13300 | 9720 | 11700 |
| Stdev | 8640 | 8770 | 8640 | 9680 | 8640 | 8390 |
| p(t-test) | | 0.24 | | 0.035 | | 0.25 |
| Min | 0.00873 | 110 | 0.00873 | 772 | 0.00873 | 174 |
| Max | 30600 | 27800 | 30600 | 30600 | 30600 | 30600 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5710 | 10900 | 5710 | 8180 | 5710 | 4400 |
| Average | 9470 | 12500 | 9470 | 10200 | 9470 | 8210 |
| Stdev | 8660 | 9100 | 8660 | 7920 | 8660 | 8360 |
| p(t-test) | | 0.0017 | | 0.46 | | 0.32 |
| Min | 0.00873 | 75.9 | 0.00873 | 303 | 0.00873 | 54.6 |
| Max | 30600 | 30600 | 30600 | 30600 | 30600 | 27300 |
| n (Samp) | 962 | 91 | 962 | 94 | 962 | 49 |
| n (Patient) | 367 | 91 | 367 | 94 | 367 | 49 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.62 | 0.57 | 0.60 | 0.59 | 0.60 | 0.55 | 0.50 | 0.58 | 0.45 |
| SE | 0.031 | 0.067 | 0.033 | 0.031 | 0.059 | 0.032 | 0.040 | 0.060 | 0.043 |
| p | 9.1E-5 | 0.28 | 0.0015 | 0.0051 | 0.078 | 0.12 | 0.95 | 0.19 | 0.28 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 | 5290 | 4780 | 4920 | 4180 | 4300 | 4180 | 2200 | 6020 | 2120 |
| Sens 1 | 71% | 70% | 70% | 71% | 73% | 70% | 70% | 72% | 71% |
| Spec 1 | 49% | 43% | 46% | 43% | 40% | 42% | 24% | 50% | 22% |
| Cutoff 2 | 3820 | 3870 | 4020 | 2940 | 3510 | 2820 | 1690 | 3240 | 1610 |
| Sens 2 | 81% | 80% | 80% | 80% | 81% | 81% | 81% | 80% | 82% |
| Spec 2 | 39% | 36% | 39% | 31% | 33% | 28% | 18% | 31% | 16% |
| Cutoff 3 | 1110 | 1110 | 1440 | 2230 | 1440 | 2000 | 989 | 1680 | 989 |
| Sens 3 | 91% | 90% | 90% | 90% | 92% | 90% | 91% | 92% | 92% |
| Spec 3 | 12% | 10% | 15% | 24% | 13% | 21% | 10% | 16% | 9% |
| Cutoff 4 | 12000 | 14500 | 14000 | 12000 | 14500 | 14000 | 12000 | 14500 | 14000 |
| Sens 4 | 47% | 45% | 42% | 38% | 50% | 28% | 32% | 40% | 24% |
| Spec 4 | 70% | 70% | 70% | 70% | 70% | 70% | 70% | 70% | 70% |
| Cutoff 5 | 18100 | 19700 | 19100 | 18100 | 19700 | 19100 | 18100 | 19700 | 19100 |
| Sens 5 | 36% | 25% | 36% | 23% | 35% | 16% | 21% | 28% | 20% |
| Spec 5 | 80% | 80% | 80% | 80% | 80% | 80% | 80% | 80% | 80% |
| Cutoff 6 | 20400 | 22100 | 22200 | 20400 | 22100 | 22200 | 20400 | 22100 | 22200 |
| Sens 6 | 17% | 10% | 13% | 11% | 15% | 7% | 11% | 8% | 8% |
| Spec 6 | 91% | 90% | 90% | 91% | 90% | 90% | 91% | 90% | 90% |
| OR Quart2 p Value 95% CI of OR Quart2 | 1.1 | 0.75 | 1.2 | 2.3 | 1.00 | 1.5 | 0.86 | 1.00 | 0.72 |
|  | 0.87 | 0.70 | 0.59 | 0.017 | 1.00 | 0.25 | 0.70 | 1.00 | 0.48 |
|  | 0.52 | 0.17 | 0.60 | 1.2 | 0.29 | 0.76 | 0.40 | 0.25 | 0.28 |
|  | 2.2 | 3.4 | 2.5 | 4.6 | 3.5 | 2.9 | 1.8 | 4.0 | 1.8 |
| OR Quart3 p Value 95% CI of OR Quart3 | 1.8 | 1.3 | 1.7 | 2.4 | 1.2 | 2.1 | 0.72 | 2.5 | 1.2 |
|  | 0.082 | 0.74 | 0.14 | 0.012 | 0.77 | 0.017 | 0.42 | 0.12 | 0.68 |
|  | 0.93 | 0.33 | 0.85 | 1.2 | 0.36 | 1.1 | 0.32 | 0.79 | 0.52 |
|  | 3.4 | 4.7 | 3.2 | 4.7 | 4.0 | 4.0 | 1.6 | 8.2 | 2.7 |
| OR Quart4 p Value 95% CI of OR Quart4 | 2.7 | 2.0 | 2.4 | 2.7 | 2.0 | 1.5 | 1.2 | 1.8 | 1.6 |
|  | 0.0015 | 0.26 | 0.0057 | 0.0042 | 0.20 | 0.25 | 0.59 | 0.37 | 0.24 |
|  | 1.5 | 0.60 | 1.3 | 1.4 | 0.68 | 0.76 | 0.60 | 0.51 | 0.73 |
|  | 5.0 | 6.8 | 4.6 | 5.2 | 6.0 | 2.9 | 2.5 | 6.1 | 3.5 |

Angiopoietin-related protein 4

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11.6 | 14.5 | 11.6 | 14.1 | 11.6 | 9.57 |
| Average | 41.8 | 50.7 | 41.8 | 76.3 | 41.8 | 55.9 |
| Stdev | 94.7 | 102 | 94.7 | 179 | 94.7 | 149 |
| p(t-test) | | 0.43 | | 0.0045 | | 0.36 |
| Min | 0.000466 | 1.68 | 0.000466 | 1.61 | 0.000466 | 0.794 |
| Max | 789 | 647 | 789 | 1370 | 789 | 878 |
| n (Samp) | 754 | 78 | 754 | 86 | 754 | 42 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.6 | 27.8 | 112.6 | 18.2 | 12.6 | 13.5 |
| Average | 45.4 | 116 | 45.4 | 86.3 | 45.4 | 103 |
| Stdev | 107 | 182 | 107 | 117 | 107 | 218 |
| p(t-test) | | 0.0067 | | 0.072 | | 0.028 |
| Min | 0.000466 | 3.69 | 0.000466 | 2.98 | 0.000466 | 2.01 |
| Max | 1370 | 647 | 1370 | 413 | 1370 | 878 |
| n (Samp) | 1164 | 18 | 1164 | 23 | 1164 | 18 |
| n (Patient) | 379 | 18 | 379 | 23 | 379 | 18 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.0 | 13.8 | 12.0 | 13.4 | 12.0 | 9.31 |
| Average | 48.1 | 35.6 | 48.1 | 70.6 | 48.1 | 38.0 |
| Stdev | 104 | 51.2 | 104 | 182 | 104 | 76.2 |
| p(t-test) | | 0.32 | | 0.089 | | 0.57 |
| Min | 0.000466 | 1.68 | 0.000466 | 0.000466 | 0.000466 | 0.794 |
| Max | 878 | 301 | 878 | 1370 | 878 | 400 |
| n (Samp) | 838 | 70 | 838 | 80 | 838 | 36 |
| n (Patient) | 306 | 70 | 306 | 80 | 306 | 36 |

| | 0hr prior to AKI stage | | | 24hr prior to AKI stage 48hr prior to AKI stage | | | | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.67 | 0.53 | 0.56 | 0.65 | 0.52 | 0.50 | 0.54 | 0.47 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.035 | 0.071 | 0.036 | 0.034 | 0.063 | 0.034 | 0.046 | 0.070 | 0.050 |
| p | 0.048 | 0.018 | 0.39 | 0.078 | 0.019 | 0.51 | 0.92 | 0.60 | 0.50 |
| nCohort 1 | 754 | 1164 | 838 | 754 | 1164 | 838 | 754 | 1164 | 838 |
| nCohort 2 | 78 | 18 | 70 | 86 | 23 | 80 | 42 | 18 | 36 |
| Cutoff 1 | 8.30 | 14.8 | 8.19 | 7.57 | 14.0 | 7.57 | 7.08 | 7.57 | 6.86 |
| Sens 1 | 71% | 72% | 70% | 71% | 74% | 70% | 71% | 72% | 72% |
| Spec 1 | 38% | 55% | 36% | 35% | 53% | 34% | 32% | 31% | 31% |
| Cutoff 2 | 6.34 | 7.15 | 5.86 | 5.80 | 10.8 | 5.34 | 5.18 | 5.18 | 4.93 |
| Sens 2 | 81% | 83% | 80% | 80% | 83% | 80% | 81% | 83% | 81% |
| Spec 2 | 29% | 29% | 25% | 26% | 43% | 23% | 24% | 21% | 21% |
| Cutoff 3 | 3.72 | 4.54 | 3.66 | 3.39 | 6.96 | 3.28 | 3.66 | 2.62 | 3.49 |
| Sens 3 | 91% | 94% | 90% | 91% | 91% | 90% | 90% | 94% | 92% |
| Spec 3 | 15% | 18% | 14% | 14% | 28% | 12% | 15% | 8% | 13% |
| Cutoff 4 | 22.1 | 24.4 | 23.7 | 22.1 | 24.4 | 23.7 | 22.1 | 24.4 | 23.7 |
| Sens 4 | 38% | 56% | 33% | 35% | 39% | 32% | 26% | 33% | 28% |
| Spec 4 | 70% | 70% | 70% | 70% | 70% | 70% | 70% | 70% | 70% |
| Cutoff 5 | 37.2 | 44.0 | 43.1 | 37.2 | 44.0 | 43.1 | 37.2 | 44.0 | 43.1 |
| Sens 5 | 31% | 39% | 27% | 30% | 39% | 26% | 21% | 28% | 22% |
| Spec 5 | 80% | 80% | 80% | 80% | 80% | 80% | 80% | 80% | 80% |
| Cutoff 6 | 93.7 | 99.5 | 133 | 93.7 | 99.5 | 133 | 93.7 | 99.5 | 133 |
| Sens 6 | 14% | 28% | 7% | 19% | 30% | 15% | 12% | 28% | 8% |
| Spec 6 | 90% | 90% | 90% | 90% | 90% | 90% | 90% | 90% | 90% |
| OR Quart 2 p Value 95% CI of OR Quart2 | 1.5 | 1.00 | 1.4 | 1.3 | 2.0 | 0.94 | 0.79 | 1.2 | 0.55 |
| | 0.29 | 1.00 | 0.37 | 0.50 | 0.42 | 0.85 | 0.63 | 0.74 | 0.29 |
| | 0.72 | 0.14 | 0.68 | 0.65 | 0.36 | 0.48 | 0.31 | 0.33 | 0.18 |
| | 3.0 | 7.1 | 2.8 | 2.5 | 11 | 1.8 | 2.0 | 4.7 | 1.7 |
| OR Quart 3 p Value 95% CI ot OR Quart3 | 1.4 | 3.6 | 1.1 | 1.3 | 4.1 | 1.1 | 1.3 | 0.75 | 1.4 |
| | 0.37 | 0.12 | 0.85 | 0.50 | 0.077 | 0.87 | 0.52 | 0.70 | 0.50 |
| | 0.68 | 0.73 | 0.51 | 0.65 | 0.86 | 0.55 | 0.57 | 0.17 | 0.56 |
| | 2.9 | 17 | 2.3 | 2.5 | 19 | 2.0 | 3.1 | 3.4 | 3.3 |
| OR Quart 4 p Value 95% CI of OR Quart4 | 1.9 | 3.5 | 1.6 | 1.7 | 4.6 | 1.2 | 1.1 | 1.5 | 1.1 |
| | 0.068 | 0.12 | 0.17 | 0.11 | 0.052 | 0.53 | 0.82 | 0.53 | 0.81 |
| | 0.95 | 0.73 | 0.81 | 0.88 | 0.98 | 0.65 | 0.46 | 0.42 | 0.45 |
| | 3.8 | 17 | 3.3 | 3.2 | 21 | 2.3 | 2.7 | 5.4 | 2.8 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.2 | 27.7 | 23.2 | 35.1 | 23.2 | 31.2 |
| Average | 54.1 | 48.4 | 54.1 | 167 | 54.1 | 73.3 |
| Stdev | 141 | 61.9 | 141 | 527 | 141 | 176 |
| p(t-test) | | 0.74 | | 2.8E-5 | | 0.42 |
| Min | 0.00401 | 1.75 | 0.00401 | 1.75 | 0.00401 | 0.00413 |
| Max | 1640 | 300 | 1640 | 3480 | 1640 | 1070 |
| n (Samp) | 597 | 69 | 597 | 76 | 597 | 38 |
| n (Patient) | 279 | 69 | 279 | 76 | 279 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.7 | 64.2 | 24.7 | 42.6 | 24.7 | 66.6 |
| Average | 59.4 | 91.9 | 59.4 | 165 | 59.4 | 94.2 |
| Stdev | 165 | 88.9 | 165 | 403 | 165 | 80.8 |
| p(t-test) | | 0.55 | | 0.012 | | 0.43 |
| Min | 0.00131 | 22.5 | 0.00131 | 6.70 | 0.00131 | 9.39 |
| Max | 2190 | 289 | 2190 | 1710 | 2190 | 246 |
| n (Samp) | 827 | 9 | 827 | 17 | 827 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.6 | 27.7 | 24.6 | 33.2 | 24.6 | 26.6 |
| Average | 51.6 | 44.1 | 51.6 | 150 | 51.6 | 121 |
| Stdev | 125 | 55.6 | 125 | 510 | 125 | 331 |
| p(t-test) | | 0.63 | | 1.2E-4 | | 0.0059 |
| Min | 0.00131 | 1.75 | 0.00131 | 1.75 | 0.00131 | 0.00413 |
| Max | 1640 | 300 | 1640 | 3480 | 1640 | 1710 |
| n (Samp) | 604 | 66 | 604 | 72 | 604 | 35 |
| n (Patient) | 263 | 66 | 263 | 72 | 263 | 35 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.76 | 0.51 | 0.60 | 0.69 | 0.58 | 0.54 | 0.74 | 0.53 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.037 | 0.093 | 0.038 | 0.036 | 0.072 | 0.037 | 0.049 | 0.077 | 0.051 |
| p | 0.24 | 0.0047 | 0.69 | 0.0080 | 0.0072 | 0.037 | 0.42 | 0.0014 | 0.60 |
| nCohort 1 | 597 | 827 | 604 | 597 | 827 | 604 | 597 | 827 | 604 |
| nCohort 2 | 69 | 9 | 66 | 76 | 17 | 72 | 38 | 14 | 35 |
| Cutoff 1 | 17.1 | 45.7 | 15.5 | 20.9 | 35.3 | 20.6 | 15.1 | 40.4 | 15.1 |
| Sens 1 | 71% | 78% | 71% | 71% | 71% | 71% | 71% | 71% | 71% |
| Spec 1 | 38 % | 71% | 33% | 46% | 63% | 44% | 34% | 67% | 32% |
| Cutoff 2 | 12.3 | 24.3 | 11.6 | 15.9 | 32.4 | 15.9 | 10.6 | 25.8 | 11.5 |
| Sens 2 | 81% | 89% | 80% | 80% | 82% | 81% | 82% | 86% | 80% |
| Spec 2 | 26% | 49% | 23% | 35% | 60% | 34% | 21% | 52% | 23% |
| Cutoff 3 | 6.31 | 22.3 | 6.31 | 4.81 | 14.6 | 4.81 | 5.07 | 25.0 | 5.07 |
| Sens 3 | 91% | 100% | 91% | 91% | 94% | 90% | 92% | 93% | 91% |
| Spec 3 | 12% | 46% | 12% | 9% | 31% | 9% | 9% | 51% | 9% |
| Cutoff 4 | 42.8 | 43.9 | 43.9 | 42.8 | 43.9 | 43.9 | 42.8 | 43.9 | 43.9 |
| Sens 4 | 32% | 78% | 29% | 39% | 47% | 36% | 42% | 57% | 37% |
| Spec 4 | 70% | 70% | 70% | 70% | 70% | 70% | 70% | 70% | 70% |
| Cutoff 5 | 53.8 | 57.2 | 55.2 | 53.8 | 57.2 | 55.2 | 53.8 | 57.2 | 55.2 |
| Sens 5 | 25% | 56% | 21% | 32% | 29% | 29% | 32% | 57% | 23% |
| Spec 5 | 80% | 80% | 80% | 80% | 80% | 80% | 80% | 80% | 80% |
| Cutoff 6 | 88.4 | 96.9 | 87.2 | 88.4 | 96.9 | 87.2 | 88.4 | 96.9 | 87.2 |
| Sens 6 | 13% | 22% | 12% | 18% | 24% | 17% | 13% | 36% | 14% |
| Spec 6 | 90% | 90% | 90% | 90% | 90% | 90% | 90% | 90% | 90% |
| OR Quart 2 p Value 95% CI of OR Quart2 | 1.1 | >2.0 | 1.1 | 1.1 | 1.0 | 1.1 | 0.99 | 0 | 0.88 |
| | 0.85 | <0.57 | 0.86 | 0.84 | 1.0 | 0.84 | 0.99 | na | 0.79 |
| | 0.49 | >0.18 | 0.50 | 0.49 | 0.062 | 0.49 | 0.38 | na | 0.33 |
| | 2.4 | na | 2.3 | 2.4 | 16 | 2.4 | 2.6 | na | 2.3 |
| OR Quart 3 p Value | 1.8 | >2.0 | 1.5 | 2.0 | 9.4 | 1.7 | 0.88 | 5.1 | 0.76 |
| | 0.11 | <0.57 | 0.28 | 0.059 | 0.035 | 0.15 | 0.79 | 0.14 | 0.60 |
| | 0.87 | >0.18 | 0.72 | 0.97 | 1.2 | 0.82 | 0.33 | 0.59 | 0.28 |
| 95% CI of OR Quart3 | 3.7 | na | 3.1 | 4.1 | 75 | 3.5 | 2.3 | 44 | 2.1 |
| OR Quart 4 p Value 95% CI of OR Quart4 | 1.6 | >5.1 | 1.2 | 2.1 | 6.1 | 2.0 | 1.4 | 8.2 | 1.2 |
| | 0.21 | <0.14 | 0.58 | 0.044 | 0.094 | 0.059 | 0.51 | 0.048 | 0.66 |
| | 0.77 | >0.59 | 0.59 | 1.0 | 0.73 | 0.97 | 0.55 | 1.0 | 0.50 |
| | 3.3 | na | 2.6 | 4.2 | 52 | 4.0 | 3.3 | 66 | 3.1 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.793 | 1.51 | 0.793 | 1.46 | 0.793 | 1.46 |
| Average | 1.42 | 1.43 | 1.42 | 1.47 | 1.42 | 1.31 |
| Stdev | 2.26 | 1.58 | 2.26 | 1.91 | 2.26 | 1.22 |
| p(t-test) | | 0.95 | | 0.85 | | 0.77 |
| Min | 0.00179 | 0.00179 | 0.00179 | 0.00179 | 0.00179 | 0.00179 |
| Max | 27.4 | 9.73 | 27.4 | 11.6 | 27.4 | 4.74 |
| n (Samp) | 597 | 69 | 597 | 76 | 597 | 38 |
| n (Patient) | 279 | 69 | 279 | 76 | 279 | 38 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 2.17 | 0.761 | 1.56 | 0.761 | 1.72 |
| Average | 1.40 | 2.29 | 1.40 | 1.31 | 1.40 | 1.71 |
| Stdev | 2.30 | 1.19 | 2.30 | 1.13 | 2.30 | 1.74 |
| p(t-test) | | 0.25 | | 0.87 | | 0.63 |
| Min | 0.00179 | 0.00342 | 0.00179 | 0.00352 | 0.00179 | 0.00246 |
| Max | 28.3 | 3.74 | 28.3 | 3.65 | 28.3 | 5.61 |
| n (Samp) | 827 | 9 | 827 | 17 | 827 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.793 | 1.11 | 0.793 | 1.46 | 0.793 | 1.43 |
| Average | 1.39 | 1.31 | 1.39 | 1.55 | 1.39 | 1.26 |
| Stdev | 2.08 | 1.57 | 2.08 | 1.98 | 2.08 | 1.24 |
| p(t-test) | | 0.76 | | 0.54 | | 0.71 |
| Min | 0.00179 | 0.00179 | 0.00179 | 0.00179 | 0.00179 | 0.00179 |
| Max | 27.4 | 9.73 | 27.4 | 11.6 | 27.4 | 4.74 |
| n (Samp) | 604 | 66 | 604 | 72 | 604 | 35 |
| n (Patient) | 263 | 66 | 263 | 72 | 263 | 35 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.52 | 0.73 | 0.50 | 0.51 | 0.55 | 0.52 | 0.53 | 0.55 | 0.52 |
| SE | 0.037 | 0.097 | 0.038 | 0.035 | 0.073 | 0.036 | 0.049 | 0.080 | 0.051 |
| p | 0.62 | 0.018 | 0.93 | 0.80 | 0.48 | 0.53 | 0.61 | 0.52 | 0.76 |
| nCohort 1 | 597 | 827 | 604 | 597 | 827 | 604 | 597 | 827 | 604 |
| nCohort 2 | 69 | 9 | 66 | 76 | 17 | 72 | 38 | 14 | 35 |
| Cutoff 1 | 0.0407 | 1.97 | 0.0305 | 0.0407 | 0.0522 | 0.0435 | 0.0742 | 0.0209 | 0.0522 |
| Sens 1 | 71% | 78% | 71% | 71% | 71% | 71% | 71% | 71% | 71% |
| Spec 1 | 27% | 73% | 25% | 27% | 33% | 31% | 33% | 25% | 32% |
| Cutoff 2 | 0.00332 | 1.10 | 0.00332 | 0.00342 | 0.0178 | 0.00342 | 0.00342 | 0.00332 | 0.00342 |
| Sens 2 | 83% | 89% | 82% | 86% | 82% | 85% | 87% | 86% | 86% |
| Spec 2 | 12% | 54% | 14% | 16% | 23% | 18% | 16% | 14% | 18% |
| Cutoff 3 | 0.00184 | 0.00332 | 0.00184 | 0.00246 | 0.00342 | 0.00246 | 0.00240 | 0.00240 | 0.00240 |
| Sens 3 | 94% | 100% | 94% | 93% | 100% | 93% | 95% | 100% | 94% |
| Spec 3 | 3% | 14% | 3% | 9% | 18% | 11% | 7% | 8% | 9% |
| Cutoff 4 | 1.87 | 1.87 | 1.87 | 1.87 | 1.87 | 1.87 | 1.87 | 1.87 | 1.87 |
| Sens 4 | 36% | 78% | 30% | 29% | 35% | 32% | 29% | 29% | 31% |
| Spec 4 | 71% | 71% | 71% | 71% | 71% | 71% | 71% | 71% | 71% |
| Cutoff 5 | 2.41 | 2.41 | 2.41 | 2.41 | 2.41 | 2.41 | 2.41 | 2.41 | 2.41 |
| Sens 5 | 26% | 44% | 23% | 21% | 18% | 24% | 21% | 29% | 20% |
| Spec 5 | 80% | 80% | 80% | 80% | 80% | 80% | 80% | 80% | 80% |
| Cutoff 6 | 3.36 | 3.36 | 3.36 | 3.36 | 3.36 | 3.36 | 3.36 | 3.36 | 3.36 |
| Sens 6 | 7% | 11% | 6% | 12% | 6% | 14% | 5% | 14% | 6% |
| Spec 6 | 91% | 91% | 90% | 91% | 91% | 90% | 91% | 91% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 0.55 | 0 | 0.83 | 0.69 | 0.50 | 1.0 | 0.99 | 0 | 0.99 |
| | 0.13 | na | 0.61 | 0.29 | 0.42 | 1.0 | 0.99 | na | 0.99 |
| | 0.25 | na | 0.41 | 0.34 | 0.090 | 0.49 | 0.36 | na | 0.36 |
| | 1.2 | na | 1.7 | 1.4 | 2.7 | 2.0 | 2.7 | na | 2.7 |
| OR Quart 3 p Value 95% CI of OR Quart3 | 0.88 | 3.0 | 0.60 | 1.1 | 1.8 | 1.1 | 1.8 | 1.0 | 1.4 |
| | 0.72 | 0.34 | 0.19 | 0.87 | 0.37 | 0.72 | 0.20 | 1.0 | 0.49 |
| | 0.44 | 0.31 | 0.28 | 0.56 | 0.51 | 0.57 | 0.74 | 0.29 | 0.55 |
| | 1.8 | 29 | 1.3 | 2.0 | 6.2 | 2.3 | 4.4 | 3.5 | 3.6 |
| OR Quart 4 p Value 95% CI of OR Quart4 | 1.2 | 5.1 | 1.0 | 0.83 | 1.0 | 1.1 | 0.99 | 0.79 | 0.99 |
| | 0.63 | 0.14 | 0.98 | 0.60 | 1.0 | 0.72 | .0.99 | .0.73 | 0.99 |
| | 0.61 | 0.59 | 0.51 | 0.43 | 0.25 | 0.57 | 0.36 | 0.21 | 0.36 |
| | 2.3 | 44 | 2.0 | 1.6 | 4.1 | 2.3 | 2.7 | 3.0 | 2.7 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5250 | 6510 | 5250 | 6350 | 5250 | 4000 |
| Average | 17300 | 18400 | 17300 | 18000 | 17300 | 12000 |
| Stdev | 31000 | 35100 | 31000 | 27900 | 31000 | 22800 |
| p(t-test) | | 0.74 | | 0.83 | | 0.20 |
| Min | 0.0130 | 444 | 0.0130 | 957 | 0.0130 | 261 |
| Max | 238000 | 227000 | 238000 | 148000 | 238000 | 149000 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5530 | 11500 | 5530 | 7760 | 5530 | 6440 |
| Average | 16600 | 39800 | 16600 | 22500 | 16600 | 22000 |
| Stdev | 28500 | 63800 | 28500 | 27900 | 28500 | 35100 |
| p(t-test) | 4.4E-4 | 4.4E-4 | | 0.30 | | 0.35 |
| Min | 0.0130 | 104 | 0.0130 | 930 | 0.0130 | 876 |
| Max | 238000 | 227000 | 238000 | 110000 | 238000 | 149000 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 6270 | 5760 | 6310 | 5760 | 4060 |
| Average | 18700 | 15800 | 18700 | 16800 | 18700 | 10300 |
| Stdev | 32100 | 28700 | 32100 | 28100 | 32100 | 14800 |
| p(t-test) | | 0.41 | | 0.57 | | 0.066 |
| Min | 0.0130 | 444 | 0.0130 | 957 | 0.0130 | 261 |
| Max | 238000 | 190000 | 238000 | 148000 | 238000 | 62900 |
| n (Samp) | 962 | 91 | 962 | 94 | 962 | 49 |
| n (Patient) | 367 | 91 | 367 | 94 | 367 | 49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.64 | 0.53 | 0.57 | 0.60 | 0.53 | 0.45 | 0.53 | 0.43 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.031 | 0.067 | 0.032 | 0.031 | 0.059 | 0.032 | 0.040 | 0.059 | 0.044 |
| p | 0.025 | 0.031 | 0.31 | 0.024 | 0.11 | 0.32 | 0.24 | 0.56 | 0.12 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 | 4740 | 6440 | 4710 | 4190 | 4220 | 4410 | 2670 | 3180 | 2670 |
| Sens 1 | 71% | 70% | 70% | 71% | 73% | 70% | 70% | 72% | 71% |
| Spec 1 | 47% | 56% | 44% | 43% | 40% | 42% | 27% | 29% | 25% |
| Cutoff 2 | 3990 | 4110 | 3970 | 3180 | 3340 | 3100 | 2090 | 2550 | 1890 |
| Sens 2 | 81% | 80% | 80% | 80% | 81% | 81% | 81% | 80% | 82% |
| Spec 2 | 41% | 39% | 38% | 32% | 31% | 29% | 19% | 22% | 15% |
| Cutoff 3 | 2150 | 3120 | 2150 | 2480 | 2420 | 2120 | 1290 | 1290 | 1290 |
| Sens 3 | 91% | 90% | 90% | 90% | 92% | 90% | 91% | 92% | 92% |
| Spec 3 | 19% | 29% | 18% | 24% | 21% | 17% | 9% | 8% | 7% |
| Cutoff 4 | 10700 | 11500 | 12800 | 10700 | 11500 | 12800 | 10700 | 11500 | 12800 |
| Sens 4 | 31% | 50% | 27% | 36% | 38% | 27% | 23% | 36% | 20% |
| Spec 4 | 70% | 70% | 70% | 70% | 70% | 70% | 70% | 70% | 70% |
| Cutoff 5 | 22400 | 22100 | 26200 | 22400 | 22100 | 26200 | 22400 | 22100 | 26200 |
| Sens 5 | 19% | 35% | 14% | 20% | 38% | 14% | 12% | 24% | 12% |
| Spec 5 | 80% | 80% | 80% | 80% | 80% | 80% | 80% | 80% | 80% |
| Cutoff 6 | 48800 | 45900 | 51900 | 48800 | 45900 | 51900 | 48800 | 45900 | 51900 |
| Sens 6 | 10% | 25% | 8% | 12% | 15% | 10% | 4% | 16% | 4% |
| Spec 6 | 90% | 90% | 90% | 90% | 90% | 90% | 90% | 90% | 90% |
| OR Quart 2 p Value 95% CI ot OR Quart2 | 2.6 | 2.0 | 2.0 | 3.4 | 2.4 | 2.8 | 1.4 | 0.83 | 1.4 |
| | 0.0074 | 0.42 | 0.038 | 6.2E-4 | 0.22 | 0.0035 | 0.40 | 0.76 | 0.48 |
| | 1.3 | 0.37 | 1.0 | 1.7 | 0.60 | 1.4 | 0.62 | 0.25 | 0.55 |
| | 5.2 | 11 | 4.0 | 6.9 | 9.2 | 5.6 | 3.3 | 2.7 | 3.5 |
| OR Quart 3 p Value 95% CI of OR Quart3 | 2.8 | 2.5 | 2.3 | 2.7 | 2.0 | 2.7 | 1.9 | 0.83 | 2.1 |
| | 0.0034 | 0.27 | 0.014 | 0.0061 | 0.33 | 0.0051 | 0.12 | 0.76 | 0.10 |
| | 1.4 | 0.49 | 1.2 | 1.3 | 0.50 | 1.3 | 0.84 | 0.25 | 0.87 |
| | 5.6 | 13 | 4.4 | 5.6 | 8.1 | 5.4 | 4.1 | 2.7 | 4.9 |
| OR Quart 4 p Value 95% CI of OR Quart4 | 2.4 | 4.6 | 1.5 | 2.8 | 3.4 | 1.8 | 1.5 | 1.5 | 1.8 |
| | 0.015 | 0.053 | 0.29 | 0.0044 | 0.065 | 0.11 | 0.31 | 0.44 | 0.19 |
| | 1.2 | 0.98 | 0.72 | 1.4 | 0.93 | 0.87 | 0.67 | 0.53 | 0.74 |
| | 4.8 | 21 | 3.0 | 5.8 | 12 | 3.8 | 3.5 | 4.3 | 4.4 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.20 | 4.21 | 3.20 | 5.03 | 3.20 | 2.79 |
| Average | 5.72 | 5.80 | 5.72 | 8.53 | 5.72 | 5.54 |
| Stdev | 13.2 | 5.24 | 13.2 | 12.4 | 13.2 | 8.89 |
| p(t-test) | | 0.96 | | 10.088 | | 0.94 |
| Min | 0.0298 | 0.235 | 0.0298 | 0.00189 | 0.0298 | 0.000104 |
| Max | 279 | 31.4 | 279 | 81.9 | 279 | 43.0 |
| n (Samp) | 581 | 67 | 581 | 72 | 581 | 35 |
| n (Patient) | 276 | 67 | 276 | 72 | 276 | 35 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.45 | 4.45 | 3.45 | 5.98 | 3.45 | 4.31 |
| Average | 6.39 | 6.33 | 6.39 | 8.93 | 6.39 | 5.53 |
| Stdev | 13.2 | 5.02 | 13.2 | 12.7 | 13.2 | 3.77 |
| p(t-test) | | 0.99 | | 0.43 | | 0.81 |
| Min | 0.000104 | 0.847 | 0.000104 | 1.11 | 0.000104 | 0.667 |
| Max | 279 | 16.5 | 279 | 57.1 | 279 | 15.2 |
| n (Samp) | 801 | 9 | 801 | 17 | 801 | 13 |
| n (Patient) | 349 | 9 | 349 | 17 | 349 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.35 | 3.67 | 3.35 | 4.63 | 3.35 | 2.79 |
| Average | 5.95 | 5.55 | 5.95 | 7.97 | 5.95 | 7.04 |
| Stdev | 13.4 | 5.21 | 13.4 | 11.4 | 13.4 | 12.7 |
| p(t-test) | | 0.81 | | 0.23 | | 0.65 |
| Min | 0.000201 | 0.235 | 0.000201 | 0.00189 | 0.000201 | 0.000104 |
| Max | 279 | 31.4 | 279 | 81.9 | 279 | 57.1 |
| n (Samp) | 585 | 64 | 585 | 68 | 585 | 33 |
| n (Patient) | 260 | 64 | 260 | 68 | 260 | 33 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.60 | 0.56 | 0.61 | 0.65 | 0.58 | 0.45 | 0.59 | 0.44 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| SE | 0.038 | 0.10 | 0.039 | 0.037 | 0.073 | 0.038 | 0.052 | 0.084 | 0.053 |
| p | 0.021 | 0.31 | 0.16 | 0.0029 | 0.041 | 0.026 | 0.29 | 0.27 | 0.28 |
| nCohort 1 | 581 | 801 | 585 | 581 | 801 | 585 | 581 | 801 | 585 |
| nCohort 2 | 67 | 9 | 64 | 72 | 17 | 68 | 35 | 13 | 33 |
| Cutoff 1 | 2.56 | 3.18 | 2.37 | 2.85 | 5.23 | 2.64 | 1.37 | 3.39 | 1.37 |
| Sens 1 | 70% | 78% | 70% | 71% | 71% | 71% | 71% | 77% | 73% |
| Spec 1 | 42% | 47% | 37% | 46% | 66% | 41% | 22% | 49% | 21% |
| Cutoff 2 | 2.17 | 3.15 | 1.88 | 1.86 | 2.56 | 1.80 | 0.806 | 2.95 | 0.806 |
| Sens 2 | 81% | 89% | 81% | 81% | 82% | 81% | 80% | 85% | 82% |
| Spec 2 | 37% | 47% | 31% | 32% | 39% | 29% | 12% | 44% | 11% |
| Cutoff 3 | 1.76 | 0.840 | 1.57 | 0.750 | 1.68 | 0.709 | 0.367 | 2.22 | 0.367 |
| Sens 3 | 91% | 100% | 91% | 90% | 94% | 91% | 91% | 92% | 91% |
| Spec 3 | 30% | 12% | 25% | 11% | 26% | 9% | 4% | 35% | 3% |
| Cutoff 4 | 5.47 | 6.00 | 5.71 | 5.47 | 6.00 | 5.71 | 5.47 | 6.00 | 5.71 |
| Sens 4 | 37% | 33% | 36% | 46% | 47% | 41% | 26% | 38% | 27% |
| Spec 4 | 70% | 70% | 70% | 70% | 70% | 70% | 70% | 70% | 70% |
| Cutoff 5 | 7.36 | 8.53 | 7.77 | 7.36 | 8.53 | 7.77 | 7.36 | 8.53 | 7.77 |
| Sens 5 | 24% | 33% | 22% | 33% | 29% | 32% | 23% | 15% | 21% |
| Spec 5 | 80% | 80% | 80% | 80% | 80% | 80% | 80% | 80% | 80% |
| Cutoff 6 | 12.1 | 13.6 | 12.5 | 12.1 | 13.6 | 12.5 | 12.1 | 13.6 | 12.5 |
| Sens 6 | 10% | 11% | 6% | 19% | 6% | 21% | 9% | 8% | 9% |
| Spec 6 | 90% | 90% | 90% | 90% | 90% | 90% | 90% | 90% | 90% |
| OR Quart 2 p Value 95% CI OR Quart2 | 3.9 | 2.0 | 3.3 | 1.0 | 3.0 | 0.84 | 0.65 | 3.0 | 0.43 |
|  | 0.0046 | 0.57 | 0.0082 | 1.0 | 0.34 | 0.67 | 0.43 | 0.34 | 0.17 |
|  | 1.5 | 0.18 | 1.4 | 0.42 | 0.31 | 0.36 | 0.23 | 0.31 | 0.13 |
|  | 9.9 | 22 | 8.0 | 2.4 | 29 | 1.9 | 1.9 | 29 | 1.4 |
| OR Quart 3 p Value 95% CI of OR Quart3 | 3.5 | 3.0 | 2.9 | 2.0 | 8.3 | 1.4 | 0.88 | 5.1 | 1.0 |
|  | 0.010 | 0.34 | 0.018 | 0.067 | 0.047 | 0.35 | 0.80 | 0.14 | 1.0 |
|  | 1.3 | 0.31 | 1.2 | 0.95 | 1.0 | 0.68 | 0.33 | 0.59 | 0.39 |
|  | 8.9 | 29 | 7.2 | 4.4 | 67 | 3.0 | 2.4 | 44 | 2.6 |
| OR Quart 4 p Value 95% CI OR Quart4 | 3.9 | 3.0 | 2.6 | 3.0 | 5.1 | 2.2 | 1.4 | 4.0 | 1.2 |
|  | 0.0046 | 0.34 | 0.041 | 0.0036 | 0.14 | 0.031 | 0.50 | 0.21 | 0.63 |
|  | 1.5 | 0.31 | 1.0 | 1.4 | 0.59 | 1.1 | 0.56 | 0.45 | 0.50 |
|  | 9.9 | 29 | 6.4 | 6.2 | 44 | 4.4 | 3.3 | 36 | 3.1 |

**Heparin-binding growth factor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 854 | 754 | 854 | 729 | 854 | 595 |
| Average | 1230 | 1000 | 1230 | 1140 | 1230 | 1060 |
| Stdev | 1510 | 922 | 1510 | 1170 | 1510 | 1420 |
| p(t-test) | | 0.14 | | 0.59 | | 0.41 |
| Min | 0.00328 | 28.3 | 0.00328 | 8.50 | 0.00328 | 13.2 |
| Max | 16700 | 5510 | 16700 | 6000 | 16700 | 7750 |
| n (Samp) | 895 | 99 | 895 | 101 | 895 | 57 |
| n (Patient) | 374 | 99 | 374 | 101 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 847 | 536 | 847 | 545 | 847 | 289 |
| Average | 1220 | 631 | 1220 | 851 | 1220 | 664 |
| Stdev | 1460 | 445 | 1460 | 753 | 1460 | 802 |
| p(t-test) | | 0.069 | | 0.20 | | 0.055 |
| Min | 0.00328 | 70.1 | 0.00328 | 90.5 | 0.00328 | 1.77 |
| Max | 16700 | 1870 | 16700 | 2920 | 16700 | 3380 |
| n (Samp) | 1357 | 20 | 1357 | 25 | 1357 | 25 |
| n (Patient) | 470 | 20 | 470 | 25 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 834 | 846 | 834 | 797 | 834 | 670 |
| Average | 1200 | 1040 | 1200 | 1170 | 1200 | 1140 |
| Stdev | 1490 | 941 | 1490 | 1190 | 1490 | 1470 |
| p(t-test) | | 0.32 | | 0.81 | | 0.78 |
| Min | 0.00328 | 28.3 | 0.00328 | 8.50 | 0.00328 | 13.2 |
| Max | 16700 | 5510 | 16700 | 6000 | 16700 | 7750 |
| n (Samp) | 961 | 91 | 961 | 94 | 961 | 49 |
| n (Patient) | 367 | 91 | 367 | 94 | 367 | 49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.37 | 0.49 | 0.49 | 0.43 | 0.50 | 0.43 | 0.33 | 0.47 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.031 | 0.067 | 0.032 | 0.030 | 0.060 | 0.031 | 0.041 | 0.060 | 0.043 |
| p | 0.35 | 0.055 | 0.79 | 0.75 | 0.24 | 0.98 | 0.10 | 0.0043 | 0.49 |
| nCohort 1 | 895 | 1357 | 961 | 895 | 1357 | 961 | 895 | 1357 | 961 |
| nCohort 2 | 99 | 20 | 91 | 101 | 25 | 94 | 57 | 25 | 49 |
| Cutoff 1 | 444 | 403 | 454 | 467 | 294 | 467 | 318 | 169 | 401 |
| Sens 1 | 71% | 70% | 70% | 70% | 72% | 70% | 70% | 72% | 71% |
| Spec 1 | 29% | 27% | 30% | 31% | 20% | 31% | 22% | 12% | 27% |
| Cutoff 2 | 249 | 242 | 280 | 359 | 247 | 359 | 192 | 77.7 | 287 |
| Sens 2 | 81% | 80% | 80% | 80% | 80% | 81% | 81% | 80% | 82% |
| Spec 2 | 17% | 17% | 19% | 24% | 17% | 24% | 13% | 6% | 19% |
| Cutoff 3 | 161 | 187 | 173 | 169 | 110 | 152 | 61.5 | 34.6 | 77.7 |
| Sens 3 | 91% | 90% | 90% | 90% | 92% | 90% | 91% | 92% | 92% |
| Spec 3 | 11% | 13% | 12% | 12% | 8% | 11% | 6% | 3% | 6% |
| Cutoff 4 | 1390 | 1400 | 1340 | 1390 | 1400 | 1340 | 1390 | 1400 | 1340 |
| Sens 4 | 26% | 5% | 29% | 27% | 20% | 27% | 25% | 20% | 27% |
| Spec 4 | 70% | 70% | 70% | 70% | 70% | 70% | 70% | 70% | 70% |
| Cutoff 5 | 1750 | 1780 | 1720 | 1750 | 1780 | 1720 | 1750 | 1780 | 1720 |
| Sens 5 | 16% | 5% | 16% | 21% | 12% | 21% | 12% | 8% | 14% |
| Spec 5 | 80% | 80% | 80% | 80% | 80% | 80% | 80% | 80% | 80% |
| Cutoff 6 | 2490 | 2520 | 2450 | 2490 | 2520 | 2450 | 2490 | 2520 | 2450 |
| Sens 6 | 6% | 0% | 8% | 11% | 4% | 12% | 9% | 4% | 8% |
| Spec 6 | 90% | 90% | 90% | 90% | 90% | 90% | 90% | 90% | 90% |
| OR Quart 2 p Value 95% CI of OR Quart2 | 1.4 | 5.1 | 1.5 | 1.0 | 1.0 | 1.4 | 1.3 | 2.5 | 1.1 |
| | 0.27 | 0.14 | 0.17 | 1.0 | 1.00 | 0.29 | 0.52 | 0.27 | 0.82 |
| | 0.76 | 0.59 | 0.83 | 0.55 | 0.29 | 0.76 | 0.57 | 0.49 | 0.48 |
| | 2.6 | 44 | 2.8 | 1.8 | 3.5 | 2.5 | 3.1 | 13 | 2.5 |
| OR Quart 3 | 1.5 | 8.2 | 1.1 | 1.3 | 1.4 | 1.3 | 1.4 | 2.5 | 1.1 |
| | 0.17 | 0.048 | 0.87 | 0.32 | 0.56 | 0.44 | 0.40 | 0.27 | 0.83 |
| p Value 95% CI of OR Quart3 | 0.83 | 1.0 | 0.55 | 0.76 | 0.44 | 0.69 | 0.62 | 0.49 | 0.47 |
| | 2.8 | 66 | 2.0 | 2.3 | 4.5 | 2.3 | 3.3 | 13 | 2.5 |
| OR Quart 4 p Value 95% CI of OR Quart4 | 1.4 | 6.1 | 1.3 | 0.91 | 1.6 | 1.1 | 2.1 | 6.7 | 1.3 |
| | 0.27 | 0.095 | 0.43 | 0.76 | 0.40 | 0.76 | 0.064 | 0.012 | 0.53 |
| | 0.76 | 0.73 | 0.69 | 0.50 | 0.52 | 0.59 | 0.96 | 1.5 | 0.58 |
| | 2.6 | 51 | 2.4 | 1.7 | 5.0 | 2.1 | 4.6 | 30 | 2.9 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 2.75E-5 | 2.96E-5 | 3.44E-5 | 2.96E-5 | 2.58E-5 |
| Average | 0.00393 | 0.0150 | 0.00393 | 0.0190 | 0.00393 | 0.00519 |
| Stdev | 0.0267 | 0.116 | 0.0267 | 0.0885 | 0.0267 | 0.0298 |
| p(t-test) | | 0.033 | 4.9E-4 | 4.9E-4 | | 0.77 |
| Min | 5.62E-6 | 5.62E-6 | 5.62E-6 | 5.62E-6 | 5.62E-6 | 5.62E-6 |
| Max | 0.476 | 1.02 | 0.476 | 0.560 | 0.476 | 0.193 |
| n (Samp) | 754 | 78 | 754 | 86 | 754 | 42 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96E-5 | 3.28E-5 | 2.96E-5 | 2.63E-5 | 2.96E-5 | 2.66E-5 |
| Average | 0.00514 | 0.0622 | 0.00514 | 0.0258 | 0.00514 | 0.000233 |
| Stdev | 0.0328 | 0.240 | 0.0328 | 0.116 | 0.0328 | 0.000850 |
| p(t-test) | | 3.9E-8 | | 0.0068 | | 0.53 |
| Min | 5.62E-6 | 7.53E-6 | 5.62E-6 | 5.62E-6 | 5.62E-6 | 5.62E-6 |
| Max | 0.476 | 1.02 | 0.476 | 0.560 | 0.476 | 0.00364 |
| n (Samp) | 1164 | 18 | 1164 | 23 | 1164 | 18 |
| n (Patient) | 379 | 18 | 379 | 23 | 379 | 18 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.68E-5 | 2.83E-5 | 3.44E-5 | 2.83E-5 | 2.58E-5 |
| Average | 0.00941 | 0.0153 | 0.00941 | 0.0138 | 0.00941 | 0.00595 |
| Stdev | 0.0925 | 0.122 | 0.0925 | 0.0684 | 0.0925 | 0.0321 |
| p(t-test) | | 0.62 | | 0.68 | | 0.82 |
| Min | 5.62E-6 | 5.62E-6 | 5.62E-6 | 5.62E-6 | 5.62E-6 | 5.62E-6 |
| Max | 2.28 | 1.02 | 2.28 | 0.470 | 2.28 | 0.193 |
| n (Samp) | 838 | 70 | 838 | 80 | 838 | 36 |
| n (Patient) | 306 | 70 | 306 | 180 | 306 | 36 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or | sCr only | UO only | sCr or UO | sCr only | UO only |
| AUC | 0.48 | 0.54 | 0.47 | 0.55 | 0.50 | 0.57 | 0.43 | 0.41 | 0.46 |
| SE | 0.035 | 0.070 | 0.036 | 0.034 | 0.061 | 0.035 | 0.047 | 0.071 | 0.050 |
| p | 0.53 | 0.54 | 0.41 | 0.12 | 0.98 | 0.055 | 0.12 | 0.22 | 0.39 |
| nCohort 1 | 754 | 1164 | 838 | 754 | 1164 | 838 | 754 | 1164 | 838 |
| nCohort 2 | 78 | 18 | 70 | 86 | 23 | 80 | 42 | 18 | 36 |
| Cutoff 1 | 1.92E-5 | 1.88E-5 | 2.17E-5 | 2.46E-5 | 1.88E-5 | 2.46E-5 | 1.92E-5 | 2.17E-5 | 1.92E-5 |
| Sens 1 | 74% | 78% | 70% | 73% | 78% | 72% | 76% | 72% | 75% |
| Spec 1 | 23% | 20% | 28% | 34% | 20% | 35% | 23% | 28% | 24% |
| Cutoff 2 | 1.82E-5 | 1.82E-5 | 1.88E-5 | 1.92E-5 | 1.82E-5 | 1.92E-5 | 1.82E-5 | 1.92E-5 | 1.82E-5 |
| Sens 2 | 86% | 89% | 80% | 81% | 87% | 81% | 86% | 83% | 83% |
| Spec 2 | 15% | 17% | 20% | 23% | 17% | 24% | 15% | 24% | 16% |
| Cutoff 3 | 1.34E-5 | 7.53E-6 | 1.34E-5 | 1.82E-5 | 1.66E-5 | 1.82E-5 | 1.34E-5 | 1.82E-5 | 1.34E-5 |
| Sens 3 | 94% | 94% | 93% | 91% | 91% | 90% | 95% | 94% | 94% |
| Spec 3 | 9% | 5% | 9% | 15% | 13% | 16% | 9% | 17% | 9% |
| Cutoff 4 | 8.63E-5 | 4.39E-5 | 4.39E-5 | 8.63E-5 | 4.39E-5 | 4.39E-5 | 8.63E-5 | 4.39E-5 | 4.39E-5 |
| Sens 4 | 22% | 39% | 21% | 35% | 35% | 41% | 14% | 11% | 22% |
| Spec 4 | 73% | 72% | 70% | 73% | 72% | 70% | 73% | 72% | 70% |
| Cutoff 5 | 0.000168 | 0.000114 | 0.000114 | 0.000168 | 0.000114 | 0.000114 | 0.000168 | 0.000114 | 0.000114 |
| Sens 5 | 15% | 33% | 16% | 27% | 26% | 31% | 12% | 11% | 14% |
| Spec 5 | 84% | 80% | 80% | 84% | 80% | 80% | 84% | 80% | 80% |
| Cutoff 6 | 0.00325 | 0.00364 | 0.00364 | 0.00325 | 0.00364 | 0.00364 | 0.00325 | 0.00364 | 0.00364 |
| Sens 6 | 13% | 28% | 9% | 14% | 17% | 11% | 10% | 0% | 8% |
| Spec 6 | 90% | 90% | 90% | 90% | 90% | 90% | 90% | 90% | 90% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or | sCr only | UO only | sCr or UO | sCr only | UO only |
| OR Quart 2 p Value 95% CI of OR Quart2 | 1.1 | 0.49 | 1.6 | 1.1 | 0.28 | 1.1 | 1.3 | 1.5 | 1.7 |
| | 0.86 | 0.32 | 0.20 | 0.74 | 0.12 | 0.73 | 0.59 | 0.65 | 0.31 |
| | 0.53 | 0.12 | 0.78 | 0.58 | 0.058 | 0.56 | 0.46 | 0.25 | 0.61 |
| | 2.1 | 2.0 | 3.5 | 2.1 | 1.4 | 2.3 | 4.0 | 9.1 | 4.8 |
| OR Quart 3 p Value 95% CI of OR Quart3 | 1.4 | 0.33 | 1.8 | 1.1 | 1.0 | 1.1 | 2.6 | 5.1 | 1.9 |
| | 0.32 | 0.18 | 0.11 | 0.87 | 1.0 | 0.86 | 0.051 | 0.036 | 0.22 |
| | 0.72 | 0.066 | 0.88 | 0.55 | 0.35 | 0.53 | 1.00 | 1.1 | 0.68 |
| | 2.7 | 1.6 | 3.8 | 2.0 | 2.9 | 2.2 | 6.9 | 24 | 5.2 |
| OR Quart 4 p Value | 1.2 | 1.2 | 1.5 | 1.4 | 1.0 | 1.9 | 2.2 | 1.5 | 1.5 |
| | 0.61 | 0.78 | 0.26 | 0.27 | 0.99 | 0.046 | 0.11 | 0.65 | 0.43 |
| | 0.61 | 0.39 | 0.73 | 0.76 | 0.35 | 1.0 | 0.84 | 0.25 | 0.53 |
| 95% CI of OR Quart4 | 2.4 | 3.5 | 3.3 | 2.7 | 2.9 | 3.6 | 6.0 | 9.1 | 4.4 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0141 | 0.0142 | 0.0141 | 0.0159 | 0.0141 | 0.0141 |
| Average | 0.134 | 0.189 | 0.134 | 0.264 | 0.134 | 0.113 |
| Stdev | 0.490 | 0.614 | 0.490 | 0.778 | 0.490 | 0.249 |
| p(t-test) | | 0.36 | | 0.031 | | 0.78 |
| Min | 1.14E-9 | 1.14E-9 | 1.14E-9 | 1.40E-9 | 1.14E-9 | 0.000186 |
| Max | 8.92 | 4.66 | 8.92 | 4.47 | 8.92 | 1.05 |
| n (Samp) | 754 | 78 | 754 | 86 | 754 | 42 |
| n (Patient) | 298 | 78 | 298 | 86 | 298 | 42 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0143 | 0.0158 | 0.0143 | 0.0371 | 0.0143 | 0.0137 |
| Average | 0.157 | 0.352 | 0.157 | 0.217 | 0.157 | 0.133 |
| Stdev | 0.510 | 1.09 | 0.510 | 0.706 | 0.510 | 0.209 |
| p(t-test) | | 0.12 | | 0.58 | | 0.85 |
| Min | 1.14E-9 | 0.00163 | 1.14E-9 | 7.54E-8 | 1.14E-9 | 0.000952 |
| Max | 8.92 | 4.66 | 8.92 | 3.42 | 8.92 | 0.635 |
| n (Samp) | 1164 | 18 | 1164 | 23 | 1164 | 18 |
| n (Patient) | 379 | 18 | 379 | 23 | 379 | 18 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0145 | 0.0134 | 0.0145 | 0.0146 | 0.0145 | 0.0141 |
| Average | 0.183 | 0.131 | 0.183 | 0.235 | 0.183 | 0.0928 |
| Stdev | 0.639 | 0.352 | 0.639 | 0.722 | 0.639 | 0.237 |
| p(t-test) | | 0.50 | | 0.50 | | 0.40 |
| Min | 1.14E-9 | 1.14E-9 | 1.14E-9 | 1.40E-9 | 1.14E-9 | 0.000186 |
| Max | 8.92 | 2.40 | 8.92 | 4.47 | 8.92 | 1.05 |
| n (Samp) | 838 | 70 | 838 | 80 | 838 | 36 |
| n (Patient) | 306 | 70 | 306 | 80 | 306 | 36 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.55 | 0.49 | 0.53 | 0.61 | 0.51 | 0.49 | 0.54 | 0.46 |
| SE | 0.035 | 0.070 | 0.036 | 0.033 | 0.063 | 0.034 | 0.046 | 0.070 | 0.050 |
| p | 0.71 | 0.48 | 0.79 | 0.31 | 0.086 | 0.77 | 0.81 | 0.60 | 0.43 |
| nCohort 1 | 754 | 1164 | 838 | 754 | 1164 | 838 | 754 | 1164 | 838 |
| nCohort 2 | 78 | 18 | 70 | 86 | 23 | 80 | 42 | 18 | 36 |
| Cutoff 1 | 0.00681 | 0.00693 | 0.00611 | 0.00657 | 0.0157 | 0.00657 | 0.00752 | 0.00639 | 0.00324 |
| Sens 1 | 71% | 72% | 70% | 71% | 74% | 70% | 71% | 72% | 72% |
| Spec 1 | 34% | 33% | 30% | 33% | 53% | 32% | 35% | 31% | 18% |
| Cutoff 2 | 0.00435 | 0.00618 | 0.00378 | 0.00479 | 0.00811 | 0.00481 | 0.00254 | 0.00583 | 0.00204 |
| Sens 2 | 81% | 83% | 80% | 80% | 83% | 80% | 81% | 83% | 81% |
| Spec 2 | 24% | 30% | 21% | 26% | 36% | 25% | 16% | 29% | 13% |
| Cutoff 3 | 0.00151 | 0.00338 | 0.00151 | 0.00123 | 0.00124 | 0.00198 | 0.00105 | 0.00254 | 0.000965 |
| Sens 3 | 91% | 94% | 90% | 91% | 91% | 90% | 90% | 94% | 92% |
| Spec 3 | 10% | 19% | 9% | 9% | 8% | 12% | 8% | 15% | 7% |
| Cutoff 4 | 0.0343 | 0.0360 | 0.0369 | 0.0343 | 0.0360 | 0.0369 | 0.0343 | 0.0360 | 0.0369 |
| Sens 4 | 29% | 33% | 29% | 38% | 57% | 34% | 29% | 39% | 31% |
| Spec 4 | 70% | 70% | 70% | 70% | 70% | 70% | 70% | 70% | 70% |
| Cutoff 5 | 0.0695 | 0.0887 | 0.0945 | 0.0695 | 0.0887 | 0.0945 | 0.0695 | 0.0887 | 0.0945 |
| Sens 5 | 26% | 33% | 21% | 24% | 22% | 24% | 19% | 28% | 14% |
| Spec 5 | 80% | 80% | 80% | 80% | 80% | 80% | 80% | 80% | 80% |
| Cutoff 6 | 0.295 | 0.363 | 0.416 | 0.295 | 0.363 | 0.416 | 0.295 | 0.363 | 0.416 |
| Sens 6 | 17% | 17% | 10% | 15% | 9% | 12% | 14% | 17% | 8% |
| Spec 6 | 90% | 90% | 90% | 90% | 90% | 90% | 90% | 90% | 90% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | only | UO only |
| OR Quart 2 p Value 95% CI of OR Quart2 | 1.5 | 1.7 | 0.88 | 1.4 | 0.49 | 1.6 | 1.1 | 2.0 | 1.6 |
| | 0.24 | 0.48 | 0.72 | 0.32 | 0.42 | 0.19 | 0.82 | 0.33 | 0.33 |
| | 0.76 | 0.40 | 0.44 | 0.72 | 0.090 | 0.80 | 0.46 | 0.50 | 0.61 |
| | 2.9 | 7.1 | 1.8 | 2.7 | 2.7 | 3.0 | 2.7 | 8.1 | 4.2 |
| OR Quart 3 p Value 95% CI of OR Quart3 | 1.2 | 1.3 | 1.1 | 1.1 | 2.0 | 0.93 | 1.0 | 1.3 | 1.0 |
| | 0.60 | 0.70 | 0.86 | 0.73 | 0.25 | 0.85 | 1.0 | 0.70 | 1.0 |
| | 0.60 | 0.30 | 0.54 | 0.57 | 0.60 | 0.45 | 0.41 | 0.30 | 0.34 |
| | 2.4 | 6.0 | 2.1 | 2.2 | 6.8 | 1.9 | 2.5 | 6.0 | 2.9 |
| OR Quart 4 p Value 95% CI or OR Quart4 | 1.3 | 2.0 | 0.94 | 1.7 | 2.3 | 1.6 | 1.1 | 1.7 | 1.6 |
| | 0.49 | 0.33 | 0.86 | 0.11 | 0.17 | 0.15 | 0.82 | 0.48 | 0.33 |
| | 0.64 | 0.50 | 0.47 | 0.88 | 0.69 | 0.84 | 0.46 | 0.40 | 0.61 |
| | 2.5 | 8.1 | 1.9 | 3.2 | 7.5 | 3.1 | 2.7 | 7.1 | 4.2 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1240 | 1920 | 1240 | 1530 | 1240 | 1120 |
| Average | 1930 | 2680 | 1930 | 4030 | 1930 | 1970 |
| Stdev | 2780 | 2580 | 2780 | 10600 | 2780 | 3040 |
| p(t-test) | | 0.011 | | 2.9E-6 | | 0.92 |
| Min | 0.0376 | 66.6 | 0.0376 | 108 | 0.0376 | 14.6 |
| Max | 45800 | 11900 | 45800 | 80100 | 45800 | 19700 |
| n (Samp) | 895 | 99 | 895 | 102 | 895 | 57 |
| n (Patient) | 374 | 99 | 374 | 102 | 374 | 57 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1370 | 1810 | 1370 | 1520 | 1370 | 1280 |
| Average | 2310 | 3240 | 2310 | 5550 | 2310 | 1900 |
| Stdev | 3600 | 2910 | 3600 | 15400 | 3600 | 1810 |
| p(t-test) | | 0.25 | | 7.9E-5 | | 0.57 |
| Min | 0.0376 | 227 | 0.0376 | 174 | 0.0376 | 201 |
| Max | 68100 | 8700 | 68100 | 80100 | 68100 | 7000 |
| n (Samp) | 1357 | 20 | 1357 | 26 | 1357 | 25 |
| n (Patient) | 470 | 20 | 470 | 26 | 470 | 25 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1280 | 1860 | 1280 | 1570 | 1280 | 1160 |
| Average | 2140 | 2550 | 2140 | 3340 | 2140 | 2160 |
| Stdev | 3830 | 2460 | 3830 | 7570 | 3830 | 3300 |
| p(t-test) | | 0.32 | | 0.010 | | 0.98 |
| Min | 0.0376 | 66.6 | 0.0376 | 108 | 0.0376 | 14.6 |
| Max | 80100 | 11900 | 80100 | 68100 | 80100 | 19700 |
| n (Samp) | 962 | 91 | 962 | 94 | 962 | 49 |
| n (Patient) | 367 | 91 | 367 | 94 | 367 | 49 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.62 | 0.57 | 0.57 | 0.57 | 0.56 | 0.47 | 0.49 | 0.46 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.031 | 0.068 | 0.032 | 0.031 | 0.059 | 0.032 | 0.040 | 0.059 | 0.043 |
| p | 0.0029 | 0.064 | 0.032 | 0.030 | 0.21 | 0.083 | 0.40 | 0.87 | 0.41 |
| nCohort 1 | 895 | 1357 | 962 | 895 | 1357 | 962 | 895 | 1357 | 962 |
| nCohort 2 | 99 | 20 | 91 | 102 | 26 | 94 | 57 | 25 | 49 |
| Cutoff 1 | 899 | 1380 | 899 | 799 | 1020 | 799 | 575 | 679 | 541 |
| Sens 1 | 71% | 70% | 70% | 71% | 73% | 70% | 70% | 72% | 71% |
| Spec 1 | 37% | 51% | 35% | 34% | 39% | 32% | 23% | 26% | 21% |
| Cutoff 2 | 612 | 878 | 612 | 501 | 686 | 498 | 450 | 589 | 450 |
| Sens 2 | 81% | 80% | 80% | 80% | 81% | 81% | 81% | 80% | 82% |
| Spec 2 | 25% | 34% | 24% | 20% | 27% | 19% | 19% | 23% | 17% |
| Cutoff 3 | 293 | 831 | 332 | 348 | 275 | 353 | 214 | 354 | 191 |
| Sens 3 | 91% | 90% | 90% | 90% | 92% | 90% | 91% | 92% | 92% |
| Spec 3 | 10% | 32% | 11% | 14% | 9% | 13% | 7% | 13% | 5% |
| Cutoff 4 | 2000 | 2300 | 2070 | 2000 | 2300 | 2070 | 2000 | 2300 | 2070 |
| Sens 4 | 48% | 40% | 48% | 41% | 35% | 44% | 25% | 24% | 27% |
| Spec 4 | 70% | 70% | 70% | 70% | 70% | 70% | 70% | 70% | 70% |
| Cutoff 5 | 2680 | 3160 | 2840 | 2680 | 3160 | 2840 | 2680 | 3160 | 2840 |
| Sens 5 | 36% | 35% | 33% | 33% | 35% | 32% | 21% | 24% | 18% |
| Spec 5 | 80% | 80% | 80% | 80% | 80% | 80% | 80% | 80% | 80% |
| Cutoff 6 | 4110 | 4870 | 4360 | 4110 | 4870 | 4360 | 4110 | 4870 | 4360 |
| Sens 6 | 21% | 25% | 16% | 20% | 19% | 17% | 11% | 8% | 12% |
| Spec 6 | 90% | 90% | 90% | 90% | 90% | 90% | 90% | 90% | 90% |
| OR Quart 2 p Value 95% CI of OR Quart2 | 1.00 | 2.0 | 1.0 | 0.55 | 1.8 | 0.62 | 0.92 | 0.83 | 1.0 |
| | 0.99 | 0.42 | 1.0 | 0.076 | 0.37 | 0.15 | 0.84 | 0.77 | 1.0 |
| | 0.52 | 0.37 | 0.52 | 0.28 | 0.51 | 0.32 | 0.41 | 0.25 | 0.43 |
| | 1.9 | 11 | 1.9 | 1.1 | 6.1 | 1.2 | 2.1 | 2.8 | 2.4 |
| OR Quart 3 p Value 95% CI ot OR Quart3 | 0.95 | 3.6 | 0.84 | 0.96 | 1.5 | 0.74 | 1.0 | 1.2 | 1.0 |
| | 0.87 | 0.12 | 0.61 | 0.88 | 0.53 | 0.35 | 1.0 | 0.78 | 1.0 |
| | 0.49 | 0.73 | 0.43 | 0.54 | 0.42 | 0.40 | 0.45 | 0.39 | 0.43 |
| | 1.8 | 17 | 1.6 | 1.7 | 5.4 | 1.4 | 2.2 | 3.5 | 2.4 |
| OR Quart 4 p Value 95% CI of OR Quart4 | 2.2 | 3.5 | 1.8 | 1.4 | 2.3 | 1.4 | 1.5 | 1.2 | 1.5 |
| | 0.0072 | 0.12 | 0.048 | 0.18 | 0.17 | 0.22 | 0.27 | 0.78 | 0.32 |
| | 1.2 | 0.73 | 1.0 | 0.84 | 0.69 | 0.82 | 0.72 | 0.39 | 0.68 |
| | 3.9 | 17 | 3.2 | 2.5 | 7.5 | 2.4 | 3.1 | 3.5 | 3.3 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.3 | 12.5 | 10.3 | 10.6 | 10.3 | 0.452 |
| Average | 17.8 | 18.7 | 17.8 | 126 | 17.8 | 22.1 |
| Stdev | 47.1 | 20.2 | 47.1 | 866 | 47.1 | 56.8 |
| p(t-test) | | 0.87 | | 0.0025 | | 0.59 |
| Min | 0.00398 | 0.00398 | 0.00398 | 0.00398 | 0.00398 | 0.00398 |
| Max | 945 | 91.5 | 945 | 7540 | 945 | 317 |
| n (Samp) | 597 | 69 | 597 | 76 | 597 | 37 |
| n (Patient) | 279 | 69 | 279 | 76 | 279 | 37 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 0.452 | 10.6 | 20.6 | 10.6 | 0.397 |
| Average | 28.3 | 5.80 | 28.3 | 21.0 | 28.3 | 12.3 |
| Stdev | 266 | 10.5 | 266 | 18.3 | 266 | 17.3 |
| p(t-test) | | 0.80 | | 0.91 | | 0.82 |
| Min | 0.00398 | 0.0132 | 0.00398 | 0.00398 | 0.00398 | 0.0184 |
| Max | 7540 | 31.7 | 7540 | 72.0 | 7540 | 50.9 |
| n (Samp) | 826 | 9 | 826 | 17 | 826 | 14 |
| n (Patient) | 352 | 9 | 352 | 17 | 352 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 12.5 | 10.6 | 9.71 | 10.6 | 2.31 |
| Average | 17.7 | 19.1 | 17.7 | 131 | 17.7 | 25.1 |
| Stdev | 46.7 | 20.3 | 46.7 | 890 | 46.7 | 58.7 |
| p(t-test) | | 0.81 | | 0.0019 | | 0.38 |
| Min | 0.00398 | 0.00398 | 0.00398 | 0.00398 | 0.00398 | 0.00398 |
| Max | 945 | 91.5 | 945 | 7540 | 945 | 317 |
| n (Samp) | 604 | 66 | 604 | 72 | 604 | 34 |
| n (Patient) | 263 | 66 | 263 | 72 | 263 | 34 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.31 | 0.56 | 0.54 | 0.62 | 0.52 | 0.43 | 0.43 | 0.48 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.037 | 0.099 | 0.038 | 0.036 | 0.073 | 0.036 | 0.050 | 0.080 | 0.051 |
| p | 0.20 | 0.054 | 0.12 | 0.33 | 0.10 | 0.63 | 0.19 | 0.41 | 0.71 |
| nCohort 1 | 597 | 826 | 604 | 597 | 826 | 604 | 597 | 826 | 604 |
| nCohort 2 | 69 | 9 | 66 | 76 | 17 | 72 | 37 | 14 | 34 |
| Cutoff 1 | 0.533 | 0.0187 | 4.89 | 1.39 | 10.0 | 0.338 | 0.117 | 0.315 | 0.315 |
| Sens 1 | 71% | 78% | 71% | 71% | 71% | 74% | 70% | 71% | 71% |
| Spec 1 | 31% | 10% | 38% | 32% | 49% | 28% | 23% | 27% | 27% |
| Cutoff 2 | 0.117 | 0.00398 | 0.315 | 0.117 | 5.43 | 0.0748 | 0.0208 | 0.0187 | 0.103 |
| Sens 2 | 81% | 100% | 82% | 80% | 82% | 83% | 81% | 86% | 82% |
| Spec 2 | 23% | 2% | 27% | 23% | 40% | 18% | 14% | 10% | 23% |
| Cutoff 3 | 0.0132 | 0.00398 | 0.0132 | 0.0190 | 0.338 | 0.0187 | 0.0179 | 0.0184 | 0.0179 |
| Sens 3 | 94% | 100% | 95% | 91% | 94% | 92% | 92% | 93% | 91% |
| Spec 3 | 3% | 2% | 4% | 12% | 29% | 11% | 7% | 9% | 8% |
| Cutoff 4 | 17.2 | 19.3 | 18.0 | 17.2 | 19.3 | 18.0 | 17.2 | 19.3 | 18.0 |
| Sens 4 | 42% | 11% | 41% | 37% | 53% | 35% | 32% | 29% | 38% |
| Spec 4 | 70% | 70% | 70% | 70% | 70% | 70% | 70% | 70% | 70% |
| Cutoff 5 | 25.6 | 28.1 | 25.8 | 25.6 | 28.1 | 25.8 | 25.6 | 28.1 | 25.8 |
| Sens 5 | 29% | 11% | 29% | 26% | 24% | 25% | 19% | 21% | 24% |
| Spec 5 | 80% | 81% | 80% | 80% | 81% | 80% | 80% | 81% | 80% |
| Cutoff 6 | 39.2 | 43.5 | 38.4 | 39.2 | 43.5 | 38.4 | 39.2 | 43.5 | 38.4 |
| Sens 6 | 13% | 0% | 14% | 16% | 6% | 17% | 14% | 7% | 15% |
| Spec 6 | 90% | 90% | 90% | 90% | 90% | 90% | 90% | 90% | 90% |
| OR Quart 2 p Value 95% CI ot OR Quart2 | 1.1 | 0 | 1.2 | 1.5 | 5.1 | 1.1 | 0.75 | 0.25 | 0.66 |
| | 0.86 | na | 0.69 | 0.23 | 0.14 | 0.86 | 0.59 | 0.21 | 0.44 |
| | 0.50 | na | 0.53 | 0.77 | 0.59 | 0.54 | 0.25 | 0.027 | 0.23 |
| | 2.3 | na | 2.6 | 3.1 | 44 | 2.1 | 2.2 | 2.2 | 1.9 |
| OR Quart 3 p Value 95% CI of OR Quart3 | 1.1 | 4.1 | 1.4 | 1.2 | 5.1 | 0.88 | 1.3 | 1.3 | 1.1 |
| | 0.85 | 0.21 | 0.43 | 0.58 | 0.14 | 0.72 | 0.63 | 0.74 | 0.81 |
| | 0.50 | 0.45 | 0.63 | 0.60 | 0.59 | 0.43 | 0.49 | 0.33 | 0.44 |
| | 2.3 | 37 | 3.0 | 2.5 | 44 | 1.8 | 3.3 | 4.7 | 2.8 |
| OR Quart 4 p Value 95% CI of OR Quart4 | 1.9 | 4.1 | 2.2 | 1.4 | 6.1 | 1.1 | 1.7 | 1.0 | 1.0 |
| | 0.067 | 0.21 | 0.039 | 0.30 | 0.095 | 0.86 | 0.26 | 1.0 | 0.99 |
| | 0.96 | 0.45 | 1.0 | 0.72 | 0.73 | 0.54 | 0.68 | 0.25 | 0.39 |
| | 3.8 | 37 | 4.5 | 2.9 | 51 | 2.1 | 4.2 | 4.1 | 2.6 |

Fig. 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Angiopoietin-related protein 4**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 11.6 | 10.1 | 15.0 | 40.4 | 10.9 | 9.76 |
| Average | 39.6 | 39.3 | 49.2 | 70.6 | 36.1 | 43.8 |
| Stdev | 103 | 85.6 | 104 | 83.2 | 93.4 | 101 |
| p(t-test) |  | 0.99 |  | 0.44 |  | 0.59 |
| Min | 0.000466 | 1.12 | 0.577 | 5.74 | 0.000466 | 0.00194 |
| Max | 789 | 645 | 624 | 276 | 789 | 645 |
| n (Samp) | 128 | 85 | 41 | 18 | 115 | 73 |
| n (Patient) | 128 | 85 | 41 | 18 | 115 | 73 |

|  | At Enrollment | | |
|---|---|---|---|
|  | sCr or UO | sCr only | UO only |
| AUC | 0.51 | 0.66 | 0.52 |
| SE | 0.041 | 0.080 | 0.043 |
| p | 0.72 | 0.045 | 0.65 |
| nCohort 1 | 128 | 41 | 115 |
| nCohort 2 | 85 | 18 | 73 |
| Cutoff 1 | 6.86 | 12.5 | 6.65 |
| Sens 1 | 71% | 72% | 71% |
| Spec 1 | 31% | 46% | 32% |
| Cutoff 2 | 4.68 | 10.1 | 4.43 |
| Sens 2 | 80% | 83% | 81% |
| Spec 2 | 23% | 39% | 17% |
| Cutoff 3 | 3.17 | 5.81 | 2.85 |
| Sens 3 | 91% | 94% | 90% |
| Spec 3 | 12% | 24% | 9% |
| Cutoff 4 | 21.9 | 35.2 | 17.4 |
| Sens 4 | 34% | 61% | 42% |
| Spec 4 | 70% | 71% | 70% |
| Cutoff 5 | 35.2 | 55.4 | 28.9 |
| Sens 5 | 25% | 39% | 25% |
| Spec 5 | 80% | 80% | 80% |
| Cutoff 6 | 77.4 | 83.9 | 65.2 |
| Sens 6 | 11% | 22% | 16% |
| Spec 6 | 91% | 90% | 90% |

(continued)

|  | At Enrollment | | |
|---|---|---|---|
|  | sCr or UO | sCr only | UO only |
| OR Quart 2 p Value 95% CI of OR Quart2 | 0.93 | 1.3 | 0.84 |
|  | 0.84 | 0.74 | 0.67 |
|  | 0.43 | 0.24 | 0.37 |
|  | 2.0 | 7.4 | 1.9 |
| OR Quart 3 p Value 95% CI of OR Quart3 | 0.62 | 1.3 | 0.57 |
|  | 0.23 | 0.74 | 0.20 |
|  | 0.28 | 0.24 | 0.24 |
|  | 1.4 | 7.4 | 1.3 |
| OR Quart 4 p Value 95% CI of OR Quart4 | 0.97 | 3.2 | 1.1 |
|  | 0.93 | 0.16 | 0.84 |
|  | 0.45 | 0.63 | 0.48 |
|  | 2.1 | 16 | 2.5 |

**Amphiregulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 25.7 | 30.6 | 32.9 | 80.6 | 24.8 | 30.2 |
| Average | 60.4 | 121 | 69.1 | 481 | 54.7 | 39.6 |
| Stdev | 164 | 467 | 93.8 | 1010 | 170 | 40.2 |
| p(t-test) |  | 0.21 |  | 0.011 |  | 0.52 |
| Min | 1.21 | 0.00389 | 6.31 | 9.15 | 1.21 | 0.00389 |
| Max | 1640 | 3480 | 426 | 3480 | 1640 | 211 |
| n (Samp) | 113 | 67 | 41 | 13 | 96 | 54 |
| n (Patient) | 113 | 67 | 41 | 13 | 96 | 54 |

|  | At Enrollment | | |
|---|---|---|---|
|  | sCr or UO | sCr only | UO only |
| AUC | 0.52 | 0.69 | 0.53 |
| SE | 0.045 | 0.090 | 0.049 |
| p | 0.59 | 0.031 | 0.60 |
| nCohort 1 | 113 | 41 | 96 |
| nCohort 2 | 67 | 13 | 54 |
| Cutoff 1 | 16.3 | 37.8 | 16.8 |
| Sens 1 | 70% | 77% | 70% |
| Spec 1 | 33% | 61% | 34% |
| Cutoff 2 | 12.2 | 28.1 | 14.0 |

(continued)

|  | At Enrollment | | |
|---|---|---|---|
|  | sCr or UO | sCr only | UO only |
| Sens 2 | 81% | 85% | 81% |
| Spec 2 | 21% | 46% | 25% |
| Cutoff 3 | 9.94 | 9.15 | 10.5 |
| Sens 3 | 91% | 92% | 91% |
| Spec 3 | 16% | 12% | 17% |
| Cutoff 4 | 42.2 | 59.5 | 42.2 |
| Sens 4 | 31% | 62% | 30% |
| Spec 4 | 71% | 71% | 71% |
| Cutoff 5 | 56.0 | 82.1 | 50.9 |
| Sens 5 | 22% | 46% | 19% |
| Spec 5 | 81% | 80% | 80% |
| Cutoff 6 | 96.9 | 189 | 72.6 |
| Sens 6 | 12% | 31% | 13% |
| Spec 6 | 90% | 90% | 91% |
| OR Quart 2 p Value 95% CI of OR Quart2 | 0.91 | 0.42 | 0.96 |
|  | 0.82 | 0.51 | 0.94 |
|  | 0.38 | 0.034 | 0.36 |
|  | 2.2 | 5.3 | 2.5 |
| OR Quart 3 p Value 95% CI of OR Quart3 | 1.4 | 2.4 | 2.0 |
|  | 0.39 | 0.36 | 0.16 |
|  | 0.62 | 0.36 | 0.77 |
|  | 3.4 | 17 | 5.1 |
| OR Quart 4 p Value 95% CI of OR Quart4 | 1.0 | 4.1 | 0.96 |
|  | 1.0 | 0.13 | 0.94 |
|  | 0.42 | 0.65 | 0.36 |
|  | 2.4 | 26 | 2.5 |

**Endostatin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 5200 | 5720 | 5200 | 31100 | 5620 | 5490 |
| Average | 14300 | 15400 | 16300 | 58800 | 14100 | 14600 |
| Stdev | 24200 | 28400 | 25000 | 67700 | 25600 | 27700 |
| p(t-test) |  | 0.75 |  | 2.6E-4 |  | 0.89 |
| Min | 0.0130 | 389 | 300 | 860 | 0.0130 | 389 |
| Max | 145000 | 173000 | 116000 | 190000 | 180000 | 148000 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| n (Samp) | 155 | 96 | 51 | 19 | 138 | 83 |
| n (Patient) | 155 | 96 | 51 | 19 | 138 | 83 |

|  | At Enrollment | | |
|---|---|---|---|
|  | sCr or UO | sCr only | UO only |
| AUC | 0.53 | 0.73 | 0.51 |
| SE | 0.038 | 0.072 | 0.040 |
| p | 0.41 | 0.0012 | 0.74 |
| nCohort 1 | 155 | 51 | 138 |
| nCohort 2 | 96 | 19 | 83 |
| Cutoff 1 | 3630 | 9260 | 4360 |
| Sens 1 | 71% | 74% | 71% |
| Spec 1 | 39% | 65% | 42% |
| Cutoff 2 | 2820 | 4640 | 3080 |
| Sens 2 | 80% | 84% | 81% |
| Spec 2 | 28% | 45% | 27% |
| Cutoff 3 | 1600 | 2300 | 1850 |
| Sens 3 | 91% | 95% | 90% |
| Spec 3 | 11% | 29% | 12% |
| Cutoff 4 | 9260 | 12800 | 10500 |
| Sens 4 | 31% | 63% | 24% |
| Spec 4 | 70% | 71% | 70% |
| Cutoff 5 | 19200 | 21900 | 18200 |
| Sens 5 | 19% | 58% | 17% |
| Spec 5 | 80% | 80% | 80% |
| Cutoff 6 | 41300 | 57800 | 35800 |
| Sens 6 | 8% | 32% | 10% |
| Spec 6 | 90% | 90% | 91% |
| OR Quart 2 | 1.7 | 1.5 | 1.6 |
| p Value | 0.16 | 0.68 | 0.24 |
| 95% CI of | 0.81 | 0.22 | 0.73 |
| OR Quart 2 | 3.5 | 10 | 3.5 |
| OR Quart2 3 | 1.6 | 2.3 | 1.5 |
| p Value | 0.22 | 0.38 | 0.33 |
| 95% CI of | 0.76 | 0.36 | 0.68 |
| OR Quart3 | 3.3 | 15 | 3.2 |

(continued)

|  | At Enrollment | | |
|---|---|---|---|
|  | sCr or UO | sCr only | UO only |
| OR Quart 4 | 1.4 | 9.4 | 0.97 |
| p Value | 0.38 | 0.012 | 0.95 |
| 95% CI of | 0.66 | 1.6 | 0.44 |
| OR Quart4 | 2.9 | 54 | 2.2 |

**Fibroblast growth factor 19**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3.21E-5 | 2.68E-5 | 4.39E-5 | 2.68E-5 | 2.83E-5 | 2.83E-5 |
| Average | 0.00453 | 0.00701 | 0.000785 | 0.00265 | 0.00506 | 0.00880 |
| Stdev | 0.0421 | 0.0426 | 0.00286 | 0.00580 | 0.0445 | 0.0466 |
| p(t-test) |  | 0.68 |  | 0.10 |  | 0.58 |
| Min | 5.62E-6 | 5.62E-6 | 5.62E-6 | 1.66E-5 | 5.62E-6 | 5.62E-6 |
| Max | 0.476 | 0.388 | 0.0164 | 0.0157 | 0.476 | 0.388 |
| n (Samp) | 128 | 85 | 41 | 18 | 115 | 73 |
| n (Patient) | 128 | 85 | 41 | 18 | 115 | 73 |

|  | At Enrollment | | |
|---|---|---|---|
|  | sCr or UO | sCr only | UO only |
| AUC | 0.48 | 0.55 | 0.49 |
| SE | 0.041 | 0.083 | 0.043 |
| p | 0.54 | 0.51 | 0.84 |
| nCohort 1 | 128 | 41 | 115 |
| nCohort 2 | 85 | 18 | 73 |
| Cutoff 1 | 1.92E-5 | 2.53E-5 | 2.17E-5 |
| Sens 1 | 75% | 72% | 71% |
| Spec 1 | 23% | 37% | 29% |
| Cutoff 2 | 1.82E-5 | 2.17E-5 | 1.82E-5 |
| Sens 2 | 86% | 83% | 85% |
| Spec 2 | 13% | 32% | 15% |
| Cutoff 3 | 1.66E-5 | 1.82E-5 | 1.66E-5 |
| Sens 3 | 91% | 94% | 90% |
| Spec 3 | 10% | 10% | 11% |
| Cutoff 4 | 0.000114 | 8.63E-5 | 8.63E-5 |
| Sens 4 | 21% | 39% | 23% |
| Spec 4 | 82% | 71% | 72% |

(continued)

| | At Enrollment | | |
|---|---|---|---|
| | sCr or UO | sCr only | UO only |
| Cutoff 5 | 0.000114 | 0.000114 | 0.000114 |
| Sens 5 | 21% | 33% | 19% |
| Spec 5 | 82% | 83% | 83% |
| Cutoff 6 | 0.00127 | 0.000197 | 0.000197 |
| Sens 6 | 15% | 22% | 18% |
| Spec 6 | 91% | 90% | 90% |
| OR Quart 2 p Value 95% CI of OR Quart2 | 0.69 | 6.9 | 1.6 |
| | 0.35 | 0.037 | 0.29 |
| | 0.31 | 1.1 | 0.68 |
| | 1.5 | 42 | 3.6 |
| OR Quart 3 p Value 95% CI of OR Quart3 | 1.2 | 0.92 | 1.2 |
| | 0.64 | 0.94 | 0.67 |
| | 0.56 | 0.11 | 0.52 |
| | 2.6 | 7.6 | 2.8 |
| OR Quart 4 p Value 95% CI of OR Quart4 | 1.0 | 4.0 | 1.2 |
| | 0.94 | 0.14 | 0.67 |
| | 0.48 | 0.65 | 0.52 |
| | 2.2 | 25 | 2.8 |

**Fibroblast growth factor 21**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.0125 | 0.0121 | 0.0255 | 0.0979 | 0.0100 | 0.0112 |
| Average | 0.122 | 0.193 | 0.147 | 0.432 | 0.132 | 0.180 |
| Stdev | 0.413 | 0.614 | 0.477 | 0.837 | 0.443 | 0.580 |
| p(t-test) | | 0.31 | | 0.10 | | 0.52 |
| Min | 1.40E-9 | 1.40E-9 | 4.16E-6 | 0.00126 | 1.40E-9 | 1.40E-9 |
| Max | 2.88 | 3.58 | 2.88 | 3.42 | 3.07 | 3.58 |
| n (Samp) | 128 | 85 | 41 | 18 | 115 | 73 |
| n (Patient) | 128 | 85 | 41 | 18 | 115 | 73 |

| | At Enrollment | | |
|---|---|---|---|
| | sCr or UO | sCr only | UO only |
| AUC | 0.52 | 0.70 | 0.52 |
| SE | 0.041 | 0.078 | 0.043 |
| p | 0.58 | 0.012 | 0.70 |

(continued)

| | At Enrollment | | |
|---|---|---|---|
| | sCr or UO | sCr only | UO only |
| nCohort 1 | 128 | 41 | 115 |
| nCohort 2 | 85 | 18 | 73 |
| Cutoff 1 | 0.00522 | 0.0330 | 0.00432 |
| Sens 1 | 71% | 72% | 71% |
| Spec 1 | 30% | 63% | 28% |
| Cutoff 2 | 0.00355 | 0.0192 | 0.00311 |
| Sens 2 | 80% | 83% | 81% |
| Spec 2 | 24% | 46% | 23% |
| Cutoff 3 | 0.00187 | 0.00324 | 0.00124 |
| Sens 3 | 91% | 94% | 90% |
| Spec 3 | 16% | 12% | 10% |
| Cutoff 4 | 0.0297 | 0.0419 | 0.0263 |
| Sens 4 | 34% | 61% | 34% |
| Spec 4 | 70% | 71% | 70% |
| Cutoff 5 | 0.0509 | 0.0696 | 0.0386 |
| Sens 5 | 24% | 50% | 26% |
| Spec 5 | 80% | 80% | 80% |
| Cutoff 6 | 0.145 | 0.135 | 0.256 |
| Sens 6 | 16% | 44% | 14% |
| Spec 6 | 91% | 90% | 90% |
| OR Quart 2 | 1.0 | 1.5 | 0.84 |
| p Value 95% CI of OR Quart2 | 1.0 | 0.69 | 0.67 |
| | 0.46 | 0.21 | 0.36 |
| | 2.2 | 11 | 1.9 |
| OR Quart 3 p Value 95% CI of OR Quart3 | 0.92 | 2.2 | 0.84 |
| | 0.84 | 0.42 | 0.67 |
| | 0.42 | 0.33 | 0.36 |
| | 2.0 | 14 | 1.9 |
| OR Quart 4 p Value 95% CI of OR Quart4 | 1.1 | 9.0 | 1.1 |
| | 0.75 | 0.018 | 0.83 |
| | 0.52 | 1.5 | 0.48 |
| | 2.4 | 55 | 2.5 |

Fig. 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6570 | 20400 | 6570 | 20400 | 6570 | 20400 |
| Average | 9580 | 18900 | 9580 | 17700 | 9580 | 17200 |
| Stdev | 8320 | 9570 | 8320 | 10100 | 8320 | 9450 |
| p(t-test) | | 5.9E-9 | | 6.4E-7 | | 8.5E-5 |
| Min | 77.7 | 647 | 77.7 | 647 | 77.7 | 1090 |
| Max | 30600 | 30600 | 30600 | 30600 | 30600 | 30600 |
| n (Samp) | 203 | 36 | 203 | 34 | 203 | 22 |
| n (Patient) | 203 | 36 | 203 | 34 | 203 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 11200 | 22300 | 11200 | 21100 | 11200 | 20400 |
| Average | 13000 | 21900 | 13000 | 20600 | 13000 | 19400 |
| Stdev | 9540 | 8480 | 9540 | 9460 | 9540 | 8650 |
| p(t-test) | | 5.9E-5 | | 6.3E-4 | | 0.011 |
| Min | 0.00873 | 647 | 0.00873 | 647 | 0.00873 | 1820 |
| Max | 30600 | 30600 | 30600 | 30600 | 30600 | 30600 |
| n (Samp) | 395 | 20 | 395 | 20 | 395 | 15 |
| n (Patient) | 395 | 20 | 395 | 20 | 395 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8170 | 20400 | 8170 | 20400 | 8170 | 20400 |
| Average | 10900 | 18000 | 10900 | 17200 | 10900 | 17600 |
| Stdev | 8900 | 9830 | 8900 | 10200 | 8900 | 10400 |
| p(t-test) | | 5.0E-4 | | 0.0029 | | 0.0090 |
| Min | 77.7 | 2260 | 77.7 | 1060 | 77.7 | 1090 |
| Max | 30600 | 30600 | 30600 | 30600 | 30600 | 30600 |
| n (Samp) | 215 | 22 | 215 | 20 | 215 | 13 |
| n (Patient) | 215 | 22 | 215 | 20 | 215 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.76 | 0.76 | 0.71 | 0.72 | 0.72 | 0.68 | 0.73 | 0.70 | 0.69 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.048 | 0.064 | 0.064 | 0.052 | 0.066 | 0.068 | 0.064 | 0.077 | 0.084 |
| p | 4.7E-8 | 6.2E-5 | 0.0013 | 1.9E-5 | 0.0011 | 0.010 | 3.8E-4 | 0.011 | 0.023 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 14600 | 20300 | 10900 | 11000 | 20300 | 10900 | 10000 | 15600 | 10000 |
| Sens 1 | 72% | 80% | 73% | 71% | 75% | 70% | 73% | 73% | 77% |
| Spec 1 | 74% | 68% | 60% | 65% | 68% | 60% | 63% | 59% | 58% |
| Cutoff 2 | 9130 | 20300 | 5040 | 5010 | 15900 | 5040 | 7710 | 11700 | 5040 |
| Sens 2 | 81% | 80% | 82% | 82% | 80% | 80% | 82% | 80% | 85% |
| Spec 2 | 61% | 68% | 39% | 44% | 60% | 39% | 56% | 52% | 39% |
| Cutoff 3 | 3680 | 9330 | 3680 | 2230 | 6050 | 3010 | 3010 | 7710 | 3010 |
| Sens 3 | 92% | 90% | 91% | 91% | 90% | 90% | 91% | 93% | 92% |
| Spec 3 | 33% | 46% | 29% | 19% | 35% | 21% | 25% | 41% | 21% |
| Cutoff 4 | 12900 | 20400 | 16200 | 12900 | 20400 | 16200 | 12900 | 20400 | 16200 |
| Sens 4 | 72% | 55% | 59% | 68% | 50% | 60% | 64% | 40% | 62% |
| Spec 4 | 70% | 79% | 70% | 70% | 79% | 70% | 70% | 79% | 70% |
| Cutoff 5 | 20000 | 20900 | 20400 | 20000 | 20900 | 20400 | 20000 | 20900 | 20400 |
| Sens 5 | 64% | 55% | 36% | 59% | 50% | 35% | 59% | 40% | 31% |
| Spec 5 | 80% | 80% | 87% | 80% | 80% | 87% | 80% | 80% | 87% |
| Cutoff 6 | 20400 | 27700 | 24000 | 20400 | 27700 | 24000 | 20400 | 27700 | 24000 |
| Sens 6 | 42% | 35% | 27% | 38% | 30% | 25% | 32% | 20% | 31% |
| Spec 6 | 90% | 90% | 90% | 90% | 90% | 90% | 90% | 90% | 90% |
| OR Quart 2 p Value | 1.3 | 2.0 | 1.5 | 0.58 | 0.99 | 0.23 | 0.32 | 2.0 | 0.49 |
| 95% CI of OR Quart2 | 0.71 | 0.57 | 0.65 | 0.47 | 0.99 | 0.20 | 0.33 | 0.57 | 0.57 |
| | 0.29 | 0.18 | 0.25 | 0.13 | 0.14 | 0.025 | 0.032 | 0.18 | 0.043 |
| | 6.2 | 22 | 9.5 | 2.5 | 7.2 | 2.1 | 3.2 | 22 | 5.6 |
| OR Quart 3 p Value | 2.9 | 6.2 | 2.6 | 1.2 | 3.1 | 0.98 | 1.7 | 6.3 | 1.0 |
| | 0.13 | 0.093 | 0.26 | 0.75 | 0.17 | 0.98 | 0.47 | 0.091 | 1.0 |
| | 0.72 | 0.74 | 0.49 | 0.35 | 0.61 | 0.23 | 0.39 | 0.75 | 0.14 |
| 95% CI of OR Quart3 | 11 | 53 | 14 | 4.2 | 16 | 4.1 | 7.6 | 53 | 7.4 |
| OR Quart 4 p Value | 10 | 12 | 7.1 | 5.4 | 5.4 | 3.1 | 5.2 | 6.2 | 4.5 |
| 95% CI of OR Quart4 | 4.0E-4 | 0.018 | 0.013 | 0.0019 | 0.033 | 0.067 | 0.014 | 0.093 | 0.065 |
| | 2.8 | 1.5 | 1.5 | 1.9 | 1.1 | 0.92 | 1.4 | 0.74 | 0.91 |
| | 36 | 95 | 33 | 16 | 25 | 10 | 19 | 53 | 22 |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 16.9 | 49.1 | 16.9 | 29.8 | 16.9 | 18.9 |
| Average | 56.0 | 143 | 56.0 | 139 | 56.0 | 108 |
| Stdev | 99.2 | 195 | 99.2 | 202 | 99.2 | 221 |
| p(t-test) | | 3.0E-4 | | 7.6E-4 | | 0.079 |
| Min | 0.00423 | 4.54 | 0.00423 | 4.54 | 0.00423 | 6.66 |
| Max | 538 | 878 | 538 | 878 | 538 | 878 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 37.6 | 21.3 | 30.7 | 21.3 | 21.5 |
| Average | 74.3 | 160 | 74.3 | 155 | 74.3 | 137 |
| Stdev | 157 | 230 | 157 | 229 | 157 | 255 |
| p(t-test) | | 0.030 | | 0.041 | | 0.17 |
| Min | 0.00423 | 14.1 | 0.00423 | 14.1 | 0.00423 | 9.39 |
| Max | 1370 | 878 | 1370 | 878 | 1370 | 878 |
| n (Samp) | 317 | 18 | 317 | 18 | 317 | 13 |
| n (Patient) | 317 | 18 | 317 | 18 | 317 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 19.2 | 36.5 | 19.2 | 19.8 | 19.2 | 12.0 |
| Average | 64.1 | 96.0 | 64.1 | 89.8 | 64.1 | 25.4 |
| Stdev | 109 | 130 | 109 | 139 | 109 | 33.3 |
| p(t-test) | | 0.24 | | 0.37 | | 0.27 |
| Min | 0.00423 | 4.54 | 0.00423 | 4.54 | 0.00423 | 6.66 |
| Max | 624 | 400 | 624 | 400 | 624 | 116 |
| n (Samp) | 172 | 19 | 172 | 17 | 172 | 10 |
| n (Patient) | 172 | 19 | 172 | 17 | 172 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.68 | 0.63 | 0.67 | 0.67 | 0.57 | 0.57 | 0.58 | 0.42 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.055 | 0.071 | 0.072 | 0.058 | 0.072 | 0.075 | 0.074 | 0.084 | 0.097 |
| p | 1.4E-4 | 0.011 | 0.076 | 0.0027 | 0.021 | 0.35 | 0.35 | 0.34 | 0.41 |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 21.0 | 21.5 | 16.6 | 17.8 | 20.7 | 16.2 | 13.7 | 16.6 | 10.6 |
| Sens 1 | 72% | 72% | 74% | 70% | 72% | 71% | 72% | 77% | 70% |
| Spec 1 | 56% | 50% | 46% | 52% | 48% | 44% | 44% | 42% | 28% |
| Cutoff 2 | 16.9 | 17.8 | 12.5 | 16.6 | 16.9 | 11.4 | 11.2 | 16.2 | 9.29 |
| Sens 2 | 81% | 83% | 84% | 80% | 83% | 82% | 83% | 85% | 80% |
| Spec 2 | 50% | 44% | 38% | 49% | 43% | 33% | 34% | 41% | 26% |
| Cutoff 3 | 13.7 | 16.2 | 10.6 | 12.5 | 16.2 | 10.6 | 9.06 | 14.0 | 9.06 |
| Sens 3 | 91% | 94% | 95% | 90% | 94% | 94% | 94% | 92% | 90% |
| Spec 3 | 44% | 41% | 28% | 42% | 41% | 28% | 29% | 37% | 26% |
| Cutoff 4 | 35.6 | 39.5 | 48.2 | 35.6 | 39.5 | 48.2 | 35.6 | 39.5 | 48.2 |
| Sens 4 | 56% | 50% | 47% | 50% | 44% | 29% | 28% | 31% | 10% |
| Spec 4 | 70% | 70% | 70% | 70% | 70% | 70% | 70% | 70% | 70% |
| Cutoff 5 | 76.7 | 76.7 | 84.1 | 76.7 | 76.7 | 84.1 | 76.7 | 76.7 | 84.1 |
| Sens 5 | 41% | 33% | 32% | 33% | 33% | 24% | 22% | 23% | 10% |
| Spec 5 | 80% | 80% | 80% | 80% | 80% | 80% | 80% | 80% | 80% |
| Cutoff 6 | 151 | 207 | 170 | 151 | 207 | 170 | 151 | 207 | 170 |
| Sens 6 | 28% | 33% | 16% | 30% | 28% | 18% | 17% | 23% | 0% |
| Spec 6 | 90% | 90% | 90% | 90% | 90% | 90% | 90% | 90% | 90% |
| OR Quart 2 p Value 95% CI of OR Quart2 | 7.9 | >6.4 | 6.6 | 11 | >7.5 | 8.0 | 8.2 | 5.2 | 2.1 |
| | 0.058 | <0.089 | 0.087 | 0.026 | <0.061 | 0.056 | 0.054 | 0.14 | 0.55 |
| | 0.93 | >0.75 | 0.76 | 1.3 | >0.91 | 0.95 | 0.96 | 0.59 | 0.18 |
| | 67 | na | 57 | 91 | na | 68 | 70 | 45 | 24 |
| OR Quart 3 p Value 95% CI of OR Quart3 | 14 | >4.2 | 5.3 | 13 | >4.2 | 5.5 | 6.8 | 4.2 | 6.8 |
| | 0.014 | <0.21 | 0.13 | 0.018 | <0.21 | 0.13 | 0.082 | 0.21 | 0.083 |
| | 1.7 | >0.45 | 0.60 | 1.5 | >0.45 | 0.61 | 0.79 | 0.45 | 0.78 |
| | 110 | na | 48 | 100 | na | 49 | 59 | 38 | 58 |
| OR Quart 4 p Value 95% CI of OR Quart4 | 17 | >8.7 | 7.9 | 12 | >7.5 | 4.2 | 4.2 | 3.0 | 1.0 |
| | 0.0073 | <0.043 | 0.059 | 0.020 | <0.061 | 0.21 | 0.21 | 0.34 | 0.99 |
| | 2.2 | >1.1 | 0.93 | 1.5 | >0.91 | 0.45 | 0.45 | 0.31 | 0.062 |
| | 140 | na | 67 | 100 | na | 39 | 39 | 30 | 17 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.292 | 0.543 | 0.292 | 0.257 | 0.292 | 0.183 |
| Average | 1.72 | 2.40 | 1.72 | 2.14 | 1.72 | 1.06 |
| Stdev | 3.60 | 4.44 | 3.60 | 4.57 | 3.60 | 1.92 |
| p(t-test) | | 0.38 | | 0.60 | | 0.47 |
| Min | 0.000122 | 0.000364 | 0.000122 | 0.000157 | 0.000122 | 0.000206 |
| Max | 25.9 | 22.4 | 25.9 | 22.4 | 25.9 | 6.31 |
| n (Samp) | 141 | 29 | 141 | 27 | 141 | 16 |
| n (Patient) | 141 | 29 | 141 | 27 | 141 | 16 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.421 | 0.399 | 0.421 | 0.399 | 0.421 | 0.218 |
| Average | 2.19 | 1.75 | 2.19 | 1.55 | 2.19 | 1.39 |
| Stdev | 5.66 | 2.25 | 5.66 | 2.20 | 5.66 | 2.13 |
| p(t-test) | | 0.76 | | 0.66 | | 0.63 |
| Min | 0.000122 | 0.000364 | 0.000122 | 0.000364 | 0.000122 | 0.000364 |
| Max | 70.5 | 6.31 | 70.5 | 6.31 | 70.5 | 6.31 |
| n (Samp) | 292 | 16 | 292 | 16 | 292 | 12 |
| n (Patient) | 292 | 16 | 292 | 16 | 292 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.360 | 0.282 | 0.360 | 0.210 | 0.360 | 0.00357 |
| Average | 1.80 | 2.56 | 1.80 | 2.33 | 1.80 | 0.178 |
| Stdev | 3.47 | 5.39 | 3.47 | 5.68 | 3.47 | 0.269 |
| p(t-test) | | 0.41 | | 0.59 | | 0.16 |
| Min | 0.000122 | 0.000364 | 0.000122 | 0.000157 | 0.000122 | 0.000206 |
| Max | 25.9 | 22.4 | 25.9 | 22.4 | 25.9 | 0.802 |
| n (Samp) | 162 | 18 | 162 | 16 | 162 | 9 |
| n (Patient) | 162 | 18 | 162 | 16 | 162 | 9 |

| | 48hr prior to AKI stage | | | 24hr prior to AKI stage | | | 0hr prior to AKI stage | | |
|---|---|---|---|---|---|---|---|---|---|
| | UO only | sCr only | sCr or UO | UO only | sCr only | sCr or UO | UO only | sCr only | sCr or UO |
| AUC | 0.30 | 0.48 | 0.45 | 0.45 | 0.52 | 0.53 | 0.52 | 0.55 | 0.59 |
| SE | 0.100 | 0.086 | 0.078 | 0.077 | 0.075 | 0.061 | 0.073 | 0.076 | 0.060 |
| p | 0.047 | 0.84 | 0.50 | 0.50 | 0.75 | 0.68 | 0.74 | 0.48 | 0.15 |
| nCohort 1 | 162 | 292 | 141 | 162 | 292 | 141 | 162 | 292 | 141 |
| nCohort 2 | 9 | 12 | 16 | 16 | 16 | 27 | 18 | 16 | 29 |
| Cutoff 1 | 0.00224 | 0.0480 | 0.00224 | 0.00224 | 0.153 | 0.111 | 0.114 | 0.153 | 0.153 |
| Sens 1 | 78% | 75% | 81% | 88% | 75% | 70% | 72% | 75% | 72% |
| Spec 1 | 19% | 31% | 19% | 19% | 36% | 38% | 32% | 36% | 41% |
| Cutoff 2 | 0.000336 | 0.00224 | 0.00224 | 0.00224 | 0.0480 | 0.00224 | 0.00224 | 0.119 | 0.0628 |
| Sens 2 | 89% | 83% | 81% | 88% | 81% | 89% | 94% | 81% | 83% |
| Spec 2 | 10% | 19% | 19% | 19% | 31% | 19% | 19% | 34% | 35% |
| Cutoff 3 | 0.000205 | 0.000336 | 0.000336 | 0.000336 | 0.000336 | 0.000336 | 0.00224 | 0.00224 | 0.00224 |
| Sens 3 | 100% | 100% | 94% | 94% | 100% | 96% | 94% | 94% | 97% |
| Spec 3 | 1% | 10% | 11% | 10% | 10% | 11% | 19% | 19% | 19% |
| Cutoff 4 | 1.45 | 1.25 | 1.28 | 1.45 | 1.25 | 1.28 | 1.45 | 1.25 | 1.28 |
| Sens 4 | 0% | 33% | 19% | 25% | 31% | 26% | 33% | 38% | 34% |
| Spec 4 | 70% | 70% | 70% | 70% | 70% | 70% | 70% | 70% | 70% |
| Cutoff 5 | 2.67 | 2.44 | 2.60 | 2.67 | 2.44 | 2.60 | 2.67 | 2.44 | 2.60 |
| Sens 5 | 0% | 25% | 19% | 25% | 25% | 26% | 28% | 31% | 31% |
| Spec 5 | 80% | 80% | 80% | 80% | 80% | 80% | 80% | 80% | 80% |
| Cutoff | 4.30 | 5.79 | 4.06 | 4.30 | 5.79 | 4.06 | 4.30 | 5.79 | 4.06 |
| Sens 6 | 0% | 8% | 12% | 12% | 6% | 19% | 17% | 6% | 21% |
| Spec 6 | 90% | 90% | 90% | 90% | 90% | 90% | 90% | 90% | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 p Value 95% CI of OR Quart2 | 2.0 | 3.2 | 1.6 | 1.0 | 1.7 | 0.24 | 1.0 | 0.66 | >2.1 |
| | 0.27 | 0.17 | 0.50 | 1.0 | 0.47 | 0.21 | 0.97 | 0.65 | <0.55 |
| | 0.60 | 0.62 | 0.41 | 0.32 | 0.39 | 0.026 | 0.19 | 0.11 | >0.18 |
| | 6.4 | 16 | 6.0 | 3.2 | 7.4 | 2.2 | 5.4 | 4.1 | na |
| OR Quart 3 p Value 95% CI of OR Quart3 | 1.2 | 1.5 | 0.73 | 0.83 | 1.4 | 1.6 | 1.4 | 1.4 | >3.2 |
| | 0.75 | 0.65 | 0.69 | 0.76 | 0.70 | 0.50 | 0.67 | 0.70 | <0.32 |
| | 0.35 | 0.25 | 0.15 | 0.25 | 0.29 | 0.41 | 0.29 | 0.29 | >0.32 |
| | 4.4 | 9.4 | 3.5 | 2.7 | 6.3 | 6.0 | 6.8 | 6.3 | na |
| OR Quart4 p Value 95% CI of OR Quart4 | 2.0 | 2.6 | 1.3 | 1.0 | 1.4 | 1.3 | 2.2 | 1.0 | >4.5 |
| | 0.27 | 0.26 | 0.73 | 1.0 | 0.70 | 0.70 | 0.28 | 1.0 | <0.19 |
| | 0.60 | 0.49 | 0.32 | 0.32 | 0.29 | 0.33 | 0.52 | 0.20 | >0.48 |
| | 6.4 | 14 | 5.1 | 3.2 | 6.3 | 5.3 | 9.7 | 5.1 | na |

102

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 26.7 | 87.0 | 26.7 | 75.5 | 26.7 | 56.9 |
| Average | 60.5 | 383 | 60.5 | 374 | 60.5 | 94.2 |
| Stdev | 127 | 783 | 127 | 799 | 127 | 92.9 |
| p(t-test) | | 4.4E-6 | | 1.1E-5 | | 0.29 |
| Min | 0.140 | 12.7 | 0.140 | 12.7 | 0.140 | 23.2 |
| Max | 1310 | 3480 | 1310 | 3480 | 1310 | 364 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 33.6 | 129 | 33.6 | 73.8 | 33.6 | 73.2 |
| Average | 85.0 | 254 | 85.0 | 239 | 85.0 | 114 |
| Stdev | 230 | 424 | 230 | 426 | 230 | 99.8 |
| p(t-test) | | 0.0090 | | 0.017 | | 0.66 |
| Min | 0.140 | 12.7 | 0.140 | 12.7 | 0.140 | 23.2 |
| Max | 2190 | 1710 | 2190 | 1710 | 2190 | 364 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 33.6 | 77.2 | 33.6 | 75.5 | 33.6 | 39.8 |
| Average | 59.7 | 422 | 59.7 | 424 | 59.7 | 67.7 |
| Stdev | 80.1 | 914 | 80.1 | 941 | 80.1 | 79.0 |
| p(t-test) | | 3.1E-6 | | 4.8E-6 | | 0.76 |
| Min | 0.140 | 13.4 | 0.140 | 13.4 | 0.140 | 13.4 |
| Max | 480 | 3480 | 480 | 3480 | 480 | 273 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.80 | 0.77 | 0.73 | 0.79 | 0.76 | 0.72 | 0.74 | 0.76 | 0.57 |
| SE | 0.052 | 0.072 | 0.070 | 0.054 | 0.073 | 0.073 | 0.071 | 0.082 | 0.097 |
| p | 7.1 E-9 | 1.5E-4 | 8.2E-4 | 8.8E-8 | 3.3E-4 | 0.0026 | 7.5E-4 | 0.0014 | 0.45 |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 | 54.4 | 56.0 | 42.9 | 54.4 | 56.0 | 42.9 | 45.3 | 56.0 | 25.6 |
| Sens 1 | 71% | 73% | 72% | 70% | 73% | 71% | 71% | 75% | 70% |
| Spec 1 | 75% | 72% | 57% | 75% | 72% | 57% | 66% | 72% | 41% |
| Cutoff 2 | 40.8 | 40.8 | 33.6 | 40.8 | 40.8 | 33.6 | 33.6 | 40.8 | 24.6 |
| Sens 2 | 82% | 80% | 83% | 81% | 80% | 82% | 82% | 83% | 80% |
| Spec 2 | 62% | 56% | 51% | 62% | 56% | 51% | 57% | 56% | 39% |
| Cutoff 3 | 25.6 | 33.6 | 24.6 | 25.6 | 33.6 | 24.6 | 24.6 | 33.6 | 22.0 |
| Sens 3 | 93% | 93% | 94% | 93% | 93% | 94% | 94% | 92% | 90% |
| Spec 3 | 49% | 50% | 39% | 49% | 50% | 39% | 46% | 50% | 36% |
| Cutoff | 48.2 | 54.5 | 53.4 | 48.2 | 54.5 | 53.4 | 48.2 | 54.5 | 53.4 |
| Sens 4 | 75% | 73% | 61% | 74% | 73% | 59% | 65% | 75% | 30% |
| Spec 4 | 70% | 70% | 70% | 70% | 70% | 70% | 70% | 70% | 70% |
| Cutoff 5 | 64.2 | 72.7 | 74.9 | 64.2 | 72.7 | 74.9 | 64.2 | 72.7 | 74.9 |
| Sens 5 | 64% | 67% | 56% | 63% | 60% | 53% | 47% | 58% | 20% |
| Spec 5 | 80% | 80% | 80% | 80% | 80% | 80% | 80% | 80% | 80% |
| Cutoff 6 | 125 | 133 | 128 | 125 | 133 | 128 | 125 | 133 | 128 |
| Sens 6 | 46% | 47% | 39% | 41% | 40% | 35% | 24% | 25% | 20% |
| Spec 6 | 91% | 90% | 90% | 91% | 90% | 90% | 91% | 90% | 90% |
| OR Quart 2 p Value 95% CI of OR Quart2 | 3.1 | 2.0 | 4.2 | 2.0 | 2.0 | 4.3 | >3.2 | >2.1 | 3.1 |
| | 0.33 | 0.57 | 0.21 | 0.58 | 0.57 | 0.20 | <0.32 | <0.56 | 0.34 |
| | 0.31 | 0.18 | 0.45 | 0.17 | 0.18 | 0.46 | >0.32 | >0.18 | 0.31 |
| | 31 | 23 | 39 | 23 | 23 | 40 | na | na | 31 |
| OR Quart 3 p Value 95% CI of OR Quart3 | 8.1 | 2.0 | 3.2 | 9.3 | 2.0 | 3.2 | >7.0 | >2.1 | 4.3 |
| | 0.055 | 0.57 | 0.33 | 0.039 | 0.57 | 0.33 | <0.077 | <0.56 | 0.20 |
| | 0.96 | 0.18 | 0.31 | 1.1 | 0.18 | 0.31 | >0.81 | >0.18 | 0.46 |
| | 69 | 23 | 32 | 78 | 23 | 32 | na | na | 41 |
| OR Quart 4 p Value 95% CI of OR Quart4 | 27 | 11 | 12 | 24 | 11 | 11 | >9.6 | >9.0 | 2.0 |
| | 0.0018 | 0.023 | 0.019 | 0.0027 | 0.023 | 0.027 | <0.037 | <0.041 | 0.58 |
| | 3.4 | 1.4 | 1.5 | 3.0 | 1.4 | 1.3 | >1.1 | >1.1 | 0.17 |
| | 220 | 90 | 100 | 190 | 90 | 90 | na | na | 23 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.56 | 2.45 | 1.56 | 2.48 | 1.56 | 2.48 |
| Average | 1.86 | 3.11 | 1.86 | 3.03 | 1.86 | 2.65 |
| Stdev | 2.43 | 2.60 | 2.43 | 2.72 | 2.43 | 1.27 |
| p(t-test) | | 0.015 | | 0.025 | | 0.19 |
| Min | 0.00230 | 0.00240 | 0.00230 | 0.00240 | 0.00230 | 0.00240 |
| Max | 23.1 | 11.6 | 23.1 | 11.6 | 23.1 | 4.42 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.72 | 2.48 | 1.72 | 2.48 | 1.72 | 2.48 |
| Average | 2.22 | 2.80 | 2.22 | 2.77 | 2.22 | 2.59 |
| Stdev | 3.19 | 1.30 | 3.19 | 1.33 | 3.19 | 1.55 |
| p(t-test) | | 0.49 | | 0.51 | | 0.70 |
| Min | 0.00230 | 0.00240 | 0.00230 | 0.00240 | 0.00230 | 0.00240 |
| Max | 28.3 | 4.42 | 28.3 | 4.42 | 28.3 | 4.42 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.56 | 2.09 | 1.56 | 2.17 | 1.56 | 2.29 |
| Average | 1.76 | 3.26 | 1.76 | 3.18 | 1.76 | 2.60 |
| Stdev | 1.81 | 3.09 | 1.81 | 3.28 | 1.81 | 1.09 |
| p(t-test) | | 0.0027 | | 0.0064 | | 0.15 |
| Min | 0.00230 | 0.00240 | 0.00230 | 0.00240 | 0.00230 | 1.11 |
| Max | 8.77 | 11.6 | 8.77 | 11.6 | 8.77 | 4.42 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.69 | 0.70 | 0.67 | 0.68 | 0.65 | 0.71 | 0.64 | 0.72 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.058 | 0.078 | 0.072 | 0.061 | 0.078 | 0.075 | 0.073 | 0.088 | 0.094 |
| p | 5.3E-4 | 0.015 | 0.0064 | 0.0050 | 0.020 | 0.040 | 0.0047 | 0.11 | 0.018 |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 | 1.87 | 1.97 | 1.87 | 1.62 | 1.74 | 1.74 | 1.97 | 1.97 | 1.97 |
| Sens 1 | 71% | 73% | 72% | 70% | 73% | 71% | 71% | 75% | 70% |
| Spec 1 | 64% | 58% | 65% | 57% | 52% | 62% | 66% | 58% | 66% |
| Cutoff 2 | 1.70 | 1.74 | 1.74 | 1.51 | 1.63 | 0.909 | 1.74 | 1.63 | 1.87 |
| Sens 2 | 82% | 80% | 83% | 81% | 80% | 82% | 82% | 83% | 80% |
| Spec 2 | 57% | 52% | 62% | 49% | 49% | 40% | 61% | 49% | 65% |
| Cutoff 3 | 0.761 | 1.51 | 0.761 | 0.0209 | 1.51 | 0.0209 | 1.03 | 0.0209 | 1.74 |
| Sens 3 | 93% | 93% | 94% | 93% | 93% | 94% | 94% | 92% | 90% |
| Spec 3 | 35% | 42% | 36% | 9% | 42% | 13% | 39% | 9% | 62% |
| Cutoff 4 | 2.41 | 2.48 | 2.35 | 2.41 | 2.48 | 2.35 | 2.41 | 2.48 | 2.35 |
| Sens 4 | 54% | 47% | 44% | 56% | 47% | 47% | 59% | 42% | 50% |
| Spec 4 | 71% | 72% | 70% | 71% | 72% | 70% | 71% | 72% | 70% |
| Cutoff 5 | 2.79 | 3.06 | 3.05 | 2.79 | 3.06 | 3.05 | 2.79 | 3.06 | 3.05 |
| Sens 5 | 39% | 47% | 28% | 41% | 47% | 29% | 35% | 42% | 30% |
| Spec 5 | 80% | 80% | 80% | 80% | 80% | 80% | 80% | 80% | 80% |
| Cutoff 6 | 3.77 | 3.96 | 4.06 | 3.77 | 3.96 | 4.06 | 3.77 | 3.96 | 4.06 |
| Sens 6 | 29% | 27% | 22% | 30% | 27% | 24% | 29% | 33% | 20% |
| Spec 6 | 91% | 90% | 90% | 91% | 90% | 90% | 91% | 90% | 90% |
| OR Quart 2 p Value 95% CI of OR Quart2 | 1.0 | 2.0 | 0.98 | 0.98 | 3.0 | 1.0 | 1.0 | 0.49 | >1.0 |
| | 1.0 | 0.57 | 0.99 | 0.98 | 0.34 | 1.0 | 1.0 | 0:57 | <1.0 |
| | 0.13 | 0.18 | 0.059 | 0.19 | 0.31 | 0.13 | 0.060 | 0.044 | >0.060 |
| | 7.4 | 23 | 16 | 5.1 | 30 | 7.5 | 17 | 5.6 | na |
| OR Quart 3 | 8.8 | 5.2 | 11 | 3.9 | 4.1 | 3.3 | 9.7 | 2.1 | >5.7 |
| | 0.0062 | 0.14 | 0.025 | 0.050 | 0.21 | 0.16 | 0.037 | 0.41 | <0.12 |
| p Value 95% CI of OR Quart3 | 1.9 | 0.59 | 1.3 | 1.00 | 0.45 | 0.63 | 1.2 | 0.37 | >0.64 |
| | 42 | 46 | 93 | 15 | 38 | 18 | 82 | 12 | na |
| OR Quart 4 p Value 95% CI of OR Quart4 | 7.0 | 7.5 | 8.0 | 4.4 | 7.5 | 3.9 | 8.0 | 2.6 | >4.3 |
| | 0.015 | 0.062 | 0.058 | 0.031 | 0.062 | 0.10 | 0.057 | 0.26 | <0.20 |
| | 1.5 | 0.90 | 0.94 | 1.1 | 0.90 | 0.76 | 0.94 | 0.49 | >0.46 |
| | 34 | 63 | 68 | 17 | 63 | 20 | 68 | 14 | na |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6560 | 29400 | 6560 | 19200 | 6560 | 10700 |
| Average | 26500 | 53100 | 26500 | 45900 | 26500 | 38500 |
| Stdev | 42900 | 61100 | 42900 | 57700 | 42900 | 62400 |
| p(t-test) | | 0.0016 | | 0.021 | | 0.24 |
| Min | 362 | 1980 | 362 | 1290 | 362 | 1790 |
| Max | 238000 | 227000 | 238000 | 227000 | 238000 | 227000 |
| n (Samp) | 203 | 36 | 203 | 34 | 203 | 22 |
| n (Patient) | 203 | 36 | 203 | 34 | 203 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8080 | 42800 | 8080 | 33800 | 8080 | 20200 |
| Average | 26200 | 62300 | 26200 | 56700 | 26200 | 45100 |
| Stdev | 39100 | 66000 | 39100 | 66100 | 39100 | 66500 |
| p(t-test) | | 1.3E-4 | | 0.0012 | | 0.077 |
| Min | 0.0130 | 1980 | 0.0130 | 1980 | 0.0130 | 3260 |
| Max | 238000 | 227000 | 238000 | 227000 | 238000 | 227000 |
| n (Samp) | 395 | 20 | 395 | 20 | 395 | 15 |
| n (Patient) | 395 | 20 | 395 | 20 | 395 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7540 | 22700 | 7540 | 16300 | 7540 | 7270 |
| Average | 28500 | 51300 | 28500 | 44500 | 28500 | 48600 |
| Stdev | 43600 | 62400 | 43600 | 57400 | 43600 | 70300 |
| p(t-test) | | 0.027 | | 0.13 | | 0.12 |
| Min | 362 | 2950 | 362 | 1290 | 362 | 1790 |
| Max | 238000 | 190000 | 238000 | 178000 | 238000 | 178000 |
| n (Samp) | 215 | 22 | 215 | 20 | 215 | 13 |
| n (Patient) | 215 | 22 | 215 | 20 | 215 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.70 | 0.66 | 0.66 | 0.67 | 0.62 | 0.58 | 0.63 | 0.55 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.052 | 0.067 | 0.066 | 0.054 | 0.068 | 0.069 | 0.067 | 0.079 | 0.085 |
| p | 1.9E-4 | 0.0023 | 0.015 | 0.0041 | 0.0097 | 0.085 | 0.20 | 0.11 | 0.56 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 10500 | 10700 | 7220 | 9350 | 10700 | 7220 | 5500 | 9440 | 4050 |
| Sens 1 | 72% | 70% | 73% | 71% | 70% | 70% | 73% | 73% | 77% |
| Spec 1 | 60% | 57% | 49% | 58% | 57% | 49% | 43% | 54% | 30% |
| Cutoff 2 | 5500 | 9440 | 4870 | 4480 | 5880 | 4480 | 4050 | 5880 | 3410 |
| Sens 2 | 81% | 80% | 82% | 82% | 80% | 80% | 82% | 80% | 85% |
| Spec 2 | 43% | 54% | 35% | 38% | 40% | 35% | 33% | 40% | 25% |
| Cutoff 3 | 4050 | 4410 | 4410 | 3120 | 4410 | 4050 | 3120 | 3410 | 2780 |
| Sens 3 | 92% | 90% | 91% | 91% | 90% | 90% | 91% | 93% | 92% |
| Spec 3 | 33% | 31% | 34% | 26% | 31% | 30% | 26% | 21% | 22% |
| Cutoff 4 | 22300 | 24500 | 26500 | 22300 | 24500 | 26500 | 22300 | 24500 | 26500 |
| Sens 4 | 56% | 60% | 45% | 47% | 55% | 40% | 32% | 40% | 31% |
| Spec 4 | 70% | 70% | 70% | 70% | 70% | 70% | 70% | 70% | 70% |
| Cutoff | 43300 | 42100 | 45300 | 43300 | 42100 | 45300 | 43300 | 42100 | 45300 |
| Sens 5 | 42% | 50% | 36% | 38% | 45% | 35% | 23% | 27% | 31% |
| Spec 5 | 80% | 80% | 80% | 80% | 80% | 80% | 80% | 80% | 80% |
| Cutoff 6 | 73500 | 73500 | 89000 | 73500 | 73500 | 89000 | 73500 | 73500 | 89000 |
| Sens 6 | 22% | 30% | 18% | 18% | 25% | 15% | 18% | 20% | 23% |
| Spec 6 | 90% | 90% | 90% | 90% | 90% | 90% | 90% | 90% | 90% |
| OR Quart 2 p Value 95% CI of OR Quart2 | 2.1 | 0.99 | 6.6 | 1.6 | 1.5 | 2.6 | 1.7 | 0.99 | 1.4 |
| | 0.32 | 0.99 | 0.086 | 0.51 | 0.66 | 0.27 | 0.47 | 0.99 | 0.70 |
| | 0.49 | 0.14 | 0.77 | 0.42 | 0.25 | 0.48 | 0.39 | 0.14 | 0.29 |
| | 8.7 | 7.2 | 56 | 5.8 | 9.2 | 14 | 7.6 | 7.2 | 6.4 |
| OR Quart 3 p Value 95% CI of OR Quart3 | 4.7 | 2.6 | 7.8 | 2.8 | 2.6 | 3.2 | 2.9 | 3.7 | 0.65 |
| | 0.022 | 0.27 | 0.058 | 0.098 | 0.27 | 0.17 | 0.13 | 0.11 | 0.65 |
| | 1.2 | 0.48 | 0.93 | 0.83 | 0.48 | 0.61 | 0.74 | 0.75 | 0.11 |
| | 18 | 13 | 66 | 9.5 | 13 | 16 | 12 | 18 | 4.1 |
| OR Quart 4 p Value | 6.2 | 6.0 | 8.9 | 4.2 | 5.4 | 3.8 | 2.1 | 2.0 | 1.4 |
| | 0.0059 | 0.022 | 0.042 | 0.017 | 0.033 | 0.11 | 0.32 | 0.42 | 0.70 |
| | 1.7 | 1.3 | 1.1 | 1.3 | 1.1 | 0.75 | 0.49 | 0.36 | 0.29 |
| 95% CI of OR Quart4 | 23 | 28 | 74 | 14 | 25 | 19 | 8.8 | 11 | 6.4 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.70 | 10.5 | 3.70 | 9.92 | 3.70 | 5.43 |
| Average | 5.74 | 15.9 | 5.74 | 15.2 | 15.74 | 9.29 |
| Stdev | 6.51 | 18.4 | 6.51 | 18.7 | 6.51 | 11.5 |
| p(t-test) | | 4.4E-7 | | 3.7E-6 | | 0.054 |
| Min | 0.123 | 0.945 | 0.123 | 0.945 | 0.123 | 0.376 |
| Max | 46.9 | 81.9 | 46.9 | 81.9 | 46.9 | 49.4 |
| n (Samp) | 147 | 28 | 147 | 27 | 147 | 17 |
| n (Patient) | 147 | 28 | 147 | 27 | 147 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.85 | 9.92 | 4.85 | 9.33 | 4.85 | 7.09 |
| Average | 9.22 | 15.2 | 9.22 | 14.6 | 9.22 | 11.9 |
| Stdev | 19.0 | 16.3 | 19.0 | 16.4 | 19.0 | 12.8 |
| p(t-test) | | 0.23 | | 0.29 | | 0.62 |
| Min | 0.123 | 1.42 | 0.123 | 1.42 | 0.123 | 3.19 |
| Max | 279 | 57.1 | 279 | 57.1 | 279 | 49.4 |
| n (Samp) | 285 | 15 | 285 | 15 | 285 | 12 |
| n (Patient) | 285 | 15 | 285 | 15 | 285 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.04 | 9.86 | 4.04 | 9.33 | 4.04 | 3.50 |
| Average | 6.57 | 13.9 | 6.57 | 13.2 | 6.57 | 4.19 |
| Stdev | 7.96 | 18.5 | 7.96 | 18.8 | 7.96 | 2.96 |
| p(t-test) | | 0.0025 | | 0.0077 | | 0.35 |
| Min | 0.123 | 0.945 | 0.123 | 0.945 | 0.123 | 0.376 |
| Max | 49.4 | 81.9 | 49.4 | 81.9 | 49.4 | 10.5 |
| n (Samp) | 151 | 18 | 151 | 17 | 151 | 10 |
| n (Patient) | 151 | 18 | 151 | 17 | 151 | 10 |

|  | 0hr prior to AKI stage | | | 24hr prior to AKI stage | | | 48hr prior to AKI stage | | |
| --- | --- | --- | --- | --- | --- | --- | --- | --- | --- |
|  | sCr or UO | sCr only | UO only | sCr or UO | sCr only | UO only | sCr or UO | sCr only | UO only |
| AUC | 0.75 | 0.69 | 0.68 | 0.74 | 0.67 | 0.66 | 0.64 | 0.66 | 0.44 |
| SE | 0.056 | 0.078 | 0.073 | 0.058 | 0.078 | 0.075 | 0.076 | 0.087 | 0.097 |
| p | 5.5E-6 | 0.014 | 0.014 | 3.8E-5 | 0.026 | 0.030 | 0.070 | 0.062 | 0.54 |
| nCohort 1 | 147 | 285 | 151 | 147 | 285 | 151 | 147 | 285 | 151 |
| nCohort 2 | 28 | 15 | 18 | 27 | 15 | 17 | 17 | 12 | 10 |
| Cutoff 1 | 5.34 | 6.73 | 3.80 | 5.34 | 6.73 | 3.80 | 4.51 | 5.34 | 2.31 |
| Sens 1 | 71% | 73% | 72% | 70% | 73% | 71% | 71% | 75% | 70% |
| Spec 1 | 65% | 61% | 49% | 65% | 61% | 49% | 59% | 54% | 27% |
| Cutoff 2 | 3.80 | 5.34 | 2.97 | 3.80 | 5.34 | 2.97 | 3.15 | 4.91 | 2.16 |
| Sens 2 | 82% | 80% | 83% | 81% | 80% | 82% | 82% | 83% | 80% |
| Spec 2 | 54% | 54% | 38% | 54% | 54% | 38% | 41% | 51% | 26% |
| Cutoff 3 | 2.16 | 3.15 | 2.16 | 2.16 | 3.15 | 2.16 | 1.99 | 4.60 | 1.99 |
| Sens 3 | 93% | 93% | 94% | 93% | 93% | 94% | 94% | 92% | 90% |
| Spec 3 | 30% | 36% | 26% | 30% | 36% | 26% | 27% | 49% | 25% |
| Cutoff 4 | 5.99 | 9.07 | 6.30 | 5.99 | 9.07 | 6.30 | 5.99 | 9.07 | 6.30 |
| Sens 4 | 68% | 60% | 61% | 67% | 53% | 59% | 47% | 42% | 20% |
| Spec 4 | 70% | 70% | 70% | 70% | 70% | 70% | 70% | 70% | 70% |
| Cutoff 5 | 8.29 | 12.5 | 8.53 | 8.29 | 12.5 | 8.53 | 8.29 | 12.5 | 8.53 |
| Sens 5 | 61% | 33% | 56% | 56% | 27% | 53% | 29% | 25% | 10% |
| Spec 5 | 80% | 80% | 80% | 80% | 80% | 80% | 80% | 80% | 80% |
| Cutoff 6 | 13.0 | 19.4 | 13.6 | 13.0 | 19.4 | 13.6 | 13.0 | 19.4 | 13.6 |
| Sens 6 | 36% | 20% | 28% | 30% | 20% | 24% | 18% | 17% | 0% |
| Spec 6 | 90% | 90% | 91% | 90% | 90% | 91% | 90% | 90% | 91% |
| OR Quart 2 p Value 95% CI of OR Quart2 | 2.0 | 2.0 | 2.1 | 2.0 | 2.0 | 2.1 | 3.2 | >3.1 | 1.0 |
|  | 0.42 | 0.57 | 0.41 | 0.42 | 0.57 | 0.41 | 0.33 | <0.33 | 0.98 |
|  | 0.36 | 0.18 | 0.36 | 0.36 | 0.18 | 0.36 | 0.31 | >0.32 | 0.14 |
|  | 12 | 23 | 12 | 12 | 23 | 12 | 32 | na | 7.7 |
| OR Quart 3 p Value 95% CI of OR Quart3 | 2.6 | 6.4 | 1.0 | 3.3 | 7.6 | 1.0 | 8.2 | >6.5 | 2.2 |
|  | 0.26 | 0.089 | 1.0 | 0.16 | 0.061 | 1.0 | 0.054 | <0.086 | 0.39 |
|  | 0.48 | 0.76 | 0.13 | 0.63 | 0.91 | 0.13 | 0.96 | >0.77 | 0.37 |
|  | 14 | 55 | 7.5 | 17 | 64 | 7.5 | 70 | na | 13 |
| OR Quart 4 p Value 95% CI of OR Quart4 | 13 | 6.4 | 6.1 | 11 | 5.3 | 5.5 | 6.9 | >3.1 | 1.0 |
|  | 0.0012 | 0.089 | 0.026 | 0.0028 | 0.13 | 0.038 | 0.081 | <0.33 | 0.98 |
|  | 2.8 | 0.76 | 1.2 | 2.3 | 0.60 | 1.1 | 0.79 | >0.31 | 0.14 |
|  | 60 | 55 | 30 | 50 | 46 | 27 | 60 | na | 7.7 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000114 | 0.000197 | 0.000114 | 0.000197 | 0.000114 | 0.000732 |
| Average | 0.0104 | 0.108 | 0.0104 | 0.0505 | 0.0104 | 0.0493 |
| Stdev | 0.0383 | 0.260 | 0.0383 | 0.143 | 0.0383 | 0.138 |
| p(t-test) | | 1.6E-5 | | 0.0032 | | 0.0070 |
| Min | 5.62E-6 | 1.88E-5 | 5.62E-6 | 1.66E-5 | 5.62E-6 | 1.66E-5 |
| Max | 0.260 | 1.02 | 0.260 | 0.560 | 0.260 | 0.560 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.000114 | 0.00341 | 0.000114 | 0.000732 | 0.000114 | 0.00127 |
| Average | 0.0111 | 0.159 | 0.0111 | 0.0551 | 0.0111 | 0.0642 |
| Stdev | 0.0451 | 0.324 | 0.0451 | 0.151 | 0.0451 | 0.162 |
| p(t-test) | | 5.1E-12 | | 0.0012 | | 5.8E-4 |
| Min | 5.62E-6 | 2.96E-5 | 5.62E-6 | 1.66E-5 | 5.62E-6 | 2.96E-5 |
| Max | 0.470 | 1.02 | 0.470 | 0.560 | 0.470 | 0.560 |
| n (Samp) | 317 | 18 | 317 | 18 | 317 | 13 |
| n (Patient) | 317 | 18 | 317 | 18 | 317 | 13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.51E-5 | 4.39E-5 | 6.51E-5 | 3.88E-5 | 6.51E-5 | 0.000118 |
| Average | 0.0127 | 0.0848 | 0.0127 | 0.0908 | 0.0127 | 0.00682 |
| Stdev | 0.0478 | 0.251 | 0.0478 | 0.265 | 0.0478 | 0.0142 |
| p(t-test) | | 0.0011 | | 7.8E-4 | | 0.70 |
| Min | 5.62E-6 | 1.88E-5 | 5.62E-6 | 1.88E-5 | 5.62E-6 | 1.66E-5 |
| Max | 0.359 | 1.02 | 0.359 | 1.02 | 0.359 | 0.0446 |
| n (Samp) | 172 | 19 | 172 | 17 | 172 | 10 |
| n (Patient) | 172 | 19 | 172 | 17 | 172 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.68 | 0.55 | 0.55 | 0.62 | 0.51 | 0.60 | 0.63 | 0.54 |
| SE | 0.057 | 0.071 | 0.071 | 0.059 | 0.072 | 0.074 | 0.074 | 0.085 | 0.096 |
| p | 0.052 | 0.012 | 0.47 | 0.36 | 0.10 | 0.94 | 0.18 | 0.14 | 0.70 |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 3.44E-5 | 8.63E-5 | 2.83E-5 | 3.21E-5 | 3.21E-5 | 2.83E-5 | 3.21 E-5 | 2.83E-5 | 2.83E-5 |
| Sens 1 | 72% | 72% | 79% | 70% | 72% | 76% | 72% | 100% | 90% |
| Spec 1 | 36% | 49% | 29% | 34% | 35% | 29% | 34% | 26% | 29% |
| Cutoff 2 | 2.83E-5 | 2.83E-5 | 2.68E-5 | 2.83E-5 | 2.83E-5 | 2.53E-5 | 2.83E-5 | 2.83E-5 | 2.83E-5 |
| Sens 2 | 88% | 100% | 84% | 83% | 94% | 82% | 94% | 100% | 90% |
| Spec 2 | 25% | 26% | 26% | 25% | 26% | 21% | 25% | 26% | 29% |
| Cutoff 3 | 2.68E-5 | 2.83E-5 | 2.46E-5 | 2.46E-5 | 2.83E-5 | 2.38E-5 | 2.83E-5 | 2.83E-5 | 2.83E-5 |
| Sens 3 | 91% | 100% | 95% | 90% | 94% | 94% | 94% | 100% | 90% |
| Spec 3 | 22% | 26% | 16% | 13% | 26% | 14% | 25% | 26% | 29% |
| Cutoff 4 | 0.00146 | 0.00127 | 0.00121 | 0.00146 | 0.00127 | 0.00121 | 0.00146 | 0.00127 | 0.00121 |
| Sens 4 | 41% | 50% | 32% | 37% | 44% | 24% | 44% | 46% | 30% |
| Spec 4 | 70% | 70% | 70% | 70% | 70% | 70% | 70% | 70% | 70% |
| Cutoff 5 | 0.00836 | 0.00672 | 0.00590 | 0.00836 | 0.00672 | 0.00590 | 0.00836 | 0.00672 | 0.00590 |
| Sens 5 | 31% | 44% | 26% | 27% | 39% | 24% | 28% | 46% | 30% |
| Spec 5 | 80% | 80% | 80% | 80% | 80% | 80% | 80% | 80% | 80% |
| Cutoff 6 | 0.0157 | 0.0171 | 0.0157 | 0.0157 | 0.0171 | 0.0157 | 0.0157 | 0.0171 | 0.0157 |
| Sens 6 | 25% | 28% | 21% | 17% | 17% | 18% | 17% | 15% | 10% |
| Spec 6 | 90% | 91% | 90% | 90% | 91% | 90% | 90% | 91% | 90% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 2.5 | >5.3 | 2.5 | 1.7 | 5.2 | 1.6 | 1.4 | >5.3 | 4.2 |
| | 0.15 | <0.13 | 0.20 | 0.37 | 0.14 | 0.51 | 0.69 | <0.13 | 0.21 |
| | 0.71 | >0.60 | 0.61 | 0.52 | 0.59 | 0.41 | 0.29 | >0.60 | 0.45 |
| | 8.8 | na | 10 | 5.8 | 45 | 6.0 | 6.5 | na | 39 |
| OR Quart 3 p Value 95% CI ot OR Quart3 | 1.8 | >4.2 | 1.3 | 1.2 | 4.1 | 0.73 | 1.0 | >2.0 | 2.0 |
| | 0.36 | <0.21 | 0.72 | 0.75 | 0.21 | 0.70 | 1.0 | <0.56 | 0.56 |
| | 0.50 | >0.45 | 0.28 | 0.35 | 0.45 | 0.15 | 0.19 | >0.18 | 0.18 |
| | 6.8 | na | 6.3 | 4.4 | 37 | 3.5 | 5.3 | na | 23 |
| OR Quart 4 p Value 95% CI of OR Quart4 | 3.6 | >10.0 | 1.7 | 2.5 | 8.6 | 0.98 | 3.0 | >6.4 | 3.1 |
| | 0.039 | <0.031 | 0.48 | 0.12 | 0.044 | 0.98 | 0.13 | <0.089 | 0.34 |
| | 1.1 | >1.2 | 0.38 | 0.80 | 1.1 | 0.23 | 0.73 | >0.75 | 0.31 |
| | 12 | na | 7.6 | 7.9 | 71 | 4.2 | 12 | na | 31 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0243 | 0.124 | 0.0243 | 0.0612 | 0.0243 | 0.0328 |
| Average | 0.188 | 0.546 | 0.188 | 0.331 | 0.188 | 0.176 |
| Stdev | 0.436 | 1.20 | 0.436 | 0.496 | 0.436 | 0.298 |
| p(t-test) | | 0.0044 | | 0.11 | | 0.91 |
| Min | 7.25E-6 | 0.00670 | 7.25E-6 | 0.00673 | 7.25E-6 | 0.00644 |
| Max | 3.07 | 6.31 | 3.07 | 1.59 | 3.07 | 1.07 |
| n (Samp) | 151 | 32 | 151 | 30 | 151 | 18 |
| n (Patient) | 151 | 32 | 151 | 30 | 151 | 18 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0253 | 0.0532 | 0.0253 | 0.0532 | 0.0253 | 0.0317 |
| Average | 0.284 | 0.556 | 0.284 | 0.290 | 0.284 | 0.139 |
| Stdev | 0.789 | 1.49 | 0.789 | 0.496 | 0.789 | 0.228 |
| p(t-test) | | 0.18 | | 0.98 | | 0.51 |
| Min | 6.78E-7 | 0.00928 | 6.78E-7 | 0.00804 | 6.78E-7 | 0.00804 |
| Max | 8.92 | 6.31 | 8.92 | 1.56 | 8.92 | 0.725 |
| n (Samp) | 317 | 18 | 317 | 18 | 317 | 13 |
| n (Patient) | 317 | 18 | 317 | 18 | 317 | 13 |

| UO only | 0hr prior to AKI stage | | 24hr prior to AKI stage | | 48hr prior to AKI stage | |
|---|---|---|---|---|---|---|
| | Cohort I | Cohort 2 | Cohort 1 | Cohort 2 | Cohort 1 | Cohort 2 |
| Median | 0.0258 | 0.226 | 0.0258 | 0.0598 | 0.0258 | 0.0249 |
| Average | 0.235 | 0.481 | 0.235 | 0.377 | 0.235 | 0.299 |
| Stdev | 0.505 | 0.718 | 0.505 | 0.546 | 0.505 | 0.551 |
| p(t-test) | | 0.056 | | 0.28 | | 0.70 |
| Min | 7.25E-6 | 0.00670 | 7.25E-6 | 0.00673 | 7.25E-6 | 0.00644 |
| Max | 3.07 | 2.69 | 3.07 | 1.59 | 3.07 | 1.56 |
| n (Samp) | 172 | 19 | 172 | 17 | 172 | 10 |
| n (Patient) | 172 | 19 | 172 | 17 | 172 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | 0.64 | 0.67 | 0.66 | 0.63 | 0.63 | 0.57 | 0.54 | 0.54 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.055 | 0.072 | 0.071 | 0.058 | 0.072 | 0.075 | 0.074 | 0.083 | 0.096 |
| p | 6.1E-4 | 0.048 | 0.016 | 0.0055 | 0.076 | 0.079 | 0.36 | 0.65 | 0.70 |
| nCohort 1 | 151 | 317 | 172 | 151 | 317 | 172 | 151 | 317 | 172 |
| nCohort 2 | 32 | 18 | 19 | 30 | 18 | 17 | 18 | 13 | 10 |
| Cutoff 1 | 0.0317 | 0.0317 | 0.0313 | 0.0317 | 0.0317 | 0.0313 | 0.0165 | 0.0148 | 0.0165 |
| Sens 1 | 72% | 72% | 74% | 70% | 72% | 71% | 72% | 77% | 70% |
| Spec 1 | 58% | 56% | 55% | 58% | 56% | 55% | 42% | 36% | 40% |
| Cutoff 2 | 0.0191 | 0.0191 | 0.0165 | 0.0171 | 0.0181 | 0.0165 | 0.0127 | 0.0134 | 0.0130 |
| Sens 2 | 81% | 83% | 84% | 80% | 83% | 82% | 83% | 85% | 80% |
| Spec 2 | 44% | 42% | 40% | 42% | 41% | 40% | 33% | 32% | 31% |
| Cutoff 3 | 0.0147 | 0.0148 | 0.00670 | 0.00912 | 0.00926 | 0.00766 | 0.00776 | 0.00926 | 0.00956 |
| Sens 3 | 91% | 94% | 95% | 90% | 94% | 94% | 94% | 92% | 90% |
| Spec 3 | 37% | 36% | 19% | 26% | 23% | 22% | 24% | 23% | 24% |
| Cutoff 4 | 0.0934 | 0.0850 | 0.144 | 0.0934 | 0.0850 | 0.144 | 0.0934 | 0.0850 | 0.144 |
| Sens 4 | 50% | 39% | 58% | 40% | 39% | 41% | 28% | 31% | 30% |
| Spec 4 | 70% | 70% | 70% | 70% | 70% | 70% | 70% | 70% | 70% |
| Cutoff 5 | 0.221 | 0.255 | 0.295 | 0.221 | 0.255 | 0.295 | 0.221 | 0.255 | 0.295 |
| Sens 5 | 44% | 28% | 42% | 33% | 28% | 35% | 22% | 23% | 20% |
| Spec 5 | 80% | 80% | 80% | 80% | 80% | 80% | 80% | 80% | 80% |
| Cutoff 6 | 0.514 | 0.740 | 0.719 | 0.514 | 0.740 | 0.719 | 0.514 | 0.740 | 0.719 |
| Sens 6 | 22% | 11% | 21% | 23% | 11% | 24% | 11% | 0% | 20% |
| Spec 6 | 90% | 90% | 90% | 90% | 90% | 90% | 90% | 90% | 90% |
| OR Quart 2 p Value 95% CI of OR Quart2 | 1.7 | 4.1 | 0.64 | 1.0 | 1.5 | 0.65 | 3.3 | 2.0 | 1.5 |
| | 0.48 | 0.21 | 0.63 | 1.0 | 0.66 | 0.65 | 0.16 | 0.42 | 0.67 |
| | 0.38 | 0.45 | 0.10 | 0.23 | 0.24 | 0.10 | 0.63 | 0.36 | 0.24 |
| | 7.6 | 37 | 4.0 | 4.3 | 9.2 | 4.1 | 18 | 11 | 9.4 |
| OR Quart 3 p Value 95% CI of OR Quart3 | 3.4 | 8.6 | 1.7 | 3.3 | 4.3 | 1.7 | 2.7 | 2.1 | 1.0 |
| | 0.082 | 0.044 | 0.48 | 0.056 | 0.072 | 0.46 | 0.25 | 0.41 | 1.0 |
| | 0.86 | 1.1 | 0.38 | 0.97 | 0.88 | 0.39 | 0.49 | 0.37 | 0.13 |
| | 14 | 71 | 7.6 | 11 | 21 | 7.8 | 15 | 12 | 7.4 |
| OR Quart 4 p Value 95% CI of OR Quart4 | 6.8 | 5.2 | 3.4 | 3.2 | 2.6 | 2.5 | 2.6 | 1.5 | 1.5 |
| | 0.0046 | 0.14 | 0.082 | 0.062 | 0.27 | 0.20 | 0.26 | 0.66 | 0.67 |
| | 1.8 | 0.59 | 0.85 | 0.94 | 0.48 | 0.61 | 0.48 | 0.24 | 0.24 |
| | 25 | 45 | 13 | 11 | 14 | 10 | 14 | 9.2 | 9.4 |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 150 | 147 | 150 | 148 | 150 | 142 |
| Average | 156 | 163 | 156 | 161 | 156 | 148 |
| Stdev | 51.8 | 74.3 | 51.8 | 77.4 | 51.8 | 46.6 |
| p(t-test) | | 0.57 | | 0.69 | | 0.55 |
| Min | 38.4 | 46.0 | 38.4 | 46.0 | 38.4 | 76.6 |
| Max | 311 | 444 | 311 | 444 | 311 | 241 |
| n (Samp) | 145 | 28 | 145 | 27 | 145 | 17 |
| n (Patient) | 145 | 28 | 145 | 27 | 145 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 151 | 170 | 151 | 170 | 151 | 156 |
| Average | 160 | 166 | 160 | 162 | 160 | 152 |
| Stdev | 62.6 | 52.1 | 62.6 | 56.8 | 62.6 | 53.9 |
| p(t-test) | | 0.69 | | 0.89 | | 0.67 |
| Min | 38.4 | 102 | 38.4 | 76.6 | 38.4 | 76.6 |
| Max | 444 | 265 | 444 | 265 | 444 | 241 |
| n (Samp) | 284 | 15 | 284 | 15 | 284 | 12 |
| n (Patient) | 284 | 15 | 284 | 15 | 284 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 153 | 144 | 153 | 142 | 153 | 127 |
| Average | 156 | 153 | 156 | 154 | 156 | 139 |
| Stdev | 52.4 | 87.0 | 52.4 | 89.7 | 52.4 | 46.9 |
| p(t-test) | | 0.87 | | 0.90 | | 0.33 |
| Min | 38.4 | 46.0 | 38.4 | 46.0 | 38.4 | 82.1 |
| Max | 311 | 444 | 311 | 444 | 311 | 241 |
| n (Samp) | 149 | 18 | 149 | 17 | 149 | 10 |
| n (Patient) | 149 | 18 | 149 | 17 | 149 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.55 | 0.44 | 0.50 | 0.52 | 0.44 | 0.45 | 0.48 | 0.40 |
| SE | 0.060 | 0.078 | 0.074 | 0.061 | 0.078 | 0.076 | 0.076 | 0.086 | 0.098 |
| p | 0.86 | 0.52 | 0.43 | 0.97 | 0.75 | 0.44 | 0.54 | 0.80 | 0.30 |
| nCohort 1 | 145 | 284 | 149 | 145 | 284 | 149 | 145 | 284 | 149 |
| nCohort 2 | 28 | 15 | 18 | 27 | 15 | 17 | 17 | 12 | 10 |
| Cutoff 1 | 131 | 128 | 110 | 128 | 118 | 110 | 119 | 105 | 119 |
| Sens 1 | 71% | 73% | 72% | 70% | 73% | 71% | 71% | 75% | 70% |
| Spec 1 | 36% | 35% | 22% | 32% | 29% | 22% | 25% | 22% | 28% |
| Cutoff 2 | 110 | 118 | 102 | 105 | 105 | 102 | 105 | 104 | 105 |
| Sens 2 | 82% | 80% | 83% | 81% | 80% | 82% | 82% | 83% | 80% |
| Spec 2 | 20% | 29% | 15% | 18% | 22% | 15% | 18% | 21% | 21% |
| Cutoff 3 | 102 | 104 | 46.0 | 74.0 | 102 | 46.0 | 94.6 | 94.8 | 94.6 |
| Sens 3 | 93% | 93% | 94% | 93% | 93% | 94% | 94% | 92% | 90% |
| Spec 3 | 13% | 21% | 1% | 3% | 19% | 1% | 10% | 14% | 12% |
| Cutoff 4 | 178 | 186 | 183 | 178 | 186 | 183 | 178 | 186 | 183 |
| Sens 4 | 32% | 27% | 17% | 33% | 27% | 18% | 24% | 17% | 10% |
| Spec 4 | 70% | 70% | 70% | 70% | 70% | 70% | 70% | 70% | 70% |
| Cutoff 5 | 194 | 212 | 198 | 194 | 212 | 198 | 194 | 212 | 198 |
| Sens 5 | 14% | 20% | 11% | 15% | 20% | 12% | 12% | 17% | 10% |
| Spec 5 | 80% | 80% | 81% | 80% | 80% | 81% | 80% | 80% | 81% |
| Cutoff 6 | 220 | 247 | 220 | 220 | 247 | 220 | 220 | 247 | 220 |
| Sens 6 | 14% | 7% | 11% | 15% | 7% | 12% | 12% | 0% | 10% |
| Spec 6 | 90% | 90% | 91% | 90% | 90% | 91% | 90% | 90% | 91% |
| OR Quart 2 p Value 95% CI of OR Quart2 | 1.2 | 1.3 | 2.7 | 1.7 | 0.73 | 2.8 | 2.8 | 2.1 | 2.1 |
| | 0.78 | 0.71 | 0.25 | 0.37 | 0.69 | 0.24 | 0.24 | 0.41 | 0.56 |
| | 0.39 | 0.29 | 0.49 | 0.52 | 0.16 | 0.51 | 0.51 | 0.37 | 0.18 |
| | 3.6 | 6.2 | 15 | 5.8 | 3.4 | 15 | 15 | 12 | 24 |
| OR Quart 3 | 1.0 | 1.7 | 2.7 | 1.5 | 1.2 | 2.1 | 3.3 | 1.0 | 4.3 |
| | 1.0 | 0.48 | 0.25 | 0.54 | 0.75 | 0.41 | 0.16 | 1.0 | 0.20 |
| p Value 95% CI of OR Quart3 | 0.32 | 0.39 | 0.49 | 0.43 | 0.32 | 0.36 | 0.63 | 0.14 | 0.46 |
| | 3.1 | 7.3 | 15 | 5.1 | 4.9 | 12 | 18 | 7.3 | 41 |
| OR Quart 4 p Value 95% CI of OR Quart4 | 0.81 | 0.99 | 3.4 | 1.5 | 0.73 | 3.4 | 2.2 | 2.1 | 3.2 |
| | 0.73 | 0.99 | 0.15 | 0.54 | 0.69 | 0.15 | 0.39 | 0.41 | 0.32 |
| | 0.25 | 0.19 | 0.65 | 0.43 | 0.16 | 0.65 | 0.37 | 0.37 | 0.32 |
| | 2.6 | 5.1 | 18 | 5.1 | 3.4 | 18 | 13 | 12 | 33 |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1570 | 3440 | 1570 | 2270 | 1570 | 1780 |
| Average | 2500 | 8940 | 2500 | 8470 | 2500 | 6780 |
| Stdev | 3600 | 17000 | 3600 | 17500 | 3600 | 16800 |
| p(t-test) | | 2.1E-6 | | 1.8E-5 | | 0.0023 |
| Min | 33.0 | 90.7 | 33.0 | 90.7 | 33.0 | 201 |
| Max | 33000 | 80100 | 33000 | 80100 | 33000 | 80100 |
| n (Samp) | 203 | 36 | 203 | 34 | 203 | 22 |
| n (Patient) | 203 | 36 | 203 | 34 | 203 | 22 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1810 | 2100 | 1810 | 1980 | 1810 | 1940 |
| Average | 3180 | 7650 | 3180 | 7290 | 3180 | 8250 |
| Stdev | 5450 | 17500 | 5450 | 17400 | 5450 | 20200 |
| p(t-test) | | 0.0029 | | 0.0061 | | 0.0033 |
| Min | 0.0376 | 90.7 | 0.0376 | 90.7 | 0.0376 | 890 |
| Max | 68100 | 80100 | 68100 | 80100 | 68100 | 80100 |
| n (Samp) | 395 | 20 | 395 | 20 | 395 | 15 |
| n (Patient) | 395 | 20 | 395 | 20 | 395 | 15 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1720 | 2890 | 1720 | 1950 | 1720 | 1250 |
| Average | 2700 | 8160 | 2700 | 7650 | 2700 | 2540 |
| Stdev | 3680 | 14700 | 3680 | 15500 | 3680 | 3590 |
| p(t-test) | | 2.1E-5 | | 2.3E-4 | | 0.88 |
| Min | 33.0 | 201 | 33.0 | 201 | 33.0 | 201 |
| Max | 33000 | 68100 | 33000 | 68100 | 33000 | 12300 |
| n (Samp) | 215 | 22 | 215 | 20 | 215 | 13 |
| n (Patient) | 215 | 22 | 215 | 20 | 215 | 13 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.71 | 0.64 | 0.63 | 0.66 | 0.61 | 0.58 | 0.60 | 0.58 | 0.43 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.051 | 0.068 | 0.066 | 0.054 | 0.068 | 0.069 | 0.067 | 0.078 | 0.085 |
| p | 5.2E-5 | 0.045 | 0.043 | 0.0023 | 0.10 | 0.24 | 0.15 | 0.33 | 0.42 |
| nCohort 1 | 203 | 395 | 215 | 203 | 395 | 215 | 203 | 395 | 215 |
| nCohort 2 | 36 | 20 | 22 | 34 | 20 | 20 | 22 | 15 | 13 |
| Cutoff 1 | 1760 | 1810 | 1220 | 1460 | 1570 | 1220 | 1170 | 1240 | 813 |
| Sens 1 | 72% | 70% | 73% | 71% | 70% | 70% | 73% | 73% | 77% |
| Spec 1 | 56% | 50% | 39% | 47% | 43% | 39% | 42% | 36% | 27% |
| Cutoff 2 | 1270 | 1660 | 952 | 1190 | 1450 | 952 | 952 | 1170 | 525 |
| Sens 2 | 81% | 80% | 82% | 82% | 80% | 80% | 82% | 80% | 85% |
| Spec 2 | 45% | 46% | 30% | 44% | 41% | 30% | 34% | 34% | 16% |
| Cutoff 3 | 813 | 1240 | 525 | 813 | 1020 | 525 | 813 | 952 | 277 |
| Sens 3 | 92% | 90% | 91% | 91% | 90% | 90% | 91% | 93% | 92% |
| Spec 3 | 31% | 36% | 16% | 31% | 29% | 16% | 31% | 28% | 10% |
| Cutoff 4 | 2360 | 2910 | 2690 | 2360 | 2910 | 2690 | 2360 | 2910 | 2690 |
| Sens 4 | 56% | 45% | 55% | 47% | 45% | 40% | 36% | 33% | 23% |
| Spec 4 | 70% | 70% | 70% | 70% | 70% | 70% | 70% | 70% | 70% |
| Cutoff 5 | 3330 | 4190 | 3610 | 3330 | 4190 | 3610 | 3330 | 4190 | 3610 |
| Sens 5 | 50% | 40% | 45% | 44% | 40% | 35% | 32% | 27% | 15% |
| Spec 5 | 80% | 80% | 80% | 80% | 80% | 80% | 80% | 80% | 80% |
| Cutoff 6 | 4930 | 6560 | 5440 | 4930 | 6560 | 5440 | 4930 | 6560 | 5440 |
| Sens 6 | 44% | 15% | 41% | 35% | 15% | 25% | 27% | 13% | 15% |
| Spec 6 | 90% | 90% | 90% | 90% | 90% | 90% | 90% | 90% | 90% |
| OR Quart 2 p Value 95% CI of OR Quart2 | 2.5 | 6.2 | 2.5 | 3.4 | 8.5 | 2.5 | 3.9 | >7.4 | 1.0 |
| | 0.21 | 0.093 | 0.20 | 0.081 | 0.046 | 0.21 | 0.10 | <0.063 | 1.0 |
| | 0.61 | 0.74 | 0.62 | 0.86 | 1.0 | 0.61 | 0.76 | >0.90 | 0.14 |
| | 10 | 53 | 10 | 13 | 69 | 10 | 19 | na | 7.4 |
| OR Quart 3 p Value 95% CI of OR Quart3 | 2.9 | 4.1 | 0.65 | 2.5 | 2.0 | 0.98 | 3.2 | >3.1 | 3.2 |
| | 0.13 | 0.21 | 0.65 | 0.20 | 0.57 | 0.98 | 0.16 | <0.33 | 0.16 |
| | 0.72 | 0.45 | 0.11 | 0.62 | 0.18 | 0.19 | 0.62 | >0.32 | 0.62 |
| | 11 | 37 | 4.1 | 10 | 22 | 5.1 | 17 | na | 17 |
| OR Quart 4 p Value | 8.0 | 9.7 | 3.7 | 6.2 | 9.7 | 2.5 | 3.8 | >5.2 | 1.5 |
| | 0.0015 | 0.033 | 0.055 | 0.0059 | 0.033 | 0.21 | 0.11 | <0.14 | 0.65 |
| | 2.2 | 1.2 | 0.97 | 1.7 | 1.2 | 0.61 | 0.75 | >0.60 | 0.25 |
| 95% CI of OR Quart4 | 29 | 78 | 14 | 23 | 78 | 10 | 19 | na | 9.5 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 13.9 | 20.6 | 13.9 | 20.6 | 13.9 | 18.2 |
| Average | 21.5 | 298 | 21.5 | 304 | 21.5 | 20.3 |
| Stdev | 43.9 | 1420 | 43.9 | 1450 | 43.9 | 19.3 |
| p(t-test) | | 0.018 | | 0.017 | | 0.92 |
| Min | 0.0190 | 0.00398 | 0.0190 | 0.00398 | 0.0190 | 0.0190 |
| Max | 470 | 7540 | 470 | 7540 | 470 | 69.1 |
| n (Samp) | 148 | 28 | 148 | 27 | 148 | 17 |
| n (Patient) | 148 | 28 | 148 | 27 | 148 | 17 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 16.2 | 20.6 | 16.2 | 20.6 | 16.2 | 18.2 |
| Average | 54.1 | 23.7 | 54.1 | 23.7 | 54.1 | 20.7 |
| Stdev | 447 | 19.8 | 447 | 19.8 | 447 | 19.8 |
| p(t-test) | | 0.79 | | 0.79 | | 0.80 |
| Min | 0.00398 | 0.338 | 0.00398 | 0.338 | 0.00398 | 0.338 |
| Max | 7540 | 69.1 | 7540 | 69.1 | 7540 | 69.1 |
| n (Samp) | 288 | 15 | 288 | 15 | 288 | 12 |
| n (Patient) | 288 | 15 | 288 | 15 | 288 | 12 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.2 | 20.6 | 15.2 | 20.6 | 15.2 | 13.0 |
| Average | 28.8 | 448 | 28.8 | 466 | 28.8 | 17.5 |
| Stdev | 86.4 | 1770 | 86.4 | 1820 | 86.4 | 17.8 |
| p(t-test) | | 0.0035 | | 0.0031 | | 0.68 |
| Min | 0.0190 | 0.00398 | 0.0190 | 0.00398 | 0.0190 | 0.0190 |
| Max | 945 | 7540 | 945 | 7540 | 945 | 46.6 |
| n (Samp) | 152 | 18 | 152 | 17 | 152 | 10 |
| n (Patient) | 152 | 18 | 152 | 17 | 152 | 10 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.55 | 0.59 | 0:62 | 0.55 | 0.57 | 0.55 | 0.50 | 0.47 |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| SE | 0.060 | 0.078 | 0.074 | 0.062 | 0.078 | 0.076 | 0.076 | 0.085 | 0.096 |
| p | 0.025 | 0.55 | 0.21 | 0.046 | 0.55 | 0.35 | 0.51 | 0.98 | 0.73 |
| nCohort 1 | 148 | 288 | 152 | 148 | 288 | 152 | 148 | 288 | 152 |
| nCohort 2 | 28 | 15 | 18 | 27 | 15 | 17 | 17 | 12 | 10 |
| Cutoff 1 | 14.8 | 8.27 | 10.0 | 14.8 | 8.27 | 10.0 | 6.41 | 7.00 | 6.24 |
| Sens 1 | 71% | 73% | 72% | 70% | 73% | 71% | 71% | 75% | 70% |
| Spec 1 | 55% | 27% | 36% | 55% | 27% | 36% | 33% | 26% | 30% |
| Cutoff 2 | 6.24 | 7.00 | 0.103 | 6.24 | 7.00 | 0.103 | 0.103 | 6.36 | 0.103 |
| Sens 2 | 82% | 80% | 89% | 81% | 80% | 88% | 88% | 83% | 80% |
| Spec 2 | 33% | 26% | 11% | 33% | 26% | 11% | 12% | 26% | 11% |
| Cutoff 3 | 0.103 | 0.103 | 0.0840 | 0.103 | 0.103 | 0.0840 | 0.0840 | 0.103 | 0.0840 |
| Sens 3 | 93% | 100% | 94% | 93% | 100% | 94% | 94% | 100% | 90% |
| Spec 3 | 12% | 10% | 10% | 12% | 10% | 10% | 10% | 10% | 10% |
| Cutoff 4 | 19.8 | 26.2 | 22.0 | 19.8 | 26.2 | 22.0 | 19.8 | 26.2 | 22.0 |
| Sens 4 | 61% | 33% | 44% | 59% | 33% | 41% | 47% | 25% | 30% |
| Spec 4 | 70% | 70% | 70% | 70% | 70% | 70% | 70% | 70% | 70% |
| Cutoff 5 | 28.1 | 36.6 | 28.1 | 28.1 | 36.6 | 28.1 | 28.1 | 36.6 | 28.1 |
| Sens 5 | 36% | 20% | 39% | 33% | 20% | 35% | 24% | 17% | 30% |
| Spec 5 | 83% | 80% | 80% | 83% | 80% | 80% | 83% | 80% | 80% |
| Cutoff 6 | 44.7 | 57.5 | 45.0 | 44.7 | 57.5 | 45.0 | 44.7 | 57.5 | 45.0 |
| Sens 6 | 21% | 7% | 22% | 19% | 7% | 18% | 12% | 8% | 10% |
| Spec 6 | 91% | 90% | 90% | 91% | 90% | 90% | 91% | 90% | 90% |
| OR Quart 2 p Value | 0.57 | 2.0 | 0.71 | 0.56 | 2.0 | 0.73 | 0.73 | 2.1 | 0.67 |
| 95% CI of OR Quart2 | 0.46 | 0.42 | 0.67 | 0.44 | 0.42 | 0.69 | 0.69 | 0.41 | 0.67 |
|  | 0.13 | 0.36 | 0.15 | 0.12 | 0.36 | 0.15 | 0.15 | 0.37 | 0.11 |
|  | 2.6 | 11 | 3.4 | 2.5 | 11 | 3.5 | 3.5 | 12 | 4.2 |
| OR Quart 3 p Value | 1.7 | 2.6 | 0.73 | 1.4 | 2.6 | 0.73 | 1.0 | 2.1 | 0.65 |
| 95% CI of OR Quart3 | 0.37 | 0.27 | 0.69 | 0.56 | 0.27 | 0.69 | 1.0 | 0.41 | 0.65 |
|  | 0.52 | 0.48 | 0.15 | 0.42 | 0.48 | 0.15 | 0.23 | 0.37 | 0.10 |
|  | 5.8 | 14 | 3.5 | 4.9 | 14 | 3.5 | 4.3 | 12 | 4.1 |
| OR Quart 4 p Value | 2.9 | 2.0 | 2.2 | 2.8 | 2.0 | 1.8 | 1.5 | 1.0 | 1.0 |
| 95% CI of OR Quart4 | 0.066 | 0.42 | 0.24 | 0.073 | 0.42 | 0.36 | 0.53 | 1.0 | 0.97 |
|  | 0.93 | 0.36 | 0.60 | 0.91 | 0.36 | 0.50 | 0.40 | 0.14 | 0.19 |
|  | 9.2 | 11 | 7.8 | 9.0 | 11 | 6.8 | 5.9 | 7.3 | 5.4 |

Fig. 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 167000 | 198000 | 167000 | 187000 | 167000 | 174000 |
| Average | 186000 | 212000 | 186000 | 183000 | 186000 | 166000 |
| Stdev | 183000 | 103000 | 183000 | 91400 | 183000 | 92300 |
| p(t-test) | | 0.27 | | 0.90 | | 0.67 |
| Min | 20700 | 61200 | 20700 | 37100 | 20700 | 34500 |
| Max | 2240000 | 548000 | 2240000 | 446000 | 2240000 | 378000 |
| n (Samp) | 158 | 73 | 158 | 56 | 158 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 175000 | 202000 | 175000 | 192000 | 175000 | 245000 |
| Average | 193000 | 200000 | 193000 | 182000 | 193000 | 197000 |
| Stdev | 144000 | 73800 | 144000 | 65100 | 144000 | 103000 |
| p(t-test) | | 0.82 | | 0.80 | | 0.95 |
| Min | 10800 | 35700 | 10800 | 61200 | 10800 | 31500 |
| Max | 2240000 | 410000 | 2240000 | 292000 | 2240000 | 308000 |
| n (Samp) | 380 | 20 | 380 | 11 | 380 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 174000 | 195000 | 174000 | 182000 | 174000 | 173000 |
| Average | 193000 | 212000 | 193000 | 186000 | 193000 | 164000 |
| Stdev | 175000 | 110000 | 175000 | 106000 | 175000 | 99200 |
| p(t-test) | | 0.42 | | 0.78 | | 0.49 |
| Min | 20700 | 23900 | 20700 | 37100 | 20700 | 34500 |
| Max | 2240000 | 548000 | 2240000 | 552000 | 2240000 | 378000 |
| n (Samp) | 184 | 64 | 184 | 62 | 184 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.58 | 0.57 | 0.52 | 0.52 | 0.49 | 0.46 | 0.56 | 0.43 |
| SE | 0.041 | 0.068 | 0.042 | 0.045 | 0.089 | 0.043 | 0.080 | 0.11 | 0.073 |
| p | 0.0094 | 0.23 | 0.093 | 0.61 | 0.84 | 0.85 | 0.64 | 0.58 | 0.31 |
| nCohort 1 | 158 | 380 | 184 | 158 | 380 | 184 | 158 | 380 | 184 |
| nCohort 2 | 73 | 20 | 64 | 56 | 11 | 62 | 15 | 7 | 18 |
| Cutoff 1 | 149000 | 179000 | 143000 | 109000 | 174000 | 109000 | 104000 | 174000 | 99900 |
| Sens 1 | 71% | 70% | 70% | 71% | 73% | 71% | 73% | 71% | 72% |
| Spec 1 | 42% | 52% | 38% | 20% | 49% | 20% | 20% | 49% | 16% |
| Cutoff 2 | 124000 | 166000 | 123000 | 92900 | 124000 | 91600 | 100000 | 88200 | 60300 |
| Sens 2 | 81% | 80% | 81% | 80% | 82% | 81% | 80% | 86% | 83% |
| Spec 2 | 30% | 47% | 27% | 12% | 29% | 12% | 16% | 12% | 6% |
| Cutoff 3 | 109000 | 123000 | 96600 | 81100 | 104000 | 81100 | 44000 | 24100 | 44000 |
| Sens 3 | 90% | 90% | 91% | 91% | 91% | 90% | 93% | 100% | 94% |
| Spec 3 | 20% | 29% | 14% | 10% | 19% | 10% | 4% | 1% | 4% |
| Cutoff 4 | 207000 | 225000 | 217000 | 207000 | 225000 | 217000 | 207000 | 225000 | 217000 |
| Sens 4 | 45% | 30% | 42% | 41% | 27% | 35% | 13% | 57% | 17% |
| Spec 4 | 70% | 70% | 70% | 70% | 70% | 70% | 70% | 70% | 70% |
| Cutoff 5 | 227000 | 253000 | 247000 | 227000 | 253000 | 247000 | 227000 | 253000 | 247000 |
| Sens 5 | 33% | 15% | 27% | 30% | 9% | 21% | 13% | 43% | 17% |
| Spec 5 | 80% | 80% | 80% | 80% | 80% | 80% | 80% | 80% | 80% |
| Cutoff 6 | 278000 | 326000 | 293000 | 278000 | 326000 | 293000 | 278000 | 326000 | 293000 |
| Sens 6 | 15% | 5% | 20% | 16% | 0% | 18% | 13% | 0% | 17% |
| Spec 6 | 91% | 90% | 90% | 91% | 90% | 90% | 91% | 90% | 90% |
| OR Quart 2 p Value 95% CI of OR Quart2 | 1.6 | 5.2 | 1.3 | 0.37 | 0.99 | 0.67 | 4.1 | 0.49 | 2.2 |
| | 0.31 | 0.14 | 0.51 | 0.039 | 0.99 | 0.34 | 0.091 | 0.56 | 0.29 |
| | 0.66 | 0.60 | 0.56 | 0.14 | 0.14 | 0.30 | 0.80 | 0.044 | 0.51 |
| | 3.6 | 45 | 3.1 | 0.95 | 7.2 | 1.5 | 21 | 5.5 | 9.3 |
| OR Quart 3 p Value 95% CI of. OR Quart3 | 1.8 | 9.8 | 1.6 | 0.69 | 2.6 | 0.38 | 0.50 | 0 | 0.65 |
| | 0.16 | 0.032 | 0.29 | 0.39 | 0.27 | 0.035 | 0.58 | na | 0.65 |
| | 0.79 | 1.2 | 0.68 | 0.29 | 0.48 | 0.16 | 0.044 | na | 0.10 |
| | 4.2 | 79 | 3.7 | 1.6 | 13 | 0.94 | 5.7 | na | 4.1 |
| OR Quart 4 p Value 95% CI ot OR Quart4 | 2.8 | 5.2 | 2.0 | 1.1 | 0.99 | 1.1 | 2.8 | 2.0 | 2.6 |
| | 0.013 | 0.14 | 0.10 | 0.89 | 0.99 | 0.80 | 0.24 | 0.42 | 0.18 |
| | 1.2 | 0.60 | 0.87 | 0.47 | 0.14 | 0.52 | 0.51 | 0.36 | 0.63 |
| | 6.5 | 45 | 4.5 | 2.4 | 7.2 | 2.4 | 15 | 11 | 11 |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.9 | 37.7 | 24.9 | 36.3 | 24.9 | 18.2 |
| Average | 53.1 | 48.0 | 53.1 | 54.1 | 53.1 | 40.0 |
| Stdev | 160 | 42.6 | 160 | 56.2 | 160 | 49.0 |
| p(t-test) | | 0.79 | | 0.96 | | 0.75 |
| Min | 1.77 | 6.18 | 1.77 | 4.89 | 1.77 | 5.00 |
| Max | 1900 | 243 | 1900 | 339 | 1900 | 176 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.1 | 52.3 | 31.1 | 51.8 | 31.1 | 43.8 |
| Average | 47.7 | 60.3 | 47.7 | 60.2 | 7.7 | 37.7 |
| Stdev | 108 | 44.6 | 108 | 34.1 | 108 | 30.4 |
| p(t-test) | | 0.60 | | 0.70 | | 0.81 |
| Min | 1.77 | 8.18 | 1.77 | 19.4 | 1.77 | 8.28 |
| Max | 1900 | 192 | 1900 | 127 | 1900 | 94.2 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.8 | 36.3 | 31.8 | 36.3 | 31.8 | 26.3 |
| Average | 61.4 | 48.0 | 61.4 | 53.4 | 61.4 | 45.6 |
| Stdev | 152 | 43.1 | 152 | 55.5 | 152 | 45.6 |
| p(t-test) | | 0.49 | | 0.69 | | 0.66 |
| Min | 2.68 | 6.18 | 2.68 | 4.89 | 2.68 | 5.00 |
| Max | 1900 | 243 | 1900 | 339 | 1900 | 176 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.68 | 0.54 | 0.63 | 0.71 | 0.55 | 0.44 | 0.49 | 0.46 |
| SE | 0.041 | 0.067 | 0.042 | 0.045 | 0.089 | 0.043 | 0.080 | 0.11 | 0.073 |
| p | 0.0019 | 0.0060 | 0.32 | 0.0040 | 0.016 | 0.25 | 0.45 | 0.95 | 0.62 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 26.3 | 40.8 | 25.8 | 23.2 | 37.1 | 22.8 | 10.7 | 15.3 | 17.6 |
| Sens 1 | 71% | 70% | 70% | 71% | 73% | 71% | 73% | 71% | 72% |
| Spec 1 | 52% | 68% | 40% | 48% | 62% | 36% | 18% | 21% | 25% |
| Cutoff 2 | 21.8 | 33.9 | 20.7 | 19.4 | 32.5 | 19.4 | 10.4 | 9.30 | 10.7 |
| Sens 2 | 81% | 80% | 81% | 80% | 82% | 81% | 80% | 86% | 83% |
| Spec 2 | 44% | 56% | 31% | 38% | 54% | 28% | 17% | 8% | 13% |
| Cutoff 3 | 13.4 | 22.2 | 14.3 | 13.5 | 28.7 | 14.6 | 8.28 | 8.28 | 9.30 |
| Sens 3 | 90% | 90% | 91% | 91% | 91% | 90% | 93% | 100% | 94% |
| Spec 3 | 24% | 35% | 19% | 24% | 45% | 19% | 10% | 7% | 8% |
| Cutoff 4 | 37.7 | 43.0 | 50.4 | 37.7 | 43.0 | 50.4 | 37.7 | 43.0 | 50.4 |
| Sens 4 | 51% | 60% | 31% | 48% | 64% | 34% | 33% | 57% | 39% |
| Spec 4 | 70% | 70% | 70% | 70% | 70% | 70% | 70% | 70% | 70% |
| Cutoff 5 | 51.6 | 57.5 | 71.1 | 51.6 | 57.5 | 71.1 | 51.6 | 57.5 | 71.1 |
| Sens 5 | 27% | 50% | 19% | 34% | 36% | 16% | 33% | 14% | 22% |
| Spec 5 | 80% | 80% | 80% | 80% | 80% | 80% | 80% | 80% | 80% |
| Cutoff 6 | 88.5 | 87.8 | 105 | 88.5 | 87.8 | 105 | 88.5 | 87.8 | 105 |
| Sens 6 | 10% | 15% | 5% | 14% | 27% | 11% | 13% | 14% | 11% |
| Spec 6 | 90% | 90% | 90% | 90% | 90% | 90% | 90% | 90% | 90% |
| OR Quart 2 p Value 95% CI of OR Quart2 | 1.6 | 0.99 | 2.1 | 1.3 | >2.0 | 1.4 | 0 | 3.1 | 0.80 |
| | 0.28 | 0.99 | 0.098 | 0.62 | <0.57 | 0.50 | na | 0.33 | 0.75 |
| | 0.67 | 0.14 | 0.87 | 0.48 | >0.18 | 0.56 | na | 0.32 | 0.20 |
| | 4.0 | 7.2 | 5.0 | 3.4 | na | 3.3 | na | 30 | 3.2 |
| OR Quart : 3 p Value 95% CI of OR Quart3 | 3.9 | 3.1 | 2.4 | 2.3 | >3.1 | 2.4 | 0.78 | 0 | 0.80 |
| | 0.0017 | 0.17 | 0.044 | 0.075 | <0.33 | 0.042 | 0.73 | na | 0.75 |
| | 1.7 | 0.61 | 1.0 | 0.92 | >0.32 | 1.0 | 0.19 | na | 0.20 |
| | 9.3 | 16 | 5.8 | 5.8 | na | 5.5 | 3.1 | na | 3.2 |
| OR Quart 4 p Value 95% CI of 6.8 OR Quart4 | 2.9 | 5.4 | 1.8 | 2.7 | >6.3 | 1.6 | 1.2 | 3.1 | 1.0 |
| | 0.017 | 0.033 | 0.20 | 0.035 | <0.091 | 0.30 | 0.75 | 0.33 | 0.97 |
| | 1.2 | 1.1 | 0.73 | 1.1 | >0.75 | 0.67 | 0.35 | 0.32 | 0.28 |
| | 6.8 | 25 | 4.3 | 6.6 | na | 3.8 | 4.4 | 30 | 3.8 |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 63.6 | 70.1 | 63.6 | 63.3 | 63.6 | 48.2 |
| Average | 73.5 | 76.7 | 73.5 | 74.2 | 73.5 | 53.6 |
| Stdev | 46.5 | 38.7 | 46.5 | 41.0 | 46.5 | 33.3 |
| p(t-test) | | 0.62 | | 0.93 | | 0.11 |
| Min | 7.54 | 28.3 | 7.54 | 3.36 | 7.54 | 4.29 |
| Max | 240 | 250 | 240 | 212 | 240 | 133 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 64.7 | 79.7 | 64.7 | 64.8 | 64.7 | 64.8 |
| Average | 71.8 | 89.9 | 71.8 | 83.3 | 71.8 | 72.3 |
| Stdev | 41.0 | 56.4 | 41.0 | 52.5 | 41.0 | 31.3 |
| p(t-test) | | 0.060 | | 0.36 | | 0.97 |
| Min | 3.36 | 36.7 | 3.36 | 38.7 | 3.36 | 47.8 |
| Max | 288 | 250 | 288 | 212 | 288 | 138 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 72.3 | 68.0 | 72.3 | 61.4 | 72.3 | 49.1 |
| Average | 82.2 | 72.7 | 82.2 | 73.4 | 82.2 | 60.5 |
| Stdev | 53.1 | 29.6 | 53.1 | 43.1 | 53.1 | 36.4 |
| p(t-test) | | 0.18 | | 0.24 | | 0.093 |
| Min | 7.54 | 28.3 | 7.54 | 3.36 | 7.54 | 4.29 |
| Max | 283 | 154 | 283 | 250 | 283 | 133 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.59 | 0.49 | 0.53 | 0.55 | 0.47 | 0.37 | 0.53 | 0.38 |
| SE | 0.041 | 0.068 | 0.042 | 0.045 | 0.091 | 0.043 | 0.080 | 0.11 | 0.073 |
| p | 0.22 | 0.19 | 0.78 | 0.56 | 0.58 | 0.45 | 0.12 | 0.80 | 0.10 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 52.7 | 48.3 | 53.7 | 49.6 | 53.7 | 47.6 | 40.3 | 53.7 | 41.8 |
| Sens 1 | 71% | 70% | 70% | 71% | 73% | 71% | 73% | 71% | 72% |
| Spec 1 | 42% | 32% | 36% | 37% | 40% | 30% | 27% | 40% | 23% |
| Cutoff | 43.4 | 46.7 | 43.4 | 43.4 | 52.1 | 42.9 | 36.5 | 48.2 | 36.5 |
| Sens 2 | 81% | 80% | 81% | 80% | 82% | 81% | 80% | 86% | 83% |
| Spec 2 | 31% | 29% | 26% | 31% | 37% | 25% | 22% | 32% | 17% |
| Cutoff 3 | 38.1 | 43.4 | 38.1 | 36.5 | 44.0 | 36.5 | 22.6 | 47.7 | 21.7 |
| Sens 3 | 90% | 90% | 91% | 91% | 91% | 90% | 93% | 100% | 94% |
| Spec 3 | 23% | 27% | 18% | 22% | 28% | 17% | 8% | 31% | 5% |
| Cutoff 4 | 89.6 | 87.7 | 92.9 | 89.6 | 87.7 | 92.9 | 89.6 | 87.7 | 92.9 |
| Sens 4 | 29% | 35% | 22% | 25% | 18% | 26% | 13% | 14% | 22% |
| Spec 4 | 70% | 70% | 70% | 70% | 70% | 70% | 70% | 70% | 70% |
| Cutoff 5 | 102 | 96.5 | 114 | 102 | 96.5 | 114 | 102 | 96.5 | 114 |
| Sens 5 | 16% | 25% | 9% | 12% | 18% | 10% | 13% | 14% | 17% |
| Spec 5 | 80% | 80% | 80% | 80% | 80% | 80% | 80% | 80% | 80% |
| Cutoff 6 | 135 | 121 | 151 | 135 | 121 | 151 | 135 | 121 | 151 |
| Sens 6 | 7% | 20% | 2% | 9% | 18% | 8% | 0% | 14% | 0% |
| Spec 6 | 90% | 90% | 90% | 90% | 90% | 90% | 90% | 90% | 90% |
| OR Quart 2 | 2.7 | 2.6 | 2.0 | 3.4 | 4.1 | 1.2 | 1.0 | >3.1 | 0.67 |
| | 0.019 | 0.27 | 0.097 | 0.010 | 0.21 | 0.63 | 1.0 | <0.34 | 0.66 |
| p Value 95% CI or OR Quart2 | 1.2 | 0.48 | 0.88 | 1.3 | 0.45 | 0.52 | 0.13 | >0.31 | 0.11 |
| | 6.4 | 13 | 4.8 | 8.7 | 37 | 3.0 | 7.4 | na | 4.2 |
| OR Quart 3 p Value 95% CI of OR Quart3 | 2.6 | 4.2 | 2.4 | 2.0 | 4.1 | 2.4 | 4.0 | >3.1 | 2.6 |
| | 0.025 | 0.073 | 0.043 | 0.16 | 0.21 | 0.041 | 0.097 | <0.33 | 0.18 |
| | 1.1 | 0.87 | 1.0 | 0.77 | 0.45 | 1.0 | 0.78 | >0.32 | 0.63 |
| | | 20 | 5.5 | 5.3 | 38 | 5.3 | 20 | na | 11 |
| OR Quart 4 p Value 95% CI of OR Quart4 | 1.7 | 2.6 | 1.3 | 2.0 | 2.0 | 1.2 | 2.1 | >1.0 | 2.2 |
| | 0.21 | 0.27 | 0.62 | 0.17 | 0.57 | 0.63 | 0.41 | <1.0 | 0.29 |
| | 0.73 | 0.48 | 0.51 | 0.75 | 0.18 | 0.52 | 0.36 | >0.062 | 0.51 |
| | 4.1 | 13 | 3.1 | 5.2 | 22 | 3.0 | 12 | na | 9.3 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20.4 | 20.9 | 20.4 | 25.2 | 20.4 | 17.7 |
| Average | 25.5 | 24.6 | 25.5 | 33.1 | 25.5 | 33.0 |
| Stdev | 17.7 | 16.7 | 17.7 | 25.5 | 17.7 | 31.9 |
| p(t-test) | | 0.70 | | 0.019 | | 0.16 |
| Min | 0.00246 | 0.00246 | 0.00246 | 5.07 | 0.00246 | 6.76 |
| Max | 121 | 72.6 | 121 | 133 | 121 | 113 |
| n (Samp) | 156 | 71 | 156 | 53 | 156 | 15 |
| n (Patient) | 87 | 71 | 87 | 53 | 87 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.3 | 20.3 | 21.3 | 31.4 | 21.3 | 43.9 |
| Average | 27.2 | 26.7 | 27.2 | 33.1 | 27.2 | 41.8 |
| Stdev | 21.5 | 17.9 | 21.5 | 14.4 | 21.5 | 29.6 |
| p(t-test) | | 0.92 | | 0.37 | | 0.079 |
| Min | 0.00246 | 7.08 | 0.00246 | 12.3 | 0.00246 | 14.3 |
| Max | 178 | 72.6 | 178 | 61.8 | 178 | 98.8 |
| n (Samp) | 372 | 19 | 372 | 11 | 372 | 7 |
| n (Patient) | 173 | 19 | 173 | 11 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.1 | 20.9 | 21.1 | 24.4 | 21.1 | 19.5 |
| Average | 26.6 | 24.6 | 26.6 | 34.1 | 26.6 | 31.7 |
| Stdev | 21.4 | 16.8 | 21.4 | 29.9 | 21.4 | 30.2 |
| p(t-test) | | 0.51 | | 0.035 | | 0.36 |
| Min | 0.00246 | 0.00246 | 0.00246 | 5.07 | 0.00246 | 4.60 |
| Max | 198 | 72.6 | 198 | 162 | 198 | 113 |
| n (Samp) | 181 | 63 | 181 | 59 | 181 | 18 |
| n (Patient) | 88 | 63 | 88 | 59 | 88 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.50 | 0.47 | 0.58 | 0.66 | 0.56 | 0.50 | 0.67 | 0.49 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.042 | 0.068 | 0.043 | 0.046 | 0.091 | 0.044 | 0.078 | 0.11 | 0.072 |
| p | 0.65 | 0.98 | 0.55 | 0.095 | 0.071 | 0.16 | 0.97 | 0.14 | 0.90 |
| nCohort 1 | 156 | 372 | 181 | 156 | 372 | 181 | 156 | 372 | 181 |
| nCohort 2 | 71 | 19 | 63 | 53 | 11 | 59 | 15 | 7 | 18 |
| Cutoff 1 | 13.6 | 14.3 | 13.6 | 16.8 | 25.6 | 16.8 | 14.7 | 21.1 | 14.7 |
| Sens 1 | 72% | 74% | 71% | 72% | 73% | 71% | 73% | 71% | 72% |
| Spec 1 | 24% | 27% | 23% | 39% | 61% | 37% | 29% | 50% | 28% |
| Cutoff 2 | 11.4 | 11.7 | 10.3 | 13.5 | 21.8 | 13.5 | 13.9 | 16.1 | 12.5 |
| Sens 2 | 80% | 84% | 81% | 81% | 82% | 81% | 80% | 86% | 83% |
| Spec 2 | 17% | 17% | 13% | 24% | 51% | 22% | 26% | 33% | 18% |
| Cutoff 3 | 8.58 | 8.58 | 8.57 | 11.0 | 18.1 | 11.0 | 6.93 | 14.3 | 6.01 |
| Sens 3 | 90% | 95% | 90% | 91% | 91% | 92% | 93% | 100% | 94% |
| Spec 3 | 12% | 10% | 10% | 15% | 41% | 14% | 6% | 26% | 4% |
| Cutoff 4 | 30.9 | 30.4 | 30.9 | 30.9 | 30.4 | 30.9 | 30.9 | 30.4 | 30.9 |
| Sens 4 | 25% | 32% | 25% | 38% | 55% | 34% | 27% | 57% | 28% |
| Spec 4 | 71% | 70% | 70% | 71% | 70% | 70% | 71% | 70% | 70% |
| Cutoff 5 | 35.6 | 37.3 | 37.0 | 35.6 | 37.3 | 37.0 | 35.6 | 37.3 | 37.0 |
| Sens 5 | 18% | 21% | 16% | 32% | 27% | 29% | 27% | 57% | 22% |
| Spec 5 | 80% | 80% | 81% | 80% | 80% | 81% | 80% | 80% | 81% |
| Cutoff 6 | 47.0 | 52.4 | 45.5 | 47.0 | 52.4 | 45.5 | 47.0 | 52.4 | 45.5 |
| Sens 6 | 11% | 11% | 11% | 23% | 9% | 22% | 20% | 29% | 22% |
| Spec 6 | 90% | 90% | 90% | 90% | 90% | 90% | 90% | 90% | 90% |
| OR Quart 2 p Value 95% CI of OR Quart2 | 1.4 | 0.99 | 1.3 | 0.79 | 0.99 | 0.74 | 1.7 | >3.1 | 1.3 |
| | 0.42 | 0.99 | 0.53 | 0.63 | 0.99 | 0.50 | 0.48 | <0.34 | 0.73 |
| | 0.63 | 0.28 | 0.57 | 0.31 | 0.061 | 0.30 | 0.38 | >0.31 | 0.32 |
| | 3.1 | 3.5 | 2.9 | 2.0 | 16 | 1.8 | 7.7 | na | 5.1 |
| OR Quart 3 p Value | 0.92 | 0.78 | 1.0 | 1.2 | 5.2 | 1.2 | 0.98 | >0 | 1.3 |
| | 0.83 | 0.72 | 1.0 | 0.65' | 0.14 | 0.67 | 0.98 | <na | 0.73 |
| | 0.40 | 0.20 | 0.43 | 0.50 | 0.59 | 0.52 | 0.19 | >na | 0.32 |
| 95% CI of OR Quart3 | 2.1 | 3.0 | 2.3 | 3.0 | 45 | 2.7 | 5.1 | na | 5.1 |
| OR Quart 4 p Value 95% CI of OR Quart4 | 1.4 | 0.99 | 1.4 | 1.6 | 4.1 | 1.4 | 1.3 | >4.1 | 1.0 |
| | 0.38 | 0.99 | 0.41 | 0.30 | 0.21 | 0.41 | 0.72 | <0.21 | 0.98 |
| | 0.64 | 0.28 | 0.62 | 0.66 | 0.45 | 0.62 | 0.28 | >0.45 | 0.24 |
| | 3.2 | 3.5 | 3.2 | 3.7 | 37 | 3.2 | 6.4 | na | 4.3 |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.02 | 3.84 | 4.02 | 4.08 | 4.02 | 3.93 |
| Average | 4.29 | 4.55 | 4.29 | 5.49 | 4.29 | 5.61 |
| Stdev | 2.94 | 6.28 | 2.94 | 5.39 | 2.94 | 3.80 |
| p(t-test) | | 0.67 | | 0.043 | | 0.12 |
| Min | 0.00226 | 0.00274 | 0.00226 | 0.00282 | 0.00226 | 1.92 |
| Max | 15.0 | 50.2 | 15.0 | 32.2 | 15.0 | 13.9 |
| n (Samp) | 155 | 72 | 155 | 54 | 155 | 14 |
| n (Patient) | 87 | 72 | 87 | 54 | 87 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.02 | 2.23 | 4.02 | 4.58 | 4.02 | 3.61 |
| Average | 4.43 | 5.58 | 4.43 | 5.03 | 4.43 | 5.58 |
| Stdev | 3.21 | 11.4 | 3.21 | 3.31 | 3.21 | 6.34 |
| p(t-test) | | 0.22 | | 0.54 | | 0.36 |
| Min | 0.00226 | 0.00282 | 0.00226 | 0.00282 | 0.00226 | 0.00274 |
| Max | 21.0 | 50.2 | 21.0 | 11.8 | 21.0 | 17.6 |
| n (Samp) | 372 | 19 | 372 | 11 | 372 | 7 |
| n (Patient) | 174 | 19 | 174 | 11 | 174 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.74 | 4.05 | 3.74 | 4.08 | 3.74 | 4.24 |
| Average | 4.87 | 3.93 | 4.87 | 5.98 | 4.87 | 5.34 |
| Stdev | 7.41 | 2.74 | 7.41 | 7.75 | 7.41 | 3.44 |
| p(t-test) | | 0.32 | | 0.32 | | 0.79 |
| Min | 0.00226 | 0.00274 | 0.00226 | 0.00289 | 0.00226 | 1.92 |
| Max | 60.7 | 12.2 | 60.7 | 50.2 | 60.7 | 13.9 |
| n (Samp) | 180 | 64 | 180 | 60 | 180 | 17 |
| n (Patient) | 88 | 64 | 88 | 60 | 88 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.38 | 0.50 | 0.54 | 0.55 | 0.56 | 0.57 | 0.50 | 0.59 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.041 | 0.070 | 0.042 | 0.046 | 0.091 | 0.044 | 0.083 | 0.11 | 0.075 |
| p | 0.45 | 0.077 | 0.93 | 0.41 | 0.55 | 0.19 | 0.40 | 0.98 | 0.24 |
| nCohort 1 | 155 | 372 | 180 | 155 | 372 | 180 | 155 | 372 | 180 |
| nCohort 2 | 72 | 19 | 64 | 54 | 11 | 60 | 14 | 7 | 17 |
| Cutoff 1 | 1.92 | 0.0204 | 2.42 | 2.92 | 3.54 | 2.92 | 3.54 | 1.59 | 3.54 |
| Sens 1 | 71% | 74% | 70% | 70% | 73% | 70% | 71% | 71% | 71% |
| Spec 1 | 23% | 9% | 31% | 34% | 42% | 40% | 41% | 16% | 46% |
| Cutoff 2 | 0.743 | 0.00282 | 1.18 | 2.26 | 2.89 | 1.92 | 2.26 | 0.0204 | 2.65 |
| Sens 2 | 81% | 89% | 83% | 81% | 82% | 80% | 93% | 86% | 82% |
| Spec 2 | 10% | 3% | 16% | 25% | 32% | 27% | 25% | 9% | 37% |
| Cutoff 3 | 0.00282 | 0.00274 | 0.00282 | 0.743 | 2.42 | 0.743 | 2.26 | 0.00226 | 2.26 |
| Sens 3 | 93% | 100% | 95% | 91% | 91% | 90% | 93% | 100% | 94% |
| Spec 3 | 4% | 2% | 8% | 10% | 26% | 14% | 25% | 1% | 31% |
| Cutoff 4 | 5.14 | 5.42 | 5.12 | 5.14 | 5.42 | 5.12 | 5.14 | 5.42 | 5.12 |
| Sens 4 | 31% | 21% | 28% | 37% | 18% | 40% | 29% | 43% | 24% |
| Spec 4 | 70% | 70% | 70% | 70% | 70% | 70% | 70% | 70% | 70% |
| Cutoff 5 | 6.20 | 6.52 | 6.20 | 6.20 | 6.52 | 6.20 | 6.20 | 6.52 | 6.20 |
| Sens 5 | 21% | 21% | 17% | 28% | 18% | 27% | 29% | 43% | 24% |
| Spec 5 | 81% | 80% | 81% | 81% | 80% | 81% | 81% | 80% | 81% |
| Cutoff 6 | 7.89 | 8.40 | 7.89 | 7.89 | 8.40 | 7.89 | 7.89 | 8.40 | 7.89 |
| Sens 6 | 12% | 11% | 9% | 19% | 18% | 17% | 29% | 29% | 24% |
| Spec 6 | 90% | 90% | 90% | 90% | 90% | 90% | 90% | 90% | 90% |
| OR Quart 2 p Value 95% CI of OR Quart2 | 0.92 | 0.49 | 1.3 | 1.2 | 3.0 | 1.5 | 1.4 | 0 | 8.0 |
| | 0.84 | 0.42 | 0.54 | 0.65 | 0.34 | 0.39 | 0.69 | na | 0.056 |
| | 0.42 | 0.088 | 0.58 | 0.50 | 0.31 | 0.62 | 0.29 | na | 0.95 |
| | 2.0 | 2.7 | 2.9 | 3.0 | 30 | 3.4 | 6.5 | na | 68 |
| OR Quart 3 p Value 95% CI of OR Quart3 | 0.85 | 0.74 | 0.91 | 1.0 | 5.2 | 1.1 | 1.0 | 0.33 | 5.5 |
| | 0.68 | 0.70 | 0.83 | 1.0 | 0.14 | 0.82 | 1.0 | 0.34 | 0.13 |
| | 0.38 | 0.16 | 0.40 | 0.40 | 0.59 | 0.46 | 0.19 | 0.033 | 0.61 |
| | 1.9 | 3.4 | 2.1 | 2.5 | 45 | 2.7 | 5.3 | 3.2 | 49 |
| OR Quart 4 p Value 95% CI ot OR Quart4 | 1.3 | 2.7 | 1.2 | 1.4 | 2.0 | 1.9 | 1.3 | 1.0 | 4.2 |
| | 0.51 | 0.10 | 0.68 | 0.41 | 0.57 | 0.15 | 0.72 | 0.99 | 0.21 |
| | 0.60 | 0.82 | 0.53 | 0.60 | 0.18 | 0.80 | 0.28 | 0.20 | 0.45 |
| | 2.8 | 8.9 | 2.7 | 3.4 | 22 | 4.3 | 6.4 | 5.1 | 39 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 35400 | 42600 | 35400 | 43100 | 35400 | 48800 |
| Average | 43000 | 57500 | 43000 | 54500 | 43000 | 49500 |
| Stdev | 32700 | 49300 | 32700 | 44800 | 32700 | 36500 |
| p(t-test) | | 0.0085 | | 0.042 | | 0.47 |
| Min | 4620 | 8430 | 4620 | 7440 | 4620 | 7440 |
| Max | 328000 | 275000 | 328000 | 241000 | 328000 | 133000 |
| n (Samp) | 158 | 73 | 158 | 56 | 158 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 38700 | 56400 | 38700 | 69400 | 38700 | 65700 |
| Average | 47300 | 62400 | 47300 | 90000 | 47300 | 74800 |
| Stdev | 34800 | 38900 | 34800 | 58000 | 34800 | 40700 |
| p(t-test) | | 0.062 | | 1.1E-4 | | 0.040 |
| Min | 3600 | 13600 | 3600 | 19800 | 3600 | 11600 |
| Max | 328000 | 165000 | 328000 | 196000 | 328000 | 133000 |
| n (Samp) | 380 | 20 | 380 | 11 | 380 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 37700 | 40000 | 37700 | 36800 | 37700 | 42800 |
| Average | 48100 | 56100 | 48100 | 49300 | 48100 | 69100 |
| Stdev | 36900 | 51500 | 36900 | 40400 | 36900 | 107000 |
| p(t-test) | | 0.18 | | 0.83 | | 0.073 |
| Min | 4620 | 8430 | 4620 | 7440 | 4620 | 7440 |
| Max | 328000 | 275000 | 328000 | 241000 | 328000 | 483000 |
| n (Samp) | 184 | 64 | 184 | 62 | 184 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.63 | 0.52 | 0.57 | 0.74 | 0.50 | 0.55 | 0.73 | 0.51 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.041 | 0.068 | 0.042 | 0.045 | 0.087 | 0.042 | 0.080 | 0.11 | 0.072 |
| p | 0.034 | 0.054 | 0.59 | 0.13 | 0.0051 | 0.99 | 0.53 | 0.033 | 0.84 |
| nCohort 1 | 158 | 380 | 184 | 158 | 380 | 184 | 158 | 380 | 184 |
| nCohort 2 | 73 | 20 | 64 | 56 | 11 | 62 | 15 | 7 | 18 |
| Cutoff 1 | 30600 | 46100 | 29800 | 30600 | 52400 | 30900 | 23500 | 58300 | 25600 |
| Sens 1 | 71% | 70% | 70% | 71% | 73% | 71% | 73% | 71% | 72% |
| Spec 1 | 40% | 61% | 33% | 40% | 69% | 37% | 23% | 75% | 23% |
| Cutoff 2 | 23500 | 31900 | 22700 | 20400 | 52100 | 20100 | 15200 | 55100 | 15700 |
| Sens 2 | 81% | 80% | 81% | 80% | 82% | 81% | 80% | 86% | 83% |
| Spec 2 | 23% | 35% | 18% | 16% | 68% | 14% | 9% | 72% | 8% |
| Cutoff 3 | 18700 | 20100 | 18700 | 14500 | 29300 | 14300 | 12500 | 11100 | 12500 |
| Sens 3 | 90% | 90% | 91% | 91% | 91% | 90% | 93% | 100% | 94% |
| Spec 3 | 13% | 14% | 11% | 7% | 30% | 6% | 4% | 4% | 4% |
| Cutoff 4 | 46600 | 53000 | 52100 | 46600 | 53000 | 52100 | 46600 | 53000 | 52100 |
| Sens 4 | 45% | 60% | 34% | 48% | 64% | 32% | 53% | 86% | 39% |
| Spec 4 | 70% | 70% | 70% | 70% | 70% | 70% | 70% | 70% | 70% |
| Cutoff 5 | 56200 | 66300 | 65300 | 56200 | 66300 | 65300 | 56200 | 66300 | 65300 |
| Sens 5 | 37% | 30% | 28% | 34% | 55% | 27% | 40% | 43% | 22% |
| Spec 5 | 80% | 80% | 80% | 80% | 80% | 80% | 80% | 80% | 80% |
| Cutoff 6 | 78000 | 84400 | 91700 | 78000 | 84400 | 91700 | 78000 | 84400 | 91700 |
| Sens 6 | 19% | 25% | 12% | 16% | 36% | 8% | 13% | 29% | 11% |
| Spec 6 | 91% | 90% | 90% | 91% | 90% | 90% | 91% | 90% | 90% |
| OR Quart 2 p Value 95% CI ot OR Quart2 | 1.1 | 0.49 | 0.92 | 0.88 | 0.99 | 0.60 | 0 | 0 | 0.46 |
| | 0.88 | 0.42 | 0.84 | 0.78 | 0.99 | 0.23 | na | na | 0.29 |
| | 0.47 | 0.088 | 0.41 | 0.36 | 0.061 | 0.26 | na | na | 0.11 |
| | 2.4 | 2.7 | 2.1 | 2.2 | 16 | 1.4 | na | na | 1.9 |
| OR Quart 3 p Value 95% CI of OR Quart3 | 0.98 | 1.5 | 0.76 | 0.90 | 3.0 | 0.85 | 0.78 | 2.0 | 0.64 |
| | 0.96 | 0.52 | 0.53 | 0.82 | 0.34 | 0.69 | 0.73 | 0.57 | 0.51 |
| | 0.42 | 0.42 | 0.33 | 0.37 | 0.31 | 0.39 | 0.19 | 0.18 | 0.17 |
| | 2.2 | 5.6 | 1.8 | 2.2 | 30 | 1.9 | 3.1 | 22 | 2.4 |
| OR Quart 4 p Value 95% CI of OR Quart4 | 2.4 | 2.1 | 1.4 | 1.7 | 6.3 | 0.87 | 1.2 | 4.1 | 0.80 |
| | 0.026 | 0.24 | 0.43 | 0.23 | 0.092 | 0.73 | 0.78 | 0.21 | 0.72 |
| | 1.1 | 0.61 | 0.63 | 0.72 | 0.74 | 0.39 | 0.34 | 0.45 | 0.23 |
| | 5.3 | 7.2 | 3.0 | 3.9 | 53 | 1.9 | 4.3 | 37 | 2.8 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.392 | 0.374 | 0.392 | 0.399 | 0.392 | 0.329 |
| Average | 0.509 | 1.12 | 0.509 | 1.16 | 0.509 | 0.604 |
| Stdev | 0.628 | 3.14 | 0.628 | 4.21 | 0.628 | 0.563 |
| p(t-test) | | 0.020 | | 0.061 | | 0.59 |
| Min | 0.000152 | 0.000152 | 0.000152 | 0.000152 | 0.000152 | 0.0793 |
| Max | 6.96 | 20.6 | 6.96 | 31.1 | 6.96 | 1.68 |
| n (Samp) | 156 | 72 | 156 | 54 | 156 | 14 |
| n (Patient) | 87 | 72 | 87 | 54 | 87 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.396 | 0.392 | 0.396 | 0.298 | 0.396 | 0.472 |
| Average | 0.667 | 0.825 | 0.667 | 0.693 | 0.667 | 0.673 |
| Stdev | 1.42 | 1.41 | 1.42 | 1.04 | 1.42 | 0.713 |
| p(t-test) | | 0.64 | | 0.95 | | 0.99 |
| Min | 0.000152 | 0.000152 | 0.000152 | 0.106 | 0.000152 | 0.192 |
| Max | 18.0 | 4.75 | 18.0 | 3.65 | 18.0 | 2.24 |
| n (Samp) | 371 | 19 | 371 | 11 | 371 | 7 |
| n (Patient) | 173 | 19 | 173 | 11 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.363 | 0.383 | 0.399 | 0.383 | 0.365 |
| Average | 0.756 | 1.14 | 0.756 | 1.06 | 0.756 | 0.550 |
| Stdev | 2.51 | 3.29 | 2.51 | 3.99 | 2.51 | 0.524 |
| p(t-test) | | 0.33 | | 0.49 | | 0.74 |
| Min | 0.000152 | 0.000152 | 0.000152 | 0.000152 | 0.000152 | 0.0793 |
| Max | 32.1 | 20.6 | 32.1 | 31.1 | 32.1 | 1.68 |
| n (Samp) | 181 | 64 | 181 | 60 | 181 | 17 |
| n (Patient) | 88 | 64 | 88 | 60 | 88 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.45 | 0.51 | 0.50 | 0.46 | 0.52 | 0.51 | 0.54 | 0.49 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.041 | 0.070 | 0.042 | 0.046 | 0.090 | 0.043 | 0.081 | 0.11 | 0.074 |
| p | 1.00 | 0.43 | 0.82 | 1.00 | 0.62 | 0.70 | 0.92 | 0.69 | 0.93 |
| nCohort 1 | 156 | 371 | 181 | 156 | 371 | 181 | 156 | 371 | 181 |
| nCohort 2 | 72 | 19 | 64 | 54 | 11 | 60 | 14 | 7 | 17 |
| Cutoff 1 | 0.188 | 0.134 | 0.192 | 0.274 | 0.188 | 0.274 | 0.238 | 0.286 | 0.238 |
| Sens 1 | 71% | 74% | 73% | 70% | 82% | 70% | 71% | 71% | 71% |
| Spec 1 | 20% | 13% | 24% | 30% | 21% | 33% | 25% | 34% | 29% |
| Cutoff 2 | 0.117 | 0.00127 | 0.126 | 0.134 | 0.188 | 0.179 | 0.139 | 0.278 | 0.139 |
| Sens 2 | 81% | 84% | 83% | 83% | 82% | 80% | 86% | 86% | 82% |
| Spec 2 | 12% | 1% | 15% | 12% | 21% | 21% | 14% | 33% | 18% |
| Cutoff 3 | 0.0201 | 0 | 0.0796 | 0.106 | 0.134 | 0.126 | 0.134 | 0.188 | 0.134 |
| Sens 3 | 90% | 100% | 91% | 91% | 91% | 90% | 93% | 100% | 94% |
| Spec 3 | 6% | 0% | 12% | 10% | 13% | 15% | 12% | 21% | 16% |
| Cutoff 4 | 0.589 | 0.645 | 0.587 | 0.589 | 0.645 | 0.587 | 0.589 | 0.645 | 0.587 |
| Sens 4 | 35% | 32% | 30% | 28% | 18% | 30% | 36% | 29% | 29% |
| Spec 4 | 71% | 70% | 70% | 71% | 70% | 70% | 71% | 70% | 70% |
| Cutoff 5 | 0.695 | 0.799 | 0.717 | 0.695 | 0.799 | 0.717 | 0.695 | 0.799 | 0.717 |
| Sens 5 | 32% | 21% | 27% | 19% | 18% | 20% | 36% | 14% | 29% |
| Spec 5 | 81% | 80% | 80% | 81% | 80% | 80% | 81% | 80% | 80% |
| Cutoff 6 | 0.913 | 1.20 | 0.967 | 0.913 | 1.20 | 0.967 | 0.913 | 1.20 | 0.967 |
| Sens 6 | 24% | 11% | 23% | 17% | 18% | 17% | 21% | 14% | 18% |
| Spec 6 | 90% | 90% | 90% | 90% | 90% | 90% | 90% | 90% | 90% |
| OR Quart 2 p Value 95% CI of OR Quart2 | 0.39 | 0.49 | 1.0 | 0.93 | 1.0 | 1.0 | 0.97 | 2.0 | 0.38 |
| | 0.028 | 0.32 | 1.0 | 0.87 | 0.99 | 1.0 | 0.97 | 0.57 | 0.27 |
| | 0.17 | 0.12 | 0.45 | 0.39 | 0.14 | 0.43 | 0.23 | 0.18 | 0.071 |
| | 0.90 | 2.0 | 2.2 | 2.2 | 7.3 | 2.3 | 4.2 | 22 | 2.1 |
| OR Quart 3 p Value 95% CI ot 0.99 OR Quart3 | 0.44 | 0.32 | 0.63 | 0.91 | 1.5 | 1.1 | 0.23 | 2.0 | 1.2 |
| | 0.046 | 0.17 | 0.29 | 0.82 | 0.65 | 0.83 | 0.20 | 0.57 | 0.75 |
| | 0.19 | 0.063 | 0.27 | 0.38 | 0.25 | 0.47 | 0.025 | 0.18 | 0.35 |
| | 0.99 | 1.6 | 1.5 | 2.2 | 9.3 | 2.5 | 2.2 | 23 | 4.3 |
| OR Quart 4 p Value 95% CI of OR Quart4 | 1.1 | 1.4 | 1.1 | 1.0 | 2.1 | 1.3 | 1.2 | 2.0 | 0.80 |
| | 0.85 | 0.57 | 0.89 | 0.95 | 0.41 | 0.57 | 0.75 | 0.57 | 0.75 |
| | 0.51 | 0.46 | 0.48 | 0.43 | 0.37 | 0.56 | 0.31 | 0.18 | 0.20 |
| | 2.3 | 4.1 | 2.3 | 2.4 | 12 | 2.9 | 5.0 | 22 | 3.2 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.170 | 0.194 | 0.170 | 0.119 | 0.170 | 0.0550 |
| Average | 0.199 | 0.224 | 0.199 | 0.162 | 0.199 | 0.107 |
| Stdev | 0.167 | 0.236 | 0.167 | 0.209 | 0.167 | 0.129 |
| p(t-test) | | 0.36 | | 0.18 | | 0.040 |
| Min | 8.23E-5 | 0.000127 | 8.23E-5 | 2.92E-5 | 8.23E-5 | 8.23E-5 |
| Max | 0.948 | 1.41 | 0.948 | 1.20 | 0.948 | 0.347 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.145 | 0.203 | 0.145 | 0.136 | 0.145 | 0.103 |
| Average | 0.193 | 0.293 | 0.193 | 0.210 | 0.193 | 0.113 |
| Stdev | 0.190 | 0.307 | 0.190 | 0.196 | 0.190 | 0.0833 |
| p(t-test) | | 0.027 | | 0.76 | | 0.27 |
| Min | 2.92E-5 | 0.000127 | 2.92E-5 | 8.23E-5 | 2.92E-5 | 8.23E-5 |
| Max | 1.41 | 1.32 | 1.41 | 0.619 | 1.41 | 0.254 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.176 | 0.168 | 0.176 | 0.129 | 0.176 | 0.0787 |
| Average | 0.219 | 0.209 | 0.219 | 0.158 | 0.219 | 0.148 |
| Stdev | 0.216 | 0.233 | 0.216 | 0.193 | 0.216 | 0.160 |
| p(t-test) | | 0.75 | | 0.048 | | 0.17 |
| Min | 8.23E-5 | 0.000127 | 8.23E-5 | 2.92E-5 | 8.23E-5 | 8.23E-5 |
| Max | 2.09 | 1.41 | 2.09 | 1.20 | 2.09 | 0.522 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO sCr | sCr only | UO only | sCr or | sCr only | UO only | sCr or UO | sCr only | UO only |
| AUC | 0.53 | 0.62 | 0.48 | 0.39 | 0.52 | 0.37 | 0.31 | 0.38 | 0.36 |

(continued)

| | 0hr prior to AKI stage | | | 24hr prior to AKI stage | | | 48hr prior to AKI stage | | |
|---|---|---|---|---|---|---|---|---|---|
| | sCr or UO sCr | sCr only | UO only | sCr or | sCr only | UO only | sCr or UO | sCr only | UO only |
| SE | 0.041 | 0.068 | 0.042 | 0.045 | 0.089 | 0.042 | 0.078 | 0.11 | 0.073 |
| p | 0.50 | 0.080 | 0.57 | 0.014 | 0.82 | 0.0016 | 0.014 | 0.29 | 0.062 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 0.107 | 0.150 | 0.106 | 0.0421 | 0.0860 | 0.0449 | 0.0123 | 0.0885 | 0.0430 |
| Sens 1 | 71% | 70% | 70% | 71% | 73% | 71% | 73% | 71% | 72% |
| Spec 1 | 34% | 51% | 31% | 17% | 33% | 14% | 11% | 33% | 14% |
| Cutoff 2 | 0.0726 | 0.125 | 0.0718 | 0.0375 | 0.0804 | 0.0375 | 8.23E-5 | 0.0423 | 8.23E-5 |
| Sens 2 | 82% | 80% | 81% | 80% | 82% | 81% | 80% | 86% | 83% |
| Spec 2 | 24% | 45% | 20% | 15% | 31% | 12% | 4% | 18% | 2% |
| Cutoff 3 | 0.0209 | 0.0804 | 0.0209 | 0.00457 | 0.0240 | 0.00457 | 0 | 2.92E-5 | 0 |
| Sens 3 | 90% | 90% | 91% | 91% | 91% | 90% | 100% | 100% | 100% |
| Spec 3 | 12% | 31% | 9% | 10% | 12% | 7% | 0% | 1% | 0% |
| Cutoff 4 | 0.255 | 0.238 | 0.267 | 0.255 | 0.238 | 0.267 | 0.255 | 0.238 | 0.267 |
| Sens 4 | 34% | 40% | 25% | 11% | 27% | 11% | 20% | 14% | 28% |
| Spec 4 | 70% | 70% | 71% | 70% | 70% | 71% | 70% | 70% | 71% |
| Cutoff 5 | 0.308 | 0.297 | 0.321 | 0.308 | 0.297 | 0.321 | 0.308 | 0.297 | 0.321 |
| Sens 5 | 16% | 30% | 11% | 11% | 27% | 10% | 13% | 0% | 22% |
| Spec 5 | 80% | 80% | 80% | 80% | 80% | 80% | 80% | 80% | 80% |
| Cutoff 6 | 0.442 | 0.401 | 0.450 | 0.442 | 0.401 | 0.450 | 0.442 | 0.401 | 0.450 |
| Sens 6 | 5% | 15% | 3% | 9% | 18% | 6% | 0% | 0% | 6% |
| Spec 6 | 90% | 90% | 90% | 90% | 90% | 90% | 90% | 90% | 90% |
| OR Quart 2 p Value 95% CI of OR Quart2 | 1.5 | 5.2 | 1.0 | 2.9 | 2.0 | 2.6 | 0.32 | >2.1 | 0.49 |
| | 0.34 | 0.14 | 1.0 | 0.048 | 0.42 | 0.060 | 0.33 | <0.56 | 0.42 |
| | 0.66 | 0.59 | 0.44 | 1.0 | 0.36 | 0.96 | 0.032 | >0.18 | 0.086 |
| | 3.3 | 45 | 2.3 | 8.2 | 11 | 6.8 | 3.2 | na | 2.8 |
| OR Quart 3 | 1.4 | 6.3 | 1.3 | 3.2 | 1.0 | 3.0 | 0.65 | >3.1 | 0.75 |
| | 0.41 | 0.092 | 0.54 | 0.030 | 1.0 | 0.024 | 0.65 | <0.33 | 0.72 |
| p Value 95% CI of OR Quart3 | 0.62 | 0.74 | 0.58 | 1.1 | 0.14 | 1.2 | 0.10 | >0.32 | 0.16 |
| | 3.1 | 53 | 2.9 | 8.9 | 7.2 | 8.0 | 4.1 | na | 3.5 |
| OR Quart 4 p Value 95% CI of OR Quart4 | 1.4 | 8.5 | 1.1 | 5.2 | 1.5 | 4.8 | 3.5 | >2.1 | 2.6 |
| | 0.45 | 0.045 | 0.79 | 0.0013 | 0.66 | 0.0012 | 0.074 | <0.56 | 0.14 |
| | 0.61 | 1.0 | 0.49 | 1.9 | 0.25 | 1.9 | 0.88 | >0.18 | 0.74 |
| | 3.1 | 69 | 2.5 | 14 | 9.2 | 12 | 14 | na | 9.0 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0454 | 0.0567 | 0.0454 | 0.0514 | 0.0454 | 0.00987 |
| Average | 0.191 | 0.198 | 0.191 | 0.179 | 0.191 | 0.0861 |
| Stdev | 0.446 | 0.496 | 0.446 | 0.296 | 0.446 | 0.134 |
| p(t-test) | | 0.91 | | 0.85 | | 0.37 |
| Min | 9.65E-6 | 7.01E-5 | 9.65E-6 | 4.60E-6 | 9.65E-6 | 0.00288 |
| Max | 3.21 | 3.36 | 3.21 | 1.29 | 3.21 | 0.438 |
| n (Samp) | 157 | 73 | 157 | 56 | 157 | 15 |
| n (Patient) | 88 | 73 | 88 | 56 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0532 | 0.0603 | 0.0532 | 0.105 | 0.0532 | 0.0913 |
| Average | 0.224 | 0.186 | 0.224 | 0.309 | 0.224 | 0.300 |
| Stdev | 0.578 | 0.355 | 0.578 | 0.413 | 0.578 | 0.421 |
| p(t-test) | | 0.77 | | 0.63 | | 0.73 |
| Min | 4.60E-6 | 0.00263 | 4.60E-6 | 0.0202 | 4.60E-6 | 0.00631 |
| Max | 5.72 | 1.39 | 5.72 | 1.25 | 5.72 | 1.15 |
| n (Samp) | 379 | 20 | 379 | 11 | 379 | 7 |
| n (Patient) | 176 | 20 | 176 | 11 | 176 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0535 | 0.0831 | 0.0535 | 0.0496 | 0.0535 | 0.0281 |
| Average | 0.213 | 0.208 | 0.213 | 0.229 | 0.213 | 0.246 |
| Stdev | 0.445 | 0.504 | 0.445 | 0.423 | 0.445 | 0.519 |
| p(t-test) | | 0.94 | | 0.80 | | 0.76 |
| Min | 9.65E-6 | 7.01E-5 | 9.65E-6 | 4.60E-6 | 9.65E-6 | 0.00288 |
| Max | 3.21 | 3.36 | 3.21 | 2.11 | 3.21 | 2.05 |
| n (Samp) | 183 | 64 | 183 | 62 | 183 | 18 |
| n (Patient) | 89 | 64 | 89 | 62 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.53 | 0.51 | 0.51 | 0.66 | 0.48 | 0.38 | 0.60 | 0.41 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.041 | 0.067 | 0.042 | 0.045 | 0.091 | 0.043 | 0.080 | 0.11 | 0.073 |
| p | 0.51 | 0.61 | 0.75 | 0.82 | 0.074 | 0.67 | 0.14 | 0.40 | 0.22 |
| nCohort 1 | 157 | 379 | 183 | 157 | 379 | 183 | 157 | 379 | 183 |
| nCohort 2 | 73 | 20 | 64 | 56 | 11 | 62 | 15 | 7 | 18 |
| Cutoff 1 | 0.0172 | 0.0301 | 0.0209 | 0.0138 | 0.0787 | 0.0139 | 0.00459 | 0.0541 | 0.00472 |
| Sens 1 | 71% | 70% | 70% | 71% | 73% | 71% | 73% | 71% | 72% |
| Spec 1 | 24% | 37% | 23% | 22% | 61% | 15% | 10% | 51% | 7% |
| Cutoff 2 | 0.0111 | 0.0164 | 0.0109 | 0.00901 | 0.0288 | 0.00901 | 0.00440 | 0.00953 | 0.00440 |
| Sens 2 | 81% | 80% | 81% | 80% | 82% | 81% | 80% | 86% | 83% |
| Spec 2 | 20% | 28% | 13% | 18% | 37% | 11% | 10% | 21% | 5% |
| Cutoff 3 | 0.00481 | 0.0157 | 0.00481 | 0.00323 | 0.0241 | 0.00323 | 0.00301 | 0.00624 | 0.00288 |
| Sens 3 | 90% | 90% | 91% | 91% | 91% | 90% | 93% | 100% | 94% |
| Spec 3 | 12% | 27% | 7% | 8% | 34% | 3% | 8% | 15% | 3% |
| Cutoff 4 | 0.0859 | 0.121 | 0.120 | 0.0859 | 0.121 | 0.120 | 0.0859 | 0.121 | 0.120 |
| Sens 4 | 44% | 30% | 38% | 43% | 45% | 37% | 27% | 43% | 33% |
| Spec 4 | 70% | 70% | 70% | 70% | 70% | 70% | 70% | 70% | 70% |
| Cutoff 5 | 0.133 | 0.224 | 0.182 | 0.133 | 0.224 | 0.182 | 0.133 | 0.224 | 0.182 |
| Sens 5 | 29% | 10% | 31% | 34% | 27% | 31% | 27% | 29% | 28% |
| Spec 5 | 80% | 80% | 80% | 80% | 80% | 80% | 80% | 80% | 80% |
| Cutoff 6 | 0.546 | 0.500 | 0.584 | 0.546 | 0.500 | 0.584 | 0.546 | 0.500 | 0.584 |
| Sens 6 | 5% | 10% | 5% | 9% | 27% | 8% | 0% | 29% | 11% |
| Spec 6 | 90% | 90% | 90% | 90% | 90% | 90% | 90% | 90% | 90% |
| OR Quart 2 p Value 95% CI of OR Quart2 | 0.53 | 4.2 | 0.46 | 0.45 | >3.1 | 0.34 | 0.23 | 0 | 0.15 |
| | 0.14 | 0.073 | 0.080 | 0.081 | <0.34 | 0.016 | 0.20 | na | 0.088 |
| | 0.23 | 0.87 | 0.19 | 0.19 | >0.31 | 0.14 | 0.025 | na | 0.018 |
| | 1.2 | 20 | 1.1 | 1.1 | na | 0.82 | 2.2 | na | 1.3 |
| OR Quart 3 p Value 95% CI ot OR Quart3 | 0.79 | 2.6 | 0.76 | 0.35 | >4.2 | 0.43 | 0.48 | 1.0 | 0.48 |
| | 0.55 | 0.27 | 0.51 | 0.027 | <0.21 | 0.048 | 0.41 | 1.0 | 0.32 |
| | 0.36 | 0.48 | 0.34 | 0.13 | >0.46 | 0.19 | 0.082 | 0.14 | 0.11 |
| | 1.7 | 13 | 1.7 | 0.89 | na | 0.99 | 2.7 | 7.2 | 2.0 |
| OR Quart 4 p Value | 1.2 | 2.6 | 1.2 | 1.1 | >4.1 | 1.0 | 2.2 | 1.5 | 1.4 |
| | 0.61 | 0.27 | 0.60 | 0.74 | <0.21 | 0.95 | 0.22 | 0.66 | 0.54 |
| | 0.57 | 0.48 | 0.57 | 0.52 | >0.45 | 0.49 | 0.62 | 0.25 | 0.46 |
| 95% CI of OR Quart4 | 2.6 | 13 | 2.6 | 2.5 | na | 2.2 | 8.0 | 9.2 | 4.5 |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 14.8 | 15.5 | 14.8 | 18.1 | 14.8 | 19.0 |
| Average | 17.6 | 20.5 | 17.6 | 26.0 | 17.6 | 22.0 |
| Stdev | 11.1 | 16.7 | 11.1 | 23.4 | 11.1 | 16.8 |
| p(t-test) | | 0.12 | | 5.2E-4 | | 0.17 |
| Min | 5.52 | 7.53 | 5.52 | 5.96 | 5.52 | 5.20 |
| Max | 98.9 | 109 | 98.9 | 113 | 98.9 | 69.4 |
| n (Samp) | 156 | 70 | 156 | 54 | 156 | 14 |
| n (Patient) | 87 | 70 | 87 | 54 | 87 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.6 | 18.1 | 15.6 | 18.3 | 15.6 | 25.9 |
| Average | 18.6 | 24.9 | 18.6 | 23.3 | 18.6 | 33.9 |
| Stdev | 12.4 | 24.3 | 12.4 | 14.1 | 12.4 | 19.4 |
| p(t-test) | | 0.042 | | 0.24 | | 0.0016 |
| Min | 5.20 | 9.73 | 5.20 | 8.57 | 5.20 | 9.67 |
| Max | 113 | 109 | 113 | 50.8 | 113 | 61.5 |
| n (Samp) | 371 | 19 | 371 | 10 | 371 | 7 |
| n (Patient) | 173 | 19 | 173 | 10 | 173 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 15.5 | 15.5 | 15.5 | 18.1 | 15.5 | 21.1 |
| Average | 20.2 | 19.5 | 20.2 | 26.9 | 20.2 | 21.5 |
| Stdev | 19.3 | 13.6 | 19.3 | 24.7 | 19.3 | 14.6 |
| p(t-test) | | 0.81 | | 0.031 | | 0.79 |
| Min | 5.52 | 7.53 | 5.52 | 5.96 | 5.52 | 5.20 |
| Max | 186 | 73.8 | 186 | 113 | 186 | 69.4 |
| n (Samp) | 180 | 62 | 180 | 60 | 180 | 17 |
| n (Patient) | 88 | 62 | 88 | 60 | 88 | 17 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.56 | 0.60 | 0.52 | 0.62 | 0.60 | 0.61 | 0.57 | 0.78 | 0.58 |
| SE | 0.042 | 0.070 | 0.043 | 0.046 | 0.096 | 0.043 | 0.083 | 0.10 | 0.075 |
| p | 0.14 | 0.14 | 0.60 | 0.0082 | 0.29 | 0.014 | 0.41 | 0.0073 | 0.28 |
| nCohort 1 | 156 | 371 | 180 | 156 | 371 | 180 | 156 | 371 | 180 |
| nCohort 2 | 70 | 19 | 62 | 54 | 10 | 60 | 14 | 7 | 17 |
| Cutoff 1 | 13.6 | 14.7 | 13.2 | 15.8 | 17.4 | 15.9 | 11.5 | 22.5 | 17.1 |
| Sens 1 | 70% | 74% | 71% | 70% | 70% | 70% | 71% | 71% | 71% |
| Spec 1 | 45% | 45% | 39% | 54% | 61% | 51% | 26% | 80% | 57% |
| Cutoff 2 | 12.8 | 13.8 | 12.4 | 12.6 | 12.1 | 13.7 | 8.14 | 21.3 | 11.2 |
| Sens 2 | 80% | 84% | 81% | 81% | 80% | 80% | 86% | 86% | 82% |
| Spec 2 | 38% | 39% | 33% | 38% | 26% | 42% | 6% | 78% | 21% |
| Cutoff 3 | 10.4 | 11.5 | 10.3 | 10.2 | 8.69 | 10.4 | 5.52 | 9.66 | 5.52 |
| Sens 3 | 90% | 95% | 90% | 91% | 90% | 90% | 93% | 100% | 94% |
| Spec 3 | 18% | 22% | 16% | 17% | 7% | 17% | 1% | 10% | 1% |
| Cutoff 4 | 19.4 | 19.5 | 20.2 | 19.4 | 19.5 | 20.2 | 19.4 | 19.5 | 20.2 |
| Sens 4 | 27% | 37% | 26% | 37% | 40% | 33% | 50% | 86% | 59% |
| Spec 4 | 71% | 70% | 70% | 71% | 70% | 70% | 71% | 70% | 70% |
| Cutoff 5 | 21.3 | 22.5 | 22.8 | 21.3 | 22.5 | 22.8 | 21.3 | 22.5 | 22.8 |
| Sens 5 | 21% | 16% | 18% | 31% | 40% | 27% | 36% | 71% | 41% |
| Spec 5 | 80% | 80% | 80% | 80% | 80% | 80% | 80% | 80% | 80% |
| Cutoff 6 | 26.6 | 29.0 | 31.2 | 26.6 | 29.0 | 31.2 | 26.6 | 29.0 | 31.2 |
| Sens 6 | 13% | 11% | 6% | 24% | 30% | 23% | 21% | 43% | 12% |
| Spec 6 | 90% | 90% | 90% | 90% | 90% | 90% | 90% | 90% | 90% |
| OR Quart 2 p Value 95% CI of OR Quart2 | 3.1 | 2.6 | 2.2 | 1.4 | 0.49 | 2.0 | 0.23 | 0 | 0 |
| | 0.010 | 0.27 | 0.072 | 0.48 | 0.57 | 0.16 | 0.19 | na | na |
| | 1.3 | 0.48 | 0.93 | 0.53 | 0.044 | 0.76 | 0.024 | na | na |
| | 7.4 | 13 | 5.1 | 3.9 | 5.5 | 5.1 | 2.1 | na | na |
| OR Quart 3 p Value 95% CI of OR Quart3 | 2.4 | 3.1 | 1.8 | 2.9 | 1.5 | 3.2 | 0.73 | 0 | 1.0 |
| | 0.055 | 0.17 | 0.20 | 0.027 | 0.65 | 0.012 | 0.69 | na | 1.0 |
| | 0.98 | 0.62 | 0.74 | 1.1 | 0.25 | 1.3 | 0.15 | na | 0.27 |
| | 5.7 | 16 | 4.2 | 7.5 | 9.3 | 8.1 | 3.5 | na | 3.7 |
| OR Quart 4 p Value 95% Clot OR Quart4 | 2.1 | 3.1 | 1.3 | 2.6 | 2.0 | 2.8 | 1.5 | 6.3 | 1.4 |
| | 0.095 | 0.17 | 0.53 | 0.049 | 0.42 | 0.030 | 0.53 | 0.092 | 0.56 |
| | 0.88 | 0.61 | 0.55 | 1.0 | 0.36 | 1.1 | 0.40 | 0.74 | 0.42 |
| | 5.1 | 16 | 3.2 | 6.7 | 11 | 7.0 | 5.9 | 53 | 4.9 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 477 | 610 | 477 | 714 | 477 | 860 |
| Average | 830 | 1060 | 830 | 1140 | 830 | 938 |
| Stdev | 1330 | 1520 | 1330 | 1500 | 1330 | 817 |
| p(t-test) | | 0.24 | | 0.15 | | 0.76 |
| Min | 94.2 | 86.2 | 94.2 | 160 | 94.2 | 143 |
| Max | 13100 | 9040 | 13100 | 9500 | 13100 | 3300 |
| n (Samp) | 158 | 73 | 158 | 55 | 158 | 15 |
| n (Patient) | 88 | 73 | 88 | 55 | 88 | 15 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 564 | 756 | 564 | 1140 | 564 | 642 |
| Average | 1010 | 1070 | 1010 | 1470 | 1010 | 664 |
| Stdev | 1680 | 983 | 1680 | 1470 | 1680 | 226 |
| p(t-test) | | 0.89 | | 0.37 | | 0.58 |
| Min | 43.9 | 205 | 43.9 | 180 | 43.9 | 385 |
| Max | 18400 | 4250 | 18400 | 5760 | 18400 | 1030 |
| n (Samp) | 378 | 20 | 378 | 11 | 378 | 7 |
| n (Patient) | 175 | 20 | 175 | 11 | 175 | 7 |

| UO only | 0hr prior to AKI stage | | 24hr prior to AKI stage | | 48 hr prior to AKI stage | |
|---|---|---|---|---|---|---|
| | Cohort 1 | Cohort 2 | Cohort 1 | Cohort 2 | Cohort 1 | Cohort 2 |
| Median | 527 | 668 | 527 | 684 | 527 | 852 |
| Average | 1330 | 1100 | 1330 | 1050 | 1330 | 942 |
| Stdev | 2900 | 1570 | 2900 | 1320 | 2900 | 738 |
| p(t-test) | | 0.55 | | 0.46 | | 0.57 |
| Min | 63.0 | 86.2 | 63.0 | 160 | 63.0 | 143 |
| Max | 26100 | 9040 | 26100 | 9500 | 26100 | 3300 |
| n (Samp) | 184 | 64 | 184 | 61 | 184 | 18 |
| n (Patient) | 89 | 64 | 89 | 61 | 89 | 18 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.59 | 0.56 | 0.60 | 0.73 | 0.55 | 0.61 | 0.55 | 0.59 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.041 | 0.068 | 0.042 | 0.046 | 0.088 | 0.043 | 0.080 | 0.11 | 0.073 |
| p | 0.019 | 0.18 | 0.18 | 0.027 | 0.0098 | 0.22 | 0.15 | 0.67 | 0.21 |
| nCohort 1 | 158 | 378 | 184 | 158 | 378 | 184 | 158 | 378 | 184 |
| nCohort 2 | 73 | 20 | 64 | 55 | 11 | 61 | 15 | 7 | 18 |
| Cutoff 1 | 417 | 486 | 434 | 357 | 1030 | 394 | 411 | 624 | 565 |
| Sens 1 | 71% | 70% | 70% | 71% | 73% | 70% | 73% | 71% | 72% |
| Spec 1 | 41% | 44% | 40% | 37% | 76% | 37% | 41% | 56% | 55% |
| Cutoff 2 | 371 | 450 | 379 | 256 | 901 | 265 | 379 | 417 | 411 |
| Sens 2 | 81% | 80% | 81% | 80% | 82% | 80% | 80% | 86% | 83% |
| Spec 2 | 40% | 39% | 36% | 18% | 70% | 20% | 41% | 35% | 38% |
| Cutoff 3 | 221 | 257 | 221 | 228 | 720 | 240 | 162 | 382 | 162 |
| Sens 3 | 90% | 90% | 91% | 91% | 91% | 90% | 93% | 100% | 94% |
| Spec 3 | 15% | 16% | 15% | 16% | 62% | 16% | 7% | 32% | 7% |
| Cutoff 4 | 678 | 901 | 886 | 678 | 901 | 886 | 678 | 901 | 886 |
| Sens 4 | 47% | 40% | 34% | 55% | 82% | 43% | 60% | 14% | 39% |
| Spec 4 | 70% | 70% | 70% | 70% | 70% | 70% | 70% | 70% | 70% |
| Cutoff 5 | 895 | 1180 | 1200 | 895 | 1180 | 1200 | 895 | 1180 | 1200 |
| Sens 5 | 34% | 35% | 23% | 45% | 45% | 31% | 40% | 0% | 17% |
| Spec 5 | 80% | 80% | 80% | 80% | 80% | 80% | 80% | 80% | 80% |
| Cutoff 6 | 1840 | 2000 | 2700 | 1840 | 2000 | 2700 | 1840 | 2000 | 2700 |
| Sens 6 | 10% | 10% | 8% | 13% | 9% | 5% | 13% | 0% | 6% |
| Spec 6 | 91% | 90% | 90% | 91% | 90% | 90% | 91% | 90% | 90% |
| OR Quart 2 p Value 95% CI of OR Quart2 | 1.4 | 1.3 | 1.5 | 0.50 | 0 | 0.82 | 0.65 | >2.0 | 0.64 |
| | 0.43 | 0.71 | 0.38 | 0.16 | na | 0.66 | 0.65 | <0.56 | 0.63 |
| | 0.61 | 0.29 | 0.62 | 0.19 | na | 0.34 | 0.10 | >0.18 | 0.10 |
| | 3.2 | 6.1 | 3.5 | 1.3 | na | 2.0 | 4.1 | na | 4.0 |
| OR Quart 3 p Value 95% CI or OR Quart3 | 1.5 | 2.1 | 2.2 | 0.65 | 2.0 | 1.0 | 1.0 | >4.2 | 3.0 |
| | 0.32 | 0.32 | 0.065 | 0.36 | 0.57 | 1.0 | 1.0 | <0.21 | 0.12 |
| | 0.66 | 0.50 | 0.95 | 0.26 | 0.18 | 0.43 | 0.19 | >0.46 | 0.74 |
| | 3.5 | 8.5 | 5.1 | 1.6 | 23 | 2.3 | 5.3 | na | 12 |
| OR Quart 4 p Value 95% CI of OR Quart4 | 2.6 | 2.4 | 1.9 | 2.1 | 8.5 | 1.7 | 2.5 | >1.0 | 1.7 |
| | 0.022 | 0.21 | 0.14 | 0.081 | 0.045 | 0.18 | 0.20 | <1.0 | 0.48 |
| | 1.1 | 0.60 | 0.81 | 0.92 | 1.0 | 0.78 | 0.61 | >0.062 | 0.38 |
| | 5.8 | 9.6 | 4.4 | 4.7 | 70 | 3.8 | 10 | na | 7.5 |

Fig. 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.6 | 39.3 | 31.6 | 42.7 | 31.6 | 47.1 |
| Average | 48.9 | 60.6 | 48.9 | 69.8 | 48.9 | 58.5 |
| Stdev | 111 | 63.3 | 111 | 69.1 | 111 | 48.0 |
| p(t-test) | | 0.58 | | 0.28 | | 0.69 |
| Min | 1.77 | 9.35 | 1.77 | 12.8 | 1.77 | 8.79 |
| Max | 1900 | 243 | 1900 | 339 | 1900 | 179 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 32.7 | 39.3 | 32.7 | 44.3 | 32.7 | 49.6 |
| Average | 50.3 | 60.2 | 50.3 | 69.5 | 50.3 | 61.2 |
| Stdev | 112 | 63.1 | 112 | 68.2 | 112 | 49.3 |
| p(t-test) | | 0.64 | | 0.32 | | 0.67 |
| Min | 2.68 | 9.35 | 2.68 | 12.8 | 2.68 | 8.79 |
| Max | 1900 | 243 | 1900 | 339 | 1900 | 179 |
| n (Samp) | 347 | 28 | 347 | 35 | 347 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | nd | 0.58 | 0.65 | nd | 0.64 | 0.63 | nd | 0.63 |
| SE | 0.058 | nd | 0.058 | 0.053 | nd | 0.052 | 0.065 | nd | 0.069 |
| p | 0.11 | nd | 0.18 | 0.0043 | nd | 0.0068 | 0.050 | nd | 0.054 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 29.1 | nd | 29.1 | 30.3 | nd | 30.3 | 25.8 | nd | 28.9 |
| Sens 1 | 71% | nd | 71% | 71% | nd | 71% | 73% | nd | 70% |
| Spec 1 | 47% | nd | 45% | 48% | nd | 46% | 42% | nd | 44% |
| Cutoff 2 | 19.0 | nd | 19.0 | 25.0 | nd | 27.0 | 22.1 | nd | 24.9 |
| Sens 2 | 82% | nd | 82% | 82% | nd | 80% | 82% | nd | 80% |
| Spec 2 | 30% | nd | 27% | 41% | nd | 41% | 37% | nd | 38% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 3 | 13.6 | nd | 13.6 | 22.1 | nd | 22.1 | 15.3 | nd | 17.6 |
| Sens 3 | 93% | nd | 93% | 91% | nd | 91% | 91% | nd | 90% |
| Spec 3 | 19% | nd | 16% | 37% | nd | 34% | 22% | nd | 24% |
| Cutoff 4 | 44.8 | nd | 45.5 | 44.8 | nd | 45.5 | 44.8 | nd | 45.5 |
| Sens 4 | 36% | nd | 36% | 47% | nd | 46% | 64% | nd | 65% |
| Spec 4 | 70% | nd | 70% | 70% | nd | 70% | 70% | nd | 70% |
| Cutoff 5 | 61.9 | nd | 62.7 | 61.9 | nd | 62.7 | 61.9 | nd | 62.7 |
| Sens 5 | 25% | nd | 25% | 32% | nd | 31% | 23% | nd | 25% |
| Spec 5 | 80% | nd | 80% | 80% | nd | 80% | 80% | nd | 80% |
| Cutoff 6 | 87.8 | nd | 88.3 | 87.8 | nd | 88.3 | 87.8 | nd | 88.3 |
| Sens 6 | 21% | nd | 18% | 24% | nd | 23% | 18% | nd | 20% |
| Spec 6 | 90% | nd | 90% | 90% | nd | 90% | 90% | nd | 90% |
| OR Quart 2 | 1.2 | nd | 1.4 | 5.4 | nd | 6.6 | 1.7 | nd | 1.3 |
|  | 0.76 | nd | 0.57 | 0.033 | nd | 0.015 | 0.48 | nd | 0.71 |
| p Value 95% CI of OR Quart2 | 0.36 | nd | 0.43 | 1.2 | nd | 1.4 | 0.39 | nd | 0.29 |
|  | 4.1 | nd | 4.6 | 25 | nd | 31 | 7.3 | nd | 6.1 |
| OR Quart 3 p Value 95% CI of OR Quart3 | 1.7 | nd | 1.6 | 5.5 | nd | 4.3 | 1.7 | nd | 1.7 |
|  | 0.39 | nd | 0.40 | 0.031 | nd | 0.071 | 0.47 | nd | 0.48 |
|  | 0.52 | nd | 0.52 | 1.2 | nd | 0.88 | 0.40 | nd | 0.39 |
|  | 5.3 | nd | 5.2 | 26 | nd | 21 | 7.3 | nd | 7.3 |
| OR Quart 4 p Value 95% CI of OR Quart4 | 1.9 | nd | 1.6 | 6.6 | nd | 7.3 | 3.2 | nd | 2.8 |
|  | 0.27 | nd | 0.40 | 0.015 | nd | 0.010 | 0.091 | nd | 0.14 |
|  | 0.61 | nd | 0.52 | 1.4 | nd | 1.6 | 0.83 | nd | 0.72 |
|  | 5.8 | nd | 5.2 | 30 | nd | 33 | 12 | nd | 11 |

**Amphiregulin**

| sCr or UO | 0hr prior to AKI stage | | 24hr prior to AKI stage | | 48 hr prior to AKI stage | |
|---|---|---|---|---|---|---|
|  | Cohort 1 | Cohort 2 | Cohort 1 | Cohort 2 | Cohort 1 | Cohort 2 |
| Median | 22.0 | 26.7 | 22.0 | 26.2 | 22.0 | 23.7 |
| Average | 27.4 | 29.5 | 27.4 | 33.1 | 27.4 | 41.5 |
| Stdev | 21.5 | 20.0 | 21.5 | 24.5 | 21.5 | 41.2 |
| p(t-test) |  | 0.61 |  | 0.16 |  | 0.0056 |
| Min | 0.00246 | 0.00246 | 0.00246 | 4.44 | 0.00246 | 2.76 |
| Max | 198 | 93.0 | 198 | 133 | 198 | 162 |
| n (Samp) | 349 | 28 | 349 | 32 | 349 | 22 |
| n (Patient) | 172 | 28 | 172 | 32 | 172 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.7 | 26.7 | 21.7 | 25.1 | 21.7 | 23.7 |
| Average | 27.1 | 29.8 | 27.1 | 32.8 | 27.1 | 40.0 |
| Stdev | 21.5 | 20.0 | 21.5 | 24.2 | 21.5 | 40.9 |
| p(t-test) | | 0.53 | | 0.16 | | 0.016 |
| Min | 0.00246 | 0.00246 | 0.00246 | 4.44 | 0.00246 | 2.76 |
| Max | 198 | 93.0 | 198 | 133 | 198 | 162 |
| n (Samp) | 340 | 28 | 340 | 33 | 340 | 20 |
| n (Patient) | 158 | 28 | 158 | 33 | 158 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | only | UO only | sCr or UO | sCr only | UO only |
| AUC | 0.55 | nd | 0.56 | 0.58 | nd | 0.58 | 0.55 | nd | 0.55 |
| SE | 0.058 | nd | 0.058 | 0.055 | nd | 0.054 | 0.065 | nd | 0.068 |
| p | 0.38 | nd | 0.31 | 0.13 | nd | 0.12 | 0.40 | nd | 0.49 |
| nCohort 1 | 349 | nd | 340 | 349 | nd | 340 | 349 | nd | 340 |
| nCohort 2 | 28 | nd | 28 | 32 | nd | 33 | 22 | nd | 20 |
| Cutoff 1 | 17.0 | nd | 17.0 | 18.8 | nd | 18.8 | 14.3 | nd | 14.3 |
| Sens 1 | 71% | nd | 71% | 72% | nd | 73% | 73% | nd | 70% |
| Spec 1 | 36% | nd | 37% | 41% | nd | 42% | 27% | nd | 27% |
| Cutoff 2 | 12.3 | nd | 12.3 | 17.0 | nd | 17.0 | 11.7 | nd | 11.7 |
| Sens 2 | 82% | nd | 82% | 81% | nd | 82% | 82% | nd | 80% |
| Spec 2 | 18% | nd | 18% | 36% | nd | 37% | 17% | nd | 17% |
| Cutoff 3 | 8.57 | nd | 8.57 | 12.3 | nd | 12.3 | 11.0 | nd | 11.0 |
| Sens 3 | 93% | nd | 93% | 91% | nd | 91% | 91% | nd | 90% |
| Spec 3 | 10% | nd | 10% | 18% | nd | 18% | 15% | nd | 15% |
| Cutoff 4 | 30.9 | nd | 30.4 | 30.9 | nd | 30.4 | 30.9 | nd | 30.4 |
| Sens 4 | 39% | nd | 43% | 47% | nd | 45% | 36% | nd | 35% |
| Spec 4 | 71% | nd | 70% | 71% | nd | 70% | 71% | nd | 70% |
| Cutoff 5 | 39.0 | nd | 37.0 | 39.0 | nd | 37.0 | 39.0 | nd | 37.0 |
| Sens 5 | 25% | nd | 29% | 25% | nd | 36% | 36% | nd | 35% |
| Spec 5 | 81% | nd | 80% | 81% | nd | 80% | 81% | nd | 80% |
| Cutoff 6 | 52.6 | nd | 50.7 | 52.6 | nd | 50.7 | 52.6 | nd | 50.7 |
| Sens 6 | 11% | nd | 11% | 16% | nd | 18% | 27% | nd | 25% |
| Spec 6 | 90% | nd | 90% | 90% | nd | 90% | 90% | nd | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | only | UO only | sCr or UO | sCr only | UO only |
| OR Quart2 p Value 95% CI of OR Quart2 | 0.55 | nd | 0.55 | 1.9 | nd | 2.1 | 0.99 | nd | 0.79 |
|  | 0.36 | nd | 0.36 | 0.27 | nd | 0.19 | 0.99 | nd | 0.73 |
|  | 0.16 | nd | 0.16 | 0.61 | nd | 0.70 | 0.28 | nd | 0.21 |
|  | 2.0 | nd | 2.0 | 5.8 | nd | 6.5 | 3.5 | nd | 3.0 |
| OR Quart3 p Value 95% CI of OR Quart3 | 1.2 | nd | 1.2 | 1.2 | nd | 1.0 | 0.78 | nd | 0.79 |
|  | 0.79 | nd | 0.79 | 0.76 | nd | 1.0 | 0.72 | nd | 0.73 |
|  | 0.40 | nd | 0.40 | 0.36 | nd | 0.28 | 0.20 | nd | 0.21 |
|  | 3.3 | nd | 3.3 | 4.1 | nd | 3.6 | 3.0 | nd | 3.0 |
| OR Quart4 p Value 95% CI OR Quart4 | 1.3 | nd | 1.3 | 2.6 | nd | 2.8 | 1.6 | nd | 1.4 |
|  | 0.62 | nd | 0.60 | 0.088 | nd | 0.058 | 0.40 | nd | 0.55 |
|  | 0.46 | nd | 0.47 | 0.87 | nd | 0.96 | 0.52 | nd | 0.44 |
|  | 3.6 | nd | 3.7 | 7.6 | nd | 8.3 | 5.2 | nd | 4.7 |

**Betacellulin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.86 | 3.89 | 3.86 | 4.40 | 3.86 | 4.54 |
| Average | 4.76 | 4.02 | 4.76 | 6.09 | 4.76 | 6.45 |
| Stdev | 5.85 | 2.57 | 5.85 | 7.48 | 5.85 | 6.05 |
| p(t-test) |  | 0.50 |  | 0.22 |  | 0.19 |
| Min | 0.00226 | 0.00282 | 0.00226 | 0.00289 | 0.00226 | 0.00274 |
| Max | 60.7 | 10.0 | 60.7 | 3.4 | 60.7 | 24.5 |
| n (Samp) | 349 | 28 | 349 | 33 | 349 | 22 |
| n (Patient) | 173 | 28 | 173 | 33 | 173 | 22 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 3.73 | 3.63 | 3.73 | 4.36 | 3.73 | 4.54 |
| Average | 4.62 | 3.87 | 4.62 | 6.00 | 4.62 | 6.21 |
| Stdev | 5.86 | 2.68 | 5.86 | 7.39 | 5.86 | 5.63 |
| p(t-test) |  | 0.50 |  | 0.20 |  | 0.24 |
| Min | 0.00226 | 0.00282 | 0.00226 | 0.00289 | 0.00226 | 0.700 |
| Max | 60.7 | 10.0 | 60.7 | 43.4 | 60.7 | 24.5 |
| n (Samp) | 340 | 28 | 340 | 34 | 340 | 20 |
| n (Patient) | 159 | 28 | 159 | 34 | 159 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | nd | 0.49 | 0.57 | nd | 0.57 | 0.58 | nd | 0.60 |
| SE | 0.057 | nd | 0.057 | 0.054 | nd | 0.053 | 0.065 | nd | 0.069 |
| p | 0.97 | nd | 0.90 | 0.23 | nd | 0.18 | 0.24 | nd | 0.16 |
| nCohort 1 | 349 | nd | 340 | 349 | nd | 340 | 349 | nd | 340 |
| nCohort 2 | 28 | nd | 28 | 33 | nd | 34 | 22 | nd | 20 |
| Cutoff 1 | 3.11 | nd | 2.65 | 3.09 | nd | 3.09 | 2.89 | nd | 3.54 |
| Sens 1 | 71% | nd | 71% | 73% | nd | 71% | 73% | nd | 70% |
| Spec 1 | 38% | nd | 35% | 38% | nd | 39% | 35% | nd | 46% |
| Cutoff 2 | 1.34 | nd | 0.549 | 2.62 | nd | 2.62 | 2.26 | nd | 2.65 |
| Sens 2 | 82% | nd | 82% | 82% | nd | 82% | 82% | nd | 80% |
| Spec 2 | 16% | nd | 14% | 32% | nd | 34% | 26% | nd | 35% |
| Cutoff 3 | 0.00282 | nd | 0.00282 | 1.78 | nd | 1.78 | 0.743 | nd | 1.92 |
| Sens 3 | 96% | nd | 96% | 91% | nd | 91% | 91% | nd | 90% |
| Spec 3 | 5% | nd | 6% | 23% | nd | 25% | 13% | nd | 26% |
| Cutoff 4 | 5.42 | nd | 5.30 | 5.42 | nd | 5.30 | 5.42 | nd | 5.30 |
| Sens 4 | 25% | nd | 32% | 33% | nd | 32% | 36% | nd | 35% |
| Spec 4 | 70% | nd | 71% | 70% | nd | 71% | 70% | nd | 71% |
| Cutoff 5 | 6.65 | nd | 6.31 | 6.65 | nd | 6.31 | 6.65 | nd | 6.31 |
| Sens 5 | 14% | nd | 18% | 24% | nd | 26% | 32% | nd | 30% |
| Spec 5 | 80% | nd | 80% | 80% | nd | 80% | 80% | nd | 80% |
| Cutoff 6 | 8.60 | nd | 8.27 | 8.60 | nd | 8.27 | 8.60 | nd | 8.27 |
| Sens 6 | 4% | nd | 4% | 15% | nd | 18% | 23% | nd | 20% |
| Spec 6 | 90% | nd | 90% | 90% | nd | 90% | 90% | nd | 90% |
| OR Quart 2 p Value 95% CI of OR Quart2 | 1.4 | nd | 1.2 | 1.6 | nd | 1.6 | 1.2 | nd | 2.6 |
| | 0.57 | nd | 0.77 | 0.40 | nd | 0.40 | 0.75 | nd | 0.26 |
| | 0.46 | nd | 0.38 | 0.52 | nd | 0.52 | 0.32 | nd | 0.49 |
| | 4.1 | nd | 3.7 | 5.2 | nd | 5.2 | 4.8 | nd | 14 |
| OR Quart 3 p Value 95% CI of OR Quart3 | 1.4 | nd | 1.4 | 2.1 | nd | 2.4 | 1.5 | nd | 3.7 |
| | 0.57 | nd | 0.58 | 0.19 | nd | 0.13 | 0.53 | nd | 0.11 |
| | 0.46 | nd | 0.45 | 0.70 | nd | 0.79 | 0.41 | nd | 0.75 |
| | 4.1 | nd | 4.1 | 6.4 | nd | 7.1 | 5.6 | nd | 18 |
| OR Quart 4 p Value 95% CI of OR Quart4 | 1.0 | nd | 1.2 | 2.1 | nd | 2.1 | 1.8 | nd | 3.1 |
| | 0.98 | nd | 0.77 | 0.19 | nd | 0.19 | 0.37 | nd | 0.17 |
| | 0.31 | nd | 0.38 | 0.69 | nd | 0.69 | 0.51 | nd | 0.62 |
| | 3.3 | nd | 3.7 | 6.4 | nd | 6.4 | 6.3 | nd | 16 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 39200 | 48000 | 39200 | 33300 | 39200 | 47800 |
| Average | 49200 | 62300 | 49200 | 53400 | 49200 | 46300 |
| Stdev | 41200 | 42600 | 41200 | 51700 | 41200 | 25400 |
| p(t-test) | | 0.11 | | 0.59 | | 0.74 |
| Min | 3600 | 13300 | 3600 | 110400 | 3600 | 12800 |
| Max | 483000 | 180000 | 483000 | 239000 | 483000 | 104000 |
| n (Samp) | 357 | 28 | 357 | 34 | 357 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40400 | 48000 | 40400 | 34000 | 40400 | 41700 |
| Average | 49800 | 59800 | 49800 | 55800 | 49800 | 44900 |
| Stdev | 41400 | 38100 | 41400 | 52800 | 41400 | 26200 |
| p(t-test) | | 0.22 | | 0.43 | | 0.60 |
| Min | 3600 | 13300 | 3600 | 10400 | 3600 | 12800 |
| Max | 483000 | 180000 | 483000 | 239000 | 483000 | 104000 |
| n (Samp) | 348 | 28 | 348 | 35 | 348 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.59 | 0.47 | nd | 0.48 | 0.52 | nd | 0.48 |
| SE | 0.058 | nd | 0.058 | 0.053 | nd | 0.052 | 0.064 | nd | 0.067 |
| p | 0.094 | nd | 0.13 | 0.62 | nd | 0.74 | 0.81 | nd | 0.81 |
| nCohort 1 | 357 | nd | 348 | 357 | nd | 348 | 357 | nd | 348 |
| nCohort 2 | 28 | nd | 28 | 34 | nd | 35 | 22 | nd | 20 |
| Cutoff 1 | 33300 | nd | 33300 | 25600 | nd | 25600 | 31800 | nd | 31800 |
| Sens 1 | 71% | nd | 71% | 71% | nd | 71% | 73% | nd | 70% |
| Spec 1 | 39% | nd | 38% | 22% | nd | 22% | 34% | nd | 33% |
| Cutoff 2 | 30600 | nd | 30600 | 18900 | nd | 21000 | 18300 | nd | 18300 |
| Sens 2 | 82% | nd | 82% | 85% | nd | 80% | 82% | nd | 80% |
| Spec 2 | 32% | nd | 31% | 12% | nd | 14% | 11% | nd | 10% |
| Cutoff 3 | 19600 | nd | 19600 | 13600 | nd | 13600 . | 14200 | nd | 14200 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 93% | nd | 93% | 91% | nd | 91% | 91% | nd | 90% |
| Spec 3 | 13% | nd | 12% | 6% | nd | 5% | 6% | nd | 5% |
| Cutoff 4 | 53100 | nd | 53500 | 53100 | nd | 53500 | 53100 | nd | 53500 |
| Sens 4 | 46% | nd | 46% | 32% | nd | 34% | 41% | nd | 35% |
| Spec 4 | 70% | nd | 70% | 70% | nd | 70% | 70% | nd | 70% |
| Cutoff 5 | 65300 | nd | 65900 | 65300 | nd | 65900 | 65300 | nd | 65900 |
| Sens 5 | 39% | nd | 39% | 29% | nd | 29% | 27% | nd | 25% |
| Spec 5 | 80% | nd | 80% | 80% | nd | 80% | 80% | nd | 80% |
| Cutoff 6 | 88800 | nd | 88800 | 88800 | nd | 88800 | 88800 | nd | 88800 |
| Sens 6 | 21% | nd | 21% | 12% | nd | 14% | 5% | nd | 5% |
| Spec 6 | 90% | nd | 90% | 90% | nd | 90% | 90% | nd | 90% |
| OR Quart 2 p Value 95% CI OR Quart2 | 1.4 | nd | 1.7 | 0.34 | nd | 0.30 | 0.64 | nd | 0.65 |
| | 0.55 | nd | 0.39 | 0.071 | nd | 0.046 | 0.51 | nd | 0.52 |
| | 0.44 | nd | 0.52 | 0.10 | nd | 0.094 | 0.18 | nd | 0.18 |
| | 4.7 | nd | 5.3 | 1.1 | nd | 0.98 | 2.4 | nd | 2.4 |
| OR Quart 3 p Value 95% CI of OR Quart3 | 0.59 | nd | 0.39 | 0.61 | nd | 0.55 | 0.81 | nd | 0.65 |
| | 0.47 | nd | 0.26 | 0.33 | nd | 0.23 | 0.74 | nd | 0.52 |
| | 0.14 | nd | 0.073 | 0.23 | nd | 0.21 | 0.24 | nd | 0.18 |
| | 2.5 | nd | 2.0 | 1.6 | nd | 1.5 | 2.8 | nd | 2.4 |
| OR Quart 4 p Value 95% CI of OR Quart4 | 2.8 | nd | 2.9 | 1.1 | nd | 1.0 | 1.2 | nd | 1.0 |
| | 0.059 | nd | 0.056 | 0.80 | nd | 0.98 | 0.79 | nd | 1.0 |
| | 0.96 | nd | 0.98 | 0.47 | nd | 0.43 | 0.38 | nd | 0.31 |
| | 8.2 | nd | 8.4 | 2.7 | nd | 2.4 | 3.6 | nd | 3.2 |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.437 | 0.383 | 0.406 | 0.383 | 0.360 |
| Average | 0.636 | 2.03 | 0.636 | 1.81 | 0.636 | 0.789 |
| Stdev | 1.83 | 4.87 | 1.83 | 5.61 | 1.83 | 0.797 |
| p(t-test) | | 0.0014 | | 0.0072 | | 0.70 |
| Min | 0.000152 | 0.0957 | 0.000152 | 0.0249 | 0.000152 | 0.0995 |
| Max | 32.1 | 20.6 | 32.1 | 31.1 | 32.1 | 2.56 |
| n (Samp) | 349 | 28 | 349 | 33 | 349 | 22 |
| n (Patient) | 172 | 28 | 172 | 33 | 172 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.383 | 0.437 | 0.383 | 0.399 | 0.383 | 0.360 |
| Average | 0.637 | 2.04 | 0.637 | 1.77 | 0.637 | 0.741 |
| Stdev | 1.86 | 4.87 | 1.86 | 5.53 | 1.86 | 0.759 |
| p(t-test) | | 0.0015 | | 0.0097 | | 0.80 |
| Min | 0.000152 | 0.0957 | 0.000152 | 0.0249 | 0.000152 | 0.0995 |
| Max | 32.1 | 20.6 | 32.1 | 31.1 | 32.1 | 2.56 |
| n (Samp) | 340 | 28 | 340 | 34 | 340 | 20 |
| n (Patient) | 158 | 28 | 158 | 34 | 158 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.60 | 0.57 | nd | 0.56 | 0.56 | nd | 0.55 |
| SE | 0.058 | nd | 0.059 | 0.054 | nd | 0.053 | 0.065 | nd | 0.068 |
| p | 0.16 | nd | 0.098 | 0.22 | nd | 0.23 | 0.33 | nd | 0.43 |
| nCohort 1 | 349 | nd | 340 | 349 | nd | 340 | 349 | nd | 340 |
| nCohort 2 | 28 | nd | 28 | 33 | nd | 34 | 22 | nd | 20 |
| Cutoff 1 | 0.321 | nd | 0.356 | 0.293 | nd | 0.293 | 0.200 | nd | 0.200 |
| Sens 1 | 71% | nd | 71% | 73% | nd | 71% | 73% | nd | 70% |
| Spec 1 | 41% | nd | 46% | 38% | nd | 39% | 28% | nd | 28% |
| Cutoff 2 | 0.224 | nd | 0.232 | 0.259 | nd | 0.259 | 0.179 | nd | 0.179 |
| Sens 2 | 82% | nd | 82% | 82% | nd | 82% | 82% | nd | 80% |
| Spec 2 | 30% | nd | 31% | 32% | nd | 32% | 21% | nd | 21% |
| Cutoff 3 | 0.134 | nd | 0.188 | 0.188 | nd | 0.188 | 0.134 | nd | 0.134 |
| Sens 3 | 96% | nd | 93% | 91% | nd | 91% | 91% | nd | 90% |
| Spec 3 | 16% | nd | 23% | 23% | nd | 23% | 16% | nd | 17% |
| Cutoff 4 | 0.589 | nd | 0.587 | 0.589 | nd | 0.587 | 0.589 | nd | 0.587 |
| Sens 4 | 36% | nd | 36% | 36% | nd | 35% | 45% | nd | 45% |
| Spec 4 | 70% | nd | 70% | 70% | nd | 70% | 70% | nd | 70% |
| Cutoff 5 | 0.734 | nd | 0.734 | 0.734 | nd | 0.734 | 0.734 | nd | 0.734 |
| Sens 5 | 29% | nd | 29% | 27% | nd | 26% | 36% | nd | 35% |
| Spec 5 | 80% | nd | 80% | 80% | nd | 80% | 80% | nd | 80% |
| Cutoff 6 | 1.09 | nd | 1.07 | 1.09 | nd | 1.07 | 1.09 | nd | 1.07 |
| Sens 6 | 21% | nd | 21% | 18% | nd | 18% | 23% | nd | 20% |
| Spec 6 | 90% | nd | 90% | 90% | nd | 90% | 90% | nd | 90% |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 1.8 | nd | 2.8 | 1.5 | nd | 2.3 | 0.99 | nd | 1.2 |
| | 0.36 | nd | 0.13 | 0.43 | nd | 0.13 | 0.98 | nd | 0.76 |
| | 0.51 | nd | 0.73 | 0.52 | nd | 0.78 | 0.31 | nd | 0.36 |
| | 6.4 | nd | 11 | 4.5 | nd | 7.0 | 3.2 | nd | 4.1 |
| OR Quart 3 p Value 95% CI of OR Quart3 | 2.4 | nd | 3.2 | 1.6 | nd | 1.9 | 0.16 | nd | 0.19 |
| | 0.16 | nd | 0.088 | 0.42 | nd | 0.27 | 0.088 | nd | 0.13 |
| | 0.71 | nd | 0.84 | 0.53 | nd | 0.61 | 0.018 | nd | 0.022 |
| | 8.0 | nd | 12 | 4.5 | nd | 5.9 | 1.3 | nd | 1.7 |
| OR Quart 4 p Value 95% CI of OR Quart4 | 2.1 | nd | 2.8 | 1.5 | nd | 1.9 | 1.5 | nd | 1.7 |
| | 0.25 | nd | 0.13 | 0.43 | nd | 0.28 | 0.43 | nd | 0.39 |
| | 0.60 | nd | 0.73 | 0.52 | nd | 0.60 | 0.52 | nd | 0.52 |
| | 7.1 | nd | 11 | 4.5 | nd | 5.8 | 4.5 | nd | 5.3 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.163 | 0.226 | 0.163 | 0.172 | 0.163 | 0.106 |
| Average | 0.200 | 0.261 | 0.200 | 0.221 | 0.200 | 0.130 |
| Stdev | 0.207 | 0.213 | 0.207 | 0.211 | 0.207 | 0.105 |
| p(t-test) | | 0.14 | | 0.59 | | 0.12 |
| Min | 2.92E-5 | 0.0472 | 2.92E-5 | 2.92E-5 | 2.92E-5 | 0.000127 |
| Max | 2.09 | 1.08 | 2.09 | 0.971 | 2.09 | 0.427 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.168 | 0.226 | 0.168 | 0.172 | 0.168 | 0.0967 |
| Average | 0.202 | 0.260 | 0.202 | 0.226 | 0.202 | 0.122 |
| Stdev | 0.207 | 0.213 | 0.207 | 0.210 | 0.207 | 0.105 |
| p(t-test) | | 0.16 | | 0.53 | | 0.086 |
| Min | 2.92E-5 | 0.0472 | 2.92E-5 | 2.92E-5 | 2.92E-5 | 0.000127 |
| Max | 2.09 | 1.08 | 2.09 | 0.971 | 2.09 | 0.427 |
| n (Samp) | 347 | 28 | 347 | 35 | 347 | 20 |
| n (Patient) | 161 | 28 | 161 | 35 | 161 | 20 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.61 | nd | 0.61 | 0.53 | nd | 0.53 | 0.40 | nd | 0.37 |
| SE | 0.058 | nd | 0.058 | 0.053 | nd | 0.052 | 0.066 | nd | 0.068 |
| p | 0.050 | nd | 0.067 | 0.63 | nd | 0.55 | 0.11 | nd | 0.049 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 0.128 | nd | 0.128 | 0.0839 | nd | 0.0820 | 0.0584 | nd | 0.0584 |
| Sens 1 | 71% | nd | 71% | 71% | nd | 71% | 73% | nd | 70% |
| Spec 1 | 43% | nd | 41% | 29% | nd | 28% | 22% | nd | 21% |
| Cutoff 2 | 0.0791 | nd | 0.0791 | 0.0536 | nd | 0.0564 | 0.0521 | nd | 0.0521 |
| Sens 2 | 82% | nd | 82% | 82% | nd | 80% | 82% | nd | 80% |
| Spec 2 | 27% | nd | 27% | 20% | nd | 20% | 20% | nd | 19% |
| Cutoff 3 | 0.0511 | nd | 0.0511 | 0.0120 | nd | 0.0120 | 0.0240 | nd | 0.0240 |
| Sens 3 | 93% | nd | 93% | 91% | nd | 91% | 91% | nd | 90% |
| Spec 3 | 20% | nd | 19% | 10% | nd | 9% | 12% | nd | 12% |
| Cutoff 4 | 0.243 | nd | 0.248 | 0.243 | nd | 0.248 | 0.243 | nd | 0.248 |
| Sens 4 | 39% | nd | 39% | 32% | nd | 34% | 18% | nd | 10% |
| Spec 4 | 70% | nd | 70% | 70% | nd | 70% | 70% | nd | 70% |
| Cutoff 5 | 0.300 | nd | 0.302 | 0.300 | nd | 0.302 | 0.300 | nd | 0.302 |
| Sens 5 | 39% | nd | 36% | 26% | nd | 29% | 5% | nd | 5% |
| Spec 5 | 80% | nd | 80% | 80% | nd | 80% | 80% | nd | 80% |
| Cutoff 6 | 0.390 | nd | 0.390 | 0.390 | nd | 0.390 | 0.390 | nd | 0.390 |
| Sens 6 | 18% | nd | 18% | 18% | nd | 20% | 5% | nd | 5% |
| Spec 6 | 90% | nd | 90% | 90% | nd | 90% | 90% | nd | 90% |
| OR Quart 2 p Value 95% CI of OR Quart2 | 1.5 | nd | 1.5 | 0.75 | nd | 0.75 | 2.6 | nd | 1.5 |
|  | 0.52 | nd | 0.53 | 0.59 | nd | 0.59 | 0.26 | nd | 0.65 |
|  | 0.42 | nd | 0.41 | 0.27 | nd | 0.27 | 0.49 | nd | 0.25 |
|  | 5.6 | nd | 5.6 | 2.1 | nd | 2.1 | 14 | nd | 9.3 |
| OR Quart 3 p Value 95% CI of OR Quart3 | 1.8 | nd | 2.1 | 0.88 | nd | 0.88 | 3.7 | nd | 3.7 |
|  | 0.36 | nd | 0.25 | 0.80 | nd | 0.80 | 0.11 | nd | 0.11 |
|  | 0.51 | nd | 0.60 | 0.32 | nd | 0.32 | 0.75 | nd | 0.75 |
|  | 6.4 | nd | 7.1 | 2.4 | nd | 2.4 | 18 | nd | 18 |
| OR Quart 4 p Value 95% CI ot OR Quart4 | 3.0 | nd | 2.6 | 1.1 | nd | 1.2 | 4.3 | nd | 4.3 |
|  | 0.070 | nd | 0.11 | 0.83 | nd | 0.65 | 0.069 | nd | 0.068 |
|  | 0.91 | nd | 0.80 | 0.43 | nd | 0.49 | 0.89 | nd | 0.90 |
|  | 9.7 | nd | 8.8 | 2.9 | nd | 3.1 | 21 | nd | 21 |

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0459 | 0.0866 | 0.0459 | 0.0750 | 0.0459 | 0.0792 |
| Average | 0.210 | 0.234 | 0.210 | 0.279 | 0.210 | 0.401 |
| Stdev | 0.518 | 0.444 | 0.518 | 0.969 | 0.518 | 0.598 |
| p(t-test) | | 0.82 | | 0.50 | | 0.097 |
| Min | 4.60E-6 | 0.00381 | 4.60E-6 | 9.65E-6 | 4.60E-6 | 0.00226 |
| Max | 4.46 | 2.26 | 4.46 | 5.72 | 4.46 | 2.11 |
| n (Samp) | 356 | 28 | 356 | 34 | 356 | 22 |
| n (Patient) | 175 | 28 | 175 | 34 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0524 | 0.0866 | 0.0524 | 0.0712 | 0.0524 | 0.0664 |
| Average | 0.224 | 0.235 | 0.224 | 0.272 | 0.224 | 0.426 |
| Stdev | 0.529 | 0.444 | 0.529 | 0.956 | 0.529 | 0.623 |
| p(t-test) | | 0.91 | | 0.64 | | 0.10 |
| Min | 4.60E-6 | 0.00381 | 4.60E-6 | 9.65E-6 | 4.60E-6 | 0.00226 |
| Max | 4.46 | 2.26 | 4.46 | 5.72 | 4.46 | 2.11 |
| n (Samp) | 347 | 28 | 347 | 35 | 347 | 20 |
| n (Patient) | 161 | 28 | 161 | 35 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.58 | nd | 0.56 | 0.57 | nd | 0.54 | 0.61 | nd | 0.58 |
| SE | 0.058 | nd | 0.058 | 0.053 | nd | 0.052 | 0.066 | nd | 0.068 |
| p | 0.18 | nd | 0.30 | 0.22 | nd | 0.44 | 0.10 | nd | 0.25 |
| nCohort 1 | 356 | nd | 347 | 356 | nd | 347 | 356 | nd | 347 |
| nCohort 2 | 28 | nd | 28 | 34 | nd | 35 | 22 | nd | 20 |
| Cutoff 1 | 0.0322 | nd | 0.0322 | 0.0325 | nd | 0.0325 | 0.0301 | nd | 0.0301 |
| Sens 1 | 71% | nd | 71% | 71% | nd | 71% | 73% | nd | 70% |
| Spec 1 | 40% | nd | 37% | 41% | nd | 38% | 38% | nd | 36% |
| Cutoff 2 | 0.0126 | nd | 0.0126 | 0.0209 | nd | 0.0252 | 0.0202 | nd | 0.0202 |
| Sens 2 | 82% | nd | 82% | 82% | nd | 80% | 82% | nd | 80% |
| Spec 2 | 24% | nd | 21% | 32% | nd | 33% | 31% | nd | 29% |
| Cutoff 3 | 0.00659 | nd | 0.00654 | 0.0141 | nd | 0.0141 | 0.00901 | nd | 0.00901 |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 3 | 93% | nd | 93% | 91% | nd | 91% | 91% | nd | 90% |
| Spec 3 | 16% | nd | 13% | 25% | nd | 22% | 20% | nd | 17% |
| Cutoff 4 | 0.122 | nd | 0.133 | 0.122 | nd | 0.133 | 0.122 | nd | 0.133 |
| Sens 4 | 36% | nd | 36% | 32% | nd | 29% | 45% | nd | 45% |
| Spec 4 | 70% | nd | 70% | 70% | nd | 70% | 70% | nd | 70% |
| Cutoff 5 | 0.214 | nd | 0.224 | 0.214 | nd | 0.224 | 0.214 | nd | 0.224 |
| Sens 5 | 21% | nd | 25% | 18% | nd | 17% | 41% | nd | 35% |
| Spec 5 | 80% | nd | 80% | 80% | nd | 80% | 80% | nd | 80% |
| Cutoff 6 | 0.474 | nd | 0.495 | 0.474 | nd | 0.495 | 0.474 | nd | 0.495 |
| Sens 6 | 18% | nd | 18% | 3% | nd | 3% | 27% | nd | 30% |
| Spec 6 | 90% | nd | 90% | 90% | nd | 90% | 90% | nd | 90% |
| OR Quart2 p Value 95% CI of OR Quart2 | 0.41 | nd | 0.41 | 2.4 | nd | 1.5 | 0.99 | nd | 0.99 |
| | 0.21 | nd | 0.20 | 0.17 | nd | 0.43 | 0.99 | nd | 0.99 |
| | 0.10 | nd | 0.10 | 0.70 | nd | 0.52 | 0.24 | nd | 0.24 |
| | 1.6 | nd | 1.6 | 7.9 | nd | 4.5 | 4.1 | nd | 4.1 |
| OR Quart3 p Value 95% CI of OR Quart3 | 1.3 | nd | 1.5 | 3.3 | nd | 1.9 | 1.3 | nd | 0.99 |
| | 0.60 | nd | 0.46 | 0.046 | nd | 0.21 | 0.73 | nd | 0.99 |
| | 0.47 | nd | 0.53 | 1.0 | nd | 0.69 | 0.33 | nd | 0.24 |
| | 3.7 | nd | 4.0 | 11 | nd | 5.5 | 4.9 | nd | 4.1 |
| OR Quart4 p Value 95% CI of OR Quart4 | 1.3 | nd | 1.1 | 2.4 | nd | 1.5 | 2.4 | nd | 2.1 |
| | 0.60 | nd | 0.80 | 0.17 | nd | 0.43 | 0.17 | nd | 0.25 |
| | 0.47 | nd | 0.40 | 0.70 | nd | 0.52 | 0.70 | nd | 0.60 |
| | 3.7 | nd | 3.3 | 7.9 | nd | 4.5 | 7.9 | nd | 7.1 |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 562 | 799 | 562 | 664 | 562 | 949 |
| Average | 1170 | 1140 | 1170 | 982 | 1170 | 1330 |
| Stdev | 2460 | 1190 | 2460 | 741 | 2460 | 1050 |
| p(t-test) | | 0.94 | | 0.67 | | 0.76 |
| Min | 43.9 | 146 | 43.9 | 167 | 43.9 | 200 |
| Max | 26100 | 5700 | 26100 | 3000 | 26100 | 4290 |
| n (Samp) | 357 | 28 | 357 | 32 | 357 | 22 |
| n (Patient) | 175 | 28 | 175 | 32 | 175 | 22 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 564 | 799 | 564 | 728 | 564 | 949 |
| Average | 1190 | 1130 | 1190 | 978 | 1190 | 1370 |
| Stdev | 2490 | 1190 | 2490 | 729 | 2490 | 1090 |
| p(t-test) | | 0.91 | | 0.63 | | 0.74 |
| Min | 43.9 | 146 | 43.9 | 167 | 43.9 | 200 |
| Max | 26100 | 5700 | 26100 | 3000 | 26100 | 4290 |
| n (Samp) | 348 | 28 | 348 | 33 | 348 | 20 |
| n (Patient) | 161 | 28 | 161 | 33 | 161 | 20 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | nd | 0.59 | 0.58 | nd | 0.58 | 0.71 | nd | 0.70 |
| SE | 0.058 | nd | 0.058 | 0.055 | nd | 0.054 | 0.063 | nd | 0.067 |
| p | 0.10 | nd | 0.13 | 0.14 | nd | 0.14 | 9.3E-4 | nd | 0.0023 |
| nCohort 1 | 357 | nd | 348 | 357 | nd | 348 | 357 | nd | 348 |
| nCohort 2 | 28 | nd | 28 | 32 | nd | 33 | 22 | nd | 20 |
| Cutoff 1 | 482 | nd | 482 | 426 | nd | 426 | 817 | nd | 817 |
| Sens 1 | 71% | nd | 71% | 72% | nd | 73% | 73% | nd | 70% |
| Spec 1 | 45% | nd | 44% | 38% | nd | 38% | 68% | nd | 67% |
| Cutoff 2 | 389 | nd | 389 | 358 | nd | 358 | 704 | nd | 704 |
| Sens 2 | 82% | nd | 82% | 81% | nd | 82% | 82% | nd | 80% |
| Spec 2 | 33% | nd | 33% | 29% | nd | 29% | 63% | nd | 62% |
| Cutoff 3 | 223 | nd | 223 | 310 | nd | 310 | 486 | nd | 486 |
| Sens 3 | 93% | nd | 93% | 91% | nd | 91% | 91% | nd | 90% |
| Spec 3 | 14% | nd | 13% | 24% | nd | 24% | 45% | nd | 44% |
| Cutoff 4 | 875 | nd | 895 | 875 | nd | 895 | 875 | nd | 895 |
| Sens 4 | 43% | nd | 43% | 44% | nd | 42% | 64% | nd | 65% |
| Spec 4 | 70% | nd | 70% | 70% | nd | 70% | 70% | nd | 70% |
| Cutoff 5 | 1160 | nd | 1170 | 1160 | nd | 1170 | 1160 | nd | 1170 |
| Sens 5 | 32% | nd | 29% | 34% | nd | 30% | 32% | nd | 35% |
| Spec 5 | 80% | nd | 80% | 80% | nd | 80% | 80% | nd | 80% |
| Cutoff 6 | 1950 | nd | 1950 | 1950 | nd | 1950 | 1950 | nd | 1950 |
| Sens 6 | 18% | nd | 18% | 12% | nd | 12% | 18% | nd | 20% |
| Spec 6 | 90% | nd | 90% | 90% | nd | 90% | 90% | nd | 90% |

(continued)

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 p Value 95% CI of OR Quart2 | 1.0 | nd | 1.0 | 2.7 | nd | 1.9 | 3.0 | nd | 3.1 |
|  | 1.0 | nd | 1.0 | 0.1 | nd | 0.27 | 0.34 | nd | 0.34 |
|  | 0.28 | nd | 0.28 | 0.81 | nd | 0.61 | 0.31 | nd | 0.31 |
|  | 3.6 | nd | 3.6 | 8.8 | nd | 5.8 | 30 | nd | 30 |
| OR Quart3 p Value 95% CI of OR Quart3 | 1.7 | nd | 1.7 | 1.0 | nd | 1.0 | 8.6 | nd | 7.5 |
|  | 0.39 | nd | 0.39 | 1.0 | nd | 1.0 | 0.045 | nd | 0.062 |
|  | 0.52 | nd | 0.52 | 0.24 | nd | 0.28 | 1.0 | nd | 0.90 |
|  | 5.3 | nd | 5.3 | 4.1 | nd | 3.6 | 70 | nd | 62 |
| OR Quart4 p Value 95% CI OR Quart4 | 2.1 | nd | 2.1 | 3.9 | nd | 3.1 | 11 | nd | 9.9 |
|  | 0.19 | nd | 0.19 | 0.021 | nd | 0.039 | 0.024 | nd | 0.032 |
|  | 0.69 | nd | 0.70 | 1.2 | nd | 1.1 | 1.4 | nd | 1.2 |
|  | 6.4 | nd | 6.5 | 12 | nd | 8.9 | 87 | nd | 80 |

Fig. 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

**Angiopoietin-related protein 4**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 37.7 | 31.0 | 52.4 | 47.4 | 35.1 | 30.5 |
| Average | 46.3 | 50.4 | 68.0 | 64.4 | 38.8 | 49.2 |
| Stdev | 43.5 | 44.4 | 65.2 | 59.7 | 22.9 | 41.0 |
| p(t-test) |  | 0.67 |  | 0.90 |  | 0.16 |
| Min | 2.71 | 8.79 | 8.18 | 20.5 | 2.71 | 8.79 |
| Max | 317 | 192 | 317 | 179 | 101 | 192 |
| n (Samp) | 65 | 31 | 26 | 6 | 50 | 26 |
| n (Patient) | 65 | 31 | 26 | 6 | 50 | 26 |

|  | At Enrollment | | |
|---|---|---|---|
|  | sCr or UO | sCr only | UO only |
| AUC | 0.50 | 0.45 | 0.53 |
| SE | 0.063 | 0.13 | 0.071 |
| P | 0.98 | 0.70 | 0.65 |
| nCohort 1 | 65 | 26 | 50 |
| nCohort 2 | 31 | 6 | 26 |
| Cutoff 1 | 24.5 | 22.3 | 25.1 |

(continued)

| | At Enrollment | | |
|---|---|---|---|
| | sCr or UO | sCr only | UO only |
| Sens 1 | 71% | 83% | 73% |
| Spec 1 | 28% | 15% | 32% |
| Cutoff 2 | 22.1 | 22.3 | 22.1 |
| Sens 2 | 81% | 83% | 81% |
| Spec 2 | 23% | 15% | 26% |
| Cutoff 3 | 15.1 | 15.1 | 14.5 |
| Sens 3 | 90% | 100% | 92% |
| Spec 3 | 14% | 12% | 12% |
| Cutoff 4 | 45.5 | 72.2 | 43.0 |
| Sens 4 | 42% | 17% | 42% |
| Spec 4 | 71% | 73% | 70% |
| Cutoff 5 | 63.2 | 78.2 | 51.6 |
| Sens 5 | 19% | 17% | 38% |
| Spec 5 | 80% | 81% | 80% |
| Cutoff 6 | 82.3 | 154 | 75.6 |
| Sens 6 | 13% | 17% | 19% |
| Spec 6 | 91% | 92% | 90% |
| OR Quart 2 p Value 95% CI of OR Quart2 | 1.0 | 2.3 | 2.0 |
| | 1.0 | 0.53 | 0.32 |
| | 0.29 | 0.17 | 0.52 |
| | 3.5 | 33 | 7.3 |
| OR Quart 3 p Value 95% CI of OR Quart3 | 1.2 | 1.0 | 0.25 |
| | 0.76 | 1.0 | 0.13 |
| | 0.36 | 0.052 | 0.044 |
| | 4.1 | 19 | 1.5 |
| OR Quart 4 p Value 95% CI of OR Quart4 | 1.5 | 2.3 | 2.0 |
| | 0.54 | 0.53 | 0.32 |
| | 0.44 | 0.17 | 0.52 |
| | 4.9 | 33 | 7.3 |

**Amphiregulin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 22.0 | 22.0 | 24.1 | 22.4 | 20.9 | 21.1 |
| Average | 31.8 | 30.9 | 39.2 | 46.8 | 27.0 | 26.9 |
| Stdev | 34.4 | 30.7 | 44.4 | 57.8 | 24.3 | 18.6 |

(continued)

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| p(t-test) | | 0.90 | | 0.72 | | 0.98 |
| Min | 2.70 | 0.00246 | 4.60 | 13.6 | 2.70 | 0.00246 |
| Max | 198 | 162 | 198 | 162 | 145 | 72.6 |
| n (Samp) | 63 | 30 | 25 | 6 | 49 | 25 |
| n (Patient) | 63 | 30 | 25 | 6 | 49 | 25 |

| | At Enrollment | | |
|---|---|---|---|
| | sCr or UO | sCr only | UO only |
| AUC | 0.51 | 0.51 | 0.53 |
| SE | 0.064 | 0.13 | 0.072 |
| p | 0.91 | 0.92 | 0.66 |
| nCohort 1 | 63 | 25 | 49 |
| nCohort 2 | 30 | 6 | 25 |
| Cutoff 1 | 16.1 | 13.9 | 17.4 |
| Sens 1 | 70% | 83% | 72% |
| Spec 1 | 32% | 24% | 41% |
| Cutoff 2 | 13.5 | 13.9 | 13.9 |
| Sens 2 | 80% | 83% | 80% |
| Spec 2 | 27% | 24% | 35% |
| Cutoff 3 | 9.97 | 11.9 | 8.92 |
| Sens 3 | 90% | 100% | 92% |
| Spec 3 | 16% | 20% | 12% |
| Cutoff 4 | 30.4 | 40.6 | 27.6 |
| Sens 4 | 30% | 33% | 28% |
| Spec 4 | 71% | 72% | 71% |
| Cutoff 5 | 40.6 | 51.0 | 36.0 |
| Sens 5 | 20% | 17% | 20% |
| Spec 5 | 81% | 80% | 82% |
| Cutoff 6 | 60.5 | 72.6 | 60.5 |
| Sens 6 | 10% | 17% | 8% |
| Spec 6 | 90% | 92% | 92% |
| OR Quart 2 | 1.8 | 2.0 | 1.5 |
| p Value | 0.35 | 0.61 | 0.56 |
| 95% CI of | 0.52 | 0.14 | 0.38 |
| OR Quart2 | 6.4 | 28 | 6.1 |

(continued)

|  | At Enrollment | | |
|---|---|---|---|
|  | sCr or UO | sCr only | UO only |
| OR Quart 3 p Value 95% CI of OR Quart3 | 1.0 | 0.86 | 1.3 |
|  | 1.0 | 0.92 | 0.72 |
|  | 0.27 | 0.044 | 0.31 |
|  | 3.7 | 17 | 5.4 |
| OR Quart 4 p Value 95% CI of OR Quart4 | 1.7 | 2.0 | 1.5 |
|  | 0.40 | 0.61 | 0.56 |
|  | 0.49 | 0.14 | 0.38 |
|  | 5.9 | 28 | 6.1 |

**Endostatin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 44200 | 37400 | 58300 | 34700 | 1500 | 34100 |
| Average | 59000 | 57100 | 77700 | 41500 | 8000 | 62200 |
| Stdev | 62600 | 61300 | 88700 | 32600 | 30200 | 69700 |
| p(t-test) |  | 0.89 |  | 0.34 |  | 0.22 |
| Min | 6940 | 9280 | 13600 | 13800 | 6940 | 9280 |
| Max | 483000 | 275000 | 483000 | 102000 | 146000 | 275000 |
| n (Samp) | 65 | 31 | 26 | 6 | 50 | 26 |
| n (Patient) | 65 | 31 | 26 | 6 | 50 | 26 |

|  | At Enrollment | | |
|---|---|---|---|
|  | sCr or UO | sCr only | UO only |
| AUC | 0.46 | 0.28 | 0.48 |
| SE | 0.064 | 0.13 | 0.070 |
| p | 0.49 | 0.077 | 0.80 |
| nCohort 1 | 65 | 26 | 50 |
| nCohort 2 | 31 | 6 | 26 |
| Cutoff 1 | 29900 | 13800 | 26300 |
| Sens 1 | 71% | 83% | 73% |
| Spec 1 | 28% | 4% | 24% |
| Cutoff 2 | 26000 | 13800 | 23600 |
| Sens 2 | 81% | 83% | 81% |
| Spec 2 | 20% | 4% | 22% |
| Cutoff 3 | 17000 | 13600 | 17000 |
| Sens 3 | 90% | 100% | 92% |

(continued)

|  | At Enrollment | | |
|---|---|---|---|
|  | sCr or UO | sCr only | UO only |
| Spec 3 | 11% | 4% | 12% |
| Cutoff 4 | 61800 | 73100 | 53400 |
| Sens 4 | 29% | 17% | 31% |
| Spec 4 | 71% | 73% | 70% |
| Cutoff 5 | 69800 | 93800 | 66900 |
| Sens 5 | 23% | 17% | 27% |
| Spec 5 | 80% | 81% | 80% |
| Cutoff 6 | 115000 | 119000 | 80400 |
| Sens 6 | 6% | 0% | 19% |
| Spec 6 | 91% | 92% | 90% |
| OR Quart 2 p Value 95% CI of OR Quart2 | 0.53 | 0 | 0.61 |
|  | 0.33 | na | 0.49 |
|  | 0.14 | na | 0.15 |
|  | 1.9 | na | 2.4 |
| OR Quart 3 p Value 95% CI of OR Quart3 | 1.4 | 4.2 | 1.0 |
|  | 0.55 | 0.27 | 1.0 |
|  | 0.44 | 0.33 | 0.27 |
|  | 4.6 | 53 | 3.7 |
| OR Quart 4 p Value 95% CI of OR Quart4 | 1.0 | 2.3 | 1.0 |
|  | 1.0 | 0.53 | 1.0 |
|  | 0.30 | 0.17 | 0.27 |
|  | 3.3 | 33 | 3.7 |

**Fibroblast growth factor 19**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.164 | 0.149 | 0.223 | 0.104 | 0.140 | 0.150 |
| Average | 0.209 | 0.236 | 0.295 | 0.149 | 0.182 | 0.240 |
| Stdev | 0.210 | 0.287 | 0.279 | 0.181 | 0.205 | 0.297 |
| p(t-test) |  | 0.60 |  | 0.23 |  | 0.32 |
| Min | 0.000127 | 8.23E-5 | 0.000127 | 8.23E-5 | 0.000127 | 0.00974 |
| Max | 1.41 | 1.32 | 1.32 | 0.482 | 1.41 | 1.32 |
| n (Samp) | 65 | 31 | 26 | 6 | 50 | 26 |
| n (Patient) | 65 | 31 | 26 | 6 | 50 | 26 |

| | At Enrollment | | |
|---|---|---|---|
| | sCr or UO | sCr only | UO only |
| AUC | 0.48 | 0.28 | 0.54 |
| SE | 0.064 | 0.13 | 0.071 |
| p | 0.71 | 0.082 | 0.61 |
| nCohort 1 | 65 | 26 | 50 |
| nCohort 2 | 31 | 6 | 26 |
| Cutoff 1 | 0.0804 | 8.23E-5 | 0.0816 |
| Sens 1 | 71% | 83% | 73% |
| Spec 1 | 23% | 0% | 28% |
| Cutoff 2 | 0.0659 | 8.23E-5 | 0.0676 |
| Sens 2 | 81% | 83% | 81% |
| Spec 2 | 22% | 0% | 24% |
| Cutoff 3 | 0.0209 | 0 | 0.0616 |
| Sens 3 | 90% | 100% | 92% |
| Spec 3 | 11% | 0% | 22% |
| Cutoff 4 | 0.265 | 0.326 | 0.215 |
| Sens 4 | 32% | 17% | 31% |
| Spec 4 | 71% | 73% | 70% |
| Cutoff 5 | 0.303 | 0.357 | 0.265 |
| Sens 5 | 23% | 17% | 31% |
| Spec 5 | 80% | 81% | 80% |
| Cutoff 6 | 0.346 | 0.543 | 0.304 |
| Sens 6 | 16% | 0% | 19% |
| Spec 6 | 91% | 92% | 90% |
| ORQuart 2 p Value 95% CI of OR Quart2 | 0.67 | 0 | 0.61 |
| | 0.53 | na | 0.49 |
| | 0.19 | na | 0.15 |
| | 2.3 | na | 2.4 |
| OR Quart 3 p Value 95% CI of OR Quart3 | 1.0 | 2.3 | 0.79 |
| | 1.0 | 0.53 | 0.73 |
| | 0.30 | 0.17 | 0.21 |
| | 3.3 | 33 | 3.0 |
| OR Quart 4 p Value 95% CI of OR Quart4 | 1.2 | 4.2 | 1.2 |
| | 0.76 | 0.27 | 0.74 |
| | 0.37 | 0.33 | 0.34 |
| | 3.9 | 53 | 4.6 |

**Fibroblast growth factor 21**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.0451 | 0.0670 | 0.0773 | 0.288 | 0.0610 | 0.0618 |
| Average | 0.212 | 0.402 | 0.257 | 0.634 | 0.191 | 0.340 |
| Stdev | 0.512 | 1.08 | 0.489 | 0.803 | 0.485 | 1.11 |
| p(t-test) |  | 0.24 |  | 0.14 |  | 0.42 |
| Min | 7.01E-5 | 0.00323 | 0.00263 | 0.0306 | 7.01E-5 | 0.00323 |
| Max | 3.36 | 5.72 | 2.05 | 2.11 | 3.36 | 5.72 |
| n (Samp) | 65 | 31 | 26 | 6 | 50 | 26 |
| n (Patient) | 65 | 31 | 26 | 6 | 50 | 26 |

|  | At Enrollment | | |
|---|---|---|---|
|  | sCr or UO | sCr only | UO only |
| AUC | 0.58 | 0.71 | 0.52 |
| SE | 0.064 | 0.13 | 0.071 |
| p | 0.21 | 0.099 | 0.73 |
| nCohort 1 | 65 | 26 | 50 |
| nCohort 2 | 31 | 6 | 26 |
| Cutoff 1 | 0.0322 | 0.0938 | 0.0231 |
| Sens 1 | 71% | 83% | 73% |
| Spec 1 | 43% | 58% | 38% |
| Cutoff 2 | 0.0231 | 0.0938 | 0.0189 |
| Sens 2 | 81% | 83% | 81% |
| Spec 2 | 40% | 58% | 32% |
| Cutoff 3 | 0.0126 | 0.0217 | 0.00762 |
| Sens 3 | 90% | 100% | 92% |
| Spec 3 | 22% | 27% | 20% |
| Cutoff 4 | 0.129 | 0.181 | 0.170 |
| Sens 4 | 35% | 50% | 31% |
| Spec 4 | 71% | 73% | 70% |
| Cutoff 5 | 0.253 | 0.206 | 0.252 |
| Sens 5 | 19% | 50% | 12% |
| Spec 5 | 80% | 81% | 80% |
| Cutoff 6 | 0.430 | 1.05 | 0.312 |
| Sens 6 | 13% | 17% | 12% |
| Spec 6 | 91% | 92% | 90% |

(continued)

|  | At Enrollment | | |
|---|---|---|---|
|  | sCr or UO | sCr only | UO only |
| OR Quart 2 p Value 95% CI of OR Quart2 | 3.0 | 0 | 3.4 |
|  | 0.11 | na | 0.094 |
|  | 0.77 | na | 0.81 |
|  | 12 | na | 14 |
| OR Quart 3 p Value 95% CI of OR Quart3 | 3.6 | 2.3 | 2.2 |
|  | 0.064 | 0.53 | 0.29 |
|  | 0.93 | 0.17 | 0.52 |
|  | 14 | 33 | 9.3 |
| OR Quart 4 p Value 95% CI of OR Quart4 | 2.5 | 4.2 | 1.7 |
|  | 0.19 | 0.27 | 0.46 |
|  | 0.64 | 0.33 | 0.40 |
|  | 9.8 | 53 | 7.5 |

**Thrombospondin-2**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 41900 | 21700 | 59900 | 18300 | 40400 | 24200 |
| Average | 66700 | 67900 | 89800 | 87900 | 65600 | 52300 |
| Stdev | 75700 | 119000 | 102000 | 169000 | 81200 | 83000 |
| p(t-test) |  | 0.95 |  | 0.97 |  | 0.50 |
| Min | 1240 | 1620 | 1400 | 4470 | 1240 | 1620 |
| Max | 434000 | 433000 | 418000 | 433000 | 434000 | 351000 |
| n (Samp) | 66 | 31 | 26 | 6 | 51 | 26 |
| n (Patient) | 66 | 31 | 26 | 6 | 51 | 26 |

|  | At Enrollment | | |
|---|---|---|---|
|  | sCr or UO | sCr only | UO only |
| AUC | 0.39 | 0.34 | 0.41 |
| SE | 0.063 | 0.13 | 0.070 |
| p | 0.076 | 0.23 | 0.18 |
| Cohort 1 | 66 | 26 | 51 |
| nCohort 2 | 31 | 6 | 26 |
| Cutoff 1 | 15500 | 9090 | 16500 |
| Sens 1 | 71% | 83% | 73% |
| Spec 1 | 26% | 15% | 27% |
| Cutoff 2 | 8620 | 9090 | 13100 |

(continued)

|  | At Enrollment | | |
|---|---|---|---|
|  | sCr or UO | sCr only | UO only |
| Sens 2 | 81% | 83% | 81% |
| Spec 2 | 14% | 15% | 20% |
| Cutoff 3 | 4470 | 2860 | 3360 |
| Sens 3 | 90% | 100% | 92% |
| Spec 3 | 6% | 8% | 6% |
| Cutoff 4 | 67700 | 106000 | 55600 |
| Sens 4 | 16% | 17% | 15% |
| Spec 4 | 71% | 73% | 71% |
| Cutoff 5 | 112000 | 123000 | 112000 |
| Sens 5 | 13% | 17% | 12% |
| Spec 5 | 80% | 81% | 80% |
| Cutoff 6 | 152000 | 237000 | 160000 |
| Sens 6 | 13% | 17% | 12% |
| Spec 6 | 91% | 92% | 90% |
| ORQuart 2 p Value 95% CI of OR Quart2 | 1.1 | 0 | 1.4 |
|  | 0.94 | na | 0.64 |
|  | 0.26 | na | 0.32 |
|  | 4.2 | na | 6.4 |
| OR Quart 3 p Value 95% CI of OR Quart3 | 4.7 | 2.3 | 5.5 |
|  | 0.016 | 0.53 | 0.019 |
|  | 1.3 | 0.17 | 1.3 |
|  | 17 | 33 | 23 |
| OR Quart 4 p Value 95% CI of OR Quart4 | 2.0 | 4.2 | 1.8 |
|  | 0.29 | 0.27 | 0.41 |
|  | 0.55 | 0.33 | 0.43 |
|  | 7.3 | 53 | 8.0 |

Fig. 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

**Angiogenin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 179000 | 204000 | 179000 | 193000 | 179000 | 250000 |
| Average | 209000 | 206000 | 209000 | 197000 | 209000 | 251000 |
| Stdev | 234000 | 104000 | 234000 | 109000 | 234000 | 96600 |
| p(t-test) |  | 0.97 |  | 0.87 |  | 0.64 |

(continued)

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 33100 | 48000 | 33100 | 48000 | 33100 | 88800 |
| Max | 2240000 | 345000 | 2240000 | 345000 | 2240000 | 345000 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 198000 | 141000 | 198000 | 140000 |
| Average | 221000 | 170000 | 221000 | 156000 |
| Stdev | 235000 | 104000 | 235000 | 106000 |
| p(t-test) | | 0.57 | | 0.47 |
| Min | 33100 | 48000 | 33100 | 48000 |
| Max | 2240000 | 345000 | 2240000 | 345000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.55 | 0.40 | 0.52 | 0.35 | 0.70 |
| SE | 0.095 | 0.12 | 0.093 | 0.12 | 0.11 |
| p | 0.61 | 0.40 | 0.87 | 0.21 | 0.083 |
| nCohort 1 | 88 | 89 | 88 | 89 | 88 |
| nCohort 2 | 11 | 7 | 11 | 7 | 7 |
| Cutoff 1 | 140000 | 109000 | 140000 | 87400 | 202000 |
| Sens 1 | 73% | 71% | 73% | 71% | 71% |
| Spec 1 | 35% | 17% | 35% | 9% | 60% |
| Cutoff 2 | 109000 | 87400 | 87400 | 76300 | 191000 |
| Sens 2 | 82% | 86% | 82% | 86% | 86% |
| Spec 2 | 17% | 9% | 9% | 6% | 56% |
| Cutoff 3 | 87400 | 33100 | 76300 | 33100 | 87400 |
| Sens 3 | 91% | 100% | 91% | 100% | 100% |
| Spec 3 | 9% | 1% | 7% | 1% | 9% |
| Cutoff 4 | 225000 | 245000 | 225000 | 245000 | 225000 |
| Sens 4 | 36% | 29% | 36% | 29% | 57% |
| Spec 4 | 70% | 71% | 70% | 71% | 70% |
| Cutoff 5 | 259000 | 271000 | 259000 | 271000 | 259000 |

EP 2 661 626 B1

(continued)

|  | 0hr prior to AKI stage | | 24hr prior to AKI stage | | 48hr prior to AKI stage |
| --- | --- | --- | --- | --- | --- |
|  | sCr or UO | UO only | sCr or UO | UO only | sCr or UO |
| Sens 5 | 27% | 14% | 27% | 14% | 43% |
| Spec 5 | 81% | 81% | 81% | 81% | 81% |
| Cutoff 6 | 285000 | 330000 | 285000 | 330000 | 285000 |
| Sens 6 | 27% | 14% | 27% | 14% | 43% |
| Spec 6 | 91% | 91% | 91% | 91% | 91% |
| OR Quart 2 p Value 95% CI of OR Quart2 | 0.29 | 2.1 | 0.61 | 1.0 | 0 |
|  | 0.30 | 0.56 | 0.61 | 1.0 | na |
|  | 0.028 | 0.18 | 0.092 | 0.059 | na |
|  | 3.0 | 25 | 4.0 | 17 | na |
| OR Quart 3 p Value 95% CI of OR Quart3 | 0.95 | 1.0 | 0.61 | 2.1 | 2.0 |
|  | 0.96 | 1.0 | 0.61 | 0.56 | 0.58 |
|  | 0.17 | 0.059 | 0.092 | 0.18 | 0.17 |
|  | 5.3 | 17 | 4.0 | 25 | 24 |
| OR Quart 4 p Value 95% CI of OR Quart4 | 1.3 | 3.3 | 1.3 | 3.3 | 4.4 |
|  | 0.73 | 0.32 | 0.73 | 0.32 | 0.20 |
|  | 0.27 | 0.32 | 0.27 | 0.32 | 0.45 |
|  | 6.7 | 34 | 6.7 | 34 | 43 |

**Angiopoietin-related protein 4**

| sCr or UO | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 23.2 | 94.8 | 23.2 | 94.8 | 23.2 | 61.9 |
| Average | 65.5 | 136 | 65.5 | 122 | 65.5 | 78.2 |
| Stdev | 212 | 101 | 212 | 89.2 | 212 | 48.4 |
| p(t-test) |  | 0.28 |  | 0.39 |  | 0.88 |
| Min | 1.77 | 34.0 | 1.77 | 34.0 | 1.77 | 34.0 |
| Max | 1900 | 339 | 1900 | 339 | 1900 | 179 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | | 48hr prior to AKI stage | |
| --- | --- | --- | --- | --- | --- | --- |
|  | Cohort 1 | Cohort 2 | Cohort 1 | Cohort 2 | Cohort 1 | Cohort 2 |
| Median | 31.8 | 179 | 31.8 | 133 | nd | nd |
| Average | 78.1 | 185 | 78.1 | 163 | nd | nd |
| Stdev | 213 | 95.1 | 213 | 88.7 | nd | nd |

(continued)

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | | 0.19 | | 0.30 | nd | nd |
| Min | 2.68 | 71.1 | 2.68 | 71.1 | nd | nd |
| Max | 1900 | 339 | 1900 | 339 | nd | nd |
| n (Samp) | 89 | 7 | 89 | 7 | nd | nd |
| n (Patient) | 89 | 7 | 89 | 7 | nd | nd |

| | 0hr prior to AKI stage | | 24hr prior to AKI stage | | 48hr prior to AKI stage |
|---|---|---|---|---|---|
| | sCr or UO | UO only | sCr or UO | sCr or UO | UO only |
| AUC | 0.88 | 0.90 | 0.87 | 0.90 | 0.83 |
| SE | 0.069 | 0.078 | 0.070 | 0.080 | 0.098 |
| p | 6.1E-8 | 2.8E-7 | 1.3E-7 | 8.7E-7 | 7.5E-4 |
| nCohort 1 | 88 | 89 | 88 | 89 | 88 |
| nCohort 2 | 11 | 7 | 11 | 7 | 7 |
| Cutoff 1 | 55.0 | 127 | 55.0 | 127 | 51.6 |
| Sens 1 | 73% | 71% | 73% | 71% | 71% |
| Spec 1 | 82% | 91% | 82% | 91% | 81% |
| Cutoff 2 | 51.6 | 93.9 | 51.6 | 93.9 | 47.5 |
| Sens 2 | 82% | 86% | 82% | 86% | 86% |
| Spec 2 | 81% | 85% | 81% | 85% | 80% |
| Cutoff 3 | 47.5 | 69.3 | 47.5 | 69.3 | 33.7 |
| Sens 3 | 91% | 100% | 91% | 100% | 100% |
| Spec 3 | 80% | 76% | 80% | 76% | 66% |
| Cutoff 4 | 36.9 | 51.6 | 36.9 | 51.6 | 36.9 |
| Sens 4 | 91% | 100% | 91% | 100% | 86% |
| Spec 4 | 70% | 71% | 70% | 71% | 70% |
| Cutoff 5 | 51.6 | 83.6 | 51.6 | 83.6 | 51.6 |
| Sens 5 | 82% | 86% | 82% | 86% | 71% |
| Spec 5 | 81% | 81% | 81% | 81% | 81% |
| Cutoff 6 | 93.9 | 127 | 93.9 | 127 | 93.9 |
| Sens 6 | 55% | 71% | 55% | 71% | 29% |
| Spec 6 | 91% | 91% | 91% | 91% | 91% |
| OR Quart 2 p Value 95% CI of OR Quart2 | >0 | >0 | >0 | >0 | >0 |
| | <na | <na | <na | <na | <na |
| | >na | >na | >na | >na | >na |
| | na | na | na | na | na |

(continued)

| | 0hr prior to AKI stage | | 24hr prior to AKI stage | | 48hr prior to AKI stage |
|---|---|---|---|---|---|
| | sCr or UO | UO only | sCr or UO | sCr or UO | UO only |
| OR Quart 3 p Value | >3.3 | >1.0 | >3.3 | >1.0 | >1.0 |
| | <0.32 | <0.98 | <0.32 | <0.98 | <1.0 |
| | >0.32 | >0.062 | >0.32 | >0.062 | >0.059 |
| 95% CI of OR Quart3 | na | na | na | na | na |
| OR Quart 4 p Value 95% CI of OR Quart4 | >11 | >8.0 | >11 | >8.0 | >7.7 |
| | <0.029 | <0.064 | <0.029 | <0.064 | <0.070 |
| | >1.3 | >0.88 | >1.3 | >0.88 | >0.85 |
| | na | na | na | na | na |

**Angiopoietin-related protein 6**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 65.2 | 95.0 | 65.2 | 85.1 | 65.2 | 96.4 |
| Average | 76.4 | 104 | 76.4 | 98.6 | 76.4 | 122 |
| Stdev | 50.5 | 69.2 | 50.5 | 70.8 | 50.5 | 80.1 |
| p(t-test) | | 0.10 | | 0.19 | | 0.030 |
| Min | 7.54 | 21.5 | 7.54 | 21.5 | 7.54 | 21.5 |
| Max | 240 | 250 | 240 | 250 | 240 | 250 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 75.7 | 82.6 | 75.7 | 51.5 |
| Average | 87.4 | 88.8 | 87.4 | 79.7 |
| Stdev | 57.4 | 63.1 | 57.4 | 63.2 |
| p(t-test) | | 0.95 | | 0.74 |
| Min | 7.54 | 21.5 | 7.54 | 21.5 |
| Max | 283 | 211 | 283 | 211 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.64 | 0.51 | 0.60 | 0.44 | 0.71 |
| SE | 0.094 | 0.11 | 0.095 | 0.12 | 0.11 |
| p | 0.13 | 0.92 | 0.29 | 0.61 | 0.065 |
| nCohort 1 | 88 | 89 | 88 | 89 | 88 |
| nCohort 2 | 11 | 7 | 11 | 7 | 7 |
| Cutoff 1 | 69.6 | 48.9 | 50.4 | 48.9 | 92.9 |
| Sens 1 | 73% | 71% | 73% | 71% | 71% |
| Spec 1 | 55% | 29% | 35% | 29% | 77% |
| Cutoff 2 | 49.9 | 42.2 | 49.9 | 42.2 | 69.6 |
| Sens 2 | 82% | 86% | 82% | 86% | 86% |
| Spec 2 | 35% | 22% | 35% | 22% | 55% |
| Cutoff 3 | 42.2 | 18.9 | 42.2 | 18.9 | 18.9 |
| Sens 3 | 91% | 100% | 91% | 100% | 100% |
| Spec 3 | 27% | 3% | 27% | 3% | 6% |
| Cutoff 4 | 89.6 | 92.9 | 89.6 | 92.9 | 89.6 |
| Sens 4 | 55% | 43% | 45% | 29% | 71% |
| Spec 4 | 70% | 71% | 70% | 71% | 70% |
| Cutoff 5 | 96.5 | 117 | 96.5 | 117 | 96.5 |
| Sens 5 | 36% | 14% | 27% | 14% | 43% |
| Spec 5 | 81% | 81% | 81% | 81% | 81% |
| Cutoff 6 | 142 | 186 | 142 | 186 | 142 |
| Sens 6 | 18% | 14% | 18% | 14% | 29% |
| Spec 6 | 91% | 91% | 91% | 91% | 91% |
| OR Quart 2 p Value 95% CI of OR Quart2 | 2.0 | 0.48 | 3.1 | 2.1 | 0 |
| | 0.58 | 0.56 | 0.34 | 0.56 | na |
| | 0.17 | 0.040 | 0.30 | 0.18 | na |
| | 24 | 5.7 | 32 | 25 | na |
| OR Quart 3 p Value 95% CI of OR Quart3 | 3.1 | 1.0 | 2.0 | 2.1 | 2.0 |
| | 0.34 | 1.0 | 0.58 | 0.56 | 0.58 |
| | 0.30 | 0.13 | 0.17 | 0.18 | 0.17 |
| | 32 | 7.7 | 24 | 25 | 24 |
| OR Quart 4 p Value 95% CI of OR Quart4 | 5.8 | 1.0 | 5.8 | 2.1 | 4.4 |
| | 0.12 | 1.0 | 0.12 | 0.56 | 0.20 |
| | 0.62 | 0.13 | 0.62 | 0.18 | 0.45 |
| | 53 | 7.7 | 53 | 25 | 43 |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.9 | 37.9 | 2.9 | 37.9 | 22.9 | 26.0 |
| Average | 28.0 | 51.7 | 28.0 | 49.9 | 28.0 | 46.3 |
| Stdev | 20.9 | 42.8 | 20.9 | 41.2 | 20.9 | 52.2 |
| p(t-test) | | 0.0028 | | 0.0051 | | 0.058 |
| Min | 0.672 | 16.1 | 0.672 | 16.1 | 0.672 | 16.1 |
| Max | 121 | 162 | 121 | 162 | 121 | 162 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 24.2 | 47.0 | 24.2 | 47.0 |
| Average | 31.0 | 65.5 | 31.0 | 62.6 |
| Stdev | 27.1 | 48.3 | 27.1 | 47.0 |
| p(t-test) | | 0.0030 | | 0.0062 |
| Min | 0.672 | 25.1 | 0.672 | 25.1 |
| Max | 198 | 162 | 198 | 162 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.73 | 0.82 | 0.73 | 0.81 | 0.63 |
| SE | 0.090 | 0.100 | 0.090 | 0.10 | 0.12 |
| p | 0.0095 | 0.0015 | 0.010 | 0.0018 | 0.27 |
| nCohort 1 | 87 | 88 | 87 | 88 | 87 |
| nCohort 2 | 11 | 7 | 11 | 7 | 7 |
| Cutoff 1 | 25.5 | 37.0 | 25.5 | 37.0 | 24.8 |
| Sens 1 | 73% | 71% | 73% | 71% | 71% |
| Spec 1 | 59% | 75% | 59% | 75% | 57% |
| Cutoff 2 | 24.8 | 32.6 | 24.8 | 32.6 | 18.3 |
| Sens 2 | 82% | 86% | 82% | 86% | 86% |
| Spec 2 | 57% | 68% | 57% | 68% | 43% |
| Cutoff 3 | 18.3 | 24.8 | 18.3 | 24.8 | 16.0 |
| Sens 3 | 91% | 100% | 91% | 100% | 100% |

(continued)

|  | 0hr prior to AKI stage | | 24hr prior to AKI stage | | 48hr prior to AKI stage |
|  | sCr or UO | UO only | sCr or UO | UO only | sCr or UO |
|---|---|---|---|---|---|
| Spec 3 | 43% | 53% | 43% | 53% | 33% |
| Cutoff 4 | 32.2 | 34.0 | 32.2 | 34.0 | 32.2 |
| Sens 4 | 64% | 71% | 64% | 71% | 29% |
| Spec 4 | 70% | 70% | 70% | 70% | 70% |
| Cutoff 5 | 41.4 | 42.2 | 41.4 | 42.2 | 41.4 |
| Sens 5 | 45% | 57% | 45% | 57% | 29% |
| Spec 5 | 80% | 82% | 80% | 82% | 80% |
| Cutoff 6 | 55.7 | 56.8 | 55.7 | 56.8 | 55.7 |
| Sens 6 | 27% | 43% | 27% | 43% | 14% |
| Spec 6 | 91% | 91% | 91% | 91% | 91% |
| OR Quart | >2.1 | >0 | >2.1 | >0 | >2.1 |
| 2 p Value 95% CI ot OR Quart2 | <0.56 | <na | <0.56 | <na | <0.56 |
|  | >0.18 | >na | >0.18 | >na | >0.18 |
|  | na | na | na | na | na |
| OR Quart 3 p Value 95% CI of OR Quart3 | >3.4 | >3.3 | >3.4 | >3.3 | >3.4 |
|  | <0.30 | <0.32 | <0.30 | <0.32 | <0.30 |
|  | >0.33 | >0.32 | >0.33 | >0.32 | >0.33 |
|  | na | na | na | na | na |
| OR Quart 4 p Value 95% CI of OR Quart4 | >7.6 | >4.6 | >7.6 | >4.6 | >2.1 |
|  | <0.071 | <0.19 | <0.071 | <0.19 | <0.56 |
|  | >0.84 | >0.47 | >0.84 | >0.47 | >0.18 |
|  | na | na | na | na | na |

**Betacellulin**

| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
|---|---|---|---|---|---|---|
| Median | 4.68 | 4.10 | 4.68 | 4.10 | 4.68 | 5.12 |
| Average | 5.23 | 11.0 | 5.23 | 11.0 | 5.23 | 15.1 |
| Stdev | 3.00 | 15.7 | 3.00 | 15.7 | 3.00 | 18.7 |
| p(t-test) |  | 0.0025 |  | 0.0025 |  | 2.2E-5 |
| Min | 0.0147 | 0.00289 | 0.0147 | 0.00289 | 0.0147 | 2.42 |
| Max | 15.0 | 50.2 | 15.0 | 50.2 | 15.0 | 50.2 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 4.80 | 3.40 | 4.80 | 3.40 |
| Average | 5.62 | 7.76 | 5.62 | 7.76 |
| Stdev | 6.59 | 11.1 | 6.59 | 11.1 |
| p(t-test) | | 0.44 | | 0.44 |
| Min | 0.00274 | 0.00289 | 0.00274 | 0.00289 |
| Max | 60.7 | 32.2 | 60.7 | 32.2 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or | UO only | sCr or UO |
| AUC | 0.51 | 0.43 | 0.51 | 0.43 | 0.60 |
| SE | 0.093 | 0.12 | 0.093 | 0.12 | 0.12 |
| p | 0.94 | 0.52 | 0.94 | 0.52 | 0.39 |
| nCohort 1 | 87 | 88 | 87 | 88 | 87 |
| nCohort 2 | 11 | 7 | 11 | 7 | 7 |
| Cutoff 1 | 3.32 | 3.09 | 3.32 | 3.09 | 4.08 |
| Sens 1 | 73% | 71% | 73% | 71% | 71% |
| Spec 1 | 24% | 24% | 24% | 24% | 39% |
| Cutoff 2 | 3.09 | 1.92 | 3.09 | 1.92 | 3.40 |
| Sens 2 | 82% | 86% | 82% | 86% | 86% |
| Spec 2 | 22% | 15% | 22% | 15% | 25% |
| Cutoff 3 | 1.92 | 0.00282 | 1.92 | 0.00282 | 1.92 |
| Sens 3 | 91% | 100% | 91% | 100% | 100% |
| Spec 3 | 11% | 2% | 11% | 2% | 11% |
| Cutoff 4 | 6.11 | 6.11 | 6.11 | 6.11 | 6.11 |
| Sens 4 | 36% | 29% | 36% | 29% | 43% |
| Spec 4 | 70% | 70% | 70% | 70% | 70% |
| Cutoff 5 | 7.64 | 7.64 | 7.64 | 7.64 | 7.64 |
| Sens 5 | 36% | 29% | 36% | 29% | 43% |
| Spec 5 | 80% | 81% | 80% | 81% | 80% |
| Cutoff 6 | 8.88 | 8.84 | 8.88 | 8.84 | 8.88 |
| Sens 6 | 27% | 29% | 27% | 29% | 29% |
| Spec 6 | 91% | 91% | 91% | 91% | 91% |

(continued)

| | 0hr prior to AKI stage | | 24hr prior to AKI stage | | 48hr prior to AKI stage |
|---|---|---|---|---|---|
| | sCr or UO | UO only | sCr or | UO only | sCr or UO |
| OR Quart 2 p Value 95% CI of OR Quart2 | 0.95 | 0 | 0.95 | 0 | 2.0 |
| | 0.96 | na | 0.96 | na | 0.58 |
| | 0.17 | na | 0.17 | na | 0.17 |
| | 5.3 | na | 5.3 | na | 24 |
| OR Quart 3 p Value 95% CI of OR Quart3 | 0.30 | 1.6 | 0.30 | 1.6 | 1.0 |
| | 0.32 | 0.64 | 0.32 | 0.64 | 1.0 |
| | 0.029 | 0.24 | 0.029 | 0.24 | 0.059 |
| | 3.2 | 10 | 3.2 | 10 | 17 |
| OR Quart 4 p Value 95% CI of OR Quart4 | 1.3 | 1.0 | 1.3 | 1.0 | 3.1 |
| | 0.73 | 0.96 | 0.73 | 0.96 | 0.34 |
| | 0.27 | 0.13 | 0.27 | 0.13 | 0.30 |
| | 6.7 | 8.1 | 6.7 | 8.1 | 33 |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 38800 | 65700 | 38800 | 65700 | 38800 | 58900 |
| Average | 49600 | 90000 | 49600 | 89000 | 49600 | 96500 |
| Stdev | 39800 | 75300 | 39800 | 75500 | 39800 | 91900 |
| p(t-test) | | 0.0058 | | 0.0071 | | 0.0094 |
| Min | 10500 | 14300 | 10500 | 14300 | 10500 | 14300 |
| Max | 328000 | 275000 | 328000 | 275000 | 328000 | 275000 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 44700 | 83700 | 44700 | 73100 |
| Average | 56000 | 110000 | 56000 | 108000 |
| Stdev | 43600 | 89900 | 43600 | 90500 |
| p(t-test) | | 0.0053 | | 0.0067 |
| Min | 10500 | 14300 | 10500 | 14300 |
| Max | 328000 | 275000 | 328000 | 275000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.70 | 0.69 | 0.69 | 0.68 | 0.68 |
| SE | 0.092 | 0.11 | 0.092 | 0.12 | 0.12 |
| p | 0.030 | 0.094 | 0.036 | 0.11 | 0.13 |
| nCohort 1 | 88 | 89 | 88 | 89 | 88 |
| nCohort 2 | 11 | 7 | 11 | 7 | 7 |
| Cutoff 1 | 51400 | 65300 | 51400 | 65300 | 51400 |
| Sens 1 | 73% | 71% | 73% | 71% | 71% |
| Spec 1 | 68% | 73% | 68% | 73% | 68% |
| Cutoff 2 | 37100 | 32400 | 37100 | 32400 | 37100 |
| Sens 2 | 82% | 86% | 82% | 86% | 86% |
| Spec 2 | 47% | 28% | 47% | 28% | 47% |
| Cutoff 3 | 32400 | 13300 | 32400 | 13300 | 13300 |
| Sens 3 | 91% | 100% | 91% | 100% | 100% |
| Spec 3 | 33% | 4% | 33% | 4% | 6% |
| Cutoff 4 | 54400 | 62700 | 54400 | 62700 | 54400 |
| Sens 4 | 64% | 71% | 64% | 71% | 57% |
| Spec 4 | 70% | 71% | 70% | 71% | 70% |
| Cutoff 5 | 67500 | 75500 | 67500 | 75500 | 67500 |
| Sens 5 | 45% | 57% | 45% | 43% | 43% |
| Spec 5 | 81% | 81% | 81% | 81% | 81% |
| Cutoff 6 | 84900 | 112000 | 84900 | 112000 | 84900 |
| Sens 6 | 27% | 43% | 27% | 43% | 29% |
| Spec 6 | 91% | 91% | 91% | 91% | 91% |
| OR Quart 2 p Value 95% CI of OR Quart2 | 2.0 | 1.0 | 2.0 | 1.0 | 0.96 |
| | 0.58 | 1.0 | 0.58 | 1.0 | 0.98 |
| | 0.17 | 0.059 | 0.17 | 0.059 | 0.056 |
| | 24 | 17 | 24 | 17 | 16 |
| OR Quart 3 p Value 95% CI of OR Quart3 | 3.1 | 1.0 | 3.1 | 1.0 | 2.0 |
| | 0.34 | 1.0 | 0.34 | 1.0 | 0.58 |
| | 0.30 | 0.059 | 0.30 | 0.059 | 0.17 |
| | 32 | 17 | 32 | 17 | 24 |
| OR Quart 4 p Value 95% CI ot OR Quart4 | 5.8 | 4.6 | 5.8 | 4.6 | 3.1 |
| | 0.12 | 0.19 | 0.12 | 0.19 | 0.34 |
| | 0.62 | 0.47 | 0.62 | 0.47 | 0.30 |
| | 53 | 45 | 53 | 45 | 33 |

**Proepiregulin**

| sCr or UO | 0hr prior to AKI stage | | 24hr prior to AKI stage | | 48r prior to AKI stage | |
|---|---|---|---|---|---|---|
| | Cohort 1 | Cohort 2 | Cohort 1 | Cohort 2 | Cohort 1 | Cohort 2 |
| Median | 0.416 | 1.00 | 0.416 | 0.626 | 0.416 | 0.561 |
| Average | 0.599 | 3.17 | 0.599 | 1.67 | 0.599 | 0.714 |
| Stdev | 0.808 | 5.62 | 0.808 | 3.24 | 0.808 | 0.508 |
| p(t-test) | | 9.6E-5 | | 0.011 | | 0.71 |
| Min | 0.000896 | 0.0249 | 0.000896 | 0.0249 | 0.000896 | 0.188 |
| Max | 6.96 | 17.1 | 6.96 | 11.3 | 6.96 | 1.47 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.472 | 1.19 | 10.472 | 0.626 |
| Average | 1.04 | 4.50 | 1.04 | 2.15 |
| Stdev | 3.51 | 6.85 | 3.51 | 4.07 |
| p(t-test) | | 0.023 | | 0.43 |
| Min | 0.000896 | 0.0249 | 0.000896 | 0.0249 |
| Max | 32.1 | 17.1 | 32.1 | 11.3 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.67 | 0.64 | 0.64 | 0.59 | 0.61 |
| SE | 0.094 | 0.12 | 0.095 | 0.12 | 0.12 |
| p | 0.067 | 0.23 | 0.15 | 0.45 | 0.36 |
| nCohort 1 | 87 | 88 | 87 | 88 | 87 |
| nCohort 2 | 11 | 7 | 11 | 7 | 7 |
| Cutoff 1 | 0.356 | 0.356 | 0.356 | 0.356 | 0.286 |
| Sens 1 | 73% | 71% | 73% | 71% | 71% |
| Spec 1 | 43% | 38% | 43% | 38% | 31% |
| Cutoff 2 | 0.261 | 0.179 | 0.261 | 0.179 | 0.261 |
| Sens 2 | 82% | 86% | 82% | 86% | 86% |
| Spec 2 | 31% | 20% | 31% | 20% | 31% |
| Cutoff 3 | 0.179 | 0.0201 | 0.179 | 0.0201 | 0.179 |
| Sens 3 | 91% | 100% | 91% | 100% | 100% |
| Spec 3 | 23% | 5% | 23% | 5% | 23% |

(continued)

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| Cutoff 4 | 0.695 | 0.717 | 0.695 | 0.717 | 0.695 |
| Sens 4 | 55% | 57% | 45% | 43% | 43% |
| Spec 4 | 71% | 70% | 71% | 70% | 71% |
| Cutoff 5 | 0.848 | 0.893 | 0.848 | 0.893 | 0.848 |
| Sens 5 | 55% | 57% | 45% | 43% | 43% |
| Spec 5 | 82% | 81% | 82% | 81% | 82% |
| Cutoff 6 | 1.00 | 1.07 | 1.00 | 1.07 | 1.00 |
| Sens 6 | 55% | 57% | 45% | 43% | 43% |
| Spec 6 | 91% | 91% | 91% | 91% | 91% |
| OR Quart 2 p Value 95% CI of OR Quart2 | 0.96 | 0.46 | 0.96 | 0.46 | 2.0 |
| | 0.97 | 0.53 | 0.97 | 0.53 | 0.58 |
| | 0.12 | 0.039 | 0.12 | 0.039 | 0.17 |
| | 7.4 | 5.4 | 7.4 | 5.4 | 24 |
| OR Quart 3 p Value 95% CI of OR Quart3 | 0.48 | 0 | 1.0 | 0.46 | 1.0 |
| | 0.56 | na | 1.0 | 0.53 | 1.0 |
| | 0.040 | na | 0.13 | 0.039 | 0.059 |
| | 5.7 | na | 7.7 | 5.4 | 17 |
| OR Quart 4 p Value | 3.5 | 2.1 | 2.8 | 1.5 | 3.1 |
| | 0.15 | 0.42 | 0.26 | 0.67 | 0.34 |
| | 0.63 | 0.35 | 0.48 | 0.23 | 0.30 |
| 95% CI of OR Quart4 | 19 | 13 | 16 | 9.9 | 33 |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.197 | 0.274 | 0.197 | 0.274 | 0.197 | 0.274 |
| Average | 0.208 | 0.379 | 0.208 | 0.379 | 0.208 | 0.275 |
| Stdev | 0.188 | 0.380 | 0.188 | 0.381 | 0.188 | 0.167 |
| p(t-test) | | 0.015 | | 0.015 | | 0.36 |
| Min | 8.23E-5 | 0.000127 | 8.23E-5 | 0.000127 | 8.23E-5 | 0.000127 |
| Max | 0.948 | 1.32 | 0.948 | 1.32 | 0.948 | 0.482 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.213 | 0.385 | 0.213 | 0.385 |
| Average | 0.255 | 0.433 | 0.255 | 0.433 |
| Stdev | 0.272 | 0.470 | 0.272 | 0.471 |
| p(t-test) | | 0.12 | | 0.12 |
| Min | 8.23E-5 | 0.000127 | 8.23E-5 | 0.000127 |
| Max | 2.09 | 1.32 | 2.09 | 1.32 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.66 | 0.60 | 0.66 | 0.60 | 0.65 |
| SE | 0.094 | 0.12 | 0.094 | 0.12 | 0.12 |
| p | 0.099 | 0.41 | 0.099 | 0.41 | 0.19 |
| nCohort 1 | 88 | 89 | 88 | 89 | 88 |
| nCohort 2 | 11 | 7 | 11 | 7 | 7 |
| Cutoff 1 | 0.125 | 0.124 | 0.125 | 0.124 | 0.260 |
| Sens 1 | 73% | 71% | 73% | 71% | 71% |
| Spec 1 | 42% | 31% | 42% | 31% | 70% |
| Cutoff 2 | 0.124 | 0.0430 | 0.124 | 0.0430 | 0.124 |
| Sens 2 | 82% | 86% | 82% | 86% | 86% |
| Spec 2 | 42% | 19% | 42% | 19% | 42% |
| Cutoff 3 | 0.0421 | 8.23E-5 | 0.0421 | 8.23E-5 | 8.23E-5 |
| Sens 3 | 91% | 100% | 91% | 100% | 100% |
| Spec 3 | 24% | 2% | 24% | 2% | 7% |
| Cutoff 4 | 0.260 | 0.285 | 0.260 | 0.285 | 0.260 |
| Sens 4 | 64% | 57% | 64% | 57% | 71% |
| Spec 4 | 70% | 71% | 70% | 71% | 70% |
| Cutoff 5 | 0.321 | 0.368 | 0.321 | 0.368 | 0.321 |
| Sens 5 | 45% | 57% | 45% | 57% | 43% |
| Spec 5 | 81% | 81% | 81% | 81% | 81% |
| Cutoff 6 | 0.485 | 0.508 | 0.485 | 0.508 | 0.485 |
| Sens 6 | 18% | 29% | 18% | 29% | 0% |
| Spec 6 | 91% | 91% | 91% | 91% | 91% |

(continued)

|  | 0hr prior to AKI stage | | 24hr prior to AKI stage | | 48hr prior to AKI stage |
|  | sCr or UO | UO only | sCr or UO | UO only | sCr or UO |
|---|---|---|---|---|---|
| OR Quart 2 p Value 95% CI of OR Quart2 | 0.96 | 0.48 | 0.96 | 0.48 | 0.96 |
|  | 0.97 | 0.56 | 0.97 | 0.56 | 0.98 |
|  | 0.12 | 0.040 | 0.12 | 0.040 | 0.056 |
|  | 7.4 | 5.7 | 7.4 | 5.7 | 16 |
| OR Quart 3 p Value 95% CI of OR Quart3 | 0.96 | 0 | 0.96 | 0 | 2.0 |
|  | 0.97 | na | 0.97 | na | 0.58 |
|  | 0.12 | na | 0.12 | na | 0.17 |
|  | 7.4 | na | 7.4 | na | 24 |
| OR Quart 4 p Value 95% CI of OR Quart4 | 2.8 | 2.2 | 2.8 | 2.2 | 3.1 |
|  | 0.26 | 0.39 | 0.26 | 0.39 | 0.34 |
|  | 0.48 | 0.36 | 0.48 | 0.36 | 0.30 |
|  | 16 | 13 | 16 | 13 | 33 |

**Fibroblast growth factor 21**

| sCr or UO | 0hr prior to AKI stage | | 24hr prior to AKI stage | | 48hr prior to AKI stage | |
|  | Cohort 1 | Cohort 2 | Cohort 1 | Cohort 2 | Cohort 1 | Cohort 2 |
|---|---|---|---|---|---|---|
| Median | 0.0451 | 0.418 | 0.0451 | 0.311 | 0.0451 | 0.295 |
| Average | 0.230 | 0.798 | 0.230 | 0.647 | 0.230 | 0.670 |
| Stdev | 0.539 | 0.794 | 0.539 | 0.655 | 0.539 | 0.799 |
| p(t-test) |  | 0.0025 |  | 0.020 |  | 0.048 |
| Min | 9.65E-6 | 0.0658 | 9.65E-6 | 0.0658 | 9.65E-6 | 0.0658 |
| Max | 3.21 | 2.26 | 3.21 | 2.11 | 3.21 | 2.11 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|  | Cohort 1 | Cohort 2 | Cohort 1 | Cohort 2 |
|---|---|---|---|---|
| Median | 0.0674 | 0.605 | 0.0674 | 0.418 |
| Average | 0.284 | 0.955 | 0.284 | 0.718 |
| Stdev | 0.564 | 0.869 | 0.564 | 0.694 |
| p(t-test) |  | 0.0046 |  | 0.057 |
| Min | 9.65E-6 | 0.159 | 9.65E-6 | 0.159 |
| Max | 3.21 | 2.26 | 3.21 | 2.11 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.87 | 0.87 | 0.86 | 0.84 | 0.84 |
| SE | 0.071 | 0.087 | 0.073 | 0.095 | 0.095 |
| p | 1.9E-7 | 1.8E-5 | 1.2E-6 | 3.3E-4 | 3.0E-4 |
| nCohort 1 | 88 | 89 | 88 | 89 | 88 |
| nCohort 2 | 11 | 7 | 11 | 7 | 7 |
| Cutoff 1 | 0.277 | 0.387 | 0.198 | 0.281 | 0.194 |
| Sens 1 | 73% | 71% | 73% | 71% | 71% |
| Spec 1 | 86% | 83% | 83% | 82% | 83% |
| Cutoff 2 | 0.186 | 0.281 | 0.186 | 0.186 | 0.186 |
| Sens 2 | 82% | 86% | 82% | 86% | 86% |
| Spec 2 | 83% | 82% | 83% | 74% | 83% |
| Cutoff 3 | 0.143 | 0.143 | 0.143 | 0.143 | 0.0619 |
| Sens 3 | 91% | 100% | 91% | 100% | 100% |
| Spec 3 | 81% | 72% | 81% | 72% | 59% |
| Cutoff 4 | 0.0949 | 0.134 | 0.0949 | 0.134 | 0.0949 |
| Sens 4 | 91% | 100% | 91% | 100% | 86% |
| Spec 4 | 70% | 71% | 70% | 71% | 70% |
| Cutoff 5 | 0.143 | 0.267 | 0.143 | 0.267 | 0.143 |
| Sens 5 | 91% | 86% | 91% | 71% | 86% |
| Spec 5 | 81% | 81% | 81% | 81% | 81% |
| Cutoff 6 | 0.687 | 1.23 | 0.687 | 1.23 | 0.687 |
| Sens 6 | 36% | 29% | 36% | 14% | 29% |
| Spec 6 | 91% | 91% | 91% | 91% | 91% |
| OR Quart. 2 p Value 95% CI of OR Quart2 | >0 | >0 | >0 | >0 | >0 |
| | <na | <na | <na | <na | <na |
| | >na | >na | >na | >na | >na |
| | na | na | na | na | na |
| OR Quart 3 p Value | >2.1 | >1.0 | >2.1 | >2.2 | >1.0 |
| | <0.56 | <0.98 | <0.56 | <0.54 | <1.0 |
| | >0.18 | >0.062 | >0.18 | >0.18 | >0.059 |
| 95% CI of OR Quart3 | na | na | na | na | na |
| OR Quart >14 4 p Value 95% CI of na OR Quart4 | >14 | >8.0 | >14 | >6.3 | >7.7 |
| | <0.018 | <0.064 | <0.018 | <0.11 | <0.070 |
| | >1.6 | >0.88 | >1.6 | >0.68 | >0.85 |
| | na | na | na | na | na |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 17.3 | 36.4 | 17.3 | 31.2 | 17.3 | 22.6 |
| Average | 20.5 | 62.5 | 20.5 | 57.2 | 20.5 | 62.8 |
| Stdev | 13.5 | 61.2 | 13.5 | 61.5 | 13.5 | 77.9 |
| p(t-test) | | 2.2E-7 | 4.7E-6 | 4.7E-6 | | 1.8E-5 |
| Min | 7.16 | 11.6 | 7.16 | 11.6 | 7.16 | 11.6 |
| Max | 98.9 | 222 | 98.9 | 222 | 98.9 | 222 |
| n (Samp) | 87 | 11 | 87 | 11 | 87 | 7 |
| n (Patient) | 87 | 11 | 87 | 11 | 87 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 18.6 | 44.7 | 18.6 | 36.4 |
| Average | 23.6 | 58.7 | 23.6 | 50.3 |
| Stdev | 22.4 | 31.2 | 22.4 | 30.7 |
| p(t-test) | | 2.0E-4 | | 0.0039 |
| Min | 7.16 | 29.4 | 7.16 | 26.9 |
| Max | 186 | 110 | 186 | 110 |
| n (Samp) | 88 | 7 | 88 | 7 |
| n (Patient) | 88 | 7 | 88 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.84 | 0.91 | 0.83 | 0.88 | 0.75 |
| SE | 0.076 | 0.075 | 0.079 | 0.085 | 0.11 |
| p | 8.6E-6 | 4.9E-8 | 3.4E-5 | 6.8E-6 | 0.025 |
| nCohort 1 | 87 | 88 | 87 | 88 | 87 |
| nCohort 2 | 11 | 7 | 11 | 7 | 7 |
| Cutoff 1 | 29.0 | 33.3 | 26.6 | 31.2 | 21.2 |
| Sens 1 | 73% | 71% | 73% | 71% | 71% |
| Spec 1 | 87% | 86% | 84% | 84% | 71% |
| Cutoff 2 | 21.8 | 31.2 | 21.8 | 29.0 | 20.9 |
| Sens 2 | 82% | 86% | 82% | 86% | 86% |
| Spec 2 | 74% | 84% | 74% | 81% | 69% |
| Cutoff 3 | 20.9 | 29.0 | 20.9 | 25.3 | 11.5 |
| Sens 3 | 91% | 100% | 91% | 100% | 100% |

(continued)

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| Spec 3 | 69% | 81% | 69% | 77% | 18% |
| Cutoff 4 | 21.1 | 21.8 | 21.1 | 21.8 | 21.1 |
| Sens 4 | 82% | 100% | 82% | 100% | 71% |
| Spec 4 | 70% | 70% | 70% | 70% | 70% |
| Cutoff 5 | 24.6 | 29.0 | 24.6 | 29.0 | 24.6 |
| Sens 5 | 73% | 100% | 73% | 86% | 43% |
| Spec 5 | 80% | 81% | 80% | 81% | 80% |
| Cutoff 6 | 31.2 | 40.1 | 31.2 | 40.1 | 31.2 |
| Sens 6 | 64% | 57% | 55% | 43% | 43% |
| Spec 6 | 91% | 91% | 91% | 91% | 91% |
| OR Quart 2 p Value 95% CI of OR Quart2 | 0 | >0 | 0 | >0 | 0 |
| | na | <na | na | <na | na |
| | na | >na | na | >na | na |
| | na | na | na | na | na |
| OR Quart 3 p Value 95% CI of OR Quart3 | 2.1 | >0 | 2.1 | >1.0 | 3.3 |
| | 0.56 | <na | 0.56 | <1.0 | 0.32 |
| | 0.18 | >na | 0.18 | >0.059 | 0.32 |
| | 25 | na | 25 | na | 34 |
| OR Quart 4 p Value 95% CI of OR Quart4 | 11 | >9.5 | 11 | >7.7 | 3.1 |
| | 0.032 | <0.044 | 0.032 | <0.070 | 0.34 |
| | 1.2 | >1.1 | 1.2 | >0.85 | 0.30 |
| | 95 | na | 95 | na | 33 |

**Thrombospondin-2**

| sCr or UO | 0hr prior to SKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40300 | 95600 | 40300 | 95600 | 40300 | 72500 |
| Average | 68700 | 147000 | 68700 | 134000 | 68700 | 111000 |
| Stdev | 81100 | 159000 | 81100 | 143000 | 81100 | 146000 |
| p(t-test) | | 0.0093 | | 0.024 | | 0.22 |
| Min | 1030 | 15000 | 1030 | 14100 | 1030 | 15000 |
| Max | 591000 | 455000 | 591000 | 433000 | 591000 | 433000 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
|---|---|---|---|---|
| | Cohort 1 | Cohort 2 | Cohort 1 | Cohort 2 |
| Median | 50300 | 95800 | 50300 | 95800 |
| Average | 75600 | 199000 | 75600 | 179000 |
| Stdev | 82100 | 180000 | 82100 | 164000 |
| p(t-test) | | 9.2E-4 | | 0.0043 |
| Min | 2770 | 24000 | 2770 | 14100 |
| Max | 591000 | 455000 | 591000 | 433000 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
|---|---|---|---|---|---|
| | sCr or UO | UO only | sCr or UO | UO only | sCr or UO |
| AUC | 0.67 | 0.74 | 0.63 | 0.69 | 0.58 |
| SE | 0.094 | 0.11 | 0.095 | 0.11 | 0.12 |
| p | 0.067 | 0.034 | 0.16 | 0.10 | 0.49 |
| nCohort 1 | 88 | 89 | 88 | 89 | 88 |
| nCohort 2 | 11 | 7 | 11 | 7 | 7 |
| Cutoff 1 | 50600 | 91600 | 27700 | 91600 | 27700 |
| Sens 1 | 73% | 71% | 73% | 71% | 71% |
| Spec 1 | 60% | 69% | 35% | 69% | 35% |
| Cutoff 2 | 27700 | 50500 | 23400 | 23400 | 23400 |
| Sens 2 | 82% | 86% | 82% | 86% | 86% |
| Spec 2 | 35% | 52% | 32% | 31% | 32% |
| Cutoff 3 | 23400 | 23400 | 14100 | 14100 | 13900 |
| Sens 3 | 91% | 100% | 91% | 100% | 100% |
| Spec 3 | 32% | 31% | 17% | 19% | 17% |
| Cutoff 4 | 77700 | 100000 | 77700 | 100000 | 77700 |
| Sens 4 | 55% | 43% | 55% | 43% | 43% |
| Spec 4 | 70% | 71% | 70% | 71% | 70% |
| Cutoff 5 | 110000 | 126000 | 110000 | 126000 | 110000 |
| Sens 5 | 36% | 43% | 36% | 43% | 29% |
| Spec 5 | 81% | 81% | 81% | 81% | 81% |
| Cutoff 6 | 159000 | 171000 | 159000 | 171000 | 159000 |
| Sens 6 | 27% | 43% | 27% | 43% | 14% |
| Spec 6 | 91% | 91% | 91% | 91% | 91% |
| OR Quart | 2.0 | >2.2 | 0.96 | 1.0 | 2.0 |

(continued)

| | 0hr prior to AKI stage | | 24hr prior to AKI stage | | 48hr prior to AKI stage |
| --- | --- | --- | --- | --- | --- |
| | sCr or UO | UO only | sCr or UO | UO only | sCr or UO |
| 2 p Value 95% CI of OR Quart2 | 0.58 | <0.54 | 0.97 | 1.0 | 0.58 |
| | 0.17 | >0.18 | 0.12 | 0.059 | 0.17 |
| | 24 | na | 7.4 | 17 | 24 |
| OR Quart 3 p Value 95% CI of OR Quart3 | 4.4 | >2.2 | 1.5 | 2.1 | 0.96 |
| | 0.20 | <0.54 | 0.67 | 0.56 | 0.98 |
| | 0.45 | >0.18 | 0.23 | 0.18 | 0.056 |
| | 42 | na | 9.9 | 25 | 16 |
| OR Quart 4 p Value 95% CI of OR Quart4 | 4.4 | >3.4 | 2.1 | 3.3 | 3.1 |
| | 0.20 | <0.30 | 0.42 | 0.32 | 0.34 |
| | 0.45 | >0.33 | 0.35 | 0.32 | 0.30 |
| | 42 | na | 13 | 34 | 33 |

**Tenascin**

| sCr or UO | 0hr prior to AKI stage | | 24hr prior to AKI stage | | 48hr prior to AKI stage | |
| --- | --- | --- | --- | --- | --- | --- |
| | Cohort 1 | Cohort 2 | Cohort 1 | Cohort 2 | Cohort 1 | Cohort 2 |
| Median | 483 | 1240 | 483 | 1240 | 483 | 1180 |
| Average | 912 | 1670 | 912 | 1410 | 912 | 1150 |
| Stdev | 1550 | 1380 | 1550 | 584 | 1550 | 333 |
| p(t-test) | | 0.13 | | 0.30 | | 0.69 |
| Min | 94.2 | 815 | 94.2 | 815 | 94.2 | 815 |
| Max | 13100 | 5700 | 13100 | 2830 | 13100 | 1780 |
| n (Samp) | 88 | 11 | 88 | 11 | 88 | 7 |
| n (Patient) | 88 | 11 | 88 | 11 | 88 | 7 |

| UO only | 0hr prior to AKI stage | | 24hr prior to AKI stage | |
| --- | --- | --- | --- | --- |
| | Cohort 1 | Cohort 2 | Cohort 1 | Cohort 2 |
| Median | 527 | 1260 | 527 | 1260 |
| Average | 1410 | 1960 | 1410 | 1550 |
| Stdev | 3250 | 1670 | 3250 | 632 |
| p(t-test) | | 0.66 | | 0.91 |
| Min | 63.0 | 959 | 63.0 | 959 |
| Max | 26100 | 5700 | 26100 | 2830 |
| n (Samp) | 89 | 7 | 89 | 7 |
| n (Patient) | 89 | 7 | 89 | 7 |

| | 0hr prior to AKI stage | | 24hr prior to AKI stage | | 48hr prior to AKI stage |
| --- | --- | --- | --- | --- | --- |
| | sCr or UO | UO only | sCr or | UO only | sCr or UO |
| AUC | 0.86 | 0.82 | 0.85 | 0.82 | 0.83 |
| SE | 0.074 | 0.099 | 0.074 | 0.100 | 0.098 |
| p | 1.4E-6 | 0.0010 | 2.3E-6 | 0.0015 | 8.7E-4 |
| nCohort 1 | 88 | 89 | 88 | 89 | 88 |
| nCohort 2 | 11 | 7 | 11 | 7 | 7 |
| Cutoff 1 | 1140 | 1220 | 1140 | 1220 | 866 |
| Sens 1 | 73% | 71% | 73% | 71% | 71% |
| Spec 1 | 85% | 80% | 85% | 80% | 80% |
| Cutoff 2 | 866 | 1200 | 866 | 1200 | 815 |
| Sens 2 | 82% | 86% | 82% | 86% | 86% |
| Spec 2 | 80% | 80% | 80% | 80% | 76% |
| Cutoff 3 | 815 | 905 | 815 | 905 | 792 |
| Sens 3 | 91% | 100% | 91% | 100% | 100% |
| Spec 3 | 76% | 72% | 76% | 72% | 76% |
| Cutoff 4 | 697 | 866 | 697 | 866 | 697 |
| Sens 4 | 100% | 100% | 100% | 100% | 100% |
| Spec 4 | 70% | 71% | 70% | 71% | 70% |
| Cutoff 5 | 969 | 1250 | 969 | 1250 | 969 |
| Sens 5 | 73% | 57% | 73% | 57% | 57% |
| Spec 5 | 81% | 81% | 81% | 81% | 81% |
| Cutoff 6 | 2080 | 2750 | 2080 | 2750 | 2080 |
| Sens 6 | 9% | 14% | 9% | 14% | 0% |
| Spec 6 | 91% | 91% | 91% | 91% | 91% |
| OR Quart 2 p Value 95% CI ot OR Quart2 | >0 | >0 | >0 | >0 | >0 |
| | <na | <na | <na | <na | <na |
| | >na | >na | >na | >na | >na |
| | na | na | na | na | na |
| OR Quart 3 p Value 95% CI ot OR Quart3 | >3.3 | >1.0 | >3.3 | >1.0 | >2.1 |
| | <0.32 | <0.98 | <0.32 | <0.98 | <0.56 |
| | >0.32 | >0.062 | >0.32 | >0.062 | >0.18 |
| | na | na | na | na | na |
| OR Quart 4 p Value 95% CI of OR Quart4 | >11 | >8.0 | >11 | >8.0 | >6.1 |
| | <0.029 | <0.064 | <0.029 | <0.064 | <0.11 |
| | >1.3 | >0.88 | >1.3 | >0.88 | >0.65 |
| | na | na | na | na | na |

Fig. 9: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

**Angiogenin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6050 | 14100 | 6050 | 10600 | 6050 | 5430 |
| Average | 9790 | 13000 | 9790 | 12400 | 9790 | 8880 |
| Stdev | 8750 | 9340 | 8750 | 9330 | 8750 | 9610 |
| p(t-test) | | 0.080 | | 0.15 | | 0.70 |
| Min | 0.00873 | 647 | 0.00873 | 849 | 0.00873 | 54.6 |
| Max | 30600 | 30600 | 30600 | 30600 | 30600 | 30600 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6110 | 14100 | 6110 | 20000 | 6110 | 11200 |
| Average | 9850 | 15100 | 9850 | 15400 | 9850 | 13100 |
| Stdev | 8800 | 10300 | 8800 | 9770 | 8800 | 9260 |
| p(t-test) | | 0.076 | | 0.036 | | 0.23 |
| Min | 0.00873 | 647 | 0.00873 | 1440 | 0.00873 | 854 |
| Max | 30600 | 30600 | 30600 | 30600 | 30600 | 30600 |
| n (Samp) | 1540 | 9 | 1540 | 11 | 1540 | 11 |
| n (Patient) | 509 | 9 | 509 | 11 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6360 | 20200 | 6360 | 9890 | nd | nd |
| Average | 10000 | 14600 | 10000 | 11000 | nd | nd |
| Stdev | 8840 | 9830 | 8840 | 9090 | nd | nd |
| p(t-test) | | 0.060 | | 0.62 | | nd | nd |
| Min | 0.00873 | 903 | 0.00873 | 849 | nd | nd |
| Max | 30600 | 28600 | 30600 | 30600 | nd | nd |
| n (Samp) | 1513 | 13 | 1513 | 18 | nd | nd |
| n (Patient) | 472 | 13 | 472 | 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.66 | 0.61 | 0.59 | 0.67 | 0.54 | 0.44 | 0.62 | nd |
| SE | 0.063 | 0.100 | 0.084 | 0.062 | 0.090 | 0.070 | 0.080 | 0.091 | nd |
| p | 0.11 | 0.11 | 0.18 | 0.14 | 0.052 | 0.55 | 0.44 | 0.19 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 5510 | 8720 | 2120 | 4960 | 6090 | 4140 | 1620 | 6520 | nd |
| Sens 1 | 74% | 78% | 77% | 71% | 73% | 72% | 71% | 73% | nd |
| Spec 1 | 47% | 58% | 20% | 44% | 50% | 37% | 15% | 51% | nd |
| Cutoff 2 | 2120 | 4780 | 1750 | 2580 | 4960 | 2480 | 844 | 5660 | nd |
| Sens 2 | 83% | 89% | 85% | 83% | 82% | 83% | 86% | 82% | nd |
| Spec 2 | 21% | 43% | 16% | 25% | 44% | 24% | 7% | 48% | nd |
| Cutoff 3 | 964 | 645 | 964 | 2260 | 3510 | 1050 | 409 | 3240 | nd |
| Sens 3 | 91% | 100% | 92% | 92% | 91% | 94% | 93% | 91% | nd |
| Spec 3 | 9% | 5% | 8% | 22% | 33% | 9% | 3% | 31% | nd |
| Cutoff 4 | 14400 | 14600 | 14900 | 14400 | 14600 | 14900 | 14400 | 14600 | nd |
| Sens 4 | 48% | 44% | 54% | 42% | 64% | 33% | 29% | 45% | nd |
| Spec 4 | 70% | 70% | 70% | 70% | 70% | 70% | 70% | 70% | nd |
| Cutoff 5 | 19800 | 20000 | 20300 | 19800 | 20000 | 20300 | 19800 | 20000 | nd |
| Sens 5 | 39% | 33% | 46% | 29% | 55% | 22% | 21% | 36% | nd |
| Spec 5 | 80% | 80% | 80% | 80% | 80% | 80% | 80% | 80% | nd |
| Cutoff 6 | 22700 | 22700 | 23400 | 22700 | 22700 | 23400 | 22700 | 22700 | nd |
| Sens 6 | 13% | 33% | 15% | 12% | 18% | 11% | 7% | 9% | nd |
| Spec 6 | 90% | 90% | 90% | 90% | 90% | 90% | 90% | 90% | nd |
| OR Quart 2 p Value 95% CI of OR Quart2 | 0.60 | 1.0 | 0 | 1.00 | 3.0 | 0.40 | 1.0 | 2.0 | nd |
| | 0.48 | 1.0 | na | 1.00 | 0.34 | 0.27 | 1.00 | 0.57 | nd |
| | 0.14 | 0.062 | na | 0.25 | 0.31 | 0.076 | 0.20 | 0.18 | nd |
| | 2.5 | 16 | na | 4.0 | 29 | 2.1 | 5.0 | 22 | nd |
| OR Quart 3 p Value 95% CI of OR Quart3 | 1.2 | 4.0 | 0.50 | 2.0 | 1.00 | 1.2 | 0.67 | 4.0 | nd |
| | 0.76 | 0.21 | 0.42 | 0.26 | 1.00 | 0.76 | 0.66 | 0.21 | nd |
| | 0.36 | 0.45 | 0.091 | 0.60 | 0.062 | 0.36 | 0.11 | 0.45 | nd |
| | 4.0 | 36 | 2.7 | 6.8 | 16 | 4.0 | 4.0 | 36 | nd |
| OR Quart 4 p Value 95% CI of OR Quart4 | 1.8 | 3.0 | 1.8 | 2.0 | 6.1 | 1.00 | 2.0 | 4.0 | nd |
| | 0.29 | 0.34 | 0.37 | 0.26 | 0.096 | 1.00 | 0.32 | 0.21 | nd |
| | 0.60 | 0.31 | 0.51 | 0.60 | 0.73 | 0.29 | 0.50 | 0.45 | nd |
| | 5.5 | 29 | 6.1 | 6.8 | 51 | 3.5 | 8.1 | 36 | nd |

**Angiopoietin-related protein 4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.5 | 52.3 | 12.5 | 17.2 | 12.5 | 8.12 |
| Average | 46.9 | 120 | 46.9 | 98.0 | 46.9 | 135 |
| Stdev | 108 | 178 | 108 | 141 | 108 | 289 |
| p(t-test) | | 0.0066 | | 0.033 | | 0.013 |
| Min | 0.000466 | 3.67 | 0.000466 | 2.98 | 0.000466 | 0.794 |
| Max | 1370 | 647 | 1370 | 400 | 1370 | 878 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.6 | 191 | 12.6 | 18.2 | 12.6 | 17.4 |
| Average | 47.7 | 205 | 47.7 | 103 | 47.7 | 169 |
| Stdev | 108 | 221 | 108 | 147 | 108 | 317 |
| p(t-test) | | 1.3E-4 | | 0.092 | | 0.0019 |
| Min | 0.000466 | 18.6 | 0.000466 | 2.98 | 0.000466 | 2.66 |
| Max | 1370 | 647 | 1370 | 413 | 1370 | 878 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 12.7 | 16.8 | 12.7 | 12.9 | nd | nd |
| Average | 48.9 | 33.5 | 48.9 | 91.1 | nd | nd |
| Stdev | 111 | 28.2 | 111 | 150 | nd | nd |
| p(t-test) | | 0.68 | | 0.15 | | nd | nd |
| Min | 0.000466 | 3.16 | 0.000466 | 0.000466 | nd | nd |
| Max | 1370 | 83.3 | 1370 | 400 | nd | nd |
| n (Samp) | 1331 | 9 | 1331 | 15 | nd | nd |
| n (Patient) | 397 | 9 | 397 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.85 | 0.60 | 0.63 | 0.66 | 0.52 | 0.44 | 0.57 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.072 | 0.091 | 0.10 | 0.066 | 0.090 | 0.076 | 0.095 | 0.11 | nd |
| p | 0.013 | 1.3E-4 | 0.32 | 0.048 | 0.072 | 0.74 | 0.50 | 0.51 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 16.2 | 56.8 | 11.2 | 14.0 | 17.2 | 7.57 | 5.34 | 9.32 | nd |
| Sens 1 | 71% | 71% | 78% | 71% | 73% | 73% | 70% | 75% | nd |
| Spec 1 | 59% | 83% | 44% | 53% | 60% | 31% | 21% | 39% | nd |
| Cutoff 2 | 8.64 | 23.3 | 8.64 | 11.2 | 14.1 | 5.81 | 4.48 | 6.86 | nd |
| Sens 2 | 82% | 86% | 89% | 81% | 82% | 80% | 80% | 88% | nd |
| Spec 2 | 37% | 67% | 36% | 45% | 53% | 23% | 17% | 28% | nd |
| Cutoff 3 | 6.65 | 18.5 | 3.15 | 5.81 | 14.0 | 2.97 | 2.29 | 2.62 | nd |
| Sens 3 | 94% | 100% | 100% | 90% | 91% | 93% | 90% | 100% | nd |
| Spec 3 | 27% | 62% | 9% | 23% | 53% | 9% | 6% | 7% | nd |
| Cutoff 4 | 24.6 | 25.5 | 24.7 | 24.6 | 25.5 | 24.7 | 24.6 | 25.5 | nd |
| Sens 4 | 53% | 71% | 44% | 38% | 36% | 33% | 20% | 25% | nd |
| Spec 4 | 70% | 70% | 70% | 70% | 70% | 70% | 70% | 70% | nd |
| Cutoff 5 | 44.7 | 47.0 | 46.0 | 44.7 | 47.0 | 46.0 | 44.7 | 47.0 | nd |
| Sens 5 | 53% | 71% | 44% | 33% | 36% | 27% | 20% | 25% | nd |
| Spec 5 | 80% | 80% | 80% | 80% | 80% | 80% | 80% | 80% | nd |
| Cutoff 6 | 118 | 125 | 130 | 118 | 125 | 130 | 118 | 125 | nd |
| Sens 6 | 29% | 57% | 0% | 24% | 27% | 20% | 20% | 25% | nd |
| Spec 6 | 90% | 90% | 90% | 90% | 90% | 90% | 90% | 90% | nd |
| OR Quart 2 p Value 95% CI of OR Quart2 | 4.0 | >0 | 2.0 | 0.66 | 0 | 0.75 | 1.0 | 2.0 | nd |
| | 0.21 | <na | 0.57 | 0.66 | na | 0.70 | 1.00 | 0.57 | nd |
| | 0.45 | >na | 0.18 | 0.11 | na | 0.17 | 0.14 | 0.18 | nd |
| | 36 | na | 22 | 4.0 | na | 3.4 | 7.2 | 22 | nd |
| OR Quart 3 p Value 95% CI of OR Quart3 | 3.0 | >2.0 | 2.0 | 2.7 | 6.1 | 0.75 | 1.0 | 3.0 | nd |
| | 0.34 | <0.57 | 0.57 | 0.14 | 0.096 | 0.70 | 1.0 | 0.34 | nd |
| | 0.31 | >0.18 | 0.18 | 0.71 | 0.73 | 0.17 | 0.14 | 0.31 | nd |
| | 29 | na | 22 | 10 | 51 | 3.4 | 7.1 | 29 | nd |
| OR Quart 4 p Value 95%Clot$_{73}$ OR Quart4 | 9.2 | >5.1 | 4.0 | 2.7 | 4.0 | 1.2 | 2.0 | 2.0 | nd |
| | 0.036 | <0.14 | 0.21 | 0.15 | 0.21 | 0.74 | 0.42 | 0.57 | nd |
| | 1.2 | >0.59 | 0.45 | 0.71 | 0.45 | 0.33 | 0.37 | 0.18 | nd |
| | 73 | na | 36 | 10 | 36 | 4.7 | 11 | 22 | nd |

**Amphiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.5 | 61.0 | 24.5 | 49.9 | 24.5 | 40.5 |
| Average | 55.8 | 106 | 55.8 | 473 | 55.8 | 45.4 |
| Stdev | 142 | 112 | 142 | 969 | 142 | 38.3 |
| p(t-test) | | 0.16 | | 9.9E-19 | | 0.82 |
| Min | 0.00131 | 9.94 | 0.00131 | 17.6 | 0.00131 | 5.13 |
| Max | 1650 | 364 | 1650 | 3480 | 1650 | 124 |
| n (Samp) | 884 | 16 | 884 | 18 | 884 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 25.1 | 44.5 | 25.1 | 72.7 |
| Average | nd | nd | 63.2 | 286 | 63.2 | 104 |
| Stdev | nd | nd | 195 | 579 | 195 | 71.9 |
| p(t-test) | nd | nd | | 0.0018 | | 0.58 |
| Min | nd | nd | 0.00131 | 29.8 | 0.00131 | 25.9 |
| Max | nd | nd | 3480 | 1710 | 3480 | 246 |
| n (Samp) | nd | nd | 916 | 8 | 916 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.6 | 36.4 | 25.6 | 45.7 | nd | nd |
| Average | 56.5 | 70.0 | 56.5 | 468 | nd | nd |
| Stdev | 145 | 71.3 | 145 | 1040 | nd | nd |
| p(t-test) | | 0.77 | | 3.4E-15 | nd | nd |
| Min | 0.00131 | 10.0 | 0.00131 | 10.1 | nd | nd |
| Max | 1710 | 229 | 1710 | 3480 | nd | nd |
| n (Samp) | 879 | 10 | 879 | 14 | nd | nd |
| n (Patient) | 342 | 10 | 342 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.69 | nd | 0.63 | 0.74 | 0.76 | 0.68 | 0.56 | 0.83 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.074 | nd | 0.095 | 0.068 | 0.099 | 0.080 | 0.095 | 0.095 | nd |
| p | 0.010 | nd | 0.17 | 4.5E-4 | 0.0075 | 0.022 | 0.50 | 4.8E-4 | nd |
| nCohort 1 | 884 | nd | 879 | 884 | 916 | 879 | 884 | 916 | nd |
| nCohort 2 | 16 | nd | 10 | 18 | 8 | 14 | 10 | 7 | nd |
| Cutoff 1 | 20.2 | nd | 20.2 | 33.8 | 40.8 | 23.2 | 19.3 | 67.3 | nd |
| Sens 1 | 75% | nd | 70% | 72% | 75% | 71% | 70% | 71% | nd |
| Spec 1 | 44% | nd | 42% | 62% | 66% | 46% | 41% | 83% | nd |
| Cutoff 2 | 19.3 | nd | 19.3 | 23.2 | 33.8 | 20.9 | 10.6 | 65.4 | nd |
| Sens 2 | 81% | nd | 80% | 83% | 88% | 86% | 80% | 86% | nd |
| Spec 2 | 41% | nd | 40% | 48% | 61% | 43% | 21% | 82% | nd |
| Cutoff 3 | 12.6 | nd | 16.5 | 20.9 | 29.8 | 18.9 | 7.58 | 25.8 | nd |
| Sens 3 | 94% | nd | 90% | 94% | 100% | 93% | 90% | 100% | nd |
| Spec 3 | 26% | nd | 35% | 45% | 56% | 39% | 14% | 51% | nd |
| Cutoff 4 | 43.9 | nd | 45.3 | 43.9 | 45.6 | 45.3 | 43.9 | 45.6 | nd |
| Sens 4 | 56% | nd | 40% | 56% | 50% | 50% | 50% | 86% | nd |
| Spec 4 | 70% | nd | 70% | 70% | 70% | 70% | 70% | 70% | nd |
| Cutoff 5 | 57.2 | nd | 57.7 | 57.2 | 58.7 | 57.7 | 57.2 | 58.7 | nd |
| Sens 5 | 50% | nd | 40% | 44% | 38% | 43% | 40% | 86% | nd |
| Spec 5 | 80% | nd | 80% | 80% | 80% | 80% | 80% | 80% | nd |
| Cutoff 6 | 96.9 | nd | 96.9 | 96.9 | 104 | 96.9 | 96.9 | 104 | nd |
| Sens 6 | 31% | nd | 20% | 33% | 38% | 29% | 10% | 43% | nd |
| Spec 6 | 90% | nd | 90% | 90% | 90% | 90% | 90% | 90% | nd |
| OR Quart 2 p Value 95% CI of OR Quart2 | 4.1 | nd | 3.0 | >5.1 | >0 | 4.1 | 0.33 | >0 | nd |
| | 0.21 | nd | 0.34 | <0.14 | <na | 0.21 | 0.34 | <na | nd |
| | 0.45 | nd | 0.31 | >0.59 | >na | 0.45 | 0.034 | >na | nd |
| | 37 | nd | 29 | na | na | 37 | 3.2 | na | nd |
| OR Quart 3 p Value 95% CI of OR Quart3 | 3.0 | nd | 2.0 | >4.1 | >5.1 | 2.0 | 0.33 | >1.0 | nd |
| | 0.34 | nd | 0.57 | <0.21 | <0.14 | 0.57 | 0.34 | <1.0 | nd |
| | 0.31 | nd | 0.18 | >0.45 | >0.59 | 0.18 | 0.034 | >0.062 | nd |
| | 29 | nd | 22 | na | na | 22 | 3.2 | na | nd |
| OR Quart 4 p Value 95% CI of OR Quart4 | 8.3 | nd | 4.0 | >9.3 | >3.0 | 7.2 | 1.7 | >6.1 | nd |
| | 0.047 | nd | 0.21 | <0.035 | <0.34 | 0.067 | 0.48 | <0.094 | nd |
| | 1.0 | nd | 0.45 | >1.2 | >0.31 | 0.87 | 0.40 | >0.73 | nd |
| | 67 | nd | 36 | na | na | 59 | 7.1 | na | nd |

**Betacellulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 2.00 | 0.761 | 1.56 | 0.761 | 1.47 |
| Average | 1.38 | 2.22 | 1.38 | 1.91 | 1.38 | 1.12 |
| Stdev | 2.23 | 1.03 | 2.23 | 2.67 | 2.23 | 0.804 |
| p(t-test) | | 0.13 | | 0.32 | | 0.72 |
| Min | 0.00179 | 0.00230 | 0.00179 | 0.00240 | 0.00179 | 0.00240 |
| Max | 28.3 | 3.96 | 28.3 | 11.6 | 28.3 | 1.94 |
| n (Samp) | 884 | 16 | 884 | 18 | 884 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 0.761 | 1.57 | 0.761 | 1.43 |
| Average | nd | nd | 1.41 | 1.73 | 1.41 | 1.33 |
| Stdev | nd | nd | 2.25 | 1.06 | 2.25 | 1.59 |
| p(t-test) | nd | nd | | 0.70 | | 0.93 |
| Min | nd | nd | 0.00179 | 0.0742 | 0.00179 | 0.00246 |
| Max | nd | nd | 28.3 | 3.65 | 28.3 | 4.42 |
| n (Samp) | nd | nd | 916 | 8 | 916 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.761 | 2.09 | 0.761 | 1.56 | nd | nd |
| Average | 1.37 | 2.33 | 1.37 | 2.05 | nd | nd |
| Stdev | 2.12 | 0.816 | 2.12 | 3.05 | nd | nd |
| p(t-test) | | 0.15 | | 0.23 | nd | nd |
| Min | 0.00179 | 1.30 | 0.00179 | 0.00240 | nd | nd |
| Max | 28.3 | 3.96 | 28.3 | 11.6 | nd | nd |
| n (Samp) | 879 | 10 | 879 | 14 | nd | nd |
| n (Patient) | 342 | 10 | 342 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.72 | nd | 0.77 | 0.59 | 0.65 | 0.57 | 0.47 | 0.47 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.073 | nd | 0.088 | 0.071 | 0.11 | 0.080 | 0.093 | 0.11 | nd |
| p | 0.0021 | nd | 0.0024 | 0.20 | 0.16 | 0.35 | 0.79 | 0.79 | nd |
| nCohort 1 | 884 | nd | 879 | 884 | 916 | 879 | 884 | 916 | nd |
| nCohort 2 | 16 | nd | 10 | 18 | 8 | 14 | 10 | 7 | nd |
| Cutoff 1 | 1.74 | nd | 1.94 | 0.135 | 1.51 | 0.108 | 1.10 | 0.0209 | nd |
| Sens 1 | 75% | nd | 70% | 72% | 75% | 71% | 70% | 71% | nd |
| Spec 1 | 69% | nd | 71% | 41% | 60% | 40% | 55% | 25% | nd |
| Cutoff 2 | 1.59 | nd | 1.74 | 0.0522 | 0.900 | 0.0209 | 0.00240 | 0.00240 | nd |
| Sens 2 | 81% | nd | 80% | 89% | 88% | 86% | 90% | 100% | nd |
| Spec 2 | 66% | nd | 69% | 34% | 51% | 27% | 7% | 7% | nd |
| Cutoff 3 | 1.10 | nd | 1.59 | 0.0209 | 0.0522 | 0.00342 | 0.00240 | 0.00240 | nd |
| Sens 3 | 94% | nd | 90% | 94% | 100% | 93% | 90% | 100% | nd |
| Spec 3 | 55% | nd | 66% | 26% | 33% | 20% | 7% | 7% | nd |
| Cutoff 4 | 1.87 | nd | 1.87 | 1.87 | 1.87 | 1.87 | 1.87 | 1.87 | nd |
| Sens 4 | 62% | nd | 70% | 33% | 38% | 43% | 10% | 14% | nd |
| Spec 4 | 71% | nd | 71% | 71% | 70% | 71% | 71% | 70% | nd |
| Cutoff 5 | 2.41 | nd | 2.41 | 2.41 | 2.42 | 2.41 | 2.41 | 2.42 | nd |
| Sens 5 | 38% | nd | 40% | 22% | 25% | 29% | 0% | 14% | nd |
| Spec 5 | 80% | nd | 80% | 80% | 81% | 80% | 80% | 81% | nd |
| Cutoff 6 | 3.36 | nd | 3.36 | 3.36 | 3.36 | 3.36 | 3.36 | 3.36 | nd |
| Sens 6 | 12% | nd | 10% | 11% | 12% | 14% | 0% | 14% | nd |
| Spec 6 | 91% | nd | 91% | 91% | 91% | 91% | 91% | 91% | nd |
| OR Quart 2 p Value 95% CI of OR Quart2 | 0 | nd | >0 | 5.1 | >1.0 | 2.0 | >7.3 | 3.0 | nd |
| | na | nd | <na | 0.14 | <1.00 | 0.42 | <0.065 | 0.34 | nd |
| | na | nd | >na | 0.59 | >0.062 | 0.37 | >0.89 | 0.31 | nd |
| | na | nd | na | 44 | na | 11 | na | 29 | nd |
| OR Quart 3 | 9.3 | nd | >5.1 | 8.3 | >5.1 | 2.0 | >0 | 1.0 | nd |
| | 0.035 | nd | <0.14 | 0.047 | <0.14 | 0.42 | <na | 1.0 | nd |
| p Value 95% CI ot OR Quart3 | 1.2 | nd | >0.59 | 1.0 | >0.59 | 0.37 | >na | 0.062 | nd |
| | 74 | nd | na | 67 | na | 11 | na | 16 | nd |
| OR Quart 4 p Value 95% CI of OR Quart4 | 6.1 | nd | >5.1 | 4.0 | >2.0 | 2.0 | >3.1 | 2.0 | nd |
| | 0.094 | nd | <0.14 | 0.21 | <0.57 | 0.42 | <0.34 | 0.57 | nd |
| | 0.73 | nd | >0.59 | 0.45 | >0.18 | 0.36 | >0.32 | 0.18 | nd |
| | 51 | nd | na | 36 | na | 11 | na | 22 | nd |

**Endostatin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5660 | 17000 | 5660 | 9540 | 5660 | 3750 |
| Average | 17000 | 44500 | 17000 | 25900 | 17000 | 20200 |
| Stdev | 29300 | 65600 | 29300 | 34200 | 29300 | 39600 |
| p(t-test) | | 1.5E-5 | | 0.14 | | 0.69 |
| Min | 0.0130 | 932 | 0.0130 | 1110 | 0.0130 | 261 |
| Max | 238000 | 227000 | 238000 | 148000 | 238000 | 149000 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 17000 | 5760 | 8130 | 5760 | 19300 |
| Average | 17200 | 56500 | 17200 | 27500 | 17200 | 36000 |
| Stdev | 29600 | 81800 | 29600 | 35200 | 29600 | 47400 |
| p(t-test) | | 9.7E-5 | | 0.25 | | 0.037 |
| Min | 0.0130 | 1980 | 0.0130 | 2050 | 0.0130 | 1790 |
| Max | 238000 | 227000 | 238000 | 110000 | 238000 | 149000 |
| n (Samp) | 1540 | 9 | 1540 | 11 | 1540 | 11 |
| n (Patient) | 509 | 9 | 509 | 11 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5760 | 16700 | 5760 | 9520 | nd | nd |
| Average | 17500 | 42400 | 17500 | 24900 | nd | nd |
| Stdev | 30400 | 63700 | 30400 | 37100 | nd | nd |
| p(t-test) | | 0.0038 | | 0.31 | | nd |
| Min | 0.0130 | 932 | 0.0130 | 1110 | nd | nd |
| Max | 267000 | 190000 | 267000 | 148000 | nd | nd |
| n (Samp) | 1513 | 13 | 1513 | 18 | nd | nd |
| n (Patient) | 472 | 13 | 472 | 18 | nd | nd |

|  | 0hr prior to AKI stage | | | 24hr prior to AKI stage | | | 48hr prior to AKI stage | | |
|---|---|---|---|---|---|---|---|---|---|
|  | sCr or UO sCr | sCr only | UO only | sCr or UO | sCr only | UO only | sCr or UO | UO sCr only | UO only |
| AUC | 0.62 | 0.68 | 0.61 | 0.61 | 0.61 | 0.57 | 0.46 | 0.66 | nd |
| SE | 0.063 | 0.099 | 0.084 | 0.062 | 0.091 | 0.071 | 0.079 | 0.090 | nd |
| p | 0.050 | 0.078 | 0.17 | 0.063 | 0.22 | 0.30 | 0.59 | 0.069 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 4710 | 4940 | 4710 | 4640 | 4410 | 4410 | 3180 | 9560 | nd |
| Sens 1 | 74% | 78% | 77% | 71% | 73% | 72% | 71% | 73% | nd |
| Spec 1 | 43% | 45% | 43% | 43% | 41% | 41% | 29% | 66% | nd |
| Cutoff 2 | 2720 | 3120 | 2150 | 3560 | 4190 | 2970 | 1420 | 3250 | nd |
| Sens 2 | 83% | 89% | 85% | 83% | 82% | 83% | 86% | 82% | nd |
| Spec 2 | 25% | 28% | 17% | 33% | 39% | 26% | 9% | 30% | nd |
| Cutoff 3 | 2010 | 1960 | 2010 | 2950 | 2970 | 1410 | 1290 | 3180 | nd |
| Sens 3 | 91% | 100% | 92% | 92% | 91% | 94% | 93% | 91% | nd |
| Spec 3 | 16% | 16% | 15% | 27% | 27% | 8% | 8% | 29% | nd |
| Cutoff 4 | 11600 | 12000 | 12000 | 11600 | 12000 | 12000 | 11600 | 12000 | nd |
| Sens 4 | 52% | 67% | 54% | 42% | 36% | 39% | 29% | 55% | nd |
| Spec 4 | 70% | 70% | 70% | 70% | 70% | 70% | 70% | 70% | nd |
| Cutoff 5 | 22600 | 22900 | 23700 | 22600 | 22900 | 23700 | 22600 | 22900 | nd |
| Sens 5 | 43% | 44% | 38% | 33% | 36% | 28% | 21% | 36% | nd |
| Spec 5 | 80% | 80% | 80% | 80% | 80% | 80% | 80% | 80% | nd |
| Cutoff 6 | 47200 | 47900 | 48800 | 47200 | 47900 | 48800 | 47200 | 47900 | nd |
| Sens 6 | 26% | 33% | 23% | 25% | 27% | 22% | 14% | 27% | nd |
| Spec 6 | 90% | 90% | 90% | 90% | 90% | 90% | 90% | 90% | nd |
| OR Quart 2 p Value 95% CI of OR Quart2 | 0.80 | 2.0 | 0.33 | 4.1 | 4.0 | 1.7 | 0.50 | 2.0 | nd |
|  | 0.73 | 0.57 | 0.34 | 0.078 | 0.21 | 0.48 | 0.42 | 0.57 | nd |
|  | 0.21 | 0.18 | 0.034 | 0.86 | 0.45 | 0.40 | 0.091 | 0.18 | nd |
|  | 3.0 | 22 | 3.2 | 19 | 36 | 7.0 | 2.7 | 22 | nd |
| OR Quart 3 p Value 95% CI ot OR Quart3 | 0.40 | 1.0 | 0.66 | 2.5 | 2.0 | 1.3 | 1.0 | 2.0 | nd |
|  | 0.27 | 1.0 | 0.66 | 0.27 | 0.57 | 0.71 | 1.00 | 0.57 | nd |
|  | 0.076 | 0.062 | 0.11 | 0.48 | 0.18 | 0.30 | 0.25 | 0.18 | nd |
|  | 2.1 | 16 | 4.0 | 13 | 22 | 6.0 | 4.0 | 22 | nd |
| OR Quart 4 p Value | 2.4 | 5.0 | 2.4 | 4.6 | 4.0 | 2.0 | 1.0 | 6.1 | nd |
|  | 0.097 | 0.14 | 0.22 | 0.053 | 0.21 | 0.33 | 1.00 | 0.096 | nd |
|  | 0.85 | 0.59 | 0.60 | 0.98 | 0.45 | 0.50 | 0.25 | 0.73 | nd |
| 95% CI of OR Quart4 | 7.0 | 43 | 9.2 | 21 | 36 | 8.1 | 4.0 | 51 | nd |

**Proepiregulin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.39 | 4.55 | 3.39 | 6.87 | 3.39 | 3.30 |
| Average | 6.14 | 9.42 | 6.14 | 14.7 | 6.14 | 4.98 |
| Stdev | 12.6 | 12.5 | 12.6 | 21.8 | 12.6 | 5.03 |
| p(t-test) | | 0.30 | | 0.0069 | | 0.77 |
| Min | 0.000104 | 1.42 | 0.000104 | 0.758 | 0.000104 | 0.376 |
| Max | 279 | 49.4 | 279 | 81.9 | 279 | 15.2 |
| n (Samp) | 854 | 16 | 854 | 17 | 854 | 10 |
| n (Patient) | 364 | 16 | 364 | 17 | 364 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 3.46 | 6.47 | 3.46 | 6.79 |
| Average | nd | nd | 6.30 | 13.5 | 6.30 | 7.21 |
| Stdev | nd | nd | 12.8 | 17.7 | 12.8 | 4.19 |
| p(t-test) | nd | nd | | 0.11 | | 0.85 |
| Min | nd | nd | 0.000104 | 5.17 | 0.000104 | 2.96 |
| Max | nd | nd | 279 | 57.1 | 279 | 15.2 |
| n (Samp) | nd | nd | 885 | 8 | 885 | 7 |
| n (Patient) | nd | nd | 377 | 8 | 377 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.52 | 3.45 | 3.52 | 7.39 | nd | nd |
| Average | 6.37 | 5.92 | 6.37 | 13.2 | nd | nd |
| Stdev | 12.8 | 7.42 | 12.8 | 21.6 | nd | nd |
| p(t-test) | | 0.91 | | 0.061 | nd | nd |
| Min | 0.000104 | 1.79 | 0.000104 | 0.758 | nd | nd |
| Max | 279 | 26.4 | 279 | 81.9 | nd | nd |
| n (Samp) | 849 | 10 | 849 | 13 | nd | nd |
| n (Patient) | 339 | 10 | 339 | 13 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | nd | 0.52 | 0.67 | 0.77 | 0.61 | 0.47 | 0.70 | nd |
| SE | 0.076 | nd | 0.093 | 0.073 | 0.098 | 0.084 | 0.094 | 0.11 | nd |
| p | 0.099 | nd | 0.79 | 0.017 | 0.0050 | 0.20 | 0.73 | 0.078 | nd |
| nCohort 1 | 854 | nd | 849 | 854 | 885 | 849 | 854 | 885 | nd |
| nCohort 2 | 16 | nd | 10 | 17 | 8 | 13 | 10 | 7 | nd |
| Cutoff 1 | 3.18 | nd | 2.37 | 5.16 | 5.96 | 1.95 | 1.37 | 4.73 | nd |
| Sens 1 | 75% | nd | 70% | 71% | 75% | 77% | 70% | 71% | nd |
| Spec 1 | 47% | nd | 35% | 66% | 70% | 30% | 21% | 63% | nd |
| Cutoff 2 | 2.22 | nd | 2.22 | 1.95 | 5.42 | 1.45 | 0.806 | 3.74 | nd |
| Sens 2 | 81% | nd | 80% | 82% | 88% | 85% | 80% | 86% | nd |
| Spec 2 | 35% | nd | 33% | 32% | 67% | 21% | 12% | 55% | nd |
| Cutoff 3 | 1.78 | nd | 2.05 | 0.944 | 5.16 | 0.944 | 0.493 | 2.95 | nd |
| Sens 3 | 94% | nd | 90% | 94% | 100% | 92% | 90% | 100% | nd |
| Spec 3 | 29% | nd | 31% | 14% | 66% | 12% | 6% | 44% | nd |
| Cutoff 4 | 5.74 | nd | 6.02 | 5.74 | 5.90 | 6.02 | 5.74 | 5.90 | nd |
| Sens 4 | 44% | nd | 30% | 59% | 75% | 54% | 40% | 57% | nd |
| Spec 4 | 70% | nd | 70% | 70% | 70% | 70% | 70% | 70% | nd |
| Cutoff 5 | 8.36 | nd | 8.53 | 8.36 | 8.47 | 8.53 | 8.36 | 8.47 | nd |
| Sens 5 | 25% | nd | 10% | 41% | 38% | 46% | 20% | 29% | nd |
| Spec 5 | 80% | nd | 80% | 80% | 80% | 80% | 80% | 80% | nd |
| Cutoff 6 Sens 6 Spec 6 | 13.0 | nd | 13.4 | 13.0 | 13.4 | 13.4 | 13.0 | 13.4 | nd |
| | 19% | nd | 10% | 24% | 12% | 23% | 10% | 14% | nd |
| | 90% | nd | 90% | 90% | 90% | 90% | 90% | 90% | nd |
| OR Quart 2 p Value 95% CI of OR Quart2 | 4.0 | nd | >6.1 | 0.33 | >0 | 0.66 | 0.66 | >1.0 | nd |
| | 0.21 | nd | <0.094 | 0.34 | <na | 0.65 | 0.66 | <1.00 | nd |
| | 0.45 | nd | >0.73 | 0.034 | >na | 0.11 | 0.11 | >0.062 | nd |
| | 36 | nd | na | 3.2 | na | 4.0 | 4.0 | na | nd |
| OR Quart 3 p Value 95% CI of OR Quart3 | 6.1 | nd | >2.0 | 1.7 | >5.1 | 0.33 | 0.33 | >3.0 | nd |
| | 0.094 | nd | <0.57 | 0.48 | <0.14 | 0.34 | 0.34 | <0.34 | nd |
| | 0.73 | nd | >0.18 | 0.40 | >0.59 | 0.034 | 0.034 | >0.31 | nd |
| | 51 | nd | na | 7.1 | na | 3.2 | 3.2 | na | nd |
| OR Quart 4 p Value 95% CI ol. OR Quart4 | 5.1 | nd | >2.0 | 2.7 | >3.0 | 2.4 | 1.3 | >3.0 | nd |
| | 0.14 | nd | <0.57 | 0.14 | <0.34 | 0.22 | 0.70 | <0.34 | nd |
| | 0.59 | nd | >0.18 | 0.71 | >0.31 | 0.60 | 0.30 | >0.31 | nd |
| | 44 | nd | na | 10 | na | 9.3 | 6.1 | na | nd |

**Heparin-binding growth factor 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 848 | 289 | 848 | 549 | 848 | 429 |
| Average | 1200 | 488 | 1200 | 967 | 1200 | 582 |
| Stdev | 1420 | 551 | 1420 | 1200 | 1420 | 595 |
| p(t-test) | | 0.016 | | 0.43 | | 0.10 |
| Min | 0.00328 | 55.5 | 0.00328 | 8.50 | 0.00328 | 35.1 |
| Max | 16700 | 2430 | 16700 | 5020 | 16700 | 1910 |
| n (Samp) | 1482 | 23 | 1482 | 23 | 1482 | 14 |
| n (Patient) | 493 | 23 | 493 | 23 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 834 | 189 | 834 | 494 | 834 | 194 |
| Average | 1180 | 335 | 1180 | 624 | 1180 | 433 |
| Stdev | 1400 | 447 | 1400 | 504 | 1400 | 506 |
| p(t-test) | | 0.070 | | 0.21 | | 0.076 |
| Min | 0.00328 | 55.5 | 0.00328 | 90.5 | 0.00328 | 32.9 |
| Max | 16700 | 1470 | 16700 | 1650 | 16700 | 1500 |
| n (Samp) | 1539 | 9 | 1539 | 10 | 1539 | 11 |
| n (Patient) | 509 | 9 | 509 | 10 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 842 | 320 | 842 | 546 | nd | nd |
| Average | 1200 | 444 | 1200 | 827 | nd | nd |
| Stdev | 1430 | 350 | 1430 | 889 | nd | nd |
| p(t-test) | | 0.056 | | 0.27 | nd | nd |
| Min | 0.00328 | 70.1 | 0.00328 | 8.50 | nd | nd |
| Max | 16700 | 1140 | 16700 | 3420 | nd | nd |
| n (Samp) | 1511 | 13 | 1511 | 18 | nd | nd |
| n (Patient) | 472 | 13 | 472 | 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.28 | 0.20 | 0.28 | 0.42 | 0.37 | 0.40 | 0.32 | 0.26 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.061 | 0.089 | 0.081 | 0.063 | 0.095 | 0.071 | 0.080 | 0.086 | nd |
| p | 2.1E-4 | 9.2E-4 | 0.0059 | 0.19 | 0.16 | 0.17 | 0.026 | 0.0045 | nd |
| nCohort 1 | 1482 | 1539 | 1511 | 1482 | 1539 | 1511 | 1482 | 1539 | nd |
| nCohort 2 | 23 | 9 | 13 | 23 | 10 | 18 | 14 | 11 | nd |
| Cutoff 1 | 155 | 110 | 155 | 364 | 449 | 364 | 125 | 77.7 | nd |
| Sens 1 | 74% | 78% | 77% | 74% | 70% | 72% | 71% | 73% | nd |
| Spec 1 | 11% | 8% | 11% | 24% | 30% | 25% | 9% | 6% | nd |
| Cutoff 2 | 145 | 69.8 | 145 | 119 | 247 | 119 | 61.5 | 61.5 | nd |
| Sens 2 | 83% | 89% | 85% | 83% | 80% | 83% | 86% | 82% | nd |
| Spec 2 | 11% | 5% | 11% | 9% | 17% | 9% | 5% | 5% | nd |
| Cutoff 3 | 110 | 55.2 | 119 | 89.7 | 91.2 | 75.4 | 51.2 | 34.6 | nd |
| Sens 3 | 91% | 100% | 92% | 91% | 90% | 94% | 93% | 91% | nd |
| Spec 3 | 8% | 5% | 9% | 7% | 7% | 6% | 4% | 3% | nd |
| Cutoff 4 | 1380 | 1340 | 1360 | 1380 | 1340 | 1360 | 1380 | 1340 | nd |
| Sens 4 | 9% | 11% | 0% | 13% | 10% | 11% | 14% | 9% | nd |
| Spec 4 | 70% | 70% | 70% | 70% | 70% | 70% | 70% | 70% | nd |
| Cutoff 5 | 1730 | 1710 | 1730 | 1730 | 1710 | 1730 | 1730 | 1710 | nd |
| Sens 5 | 4% | 0% | 0% | 13% | 0% | 11% | 7% | 0% | nd |
| Spec 5 | 80% | 80% | 80% | 80% | 80% | 80% | 80% | 80% | nd |
| Cutoff 6 | 2450 | 2430 | 2460 | 2450 | 2430 | 2460 | 2450 | 2430 | nd |
| Sens 6 | 0% | 0% | 0% | 13% | 0% | 11% | 0% | 0% | nd |
| Spec 6 | 90% | 90% | 90% | 90% | 90% | 90% | 90% | 90% | nd |
| OR Quart 2 p Value 95% CI of OR Quart2 | 3.0 | >1.0 | >3.0 | 2.0 | 2.0 | 2.5 | 3.0 | >2.0 | nd |
| | 0.34 | <1.00 | <0.34 | 0.32 | 0.57 | 0.27 | 0.34 | <0.57 | nd |
| | 0.31 | >0.062 | >0.31 | 0.50 | 0.18 | 0.49 | 0.31 | >0.18 | nd |
| | 29 | na | na | 8.1 | 22 | 13 | 29 | na | nd |
| OR Quart 3 p Value 95% CI of OR Quart3 | 5.1 | >1.0 | >2.0 | 2.4 | 4.0 | 2.5 | 3.0 | >2.0 | nd |
| | 0.14 | <1.00 | <0.57 | 0.21 | 0.21 | 0.27 | 0.34 | <0.57 | nd |
| | 0.59 | >0.062 | >0.18 | 0.61 | 0.45 | 0.49 | 0.31 | >0.18 | nd |
| | 44 | na | na | 9.2 | 36 | 13 | 29 | na | nd |
| OR Quart 4 p Value 95% CI ot OR Quart4 | 15 | >7.1 | >8.2 | 2.4 | 3.0 | 3.0 | 7.1 | >7.1 | nd |
| | 0.0099 | <0.067 | <0.048 | 0.21 | 0.34 | 0.17 | 0.067 | <0.066 | nd |
| | 1.9 | >0.87 | >1.0 | 0.61 | 0.31 | 0.61 | 0.87 | >0.88 | nd |
| | 110 | na | na | 9.2 | 29 | 15 | 58 | na | nd |

**Fibroblast growth factor 19**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 0.000168 | 2.83E-5 | 2.63E-5 |
| Average | 0.00494 | 0.0778 | 0.00494 | 0.0504 | 0.00494 | 0.00105 |
| Stdev | 0.0336 | 0.249 | 0.0336 | 0.155 | 0.0336 | 0.00323 |
| p(t-test) | | 9.9E-12 | | 9.4E-8 | | 0.71 |
| Min | 5.62E-6 | 5.62E-6 | 5.62E-6 | 1.66E-5 | 5.62E-6 | 2.17E-5 |
| Max | 0.634 | 1.02 | 0.634 | 0.560 | 0.634 | 0.0102 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 0.00600 | 2.83E-5 | 0.000197 | 2.83E-5 | 2.55E-5 |
| Average | 0.00524 | 0.188 | 0.00524 | 0.0539 | 0.00524 | 2.62E-5 |
| Stdev | 0.0354 | 0.377 | 0.0354 | 0.168 | 0.0354 | 3.91E-6 |
| p(t-test) | | 2.0E-27 | | 2.7E-5 | | 0.68 |
| Min | 5.62E-6 | 7.53E-6 | 5.62E-6 | 1.88E-5 | 5.62E-6 | 2.17E-5 |
| Max | 0.634 | 1.02 | 0.634 | 0.560 | 0.634 | 3.21E-5 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.83E-5 | 2.83E-5 | 2.83E-5 | 4.39E-5 | nd | nd |
| Average | 0.00839 | 0.113 | 0.00839 | 0.0349 | nd | nd |
| Stdev | 0.0779 | 0.340 | 0.0779 | 0.121 | nd | nd |
| p(t-test) | | 1.3E-4 | | 0.19 | nd | nd |
| Min | 5.62E-6 | 1.88E-5 | 5.62E-6 | 1.66E-5 | nd | nd |
| Max | 2.28 | 1.02 | 2.28 | 0.470 | nd | nd |
| n (Samp) | 1331 | 9 | 1331 | 15 | nd | nd |
| n (Patient) | 397 | 9 | 397 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.55 | 0.61 | 0.54 | 0.66 | 0.66 | 0.62 | 0.47 | 0.41 | nd |
| SE | 0.072 | 0.11 | 0.099 | 0.066 | 0.090 | 0.078 | 0.093 | 0.11 | nd |
| p | 0.52 | 0.34 | 0.69 | 0.015 | 0.075 | 0.12 | 0.77 | 0.37 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 1.92E-5 | 1.82E-5 | 2.17E-5 | 2.53E-5 | 2.17E-5 | 2.46E-5 | 2.38E-5 | 2.17E-5 | nd |
| Sens 1 | 71% | 71% | 78% | 76% | 73% | 73% | 70% | 75% | nd |
| Spec 1 | 26% | 18% | 30% | 39% | 29% | 36% | 34% | 29% | nd |
| Cutoff 2 | 1.82E-5 | 1.34E-5 | 1.88E-5 | 1.92E-5 | 1.88E-5 | 1.92E-5 | 2.17E-5 | 1.92E-5 | nd |
| Sens 2 | 88% | 86% | 89% | 81% | 82% | 80% | 80% | 100% | nd |
| Spec 2 | 18% | 10% | 22% | 26% | 22% | 26% | 29% | 26% | nd |
| Cutoff 3 | 1.34E-5 | 5.62E-6 | 1.82E-5 | 1.82E-5 | 1.82E-5 | 1.82E-5 | 1.92E-5 | 1.92E-5 | nd |
| Sens 3 | 94% | 100% | 100% | 95% | 100% | 93% | 100% | 100% | nd |
| Spec 3 | 10% | 4% | 18% | 18% | 18% | 18% | 26% | 26% | nd |
| Cutoff 4 | 4.39E-5 | 4.39E-5 | 4.39E-5 | 4.39E-5 | 4.39E-5 | 4.39E-5 | 4.39E-5 | 4.39E-5 | nd |
| Sens 4 | 35% | 57% | 11% | 57% | 64% | 47% | 10% | 0% | nd |
| Spec 4 | 72% | 73% | 73% | 72% | 73% | 73% | 72% | 73% | nd |
| Cutoff 5 Sens 5 | 0.000114 | 0.000114 | 0.000114 | 0.000114 | 0.000114 | 0.000114 | 0.000114 | 0.000114 | nd |
| Spec 5 | 35% | 57% | 11% | 52% | 55% | 47% | 10% | 0% | nd |
| | 81% | 81% | 81% | 81% | 81% | 81% | 81% | 81% | nd |
| Cutoff 6 | 0.00364 | 0.00364 | 0.00364 | 0.00364 | 0.00364 | 0.00364 | 0.00364 | 0.00364 | nd |
| Sens 6 | 29% | 57% | 11% | 19% | 36% | 20% | 10% | 0% | nd |
| Spec 6 | 90% | 90% | 90% | 90% | 90% | 90% | 90% | 90% | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 2 p Value 95% CI of OR Quart2 | 0.60 | 0 | 1.0 | 0.75 | 0.33 | 1.00 | 3.0 | >3.0 | nd |
| | 0.48 | na | 1.0 | 0.70 | 0.34 | 1.00 | 0.34 | <0.34 | nd |
| | 0.14 | na | 0.14 | 0.17 | 0.034 | 0.20 | 0.31 | >0.31 | nd |
| | 2.5 | na | 7.1 | 3.4 | 3.2 | 5.0 | 29 | na | nd |
| OR Quart 3 p Value 95% CI of OR Quart3 | 0.60 | 0 | 2.0 | 0.50 | 0 | 0.66 | 6.1 | >5.1 | nd |
| | 0.48 | na | 0.42 | 0.42 | na | 0.66 | 0.095 | <0.14 | nd |
| | 0.14 | na | 0.37 | 0.090 | na | 0.11 | 0.73 | >0.59 | nd |
| | 2.5 | na | 11 | 2.7 | na | 4.0 | 51 | na | nd |
| OR Quart 4 p Value 95% CI of OR Quart4 | 1.2 | 1.3 | 0.50 | 3.1 | 2.4 | 2.4 | 0 | >0 | nd |
| | 0.77 | 0.71 | 0.57 | 0.054 | 0.22 | 0.22 | na | <na | nd |
| | 0.36 | 0.30 | 0.045 | 0.98 | 0.60 | 0.60 | na | >na | nd |
| | 4.0 | 6.0 | 5.5 | 9.6 | 9.2 | 9.2 | na | na | nd |

EP 2 661 626 B1

**Fibroblast growth factor 21**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0140 | 0.0567 | 0.0140 | 0.0466 | 0.0140 | 0.00852 |
| Average | 0.168 | 0.249 | 0.168 | 0.214 | 0.168 | 0.0619 |
| Stdev | 0.559 | 0.393 | 0.559 | 0.393 | 0.559 | 0.149 |
| p(t-test) | | 0.55 | | 0.71 | | 0.55 |
| Min | 1.14E-9 | 0.00290 | 1.14E-9 | 7.54E-8 | 1.14E-9 | 0.000662 |
| Max | 8.92 | 1.53 | 8.92 | 1.59 | 8.92 | 0.482 |
| n (Samp) | 1276 | 17 | 1276 | 21 | 1276 | 10 |
| n (Patient) | 400 | 17 | 400 | 21 | 400 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0145 | 0.318 | 0.0145 | 0.0466 | 0.0145 | 0.0115 |
| Average | 0.174 | 0.434 | 0.174 | 0.0590 | 0.174 | 0.110 |
| Stdev | 0.558 | 0.553 | 0.558 | 0.0659 | 0.558 | 0.185 |
| p(t-test) | | 0.22 | | 0.50 | | 0.75 |
| Min | 1.14E-9 | 0.00620 | 1.14E-9 | 7.54E-8 | 1.14E-9 | 0.00255 |
| Max | 8.92 | 1.53 | 8.92 | 0.206 | 8.92 | 0.482 |
| n (Samp) | 1324 | 7 | 1324 | 11 | 1324 | 8 |
| n (Patient) | 414 | 7 | 414 | 11 | 414 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0142 | 0.0809 | 0.0142 | 0.0598 | nd | nd |
| Average | 0.175 | 0.176 | 0.175 | 0.287 | nd | nd |
| Stdev | 0.590 | 0.183 | 0.590 | 0.449 | nd | nd |
| p(t-test) | | 1.00 | | 0.46 | nd | nd |
| Min | 1.14E-9 | 0.00163 | 1.14E-9 | 7.54E-8 | nd | nd |
| Max | 8.92 | 0.442 | 8.92 | 1.59 | nd | nd |
| n (Samp) | 1331 | 9 | 1331 | 15 | nd | nd |
| n (Patient) | 397 | 9 | 397 | 15 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.71 | 0.66 | 0.59 | 0.56 | 0.60 | 0.42 | 0.50 | nd |
| SE | 0.073 | 0.11 | 0.100 | 0.066 | 0.090 | 0.078 | 0.095 | 0.10 | nd |
| p | 0.029 | 0.052 | 0.10 | 0.20 | 0.50 | 0.18 | 0.37 | 1.00 | nd |
| nCohort 1 | 1276 | 1324 | 1331 | 1276 | 1324 | 1331 | 1276 | 1324 | nd |
| nCohort 2 | 17 | 7 | 9 | 21 | 11 | 15 | 10 | 8 | nd |
| Cutoff 1 | 0.0171 | 0.0191 | 0.0172 | 0.00670 | 0.0116 | 0.00670 | 0.00324 | 0.00583 | nd |
| Sens 1 | 71% | 71% | 78% | 71% | 73% | 73% | 70% | 75% | nd |
| Spec 1 | 56% | 57% | 56% | 32% | 43% | 32% | 18% | 28% | nd |
| Cutoff 2 | 0.00618 | 0.0115 | 0.00289 | 0.00514 | 0.00484 | 0.00544 | 0.00254 | 0.00324 | nd |
| Sens 2 | 82% | 86% | 89% | 81% | 82% | 80% | 80% | 88% | nd |
| Spec 2 | 30% | 43% | 17% | 26% | 25% | 27% | 15% | 18% | nd |
| Cutoff 3 | 0.00293 | 0.00618 | 0.00162 | 0.00228 | 0.00108 | 0.00228 | 0.00105 | 0.00254 | nd |
| Sens 3 | 94% | 100% | 100% | 90% | 91% | 93% | 90% | 100% | nd |
| Spec 3 | 17% | 30% | 10% | 14% | 8% | 14% | 8% | 15% | nd |
| Cutoff 4 | 0.0347 | 0.0374 | 0.0353 | 0.0347 | 0.0374 | 0.0353 | 0.0347 | 0.0374 | nd |
| Sens 4 | 59% | 57% | 67% | 52% | 55% | 53% | 30% | 38% | nd |
| Spec 4 | 70% | 70% | 70% | 70% | 70% | 70% | 70% | 70% | nd |
| Cutoff 5 | 0.0850 | 0.0996 | 0.0863 | 0.0850 | 0.0996 | 0.0863 | 0.0850 | 0.0996 | nd |
| Sens 5 | 41% | 57% | 44% | 33% | 18% | 40% | 10% | 25% | nd |
| Spec 5 | 80% | 80% | 80% | 80% | 80% | 80% | 80% | 80% | nd |
| Cutoff 6 | 0.374 | 0.400 | 0.374 | 0.374 | 0.400 | 0.374 | 0.374 | 0.400 | nd |
| Sens 6 | 24% | 43% | 22% | 19% | 0% | 27% | 10% | 12% | nd |
| Spec 6 | 90% | 90% | 90% | 90% | 90% | 90% | 90% | 90% | nd |
| OR Quart 2 p Value | 0.33 | >2.0 | 0 | 1.7 | 0.33 | 2.0 | 1.0 | 1.0 | nd |
| | 0.34 | <0.57 | na | 0.48 | 0.34 | 0.42 | 1.00 | 1.0 | nd |
| | 0.034 | >0.18 | na | 0.40 | 0.034 | 0.36 | 0.14 | 0.14 | nd |
| 95% CI of OR Quart2 | 3.2 | na | na | 7.1 | 3.2 | 11 | 7.2 | 7.1 | nd |
| OR Quart 3 p Value 95% CI of OR Quart3 | 1.3 | >1.0 | 0.50 | 1.0 | 0.66 | 0.50 | 1.0 | 1.0 | nd |
| | 0.70 | <1.0 | 0.57 | 1.0 | 0.65 | 0.57 | 1.0 | 1.0 | nd |
| | 0.30 | >0.062 | 0.045 | 0.20 | 0.11 | 0.045 | 0.14 | 0.14 | nd |
| | 6.0 | na | 5.5 | 5.0 | 4.0 | 5.5 | 7.1 | 7.1 | nd |
| OR Quart p Value 95% CI ot OR Quart4 | 3.0 | >4.0 | 3.0 | 3.4 | 1.7 | 4.1 | 2.0 | 1.0 | nd |
| | 0.097 | <0.21 | 0.18 | 0.065 | 0.48 | 0.078 | 0.42 | 1.0 | nd |
| | 0.82 | >0.45 | 0.61 | 0.93 | 0.40 | 0.86 | 0.37 | 0.14 | nd |
| | 11 | na | 15 | 12 | 7.1 | 19 | 11 | 7.1 | nd |

**Heparin-binding EGF-like growth factor**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 131 | 135 | 131 | 157 | 131 | 136 |
| Average | 140 | 141 | 140 | 163 | 140 | 129 |
| Stdev | 58.5 | 43.3 | 58.5 | 90.7 | 58.5 | 38.1 |
| p(t-test) | | 0.94 | | 0.14 | | 0.56 |
| Min | 31.0 | 83.4 | 31.0 | 53.4 | 31.0 | 69.9 |
| Max | 360 | 234 | 360 | 444 | 360 | 177 |
| n (Samp) | 816 | 16 | 816 | 16 | 816 | 10 |
| n (Patient) | 362 | 16 | 362 | 16 | 362 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 130 | 151 | 130 | 170 |
| Average | nd | nd | 140 | 152 | 140 | 160 |
| Stdev | nd | nd | 58.9 | 61.3 | 58.9 | 42.7 |
| p(t-test) | nd | nd | | 0.57 | | 0.37 |
| Min | nd | nd | 31.0 | 74.6 | 31.0 | 76.6 |
| Max | nd | nd | 444 | 265 | 444 | 217 |
| n (Samp) | nd | nd | 847 | 8 | 847 | 7 |
| n (Patient) | nd | nd | 375 | 8 | 375 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 130 | 116 | 130 | 124 | nd | nd |
| Average | 140 | 129 | 140 | 149 | nd | nd |
| Stdev | 58.8 | 48.9 | 58.8 | 96.8 | nd | nd |
| p(t-test) | | 0.58 | | 0.56 | nd | nd |
| Min | 31.0 | 77.1 | 31.0 | 53.4 | nd | nd |
| Max | 360 | 234 | 360 | 444 | nd | nd |
| n (Samp) | 811 | 10 | 811 | 13 | nd | nd |
| n (Patient) | 338 | 10 | 338 | 13 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | nd | 0.46 | 0.56 | 0.56 | 0.49 | 0.47 | 0.64 | nd |
| SE | 0.074 | nd | 0.094 | 0.075 | 0.11 | 0.081 | 0.093 | 0.11 | nd |
| p | 0.72 | nd | 0.63 | 0.39 | 0.56 | 0.91 | 0.76 | 0.22 | nd |
| nCohort 1 | 816 | nd | 811 | 816 | 847 | 811 | 816 | 847 | nd |
| nCohort 2 | 16 | nd | 10 | 16 | 8 | 13 | 10 | 7 | nd |
| Cutoff 1 | 118 | nd | 100 | 110 | 102 | 100 | 120 | 164 | nd |
| Sens 1 | 75% | nd | 70% | 75% | 75% | 77% | 70% | 71% | nd |
| Spec 1 | 42% | nd | 31% | 36% | 31% | 31% | 42% | 70% | nd |
| Cutoff 2 | 107 | nd | 92.4 | 102 | 98.4 | 99.1 | 96.4 | 143 | nd |
| Sens 2 | 81% | nd | 80% | 81% | 88% | 85% | 80% | 86% | nd |
| Spec 2 | 34% | nd | 25% | 31% | 29% | 30% | 27% | 58% | nd |
| Cutoff 3 | 84.8 | nd | 84.8 | 74.4 | 74.4 | 81.8 | 76.5 | 76.5 | nd |
| Sens 3 | 94% | nd | 90% | 94% | 100% | 92% | 90% | 100% | nd |
| Spec 3 | 17% | nd | 18% | 10% | 11% | 16% | 12% | 12% | nd |
| Cutoff 4 | 166 | nd | 166 | 166 | 165 | 166 | 166 | 165 | nd |
| Sens 4 | 25% | nd | 20% | 31% | 38% | 23% | 20% | 57% | nd |
| Spec 4 | 70% | nd | 70% | 70% | 70% | 70% | 70% | 70% | nd |
| Cutoff 5 | 192 | nd | 192 | 192 | 190 | 192 | 192 | 190 | nd |
| Sens 5 | 12% | nd | 10% | 12% | 12% | 8% | 0% | 14% | nd |
| Spec 5 | 80% | nd | 80% | 80% | 80% | 80% | 80% | 80% | nd |
| Cutoff 6 | 222 | nd | 220 | 222 | 220 | 220 | 222 | 220 | nd |
| Sens 6 | 6% | nd | 10% | 12% | 12% | 8% | 0% | 0% | nd |
| Spec 6 | 90% | nd | 90% | 90% | 90% | 90% | 90% | 90% | nd |
| OR Quart 2 p Value 95% CI of OR Quart2 | 3.1 | nd | 1.0 | 2.0 | 2.0 | 2.0 | >6.2 | 0 | nd |
| | 0.17 | nd | 1.00 | 0.42 | 0.57 | 0.42 | <0.092 | na | nd |
| | 0.61 | nd | 0.14 | 0.37 | 0.18 | 0.37 | >0.74 | na | nd |
| | 15 | nd | 7.2 | 11 | 22 | 11 | na | na | nd |
| OR Quart 3 p Value 95% CI of OR Quart3 | 2.0 | nd | 2.0 | 3.1 | 2.0 | 2.5 | >2.0 | 4.1 | nd |
| | 0.42 | nd | 0.42 | 0.17 | 0.57 | 0.27 | <0.57 | 0.21 | nd |
| | 0.37 | nd | 0.37 | 0.61 | 0.18 | 0.49 | >0.18 | 0.45 | nd |
| | 11 | nd | 11 | 15 | 22 | 13 | na | 37 | nd |
| OR Quart 4 p Value 95% CI of OR Quart4 | 2.0 | nd | 1.0 | 2.0 | 3.0 | 1.0 | >2.0 | 2.0 | nd |
| | 0.42 | nd | 1.00 | 0.42 | 0.34 | 1.0 | <0.56 | 0.57 | nd |
| | 0.37 | nd | 0.14 | 0.37 | 0.31 | 0.14 | >0.18 | 0.18 | nd |
| | 11 | nd | 7.2 | 11 | 29 | 7.2 | na | 22 | nd |

**Thrombospondin-2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1300 | 2180 | 1300 | 1520 | 1300 | 1450 |
| Average | 2160 | 3460 | 2160 | 8940 | 2160 | 1480 |
| Stdev | 2890 | 3590 | 2890 | 20600 | 2890 | 1120 |
| p(t-test) | | 0.032 | | 2.1E-17 | | 0.38 |
| Min | 0.0376 | 90.7 | 0.0376 | 113 | 0.0376 | 14.6 |
| Max | 45800 | 14600 | 45800 | 80100 | 45800 | 3740 |
| n (Samp) | 1483 | 23 | 1483 | 24 | 1483 | 14 |
| n (Patient) | 493 | 23 | 493 | 24 | 493 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1340 | 1920 | 1340 | 1840 | 1340 | 1590 |
| Average | 2320 | 3280 | 2320 | 9720 | 2320 | 1770 |
| Stdev | 3500 | 4440 | 3500 | 23400 | 3500 | 1070 |
| p(t-test) | | 0.41 | | 9.0E-10 | | 0.60 |
| Min | 0.0376 | 90.7 | 0.0376 | 679 | 0.0376 | 419 |
| Max | 68100 | 14600 | 68100 | 80100 | 68100 | 3740 |
| n (Samp) | 1540 | 9 | 1540 | 11 | 1540 | 11 |
| n (Patient) | 509 | 9 | 509 | 11 | 509 | 11 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1370 | 1670 | 1370 | 1250 | nd | nd |
| Average | 2280 | 2790 | 2280 | 6600 | nd | nd |
| Stdev | 3540 | 2840 | 3540 | 16200 | nd | nd |
| p(t-test) | | 0.60 | | 3.5E-6 | nd | nd |
| Min | 0.0376 | 150 | 0.0376 | 113 | nd | nd |
| Max | 80100 | 8180 | 80100 | 68100 | nd | nd |
| n (Samp) | 1513 | 13 | 1513 | 18 | nd | nd |
| n (Patient) | 472 | 13 | 472 | 18 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.58 | 0.53 | 0.57 | 0.66 | 0.48 | 0.46 | 0.54 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.063 | 0.100 | 0.082 | 0.061 | 0.090 | 0.069 | 0.079 | 0.089 | nd |
| p | 0.12 | 0.44 | 0.71 | 0.27 | 0.071 | 0.80 | 0.61 | 0.66 | nd |
| nCohort 1 | 1483 | 1540 | 1513 | 1483 | 1540 | 1513 | 1483 | 1540 | nd |
| nCohort 2 | 23 | 9 | 13 | 24 | 11 | 18 | 14 | 11 | nd |
| Cutoff 1 | 831 | 878 | 481 | 1020 | 1460 | 560 | 813 | 1250 | nd |
| Sens 1 | 74% | 78% | 77% | 71% | 73% | 72% | 71% | 73% | nd |
| Spec 1 | 33% | 34% | 17% | 40% | 54% | 21% | 32% | 47% | nd |
| Cutoff 2 | 404 | 831 | 333 | 560 | 1330 | 360 | 276 | 1020 | nd |
| Sens 2 | 83% | 89% | 85% | 83% | 82% | 83% | 86% | 82% | nd |
| Spec 2 | 16% | 33% | 11% | 22% | 50% | 12% | 9% | 40% | nd |
| Cutoff 3 | 163 | 90.6 | 163 | 291 | 1020 | 167 | 72.4 | 619 | nd |
| Sens 3 | 91% | 100% | 92% | 92% | 91% | 94% | 93% | 91% | nd |
| Spec 3 | 4% | 1% | 4% | 10% | 40% | 4% | 1% | 24% | nd |
| Cutoff 4 | 2250 | 2330 | 2330 | 2250 | 2330 | 2330 | 2250 | 2330 | nd |
| Sens 4 | 48% | 33% | 46% | 38% | 36% | 28% | 21% | 18% | nd |
| Spec 4 | 70% | 70% | 70% | 70% | 70% | 70% | 70% | 70% | nd |
| Cutoff 5 | 3140 | 3250 | 3240 | 3140 | 3250 | 3240 | 3140 | 3250 | nd |
| Sens 5 | 39% | 22% | 31% | 29% | 36% | 22% | 7% | 18% | nd |
| Spec 5 | 80% | 80% | 80% | 80% | 80% | 80% | 80% | 80% | nd |
| Cutoff 6 | 4640 | 5100 | 4800 | 4640 | 5100 | 4800 | 4640 | 5100 | nd |
| Sens 6 | 35% | 11% | 31% | 21% | 27% | 17% | 0% | 0% | nd |
| Spec 6 | 90% | 90% | 90% | 90% | 90% | 90% | 90% | 90% | nd |
| OR Quart 2 p Value 95% CI of OR Quart2 | 0.80 | 2.0 | 0.50 | 1.00 | >3.0 | 0.60 | 1.7 | 1.5 | nd |
| | 0.73 | 0.57 | 0.42 | 1.00 | <0.34 | 0.48 | 0.48 | 0.66 | nd |
| | 0.21 | 0.18 | 0.090 | 0.29 | >0.31 | 0.14 | 0.40 | 0.25 | nd |
| | 3.0 | 22 | 2.7 | 3.5 | na | 2.5 | 7.1 | 9.0 | nd |
| OR Quart 3 p Value 95% CI of OR Quart3 | 0.60 | 4.0 | 0.50 | 1.2 | >4.0 | 0.80 | 0.67 | 2.0 | nd |
| | 0.48 | 0.21 | 0.42 | 0.77 | <0.21 | 0.74 | 0.66 | 0.42 | nd |
| | 0.14 | 0.45 | 0.091 | 0.36 | >0.45 | 0.21 | 0.11 | 0.37 | nd |
| | 2.5 | 36 | 2.7 | 4.0 | na | 3.0 | 4.0 | 11 | nd |
| OR Quart 4 p Value 95% CI of 6.5 OR Quart4 | 2.2 | 2.0 | 1.2 | 1.6 | >4.0 | 1.2 | 1.3 | 1.00 | nd |
| | 0.14 | 0.57 | 0.74 | 0.41 | <0.21 | 0.76 | 0.70 | 1.00 | nd |
| | 0.77 | 0.18 | 0.33 | 0.52 | >0.45 | 0.37 | 0.30 | 0.14 | nd |
| | 6.5 | 22 | 4.7 | 5.0 | na | 4.0 | 6.0 | 7.1 | nd |

**Tenascin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.6 | 9.54 | 10.6 | 15.6 | 10.6 | 0.221 |
| Average | 19.1 | 19.1 | 19.1 | 437 | 19.1 | 10.9 |
| Stdev | 50.4 | 34.4 | 50.4 | 1770 | 50.4 | 20.2 |
| p(t-test) | | 1.00 | | 3.5E-12 | | 0.61 |
| Min | 0.00398 | 0.0190 | 0.00398 | 0.0190 | 0.00398 | 0.0184 |
| Max | 945 | 141 | 945 | 7540 | 945 | 50.9 |
| n (Samp) | 883 | 16 | 883 | 18 | 883 | 10 |
| n (Patient) | 367 | 16 | 367 | 18 | 367 | 10 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 10.6 | 20.6 | 10.6 | 0.338 |
| Average | nd | nd | 27.3 | 23.6 | 27.3 | 9.59 |
| Stdev | nd | nd | 254 | 9.89 | 254 | 19.1 |
| p(t-test) | nd | nd | | 0.97 | | 0.85 |
| Min | nd | nd | 0.00398 | 9.36 | 0.00398 | 0.0184 |
| Max | nd | nd | 7540 | 39.5 | 7540 | 50.9 |
| n (Samp) | nd | nd | 915 | 8 | 915 | 7 |
| n (Patient) | nd | nd | 380 | 8 | 380 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 10.7 | 11.4 | 10.7 | 4.85 | nd | nd |
| Average | 19.4 | 26.0 | 19.4 | 552 | nd | nd |
| Stdev | 50.4 | 42.2 | 50.4 | 2010 | nd | nd |
| p(t-test) | | 0.68 | | 4.9E-15 | nd | nd |
| Min | 0.00398 | 0.0208 | 0.00398 | 0.00398 | nd | nd |
| Max | 945 | 141 | 945 | 7540 | nd | nd |
| n (Samp) | 878 | 10 | 878 | 14 | nd | nd |
| n (Patient) | 342 | 10 | 342 | 14 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | nd | 0.56 | 0.58 | 0.73 | 0.45 | 0.35 | 0.34 | nd |

(continued)

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| SE | 0.073 | nd | 0.095 | 0.071 | 0.10 | 0.080 | 0.095 | 0.11 | nd |
| p | 0.91 | nd | 0.54 | 0.28 | 0.026 | 0.52 | 0.12 | 0.17 | nd |
| nCohort 1 | 883 | nd | 878 | 883 | 915 | 878 | 883 | 915 | nd |
| nCohort 2 | 16 | nd | 10 | 18 | 8 | 14 | 10 | 7 | nd |
| Cutoff 1 | 0.338 | nd | 7.28 | 8.63 | 20.5 | 0.0748 | 0.0187 | 0.0187 | nd |
| Sens 1 | 75% | nd | 70% | 72% | 75% | 79% | 70% | 71% | nd |
| Spec 1 | 28% | nd | 43% | 46% | 72% | 18% | 11% | 11% | nd |
| Cutoff 2 | 0.315 | nd | 0.338 | 0.0840 | 15.6 | 0.0187 | 0.0184 | 0.0184 | nd |
| Sens 2 | 88% | nd | 80% | 83% | 88% | 93% | 80% | 86% | nd |
| Spec 2 | 26% | nd | 28% | 20% | 63% | 11% | 9% | 9% | nd |
| Cutoff 3 | 0.0190 | nd | 0.315 | 0.0748 | 8.63 | 0.0187 | 0.0179 | 0.0179 | nd |
| Sens 3 | 94% | nd | 90% | 94% | 100% | 93% | 100% | 100% | nd |
| Spec 3 | 13% | nd | 26% | 17% | 46% | 11% | 8% | 8% | nd |
| Cutoff 4 | 19.5 | nd | 19.8 | 19.5 | 19.5 | 19.8 | 19.5 | 19.5 | nd |
| Sens 4 | 31% | nd | 40% | 39% | 75% | 29% | 20% | 14% | nd |
| Spec 4 | 70% | nd | 70% | 70% | 70% | 70% | 70% | 70% | nd |
| Cutoff 5 | 27.8 | nd | 28.1 | 27.8 | 27.8 | 28.1 | 27.8 | 27.8 | nd |
| Sens 5 | 19% | nd | 30% | 22% | 38% | 14% | 20% | 14% | nd |
| Spec 5 | 80% | nd | 81% | 80% | 80% | 81% | 80% | 80% | nd |
| Cutoff 6 | 40.0 | nd | 41.6 | 40.0 | 40.0 | 41.6 | 40.0 | 40.0 | nd |
| Sens 6 | 6% | nd | 10% | 11% | 0% | 14% | 20% | 14% | nd |
| Spec 6 | 90% | nd | 90% | 90% | 90% | 90% | 90% | 90% | nd |
| OR Quart 2 p Value 95% CI of OR Quart2 | 4.1 | nd | 4.1 | 0.75 | >1.0 | 2.0 | 0.50 | 1.0 | nd |
| | 0.077 | nd | 0.21 | 0.70 | <1.0 | 0.42 | 0.57 | 1.00 | nd |
| | 0.86 | nd | 0.45 | 0.17 | >0.062 | 0.37 | 0.045 | 0.062 | nd |
| | 19 | nd | 37 | 3.4 | na | 11 | 5.6 | 16 | nd |
| OR Quart 3 p Value 95% CI of OR Quart3 | 1.00 | nd | 1.0 | 1.8 | >4.1 | 1.0 | 1.0 | 2.0 | nd |
| | 1.00 | nd | 1.0 | 0.37 | <0.21 | 1.0 | 1.00 | 0.57 | nd |
| | 0.14 | nd | 0.062 | 0.51 | >0.45 | 0.14 | 0.14 | 0.18 | nd |
| | 7.1 | nd | 16 | 6.1 | na | 7.2 | 7.2 | 22 | nd |
| OR Quart 4 p Value 95% CI of OR Quart4 | 2.0 | nd | 4.1 | 1.00 | >3.0 | 3.1 | 2.5 | 3.0 | nd |
| | 0.42 | nd | 0.21 | 0.99 | <0.34 | 0.17 | 0.27 | 0.34 | nd |
| | 0.36 | nd | 0.45 | 0.25 | >0.31 | 0.61 | 0.49 | 0.31 | nd |
| | 11 | nd | 37 | 4.0 | na | 15 | 13 | 29 | nd |

Fig. 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

**Angiopoietin-related protein 4**

| sCr or UO | 24hr prior to AKI stage | |
|---|---|---|
| | Cohort 1 | Cohort 2 |
| Median | 32.9 | 96.2 |
| Average | 49.1 | 127 |
| Stdev | 97.1 | 115 |
| p(t-test) | | 0.051 |
| Min | 1.77 | 25.0 |
| Max | 1900 | 339 |
| n (Samp) | 487 | 6 |
| n (Patient) | 218 | 6 |

| | 24hr prior to AKI stage | | |
|---|---|---|---|
| | sCr or UO | sCr only | UO only |
| AUC | 0.80 | nd | nd |
| SE | 0.11 | nd | nd |
| p | 0.0049 | nd | nd |
| nCohort 1 | 487 | nd | nd |
| nCohort 2 | 6 | nd | nd |
| Cutoff 1 | 52.4 | nd | nd |
| Sens 1 | 83% | nd | nd |
| Spec 1 | 74% | nd | nd |
| Cutoff 2 | 52.4 | nd | nd |
| Sens 2 | 83% | nd | nd |
| Spec 2 | 74% | nd | nd |
| Cutoff 3 | 25.0 | nd | nd |
| Sens 3 | 100% | nd | nd |
| Spec 3 | 37% | nd | nd |
| Cutoff 4 | 47.5 | nd | nd |
| Sens 4 | 83% | nd | nd |
| Spec 4 | 70% | nd | nd |
| Cutoff 5 | 61.9 | nd | nd |
| Sens 5 | 50% | nd | nd |
| Spec 5 | 80% | nd | nd |
| Cutoff 6 | 92.2 | nd | nd |
| Sens 6 | 50% | nd | nd |
| Spec 6 | 90% | nd | nd |
| OR | >1.0 | nd | nd |

(continued)

|  | 24hr prior to AKI stage | | |
|---|---|---|---|
|  | sCr or UO | sCr only | UO only |
| Quart 2 | <1.00 | nd | nd |
| p Value | >0.062 | nd | nd |
| 95% CI of OR Quart2 | na | nd | nd |
| OR Quart 3 p Value 95% CI of OR Quart3 | >1.0 | nd | nd |
|  | <1.00 | nd | nd |
|  | >0.062 | nd | nd |
|  | na | nd | nd |
| OR Quart 4 p Value 95% CI of OR Quart4 | >4.1 | nd | nd |
|  | <0.21 | nd | nd |
|  | >0.45 | nd | nd |
|  | na | nd | nd |

**Amphiregulin**

| sCr or UO | 24hr prior to AKI stage | |
|---|---|---|
|  | Cohort 1 | Cohort 2 |
| Median | 22.4 | 41.7 |
| Average | 28.3 | 40.8 |
| Stdev | 22.9 | 16.4 |
| p(t-test) |  | 0.18 |
| Min | 0.00246 | 19.8 |
| Max | 198 | 60.8 |
| n (Samp) | 478 | 6 |
| n (Patient) | 214 | 6 |

|  | 24hr prior to AKI stage | | |
|---|---|---|---|
|  | sCr or UO | sCr only | UO only |
| AUC | 0.75 | nd | nd |
| SE | 0.12 | nd | nd |
| p | 0.034 | nd | nd |
| nCohort 1 | 478 | nd | nd |
| nCohort 2 | 6 | nd | nd |
| Cutoff 1 | 24.8 | nd | nd |
| Sens 1 | 83% | nd | nd |
| Spec 1 | 56% | nd | nd |
| Cutoff 2 | 24.8 | nd | nd |
| Sens 2 | 83% | nd | nd |

(continued)

| | 24hr prior to AKI stage | | |
| --- | --- | --- | --- |
| | sCr or UO | sCr only | UO only |
| Spec 2 | 56% | nd | nd |
| Cutoff 3 | 19.6 | nd | nd |
| Sens 3 | 100% | nd | nd |
| Spec 3 | 43% | nd | nd |
| Cutoff 4 | 32.2 | nd | nd |
| Sens 4 | 67% | nd | nd |
| Spec 4 | 70% | nd | nd |
| Cutoff 5 | 39.9 | nd | nd |
| Sens 5 | 50% | nd | nd |
| Spec 5 | 80% | nd | nd |
| Cutoff 6 | 54.0 | nd | nd |
| Sens 6 | 33% | nd | nd |
| Spec 6 | 90% | nd | nd |
| OR Quart 2 p Value 95% CI of OR Quart2 | >1.0 | nd | nd |
| | <1.00 | nd | nd |
| | >0.062 | nd | nd |
| | na | nd | nd |
| OR Quart 3 p Value 95% CI of OR Quart3 | >1.0 | nd | nd |
| | <1.00 | nd | nd |
| | >0.062 | nd | nd |
| | na | nd | nd |
| OR Quart 4 p Value | >4.1 | nd | nd |
| | <0.21 | nd | nd |
| | >0.46 | nd | nd |
| 95% CI of OR Quart4 | na | nd | nd |

**Betacellulin**

| sCr or UO | 24hr prior to AKI stage | |
| --- | --- | --- |
| | Cohort 1 | Cohort 2 |
| Median | 4.02 | 6.55 |
| Average | 4.75 | 12.8 |
| Stdev | 5.32 | 16.1 |
| p(t-test) | 4.7E-4 | 4.7E-4 |
| Min | 0.00226 | 0.00289 |
| Max | 60.7 | 43.4 |
| n (Samp) | 479 | 6 |

(continued)

| sCr or UO | 24hr prior to AKI stage | |
| --- | --- | --- |
| | Cohort 1 | Cohort 2 |
| n (Patient) | 216 | 6 |

| | 24hr prior to AKI stage | | |
| --- | --- | --- | --- |
| | sCr or UO | sCr only | UO only |
| AUC | 0.64 | nd | nd |
| SE | 0.12 | nd | nd |
| p | 0.25 | nd | nd |
| nCohort 1 | 479 | nd | nd |
| nCohort 2 | 6 | nd | nd |
| Cutoff 1 | 3.09 | nd | nd |
| Sens 1 | 83% | nd | nd |
| Spec 1 | 36% | nd | nd |
| Cutoff 2 | 3.09 | nd | nd |
| Sens 2 | 83% | nd | nd |
| Spec 2 | 36% | nd | nd |
| Cutoff 3 | 0.00282 | nd | nd |
| Sens 3 | 100% | nd | nd |
| Spec 3 | 5% | nd | nd |
| Cutoff 4 | 5.42 | nd | nd |
| Sens 4 | 50% | nd | nd |
| Spec 4 | 70% | nd | nd |
| Cutoff 5 | 6.66 | nd | nd |
| Sens 5 | 50% | nd | nd |
| Spec 5 | 80% | nd | nd |
| Cutoff 6 | 8.60 | nd | nd |
| Sens 6 | 50% | nd | nd |
| Spec 6 | 90% | nd | nd |
| OR Quart 2 p Value 95% CI of OR Quart2 | 1.0 | nd | nd |
| | 1.0 | nd | nd |
| | 0.062 | nd | nd |
| | 16 | nd | nd |
| OR Quart 3 p Value 95% CI of OR Quart3 | 1.0 | nd | nd |
| | 1.0 | nd | nd |
| | 0.062 | nd | nd |
| | 16 | nd | nd |

(continued)

|  | 24hr prior to AKI stage | | |
|---|---|---|---|
|  | sCr or UO | sCr only | UO only |
| OR Quart 4 p Value 95% CI of OR Quart4 | 3.0 | nd | nd |
|  | 0.34 | nd | nd |
|  | 0.31 | nd | nd |
|  | 29 | nd | nd |

**Proepiregulin**

| sCr or UO | 24hr prior to AKI stage | |
|---|---|---|
|  | Cohort 1 | Cohort 2 |
| Median | 0.392 | 1.07 |
| Average | 0.771 | 2.58 |
| Stdev | 2.33 | 4.32 |
| p(t-test) |  | 0.063 |
| Min | 0.000152 | 0.0249 |
| Max | 32.1 | 11.3 |
| n (Samp) | 478 | 6 |
| n (Patient) | 215 | 6 |

|  | 24hr prior to AKI stage | | |
|---|---|---|---|
|  | sCr or UO | sCr only | UO only |
| AUC | 0.72 | nd | nd |
| SE | 0.12 | nd | nd |
| p | 0.067 | nd | nd |
| nCohort 1 | 478 | nd | nd |
| nCohort 2 | 6 | nd | nd |
| Cutoff 1 | 0.513 | nd | nd |
| Sens 1 | 83% | nd | nd |
| Spec 1 | 61% | nd | nd |
| Cutoff 2 | 0.513 | nd | nd |
| Sens 2 | 83% | nd | nd |
| Spec 2 | 61% | nd | nd |
| Cutoff 3 | 0.0201 | nd | nd |
| Sens 3 | 100% | nd | nd |
| Spec 3 | 5% | nd | nd |
| Cutoff 4 | 0.645 | nd | nd |
| Sens 4 | 67% | nd | nd |
| Spec 4 | 70% | nd | nd |

(continued)

|  | 24hr prior to AKI stage | | |
|---|---|---|---|
|  | sCr or UO | sCr only | UO only |
| Cutoff 5 | 0.806 | nd | nd |
| Sens 5 | 67% | nd | nd |
| Spec 5 | 81% | nd | nd |
| Cutoff 6 | 1.24 | nd | nd |
| Sens 6 | 50% | nd | nd |
| Spec 6 | 90% | nd | nd |
| OR Quart 2 p Value 95% CI of OR Quart2 | 0 | nd | nd |
|  | na | nd | nd |
|  | na | nd | nd |
|  | na | nd | nd |
| OR Quart 3 p Value | 1.0 | nd | nd |
|  | 1.0 | nd | nd |
|  | 0.062 | nd | nd |
| 95% CI of OR Quart3 | 16 | nd | nd |
| OR | 4.1 | nd | nd |
| Quart 4 | 0.21 | nd | nd |
| p Value | 0.45 | nd | nd |
| 95% CI of OR Quart4 | 37 | nd | nd |

**Fibroblast growth factor 19**

| sCr or UO | 24hr prior to AKI stage | |
|---|---|---|
|  | Cohort 1 | Cohort 2 |
| Median | 0.157 | 0.271 |
| Average | 0.198 | 0.414 |
| Stdev | 0.198 | 0.369 |
| p(t-test) |  | 0.0090 |
| Min | 2.92E-5 | 0.0449 |
| Max | 2.09 | 0.971 |
| n (Samp) | 487 | 6 |
| n (Patient) | 218 | 6 |

|  | 24hr prior to AKI stage | | |
|---|---|---|---|
|  | sCr or UO | sCr only | UO only |
| AUC | 0.68 | nd | nd |
| SE | 0.12 | nd | nd |
| p | 0.14 | nd | nd |

(continued)

|  | 24hr prior to AKI stage | | |
|---|---|---|---|
|  | sCr or UO | sCr only | UO only |
| nCohort 1 | 487 | nd | nd |
| nCohort 2 | 6 | nd | nd |
| Cutoff 1 | 0.172 | nd | nd |
| Sens 1 | 83% | nd | nd |
| Spec 1 | 54% | nd | nd |
| Cutoff 2 | 0.172 | nd | nd |
| Sens 2 | 83% | nd | nd |
| Spec 2 | 54% | nd | nd |
| Cutoff 3 | 0.0430 | nd | nd |
| Sens 3 | 100% | nd | nd |
| Spec 3 | 16% | nd | nd |
| Cutoff 4 | 0.233 | nd | nd |
| Sens 4 | 50% | nd | nd |
| Spec 4 | 70% | nd | nd |
| Cutoff 5 | 0.300 | nd | nd |
| Sens 5 | 50% | nd | nd |
| Spec 5 | 80% | nd | nd |
| Cutoff 6 | 0.395 | nd | nd |
| Sens 6 | 33% | nd | nd |
| Spec 6 | 90% | nd | nd |
| OR Quart 2 p Value 95% CI of OR Quart2 | 0 | nd | nd |
|  | na | nd | nd |
|  | na | nd | nd |
|  | na | nd | nd |
| OR Quart 3 p Value 95% CI of OR Quart3 | 2.0 | nd | nd |
|  | 0.57 | nd | nd |
|  | 0.18 | nd | nd |
|  | 23 | nd | nd |
| OR Quart 4 p Value 95% CI of OR Quart4 | 3.0 | nd | nd |
|  | 0.34 | nd | nd |
|  | 0.31 | nd | nd |
|  | 29 | nd | nd |

**Fibroblast growth factor 21**

| sCr or UO | 24hr prior to AKI stage | |
|---|---|---|
| | Cohort 1 | Cohort 2 |
| Median | 0.0538 | 0.103 |
| Average | 0.214 | 0.141 |
| Stdev | 0.529 | 0.145 |
| p(t-test) | | 0.74 |
| Min | 4.60E-6 | 0.0212 |
| Max | 5.72 | 0.418 |
| n (Samp) | 487 | 6 |
| n (Patient) | 218 | 6 |

| | 24hr prior to AKI stage | | |
|---|---|---|---|
| | sCr or UO | sCr only | UO only |
| AUC | 0.61 | nd | nd |
| SE | 0.12 | nd | nd |
| p | 0.39 | nd | nd |
| nCohort 1 | 487 | nd | nd |
| nCohort 2 | 6 | nd | nd |
| Cutoff 1 | 0.0388 | nd | nd |
| Sens 1 | 83% | nd | nd |
| Spec 1 | 42% | nd | nd |
| Cutoff 2 | 0.0388 | nd | nd |
| Sens 2 | 83% | nd | nd |
| Spec 2 | 42% | nd | nd |
| Cutoff 3 | 0.0209 | nd | nd |
| Sens 3 | 100% | nd | nd |
| Spec 3 | 29% | nd | nd |
| Cutoff 4 | 0.129 | nd | nd |
| Sens 4 | 33% | nd | nd |
| Spec 4 | 70% | nd | nd |
| Cutoff 5 | 0.224 | nd | nd |
| Sens 5 | 17% | nd | nd |
| Spec 5 | 80% | nd | nd |
| Cutoff 6 | 0.495 | nd | nd |
| Sens 6 | 0% | nd | nd |
| Spec 6 | 90% | nd | nd |

(continued)

|  | 24hr prior to AKI stage | | |
|---|---|---|---|
|  | sCr or UO | sCr only | UO only |
| OR Quart 2 p Value | >2.0 | nd | nd |
|  | <0.56 | nd | nd |
|  | >0.18 | nd | nd |
| 95% CI of OR Quart2 | na | nd | nd |
| OR Quart 3 p Value 95% CI of OR Quart3 | >3.1 | nd | nd |
|  | <0.33 | nd | nd |
|  | >0.32 | nd | nd |
|  | na | nd | nd |
| OR Quart 4 p Value 95% CI of OR Quart4 | >1.0 | nd | nd |
|  | <1.0 | nd | nd |
|  | >0.062 | nd | nd |
|  | na | nd | nd |

**Heparin-binding EGF-like growth factor**

| sCr or UO | 24hr prior to AKI stage | |
|---|---|---|
|  | Cohort 1 | Cohort 2 |
| Median | 16.3 | 46.9 |
| Average | 20.0 | 74.3 |
| Stdev | 15.8 | 74.4 |
| p(t-test) |  | 2.1E-13 |
| Min | 5.20 | 21.0 |
| Max | 186 | 222 |
| n (Samp) | 476 | 6 |
| n (Patient) | 215 | 6 |

|  | 24hr prior to AKI stage | | |
|---|---|---|---|
|  | sCr or UO | sCr only | UO only |
| AUC | 0.92 | nd | nd |
| SE | 0.076 | nd | nd |
| p | 2.4E-8 | nd | nd |
| nCohort 1 | 476 | nd | nd |
| nCohort 2 | 6 | nd | nd |
| Cutoff 1 | 35.8 | nd | nd |
| Sens 1 | 83% | nd | nd |
| Spec 1 | 92% | nd | nd |
| Cutoff 2 | 35.8 | nd | nd |

(continued)

| | 24hr prior to AKI stage | | |
|---|---|---|---|
| | sCr or UO | sCr only | UO only |
| Sens 2 | 83% | nd | nd |
| Spec 2 | 92% | nd | nd |
| Cutoff 3 | 20.9 | nd | nd |
| Sens 3 | 100% | nd | nd |
| Spec 3 | 73% | nd | nd |
| Cutoff 4 | 20.2 | nd | nd |
| Sens 4 | 100% | nd | nd |
| Spec 4 | 70% | nd | nd |
| Cutoff 5 | 23.6 | nd | nd |
| Sens 5 | 83% | nd | nd |
| Spec 5 | 80% | nd | nd |
| Cutoff 6 | 31.9 | nd | nd |
| Sens 6 | 83% | nd | nd |
| Spec 6 | 90% | nd | nd |
| OR Quart 2 p Value 95% CI of OR Quart2 | >0 | nd | nd |
| | <na | nd | nd |
| | >na | nd | nd |
| | na | nd | nd |
| OR Quart 3 p Value 95% CI of OR Quart3 | >1.0 | nd | nd |
| | <1.00 | nd | nd |
| | >0.062 | nd | nd |
| | na | nd | nd |
| OR | >5.2 | nd | nd |
| Quart 4 | <0.14 | nd | nd |
| p Value | >0.60 | nd | nd |
| 95% CI of OR Quart4 | na | nd | nd |

**Tenascin**

| sCr or UO | 24hr prior to AKI stage | |
|---|---|---|
| | Cohort 1 | Cohort 2 |
| Median | 593 | 1520 |
| Average | 1170 | 1380 |
| Stdev | 2220 | 718 |
| p(t-test) | | 0.82 |
| Min | 43.9 | 323 |
| Max | 26100 | 2220 |

(continued)

| sCr or UO | 24hr prior to AKI stage | |
| --- | --- | --- |
| | Cohort 1 | Cohort 2 |
| n (Samp) | 486 | 6 |
| n (Patient) | 217 | 6 |

| | 24hr prior to AKI stage | | |
| --- | --- | --- | --- |
| | sCr or UO | sCr only | UO only |
| AUC | 0.72 | nd | nd |
| SE | 0.12 | nd | nd |
| p | 0.062 | nd | nd |
| nCohort 1 | 486 | nd | nd |
| nCohort 2 | 6 | nd | nd |
| Cutoff 1 | 814 | nd | nd |
| Sens 1 | 83% | nd | nd |
| Spec 1 | 64% | nd | nd |
| Cutoff 2 | 814 | nd | nd |
| Sens 2 | 83% | nd | nd |
| Spec 2 | 64% | nd | nd |
| Cutoff 3 | 322 | nd | nd |
| Sens 3 | 100% | nd | nd |
| Spec 3 | 23% | nd | nd |
| Cutoff 4 | 964 | nd | nd |
| Sens 4 | 67% | nd | nd |
| Spec 4 | 70% | nd | nd |
| Cutoff 5 | 1250 | nd | nd |
| Sens 5 | 67% | nd | nd |
| Spec 5 | 80% | nd | nd |
| Cutoff 6 | 2140 | nd | nd |
| Sens 6 | 17% | nd | nd |
| Spec 6 | 90% | nd | nd |
| OR Quart 2 p Value 95% CI of OR Quart2 | 0 | nd | nd |
| | na | nd | nd |
| | na | nd | nd |
| | na | nd | nd |
| OR Quart 3 p Value 95% CI of OR Quart3 | 1.0 | nd | nd |
| | 1.0 | nd | nd |
| | 0.062 | nd | nd |
| | 16 | nd | nd |

(continued)

|  | 24hr prior to AKI stage | | |
|---|---|---|---|
|  | sCr or UO | sCr only | UO only |
| OR Quart 4 p Value 95% CI of OR Quart4 | 4.1 | nd | nd |
|  | 0.21 | nd | nd |
|  | 0.45 | nd | nd |
|  | 37 | nd | nd |

Fig. 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

**Angiogenin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 4420 | 8700 | 5060 | 8890 | 4570 | 8690 |
| Average | 7680 | 11200 | 8320 | 11600 | 7900 | 10900 |
| Stdev | 7650 | 8940 | 7980 | 10100 | 7910 | 8530 |
| p(t-test) |  | 1.9E-4 |  | 0.092 |  | 0.0030 |
| Min | 0.00873 | 54.6 | 0.00873 | 647 | 0.00873 | 54.6 |
| Max | 30600 | 30600 | 30600 | 30600 | 30600 | 30600 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.62 | 0.58 | 0.61 |
| SE | 0.034 | 0.072 | 0.036 |
| p | 6.5E-4 | 0.28 | 0.0017 |
| nCohort 1 | 352 | 421 | 343 |
| nCohort 2 | 93 | 18 | 82 |
| Cutoff 1 | 4290 | 4330 | 4330 |
| Sens 1 | 71% | 72% | 71% |
| Spec 1 | 50% | 46% | 50% |
| Cutoff 2 | 2200 | 995 | 2580 |
| Sens 2 | 81% | 83% | 80% |
| Spec 2 | 28% | 12% | 33% |
| Cutoff 3 | 985 | 831 | 1050 |
| Sens 3 | 90% | 94% | 90% |
| Spec 3 | 12% | 9% | 13% |
| Cutoff 4 | 9070 | 10600 | 9500 |

(continued)

| | At Enrollment | | |
|---|---|---|---|
| | sCr or UO | sCr only | UO only |
| Sens 4 | 49% | 44% | 48% |
| Spec 4 | 70% | 70% | 70% |
| Cutoff 5 | 15600 | 16600 | 15800 |
| Sens 5 | 33% | 33% | 29% |
| Spec 5 | 80% | 80% | 80% |
| Cutoff 6 | 20400 | 20400 | 20400 |
| Sens 6 | 14% | 17% | 12% |
| Spec 6 | 93% | 92% | 92% |
| OR Quart 2 p Value 95% CI of OR Quart2 | 0.93 | 0.39 | 1.4 |
| | 0.85 | 0.26 | 0.43 |
| | 0.44 | 0.073 | 0.63 |
| | 2.0 | 2.0 | 3.0 |
| OR Quart 3 p Value 95% CI of OR Quart3 | 1.5 | 0.78 | 2.0 |
| | 0.23 | 0.72 | 0.071 |
| | 0.77 | 0.21 | 0.94 |
| | 3.0 | 3.0 | 4.2 |
| OR Quart 4 p Value | 2.6 | 1.4 | 2.7 |
| | 0.0038 | 0.57 | 0.0078 |
| 95% CI of | 1.4 | 0.43 | 1.3 |
| OR Quart4 | 5.0 | 4.6 | 5.5 |

**Angiopoietin-related protein 4**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 9.75 | 14.6 | 10.6 | 20.7 | 10.6 | 13.0 |
| Average | 39.3 | 51.6 | 40.1 | 74.6 | 39.8 | 49.3 |
| Stdev | 89.3 | 95.9 | 89.2 | 118 | 89.5 | 96.1 |
| p(t-test) | | 0.29 | | 10.13 | | 0.43 |
| Min | 0.000466 | 0.000466 | 0.000466 | 0.000466 | 0.000466 | 0.000466 |
| Max | 708 | 413 | 708 | 413 | 708 | 413 |
| n (Samp) | 294 | 79 | 351 | 17 | 296 | 69 |
| n (Patient) | 294 | 79 | 351 | 17 | 296 | 69 |

| | At Enrollment | | |
|---|---|---|---|
| | sCr or UO | sCr only | UO only |
| AUC | 0.58 | 0.61 | 0.56 |

(continued)

| | At Enrollment | | |
|---|---|---|---|
| | sCr or UO | sCr only | UO only |
| SE | 0.037 | 0.074 | 0.039 |
| p | 0.036 | 0.12 | 0.13 |
| nCohort 1 | 294 | 351 | 296 |
| nCohort 2 | 79 | 17 | 69 |
| Cutoff 1 | 7.09 | 8.48 | 7.09 |
| Sens 1 | 71% | 71% | 71% |
| Spec 1 | 38% | 43% | 38% |
| Cutoff 2 | 5.29 | 5.29 | 5.10 |
| Sens 2 | 81% | 82% | 81% |
| Spec 2 | 30% | 28% | 28% |
| Cutoff 3 | 3.28 | 2.94 | 3.21 |
| Sens 3 | 91% | 94% | 91% |
| Spec 3 | 15% | 13% | 14% |
| Cutoff 4 | 18.1 | 19.6 | 19.4 |
| Sens 4 | 46% | 53% | 39% |
| Spec 4 | 70% | 70% | 70% |
| Cutoff 5 | 36.6 | 36.2 | 37.2 |
| Sens 5 | 25% | 47% | 22% |
| Spec 5 | 80% | 80% | 80% |
| Cutoff 6 | 96.2 | 96.5 | 96.2 |
| Sens 6 | 15% | 24% | 14% |
| Spec 6 | 90% | 90% | 90% |
| OR Quart 2 p Value 95% CI of | 1.5 | 1.3 | 1.4 |
| | 0.26 | 0.70 | 0.43 |
| | 0.73 | 0.29 | 0.63 |
| OR Quart2 | 3.3 | 6.2 | 3.0 |
| OR Quart 3 p Value 95% CI of OR Quart3 | 1.4 | 0.66 | 1.4 |
| | 0.44 | 0.65 | 0.43 |
| | 0.63 | 0.11 | 0.63 |
| | 2.9 | 4.0 | 3.0 |
| OR Quart 4 p Value 95% CI of OR Quart4 | 2.3 | 2.8 | 1.9 |
| | 0.026 | 0.13 | 0.10 |
| | 1.1 | 0.73 | 0.88 |
| | 4.7 | 11 | 4.0 |

**Amphiregulin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 23.5 | 39.4 | 25.0 | 42.8 | 25.2 | 35.9 |
| Average | 72.3 | 177 | 90.2 | 248 | 73.1 | 162 |
| Stdev | 202 | 527 | 298 | 478 | 211 | 522 |
| p(t-test) | | 0.012 | | 0.082 | | 0.043 |
| Min | 0.00131 | 2.63 | 0.00131 | 10.1 | 0.00131 | 2.63 |
| Max | 1640 | 3480 | 3480 | 1710 | 1710 | 3480 |
| n (Samp) | 245 | 68 | 296 | 12 | 232 | 60 |
| n (Patient) | 245 | 68 | 296 | 12 | 232 | 60 |

| | At Enrollment | | |
|---|---|---|---|
| | sCr or UO | sCr only | UO only |
| AUC | 0.61 | 0.65 | 0.59 |
| SE | 0.040 | 0.088 | 0.042 |
| p | 0.0062 | 0.081 | 0.027 |
| nCohort 1 | 245 | 296 | 232 |
| nCohort 2 | 68 | 12 | 60 |
| Cutoff 1 | 18.9 | 18.9 | 18.9 |
| Sens 1 | 72% | 83% | 70% |
| Spec 1 | 41% | 39% | 39% |
| Cutoff 2 | 15.0 | 18.9 | 15.0 |
| Sens 2 | 81% | 83% | 80% |
| Spec 2 | 35% | 39% | 34% |
| Cutoff 3 | 10.0 | 12.6 | 10.0 |
| Sens 3 | 91% | 92% | 90% |
| Spec 3 | 21% | 26% | 20% |
| Cutoff 4 | 45.7 | 49.1 | 45.9 |
| Sens 4 | 44% | 42% | 45% |
| Spec 4 | 70% | 70% | 70% |
| Cutoff 5 | 59.5 | 68.6 | 59.5 |
| Sens 5 | 34% | 42% | 33% |
| Spec 5 | 80% | 80% | 80% |
| Cutoff 6 | 118 | 133 | 114 |
| Sens 6 | 22% | 42% | 20% |
| Spec 6 | 90% | 90% | 90% |

(continued)

| | At Enrollment | | |
|---|---|---|---|
| | sCr or UO | sCr only | UO only |
| OR Quart 2 p Value 95% CI of OR Quart2 | 2.0 | 3.1 | 1.9 |
| | 0.099 | 0.33 | 0.14 |
| | 0.87 | 0.31 | 0.81 |
| | 4.8 | 30 | 4.5 |
| OR Quart 3 p Value 95% CI of OR Quart3 | 1.5 | 3.1 | 1.0 |
| | 0.38 | 0.33 | 1.0 |
| | 0.62 | 0.31 | 0.39 |
| | 3.6 | 30 | 2.6 |
| OR Quart 4 p Value 95% CI of OR Quart4 | 3.3 | 5.3 | 2.9 |
| | 0.0037 | 0.13 | 0.012 |
| | 1.5 | 0.60 | 1.3 |
| | 7.5 | 46 | 6.6 |

**Betacellulin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.793 | 1.22 | 0.777 | 1.80 | 0.909 | 0.777 |
| Average | 1.41 | 1.56 | 1.42 | 2.12 | 1.35 | 1.47 |
| Stdev | 2.13 | 1.94 | 2.11 | 1.52 | 1.63 | 1.98 |
| p(t-test) | | 0.59 | | 0.25 | | 0.62 |
| Min | 0.00179 | 0.00240 | 0.00179 | 0.00352 | 0.00179 | 0.00240 |
| Max | 23.1 | 11.6 | 23.1 | 4.57 | 9.19 | 11.6 |
| n (Samp) | 245 | 68 | 296 | 12 | 232 | 60 |
| n (Patient) | 245 | 68 | 296 | 12 | 232 | 60 |

| | At Enrollment | | |
|---|---|---|---|
| | sCr or UO | sCr only | UO only |
| AUC | 0.53 | 0.67 | 0.50 |
| SE | 0.040 | 0.087 | 0.042 |
| p | 0.48 | 0.049 | 0.94 |
| nCohort 1 | 245 | 296 | 232 |
| nCohort 2 | 68 | 12 | 60 |
| Cutoff 1 | 0.0407 | 1.51 | 0.0407 |
| Sens 1 | 74% | 75% | 70% |
| Spec 1 | 28% | 59% | 28% |
| Cutoff 2 | 0.00342 | 0.793 | 0.00342 |

(continued)

|  | At Enrollment | | |
|---|---|---|---|
|  | sCr or UO | sCr only | UO only |
| Sens 2 | 82% | 83% | 80% |
| Spec 2 | 18% | 51% | 19% |
| Cutoff 3 | 0.00246 | 0.0522 | 0.00246 |
| Sens 3 | 91% | 92% | 90% |
| Spec 3 | 10% | 31% | 11% |
| Cutoff 4 | 1.76 | 1.87 | 1.78 |
| Sens 4 | 37% | 50% | 33% |
| Spec 4 | 70% | 70% | 70% |
| Cutoff 5 | 2.41 | 2.42 | 2.41 |
| Sens 5 | 25% | 33% | 23% |
| Spec 5 | 80% | 81% | 80% |
| Cutoff 6 | 3.36 | 3.36 | 3.36 |
| Sens 6 | 15% | 25% | 13% |
| Spec 6 | 91% | 90% | 91% |
| OR Quart 2 | 0.85 | 1.0 | 0.78 |
| p Value | 0.69 | 1.0 | 0.54 |
| 95% CI of | 0.39 | 0.061 | 0.35 |
| OR Quart2 | 1.9 | 16 | 1.7 |
| OR Quart 3 p Value 95% CI of OR Quart3 | 1.0 | 5.3 | 0.78 |
|  | 1.0 | 0.13 | 0.54 |
|  | 0.47 | 0.60 | 0.35 |
|  | 2.1 | 46 | 1.7 |
| OR Quart 4 p Value 95% CI of OR Quart4 | 1.1 | 5.3 | 0.85 |
|  | 0.74 | 0.13 | 0.69 |
|  | 0.54 | 0.60 | 0.39 |
|  | 2.4 | 46 | 1.9 |

**Endostatin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 4620 | 5530 | 4710 | 9920 | 4690 | 5400 |
| Average | 16600 | 17200 | 16300 | 24300 | 16400 | 16900 |
| Stdev | 32000 | 27800 | 31100 | 28800 | 31000 | 28600 |
| p(t-test) |  | 0.87 |  | 0.28 |  | 0.90 |
| Min | 0.0130 | 261 | 0.0130 | 748 | 0.0130 | 261 |
| Max | 238000 | 148000 | 238000 | 110000 | 238000 | 148000 |

(continued)

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.56 | 0.61 | 0.55 |
| SE | 0.034 | 0.072 | 0.036 |
| p | 0.096 | 0.12 | 0.20 |
| nCohort 1 | 352 | 421 | 343 |
| nCohort 2 | 93 | 18 | 82 |
| Cutoff 1 | 3530 | 2970 | 3530 |
| Sens 1 | 71% | 72% | 71% |
| Spec 1 | 42% | 35% | 41% |
| Cutoff 2 | 2540 | 2540 | 2570 |
| Sens 2 | 81% | 83% | 80% |
| Spec 2 | 32% | 30% | 32% |
| Cutoff 3 | 1510 | 1410 | 1510 |
| Sens 3 | 90% | 94% | 90% |
| Spec 3 | 15% | 13% | 15% |
| Cutoff 4 | 9580 | 9440 | 10000 |
| Sens 4 | 33% | 50% | 32% |
| Spec 4 | 70% | 70% | 70% |
| Cutoff 5 | 20800 | 20000 | 21900 |
| Sens 5 | 23% | 44% | 21% |
| Spec 5 | 80% | 80% | 80% |
| Cutoff 6 | 43700 | 43700 | 43700 |
| Sens 6 | 12% | 17% | 11% |
| Spec 6 | 90% | 90% | 90% |
| OR Quart 2 p Value 95% CI of OR Quart2 | 1.4 | 0.99 | 1.3 |
|  | 0.38 | 0.99 | 0.47 |
|  | 0.68 | 0.20 | 0.64 |
|  | 2.7 | 5.0 | 2.7 |
| OR Quart 3 p Value 95% CI of OR Quart3 | 1.9 | 1.3 | 1.6 |
|  | 0.069 | 0.71 | 0.16 |
|  | 0.95 | 0.29 | 0.82 |
|  | 3.6 | 6.1 | 3.3 |

(continued)

|  | At Enrollment | | |
|---|---|---|---|
|  | sCr or UO | sCr only | UO only |
| OR Quart 4 p Value 95% CI of OR Quart4 | 1.7 | 2.8 | 1.5 |
|  | 0.14 | 0.14 | 0.30 |
|  | 0.85 | 0.72 | 0.72 |
|  | 3.3 | 11 | 3.0 |

**Proepiregulin**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3.42 | 5.13 | 3.55 | 3.48 | 3.52 | 5.13 |
| Average | 6.98 | 13.9 | 8.25 | 15.1 | 7.67 | 13.0 |
| Stdev | 19.3 | 22.1 | 20.3 | 20.4 | 20.3 | 21.9 |
| p(t-test) |  | 0.014 |  | 0.27 |  | 0.081 |
| Min | 0.0298 | 0.376 | 0.0298 | 1.42 | 0.0298 | 0.376 |
| Max | 279 | 134 | 279 | 57.1 | 279 | 134 |
| n (Samp) | 237 | 64 | 285 | 11 | 223 | 57 |
| n (Patient) | 237 | 64 | 285 | 11 | 223 | 57 |

|  | At Enrollment | | |
|---|---|---|---|
|  | sCr or UO | sCr only | UO only |
| AUC | 0.63 | 0.60 | 0.61 |
| SE | 0.041 | 0.092 | 0.043 |
| p | 0.0020 | 0.29 | 0.015 |
| nCohort 1 | 237 | 285 | 223 |
| nCohort 2 | 64 | 11 | 57 |
| Cutoff 1 | 2.85 | 2.57 | 2.85 |
| Sens 1 | 70% | 73% | 70% |
| Spec 1 | 43% | 38% | 42% |
| Cutoff 2 | 1.81 | 2.10 | 1.81 |
| Sens 2 | 81% | 82% | 81% |
| Spec 2 | 31% | 32% | 30% |
| Cutoff 3 | 0.971 | 1.46 | 0.750 |
| Sens 3 | 91% | 91% | 91% |
| Spec 3 | 16% | 22% | 9% |
| Cutoff 4 | 5.81 | 6.55 | 6.30 |
| Sens 4 | 48% | 45% | 46% |
| Spec 4 | 70% | 70% | 70% |

(continued)

|  | At Enrollment | | |
|---|---|---|---|
|  | sCr or UO | sCr only | UO only |
| Cutoff 5 | 8.47 | 10.2 | 9.47 |
| Sens 5 | 39% | 36% | 37% |
| Spec 5 | 80% | 80% | 80% |
| Cutoff 6 | 13.6 | 17.1 | 14.6 |
| Sens 6 | 27% | 27% | 23% |
| Spec 6 | 90% | 90% | 90% |
| OR Quart 2 p Value 95% CI of OR Quart2 | 1.9 | 2.1 | 1.5 |
|  | 0.14 | 0.41 | 0.37 |
|  | 0.81 | 0.37 | 0.62 |
|  | 4.5 | 12 | 3.7 |
| OR Quart 3 p Value 95% CI of OR Quart3 | 1.1 | 0.49 | 1.1 |
|  | 0.81 | 0.57 | 0.81 |
|  | 0.44 | 0.044 | 0.44 |
|  | 2.8 | 5.6 | 2.8 |
| OR Quart 4 p Value 95% CI of OR Quart4 | 3.4 | 2.1 | 2.8 |
|  | 0.0035 | 0.41 | 0.018 |
|  | 1.5 | 0.37 | 1.2 |
|  | 7.7 | 12 | 6.4 |

**Heparin-binding growth factor 1**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 867 | 712 |  | 396 | 861 | 820 |
| Average | 1250 | 1160 | 1250 | 768 | 1240 | 1200 |
| Stdev | 1400 | 1340 | 1390 | 1230 | 1420 | 1310 |
| p(t-test) |  | 0.58 |  | 0.15 |  | 0.82 |
| Min | 0.00328 | 2.62 | 0.00328 | 2.62 | 0.00328 | 2.62 |
| Max | 12300 | 6460 | 12300 | 5360 | 12300 | 6460 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.46 | 0.32 | 0.48 |
| SE | 0.034 | 0.071 | 0.036 |
| p | 0.21 | 0.013 | 0.66 |

(continued)

| | At Enrollment | | |
|---|---|---|---|
| | sCr or UO | sCr only | UO only |
| nCohort 1 | 352 | 421 | 343 |
| nCohort 2 | 93 | 18 | 82 |
| Cutoff 1 | 338 | 221 | 417 |
| Sens 1 | 71% | 72% | 71% |
| Spec 1 | 22% | 14% | 29% |
| Cutoff 2 | 226 | 101 | 280 |
| Sens 2 | 81% | 83% | 80% |
| Spec 2 | 13% | 8% | 18% |
| Cutoff 3 | 101 | 49.5 | 119 |
| Sens 3 | 90% | 94% | 90% |
| Spec 3 | 8% | 4% | 9% |
| Cutoff 4 | 1430 | 1420 | 1420 |
| Sens 4 | 26% | 17% | 27% |
| Spec 4 | 70% | 70% | 70% |
| Cutoff 5 | 1850 | 1850 | 1820 |
| Sens 5 | 18% | 6% | 23% |
| Spec 5 | 80% | 80% | 80% |
| Cutoff 6 | 2630 | 2670 | 2670 |
| Sens 6 | 11% | 6% | 11% |
| Spec 6 | 90% | 90% | 90% |
| OR Quart 2 p Value 95% CI of OR Quart2 | 1.1 | 4.1 | 1.1 |
| | 0.71 | 0.21 | 0.84 |
| | 0.59 | 0.45 | 0.54 |
| | 2.2 | 37 | 2.1 |
| OR Quart 3 p Value 95% CI of OR Quart3 | 0.95 | 4.1 | 0.95 |
| | 0.89 | 0.21 | 0.88 |
| | 0.48 | 0.45 | 0.47 |
| | 1.9 | 37 | 1.9 |
| OR Quart 4 p Value 95% CI of OR Quart4 | 1.5 | 9.8 | 1.1 |
| | 0.19 | 0.032 | 0.71 |
| | 0.81 | 1.2 | 0.58 |
| | 2.9 | 79 | 2.2 |

**Thrombospondin-2**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 988 | 1300 | 1050 | 2210 | 1050 | 1300 |
| Average | 1640 | 3290 | 1940 | 3080 | 1740 | 3270 |
| Stdev | 2280 | 7500 | 4070 | 3520 | 2430 | 7860 |
| p(t-test) |  | 4.1E-4 |  | 0.24 |  | 0.0024 |
| Min | 0.0376 | 14.6 | 0.0376 | 90.7 | 0.0376 | 14.6 |
| Max | 26900 | 68100 | 68100 | 14600 | 26900 | 68100 |
| n (Samp) | 352 | 93 | 421 | 18 | 343 | 82 |
| n (Patient) | 352 | 93 | 421 | 18 | 343 | 82 |

|  | At Enrollment | | |
|---|---|---|---|
|  | sCr or UO | sCr only | UO only |
| AUC | 0.60 | 0.61 | 0.58 |
| SE | 0.034 | 0.072 | 0.036 |
| p | 0.0038 | 0.14 | 0.019 |
| nCohort 1 | 352 | 421 | 343 |
| nCohort 2 | 93 | 18 | 82 |
| Cutoff 1 | 760 | 679 | 760 |
| Sens 1 | 71% | 72% | 71% |
| Spec 1 | 39% | 34% | 37% |
| Cutoff 2 | 498 | 360 | 502 |
| Sens 2 | 81% | 83% | 80% |
| Spec 2 | 25% | 17% | 25% |
| Cutoff 3 | 332 | 122 | 360 |
| Sens 3 | 90% | 94% | 90% |
| Spec 3 | 16% | 5% | 18% |
| Cutoff 4 | 1770 | 1830 | 1840 |
| Sens 4 | 41% | 56% | 39% |
| Spec 4 | 70% | 70% | 70% |
| Cutoff 5 | 2270 | 2430 | 2350 |
| Sens 5 | 40% | 44% | 37% |
| Spec 5 | 80% | 80% | 80% |
| Cutoff 6 | 3270 | 4010 | 3620 |
| Sens 6 | 28% | 28% | 22% |
| Spec 6 | 90% | 90% | 90% |

(continued)

|  | At Enrollment | | |
|  | sCr or UO | sCr only | UO only |
| --- | --- | --- | --- |
| OR Quart 2 p Value 95% CI of OR Quart2 | 0.71 | 0.19 | 0.86 |
|  | 0.36 | 0.13 | 0.70 |
|  | 0.34 | 0.022 | 0.41 |
|  | 1.5 | 1.7 | 1.8 |
| OR Quart 3 p Value 95% CI of OR Quart3 | 1.1 | 0.58 | 1.1 |
|  | 0.86 | 0.47 | 0.85 |
|  | 0.54 | 0.14 | 0.52 |
|  | 2.1 | 2.5 | 2.2 |
| OR Quart 4 | 2.2 | 1.9 | 2.2 |
| p Value | 0.011 | 0.28 | 0.018 |
| 95% CI of | 1.2 | 0.60 | 1.2 |
| OR Quart4 | 4.2 | 5.7 | 4.3 |

Fig. 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

**Angiogenin**

|  | sCr or UO | | UO only | |
|  | Cohort 1 | Cohort 2 | Cohort 1 | Cohort 2 |
| --- | --- | --- | --- | --- |
| Median | 179000 | 117000 | 179000 | 110000 |
| Average | 197000 | 159000 | 197000 | 157000 |
| Stdev | 197000 | 107000 | 201000 | 108000 |
| p(t-test) |  | 0.32 |  | 0.30 |
| Min | 10800 | 24100 | 10800 | 24100 |
| Max | 2240000 | 430000 | 2240000 | 430000 |
| n (Samp) | 141 | 30 | 135 | 29 |
| n (Patient) | 141 | 30 | 135 | 29 |

|  | At Enrollment | |
|  | sCr or UO | UO only |
| --- | --- | --- |
| AUC | 0.40 | 0.39 |
| SE | 0.059 | 0.060 |
| p | 0.079 | 0.056 |
| nCohort 1 | 141 | 135 |
| nCohort 2 | 30 | 29 |
| Cutoff 1 | 90900 | 88800 |
| Sens 1 | 70% | 72% |

(continued)

|  | At Enrollment | |
|---|---|---|
|  | sCr or UO | UO only |
| Spec 1 | 15% | 14% |
| Cutoff 2 | 82700 | 80900 |
| Sens 2 | 80% | 83% |
| Spec 2 | 13% | 13% |
| Cutoff 3 | 76300 | 44000 |
| Sens 3 | 90% | 93% |
| Spec 3 | 12% | 4% |
| Cutoff 4 | 218000 | 215000 |
| Sens 4 | 23% | 24% |
| Spec 4 | 70% | 70% |
| Cutoff 5 | 241000 | 239000 |
| Sens 5 | 20% | 21% |
| Spec 5 | 80% | 80% |
| Cutoff 6 | 314000 | 332000 |
| Sens 6 | 10% | 10% |
| Spec 6 | 90% | 90% |
| OR Quart 2 p Value 95% CI of OR Quart2 | 0.81 | 0.63 |
|  | 0.75 | 0.50 |
|  | 0.23 | 0.16 |
|  | 2.9 | 2.4 |
| OR Quart 3 p Value 95% CI of OR Quart3 | 1.2 | 1.2 |
|  | 0.76 | 0.76 |
|  | 0.37 | 0.37 |
|  | 3.9 | 3.9 |
| OR Quart 4 p Value 95% CI of OR Quart4 | 2.5 | 2.4 |
|  | 0.11 | 0.12 |
|  | 0.83 | 0.81 |
|  | 7.4 | 7.2 |

**Angiopoietin-related protein 4**

|  | sCr or UO | | UO only | |
|---|---|---|---|---|
|  | Cohort 1 | Cohort 2 | Cohort 1 | Cohort 2 |
| Median | 34.2 | 47.0 | 35.0 | 47.4 |
| Average | 63.6 | 73.3 | 65.6 | 75.0 |
| Stdev | 166 | 66.6 | 169 | 67.2 |
| p(t-test) |  | 0.75 |  | 0.77 |

(continued)

|  | sCr or UO | | UO only | |
|---|---|---|---|---|
|  | Cohort 1 | Cohort 2 | Cohort 1 | Cohort 2 |
| Min | 1.77 | 16.4 | 2.71 | 16.4 |
| Max | 1900 | 339 | 1900 | 339 |
| n (Samp) | 141 | 30 | 135 | 29 |
| n (Patient) | 141 | 30 | 135 | 29 |

|  | At Enrollment | |
|---|---|---|
|  | sCr or UO | UO only |
| AUC | 0.65 | 0.65 |
| SE | 0.058 | 0.060 |
| p | 0.0094 | 0.011 |
| nCohort 1 | 141 | 135 |
| nCohort 2 | 30 | 29 |
| Cutoff 1 | 35.6 | 35.6 |
| Sens 1 | 70% | 72% |
| Spec 1 | 53% | 52% |
| Cutoff 2 | 29.1 | 29.1 |
| Sens 2 | 80% | 83% |
| Spec 2 | 41% | 39% |
| Cutoff 3 | 24.9 | 22.7 |
| Sens 3 | 90% | 93% |
| Spec 3 | 35% | 27% |
| Cutoff 4 | 51.8 | 53.8 |
| Sens 4 | 43% | 45% |
| Spec 4 | 70% | 70% |
| Cutoff 5 | 71.1 | 71.8 |
| Sens 5 | 30% | 31% |
| Spec 5 | 80% | 80% |
| Cutoff 6 | 98.7 | 104 |
| Sens 6 | 27% | 28% |
| Spec 6 | 90% | 90% |
| OR Quart 2 p Value 95% CI of OR Quart2 | 4.6 | 2.2 |
|  | 0.065 | 0.30 |
|  | 0.91 | 0.50 |
|  | 23 | 9.4 |

(continued)

|  | At Enrollment | |
| --- | --- | --- |
|  | sCr or UO | UO only |
| OR Quart 3 p Value 95% CI of OR Quart3 | 6.1 | 4.1 |
|  | 0.026 | 0.045 |
|  | 1.2 | 1.0 |
|  | 30 | 16 |
| OR Quart 4 p Value 95% CI of OR Quart4 | 6.1 | 4.1 |
|  | 0.026 | 0.045 |
|  | 1.2 | 1.0 |
|  | 30 | 16 |

**Amphiregulin**

|  | sCr or UO | | UO only | |
| --- | --- | --- | --- | --- |
|  | Cohort 1 | Cohort 2 | Cohort 1 | Cohort 2 |
| Median | 24.9 | 27.5 | 24.5 | 31.5 |
| Average | 32.9 | 37.1 | 32.6 | 38.1 |
| Stdev | 29.0 | 32.2 | 29.3 | 32.3 |
| p(t-test) |  | 0.50 |  | 0.38 |
| Min | 0.00246 | 2.76 | 0.00246 | 2.76 |
| Max | 198 | 162 | 198 | 162 |
| n (Samp) | 136 | 29 | 130 | 28 |
| n (Patient) | 136 | 29 | 130 | 28 |

|  | At Enrollment | |
| --- | --- | --- |
|  | sCr or UO | UO only |
| AUC | 0.53 | 0.56 |
| SE | 0.060 | 0.061 |
| p | 0.58 | 0.37 |
| nCohort 1 | 136 | 130 |
| nCohort 2 | 29 | 28 |
| Cutoff 1 | 16.8 | 17.0 |
| Sens 1 | 72% | 71% |
| Spec 1 | 29% | 30% |
| Cutoff 2 | 11.1 | 11.1 |
| Sens 2 | 83% | 86% |
| Spec 2 | 12% | 14% |
| Cutoff 3 | 8.21 | 8.93 |
| Sens 3 | 97% | 93% |

(continued)

| | At Enrollment | |
|---|---|---|
| | sCr or UO | UO only |
| Spec 3 | 9% | 12% |
| Cutoff 4 | 34.0 | 34.0 |
| Sens 4 | 48% | 50% |
| Spec 4 | 71% | 72% |
| Cutoff 5 | 44.7 | 44.1 |
| Sens 5 | 34% | 36% |
| Spec 5 | 80% | 80% |
| Cutoff 6 | 67.0 | 60.3 |
| Sens 6 | 14% | 14% |
| Spec 6 | 90% | 90% |
| OR Quart 2 p Value 95% CI of OR Quart2 | 0.57 | 0.65 |
| | 0.37 | 0.50 |
| | 0.17 | 0.19 |
| | 1.9 | 2.3 |
| OR Quart 3 p Value 95% CI of OR Quart3 | 0.71 | 0.83 |
| | 0.56 | 0.76 |
| | 0.22 | 0.25 |
| | 2.3 | 2.7 |
| OR Quart 4 | 1.3 | 1.5 |
| p Value | 0.64 | 0.45 |
| 95% CI of | 0.45 | 0.51 |
| OR Quart4 | 3.7 | 4.5 |

**Betacellulin**

| | sCr or UO | | UO only | |
|---|---|---|---|---|
| | Cohort 1 | Cohort 2 | Cohort 1 | Cohort 2 |
| Median | 3.93 | 3.11 | 3.73 | 3.11 |
| Average | 5.07 | 5.36 | 4.89 | 5.50 |
| Stdev | 5.50 | 7.04 | 5.49 | 7.12 |
| p(t-test) | | 0.80 | | 0.61 |
| Min | 0.00226 | 0.00274 | 0.00226 | 0.00274 |
| Max | 52.9 | 32.2 | 52.9 | 32.2 |
| n (Samp) | 137 | 30 | 131 | 29 |
| n (Patient) | 137 | 30 | 131 | 29 |

| | At Enrollment | |
|---|---|---|
| | sCr or UO | UO only |
| AUC | 0.45 | 0.47 |
| SE | 0.059 | 0.060 |
| p | 0.36 | 0.60 |
| nCohort 1 | 137 | 131 |
| nCohort 2 | 30 | 29 |
| Cutoff 1 | 2.26 | 2.26 |
| Sens 1 | 70% | 72% |
| Spec 1 | 25% | 27% |
| Cutoff 2 | 1.78 | 1.78 |
| Sens 2 | 80% | 83% |
| Spec 2 | 21% | 23% |
| Cutoff 3 | 0.00282 | 0.00282 |
| Sens 3 | 97% | 97% |
| Spec 3 | 4% | 5% |
| Cutoff 4 | 5.88 | 5.83 |
| Sens 4 | 23% | 24% |
| Spec 4 | 70% | 70% |
| Cutoff 5 | 7.71 | 7.22 |
| Sens 5 | 17% | 17% |
| Spec 5 | 80% | 80% |
| Cutoff 6 | 10.1 | 9.07 |
| Sens 6 | 13% | 14% |
| Spec 6 | 91% | 90% |
| OR Quart 2 p Value 95% CI of OR Quart2 | 1.0 | 1.0 |
| | 1.0 | 1.0 |
| | 0.29 | 0.29 |
| | 3.4 | 3.4 |
| OR Quart 3 p Value 95% CI of OR Quart3 | 1.6 | 1.9 |
| | 0.40 | 0.27 |
| | 0.53 | 0.61 |
| | 5.1 | 5.8 |
| OR Quart 4 p Value 95% CI of OR Quart4 | 1.7 | 1.2 |
| | 0.37 | 0.76 |
| | 0.54 | 0.37 |
| | 5.3 | 4.0 |

**Proepiregulin**

|  | sCr or UO | | UO only | |
|---|---|---|---|---|
|  | Cohort 1 | Cohort 2 | Cohort 1 | Cohort 2 |
| Median | 0.411 | 0.383 | 0.406 | 0.394 |
| Average | 0.804 | 0.981 | 0.805 | 1.01 |
| Stdev | 2.80 | 2.10 | 2.86 | 2.13 |
| p(t-test) |  | 0.75 |  | 0.72 |
| Min | 0.000152 | 0.0249 | 0.000152 | 0.0249 |
| Max | 32.1 | 11.3 | 32.1 | 11.3 |
| n (Samp) | 136 | 29 | 130 | 28 |
| n (Patient) | 136 | 29 | 130 | 28 |

|  | At Enrollment | |
|---|---|---|
|  | sCr or UO | UO only |
| AUC | 0.51 | 0.54 |
| SE | 0.060 | 0.061 |
| p | 0.84 | 0.56 |
| nCohort 1 | 136 | 130 |
| nCohort 2 | 29 | 28 |
| Cutoff 1 | 0.242 | 0.242 |
| Sens 1 | 72% | 75% |
| Spec 1 | 31% | 32% |
| Cutoff 2 | 0.200 | 0.200 |
| Sens 2 | 83% | 82% |
| Spec 2 | 25% | 27% |
| Cutoff 3 | 0.0796 | 0.117 |
| Sens 3 | 93% | 93% |
| Spec 3 | 12% | 18% |
| Cutoff 4 | 0.647 | 0.589 |
| Sens 4 | 24% | 36% |
| Spec 4 | 72% | 71% |
| Cutoff 5 | 0.806 | 0.750 |
| Sens 5 | 21% | 25% |
| Spec 5 | 80% | 80% |
| Cutoff 6 | 1.24 | 1.20 |
| Sens 6 | 17% | 18% |
| Spec 6 | 90% | 90% |

(continued)

|  | At Enrollment | |
| --- | --- | --- |
|  | sCr or UO | UO only |
| OR Quart 2 p Value 95% CI of OR Quart2 | 1.9 | 2.0 |
|  | 0.27 | 0.27 |
|  | 0.61 | 0.60 |
|  | 5.8 | 6.5 |
| OR Quart 3 p Value 95% CI of OR Quart3 | 1.0 | 1.5 |
|  | 1.0 | 0.53 |
|  | 0.29 | 0.43 |
|  | 3.4 | 5.2 |
| OR Quart 4 p Value 95% CI of OR Quart4 | 1.2 | 1.4 |
|  | 0.80 | 0.56 |
|  | 0.36 | 0.42 |
|  | 3.8 | 5.0 |

The examples provided herein are representative of preferred embodiments, are exemplary, and are not intended as limitations on the scope of the invention. Modifications therein and other uses will occur to those skilled in the art.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

**Claims**

1. A method for evaluating renal status in a subject, comprising:

performing one or more assays configured to detect one or more biomarkers on a body fluid sample obtained from the subject to provide an assay result; and
correlating the assay result(s) to the renal status of the subject,
wherein said correlating step comprises assigning a likelihood of a future change in renal status to the subject based on the assay result(s),
wherein said one or more future changes in renal status comprise future acute renal failure (ARF), and
the likelihood of the future change in renal status is that an event of interest is likely to occur within a period 24 hours of the time at which the body fluid sample is obtained from the subject,
wherein the one or more biomarkers comprise Fibroblast growth factor 21, and optionally one or more of Proheparin-binding EGF-like growth factor, Tenascin C, Angiopoietin-related protein 4, Fibroblast growth factor 19, Heparin-binding growth factor 1, Angiopoietin-related protein 6, Proepiregulin, Probetacellulin, Amphiregulin, Angiogenin, Thrombospondin-2, and Collagen alpha-1(XVIII) chain.

**2.** The method of claim 1, wherein said correlating step comprises

(a) assigning a diagnosis of the occurrence or nonoccurrence of one or more of an injury to renal function, reduced renal function, or ARF to the subject based on the assay result(s),
(b) assessing whether or not renal function is improving or worsening in a subject who has suffered from an injury to renal function, reduced renal function, or ARF based on the assay result(s),
(c) assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine,
(d) assigning one or more of: a likelihood that within 24 hours the subject will (i) experience a 2-fold or greater increase in serum creatinine, (ii) have a urine output of less than 0.5 ml/kg/hr over a 6 hour period, or (iii) experience a 2-fold or greater increase in serum creatinine, or
(e) assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period.

**3.** A method according to one of claims 1 and 2, wherein said assay results comprise at least 2, 3, or 4 of:

a measured concentration of Fibroblast growth factor 21,
a measured concentration of Proheparin-binding EGF-like growth factor,
a measured concentration of Tenascin C,
a measured concentration of Angiopoietin-related protein 4,
a measured concentration of Fibroblast growth factor 19,
a measured concentration of Heparin-binding growth factor 1,
a measured concentration of Angiopoietin-related protein 6,
a measured concentration of Proepiregulin,
a measured concentration of Probetacellulin,
a measured concentration of Amphiregulin,
a measured concentration of Angiogenin,
a measured concentration of Thrombospondin-2, and
a measured concentration of Collagen alpha-1(XVIII) chain.

**4.** A method according to one of claims 1-3, wherein a plurality of assay results are combined using a function that converts the plurality of assay results into a single composite result.

**5.** A method according to claim 1, wherein said future change in renal status comprises a clinical outcome related to a renal injury suffered by the subject.

**6.** A method according to one of claims 1-3, wherein the subject is selected for evaluation of renal status based on

(a) the pre-existence in the subject of one or more known risk factors for prerenal, intrinsic renal, or postrenal ARF or
(b) an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

**7.** A method according to one of claims 1-3, wherein said method is a method of diagnosing the occurrence or nonoccurrence of acute renal failure in said subject,
a method of diagnosing the occurrence or nonoccurrence of a need for renal replacement therapy in said subject,
a method of diagnosing the occurrence or nonoccurrence of a need for renal transplantation in said subject,
a method of assigning a risk of the future occurrence or nonoccurrence of acute renal failure in said subject,
a method of assigning a risk of the future occurrence or nonoccurrence of a need for renal replacement therapy in said subject, or
a method of assigning a risk of the future occurrence or nonoccurrence of a need for renal transplantation in said subject.

**8.** A method according to one of claims 1-3, wherein said future change in renal status comprises ARF within 12 hours of the time at which the body fluid sample is obtained.

**9.** A method according to one of claims 1-3, wherein

(A) the subject is in RIFLE stage 0 or R, wherein

(a) the subject optionally is in RIFLE stage 0, and said correlating step comprises assigning a likelihood that the subject will reach RIFLE stage R, I or F within 24 hours. wherein the subject is optionally in RIFLE stage 0, and said correlating step comprises assigning a likelihood that the subject will reach RIFLE stage I or F within 24 hours, (b) the subject optionally is in RIFLE stage 0 or R, and said correlating step comprises assigning a likelihood that the subject will reach RIFLE stage I or F within 24 hours or (c) the subject optionally is in RIFLE stage R, and said correlating step comprises assigning a likelihood that the subject will reach RIFLE stage I or F within 24 hours, or

(B) wherein the subject is in RIFLE stage 0, R, or I, and said correlating step comprises assigning a likelihood that the subject will reach RIFLE stage F within 24 hours.

**10.** A method according to one of claims 1-3, wherein

(a) the subject is not in acute renal failure,
(b) the subject has not experienced a 1.5-fold or greater increase in serum creatinine over a baseline value determined prior to the time at which the body fluid sample is obtained,
(c) the subject has a urine output of at least 0.5 ml/kg/hr over the 6 hours preceding the time at which the body fluid sample is obtained,
(d) the subject has not experienced an increase of 0.3 mg/dL or greater in serum creatinine over a baseline value determined prior to the time at which the body fluid sample is obtained,
(e) the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained,
(f) the subject has not experienced a 1.5-fold or greater increase in serum creatinine over a baseline value determined prior to the time at which the body fluid sample is obtained,
(g) the subject has a urine output of at least 0.5 ml/kg/hr over the 6 hours preceding the time at which the body fluid sample is obtained,
(h) the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained,
(i) the subject has not experienced a 2-fold or greater increase in serum creatinine over a baseline value determined prior to the time at which the body fluid sample is obtained,
(j) the subject has a urine output of at least 0.5 ml/kg/hr over the 12 hours preceding the time at which the body fluid sample is obtained,
(k) the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained,
(l) the subject has not experienced a 3-fold or greater increase in serum creatinine over a baseline value determined prior to the time at which the body fluid sample is obtained,
(m) the subject has a urine output of at least 0.3 ml/kg/hr over the 24 hours preceding the time at which the body fluid sample is obtained, or anuria over the 12 hours preceding the time at which the body fluid sample is obtained or

(n) the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained.

**11.** A method according to one of claims 1-10, wherein the body fluid sample is a urine sample.

**12.** A method according to one of claims 1-11, wherein said method comprises performing assays that detect one, two or three, or more of Fibroblast growth factor 21, Proheparin-binding EGF-like growth factor, Tenascin C, Angiopoietin-related protein 4, Fibroblast growth factor 19, Heparin-binding growth factor 1, Angiopoietin-related protein 6, Proepiregulin, Probetacellulin, Amphiregulin, Angiogenin, Thrombospondin-2, and Collagen alpha-1(XVIII) chain.

**13.** The use of Fibroblast growth factor 21, and optionally of one or more biomarkers selected from the group consisting of Proheparin-binding EGF-like growth factor, Tenascin C, Angiopoietin-related protein 4, Fibroblast growth factor 19, Heparin-binding growth factor 1, Angiopoietin-related protein 6, Proepiregulin, Probetacellulin, Amphiregulin, Angiogenin, Thrombospondin-2, and Collagen alpha-1(XVIII) chain for the evaluation of future acute renal failure, wherein the future acute renal failure is likely to occur within a period 24 hours of the time at which the body fluid sample is obtained from a subject.

**14.** The use of claim 13 comprising using a kit, comprising:

reagents for performing one or more assays configured to detect Fibroblast growth factor 21, and optionally one or more biomarkers selected from the group consisting of Proheparin-binding EGF-like growth factor, Tenascin C, Angiopoietin-related protein 4, Fibroblast growth factor 19, Heparin-binding growth factor 1, Angiopoietin-related protein 6, Proepiregulin, Probetacellulin, Amphiregulin, Angiogenin, Thrombospondin-2, and Collagen alpha-1(XVIII) chain.

**Patentansprüche**

**1.** Verfahren für die Bewertung des Nierenstatus bei einem Subjekt, das Folgendes umfasst:

Durchführung von einer oder mehreren Untersuchungen, die zum Nachweis von einem oder mehreren Biomarkern bei einer Körperflüssigkeitsprobe ausgelegt sind, die von dem Subjekt erhalten wurde, um ein Untersuchungsergebnis bereitzustellen; und
Korrelation des/der Untersuchungsergebnisses/Untersuchungsergebnisse mit dem Nierenstatus des Subjekts, wobei der Korrelationsschritt die Bestimmung einer Wahrscheinlichkeit einer zukünftigen Veränderung des Nierenstatus bei dem Subjekt auf der Grundlage des/der Untersuchungsergebnisses/Untersuchungsergebnisse umfasst,
wobei die eine oder mehreren zukünftigen Veränderungen des Nierenstatus zukünftiges akutes Nierenversagen (ARF) umfassen, und
die Wahrscheinlichkeit der zukünftigen Veränderung des Nierenstatus ist, dass ein Ereignis von Interesse wahrscheinlich innerhalb einer Zeitdauer von 24 Stunden von dem Zeitpunkt auftritt, zu dem die Körperflüssigkeitsprobe von dem Subjekt erhalten wurde,
wobei der eine oder die mehreren Biomarker Fibroblasten-Wachstumsfaktor 21 und optional einen oder mehrere von Folgenden umfassen: Proheparin-bindendem EGF-ähnlichem Wachstumsfaktor, Tenascin C, Angiopoietin-verwandtem Protein 4, Fibroblasten-Wachstumsfaktor 19, Heparinbindendem Wachstumsfaktor 1, Angiopoietin-verwandtem Protein 6, Proepiregulin, Probetacellulin, Amphiregulin, Angiogenin, Thrombospondin-2 und Kollagen-alpha-1(XVIII)-Kette.

**2.** Verfahren nach Anspruch 1, wobei der Korrelationsschritt Folgendes umfasst:

(a) Bestimmen einer Diagnose des Auftretens oder Nichtauftretens von einer oder mehreren von einer Verletzung der Nierenfunktion, eingeschränkter Nierenfunktion oder ARF bei dem Subjekt auf der Grundlage des/der Untersuchungsergebnisses/Untersuchungsergebnisse,
(b) Beurteilen, ob sich die Nierenfunktion bei einem Subjekt, das eine Verletzung der Nierenfunktion, eingeschränkte Nierenfunktion oder ARF erlitten hat, verbessert oder verschlechtert hat, auf der Grundlage des/der Untersuchungsergebnisses/Untersuchungsergebnisse,

(c) Bestimmen von einem oder mehreren der Folgenden: einer Wahrscheinlichkeit, dass innerhalb von 24 Stunden das Subjekt (i) einen 1,5-fachen oder größeren Anstieg des Serumkreatinins erfahren wird, (ii) eine Urinausscheidung von weniger als 0,5 ml/kg/Std. über eine Zeitdauer von 6 Stunden haben wird oder (iii) einen Anstieg des Serumkreatinins von 0,3 mg/dl oder größer erfahren wird,

(d) Bestimmen von einem oder mehreren der Folgenden: einer Wahrscheinlichkeit, dass innerhalb von 24 Stunden das Subjekt (i) einen 2-fachen oder größeren Anstieg des Serumkreatinins erfahren wird, (ii) eine Urinausscheidung von weniger als 0,5 ml/kg/Std. über eine Zeitdauer von 6 Stunden haben wird oder (iii) einen 2-fachen oder größeren Anstieg des Serumkreatinins erfahren wird, oder

(e) Bestimmen von einem oder mehreren der Folgenden: einer Wahrscheinlichkeit, dass innerhalb von 24 Stunden das Subjekt (i) einen 3-fachen oder größeren Anstieg des Serumkreatinins erfahren wird oder (ii) eine Urinausscheidung von weniger als 0,3 ml/kg/Std. über eine Zeitdauer von 24 Stunden oder Anurie über eine Zeitdauer von 12 Stunden haben wird.

3. Verfahren nach einem der Ansprüche 1 und 2, wobei die Untersuchungsergebnisse mindestens 2, 3 oder 4 der Folgenden umfassen:

eine gemessene Konzentration von Fibroblasten-Wachstumsfaktor 21,
eine gemessene Konzentration von Proheparin-bindendem EGF-ähnlichem Wachstumsfaktor,
eine gemessene Konzentration von Tenascin C,
eine gemessene Konzentration von Angiopoietin-verwandtem Protein 4,
eine gemessene Konzentration von Fibroblasten-Wachstumsfaktor 19,
eine gemessene Konzentration von Heparin-bindendem Wachstumsfaktor 1,
eine gemessene Konzentration von Angiopoietin-verwandtem Protein 6,
eine gemessene Konzentration von Proepiregulin,
eine gemessene Konzentration von Probetacellulin,
eine gemessene Konzentration von Amphiregulin,
eine gemessene Konzentration von Angiogenin,
eine gemessene Konzentration von Thrombospondin-2 und
eine gemessene Konzentration von Kollagen-alpha-1(XVIII)-Kette.

4. Verfahren nach einem der Ansprüche 1-3, wobei eine Vielzahl von Untersuchungsergebnissen unter Verwendung einer Funktion kombiniert werden, die die Vielzahl von Untersuchungsergebnissen in ein einziges zusammengesetztes Ergebnis umwandelt.

5. Verfahren nach Anspruch 1, wobei die zukünftige Veränderung des Nierenstatus ein klinisches Ergebnis umfasst, das mit einer Nierenverletzung, die das Subjekt erlitt, verbunden ist.

6. Verfahren nach einem der Ansprüche 1-3, wobei das Subjekt für die Bewertung des Nierenstatus auf der Grundlage von Folgendem ausgewählt ist:

(a) dem vorherigen Bestehen bei dem Subjekt von einem oder mehreren bekannten Risikofaktoren für prärenales, intrinsisch renales oder postrenales ARF oder

(b) einer bestehenden Diagnose von einem oder mehreren von Folgenden: kongestiver Herzinsuffizienz, Präeklampsie, Eklampsie, Diabetes mellitus, Bluthochdruck, koronarer Herzkrankheit, Proteinurie, Niereninsuffizienz, glomerulärer Filtration unterhalb des normalen Bereichs, Zirrhose, Serumkreatinin oberhalb des normalen Bereichs, Sepsis, Verletzung der Nierenfunktion, eingeschränkter Nierenfunktion oder ARF, oder auf der Grundlage von größerer Gefäßoperation, Koronararterienbypass oder anderer Herzoperation, der sich unterzogen wird oder wurde, oder auf der Grundlage der Exposition gegenüber NSAIDs, Cyclosporinen, Tacrolismus, Aminoglycosiden, Foscarnet, Ethylenglycol, Hämoglobin, Myoglobin, Ifosfamid, Schwermetallen, Methotrexat, radioopaken Kontrastmitteln oder Streptozotocin.

7. Verfahren nach einem der Ansprüche 1-3, wobei das Verfahren ein Verfahren zur Diagnose des Auftretens oder Nichtauftretens von akutem Nierenversagen bei dem Subjekt, ein Verfahren zur Diagnose des Auftretens oder Nichtauftretens eines Erfordernisses für Nierenersatztherapie bei dem Subjekt, ein Verfahren zur Diagnose des Auftretens oder Nichtauftretens eines Erfordernisses für Nierentransplantation bei dem Subjekt,

ein Verfahren zur Bestimmung eines Risikos des zukünftigen Auftretens oder Nichtauftretens von akutem Nierenversagen bei dem Subjekt,

ein Verfahren zur Bestimmung eines Risikos des zukünftigen Auftretens oder Nichtauftretens eines Erfordernisses für Nierenersatztherapie bei dem Subjekt oder

ein Verfahren zur Bestimmung eines Risikos des zukünftigen Auftretens oder Nichtauftretens eines Erfordernisses für Nierentransplantation bei dem Subjekt ist.

8. Verfahren nach einem der Ansprüche 1-3, wobei die zukünftige Veränderung des Nierenstatus ein ARF innerhalb von 12 Stunden von dem Zeitpunkt umfasst, zu dem die Körperflüssigkeitsprobe von dem Subjekt erhalten wurde.

9. Verfahren nach einem der Ansprüche 1-3, wobei

(A) das Subjekt im RIFLE-Stadium 0 oder R ist, wobei

(a) das Subjekt optional im RIFLE-Stadium 0 ist und der Korrelationsschritt die Bestimmung einer Wahrscheinlichkeit umfasst, dass das Subjekt RIFLE-Stadium R, I oder F innerhalb von 24 Stunden erreichen wird,

wobei das Subjekt optional im RIFLE-Stadium 0 ist und der Korrelationsschritt die Bestimmung einer Wahrscheinlichkeit umfasst, dass das Subjekt RIFLE-Stadium I oder F innerhalb von 24 Stunden erreichen wird,

(b) das Subjekt optional im RIFLE-Stadium 0 oder R ist und der Korrelationsschritt die Bestimmung einer Wahrscheinlichkeit umfasst, dass das Subjekt RIFLE-Stadium I oder F innerhalb von 24 Stunden erreichen wird, oder

(c) das Subjekt optional im RIFLE-Stadium R ist und der Korrelationsschritt die Bestimmung einer Wahrscheinlichkeit umfasst, dass das Subjekt RIFLE-Stadium I oder F innerhalb von 24 Stunden erreichen wird, oder (B) wobei das Subjekt im RIFLE-Stadium 0, R oder I ist und der Korrelationsschritt die Bestimmung einer Wahrscheinlichkeit umfasst, dass das Subjekt RIFLE-Stadium F innerhalb von 24 Stunden erreichen wird.

10. Verfahren nach einem der Ansprüche 1-3, wobei

(a) bei dem Subjekt kein akutes Nierenversagen vorliegt,

(b) das Subjekt keinen 1,5-fachen oder größeren Anstieg des Serumkreatinins über einem Ausgangswert erfahren hat, der vor dem Zeitpunkt bestimmt wurde, zu dem die Körperflüssigkeitsprobe erhalten wurde,

(c) das Subjekt eine Urinausscheidung von mindestens 0,5 ml/kg/Std. über die 6 Stunden vor dem Zeitpunkt hat, zu dem die Körperflüssigkeitsprobe erhalten wurde,

(d) das Subjekt keinen Anstieg des Serumkreatinins von 0,3 mg/dl oder größer über einem Ausgangswert erfahren hat, der vor dem Zeitpunkt bestimmt wurde, zu dem die Körperflüssigkeitsprobe erhalten wurde,

(e) das Subjekt (i) keinen 1,5-fachen oder größeren Anstieg des Serumkreatinins über einem Ausgangswert erfahren hat, der vor dem Zeitpunkt bestimmt wurde, zu dem die Körperflüssigkeitsprobe erhalten wurde, (ii) eine Urinausscheidung von mindestens 0,5.ml/kg/Std. über die 6 Stunden vor dem Zeitpunkt hat, zu dem die Körperflüssigkeitsprobe erhalten wurde, und (iii) keinen Anstieg des Serumkreatinins von 0,3 mg/dl oder größer über einem Ausgangswert erfahren hat, der vor dem Zeitpunkt bestimmt wurde, zu dem die Körperflüssigkeitsprobe erhalten wurde,

(f) das Subjekt keinen 1,5-fachen oder größeren Anstieg des Serumkreatinins über einem Ausgangswert erfahren hat, der vor dem Zeitpunkt bestimmt wurde, zu dem die Körperflüssigkeitsprobe erhalten wurde,

(g) das Subjekt eine Urinausscheidung von mindestens 0,5 ml/kg/Std. über die 6 Stunden vor dem Zeitpunkt hat, zu dem die Körperflüssigkeitsprobe erhalten wurde,

(h) das Subjekt (i) keinen 1,5-fachen oder größeren Anstieg des Serumkreatinins über einem Ausgangswert erfahren hat, der vor dem Zeitpunkt bestimmt wurde, zu dem die Körperflüssigkeitsprobe erhalten wurde, (ii) eine Urinausscheidung von mindestens 0,5 ml/kg/Std. über die 12 Stunden vor dem Zeitpunkt hat, zu dem die Körperflüssigkeitsprobe erhalten wurde, und (iii) keinen Anstieg des Serumkreatinins von 0,3 mg/dl oder größer über einem Ausgangswert erfahren hat, der vor dem Zeitpunkt bestimmt wurde, zu dem die Körperflüssigkeitsprobe erhalten wurde,

(i) das Subjekt keinen 2-fachen oder größeren Anstieg des Serumkreatinins über einem Ausgangswert erfahren hat, der vor dem Zeitpunkt bestimmt wurde, zu dem die Körperflüssigkeitsprobe erhalten wurde,

(j) das Subjekt eine Urinausscheidung von mindestens 0,5 ml/kg/Std. über die 12 Stunden vor dem Zeitpunkt hat, zu dem die Körperflüssigkeitsprobe erhalten wurde,

(k) das Subjekt (i) keinen 2-fachen oder größeren Anstieg des Serumkreatinins über einem Ausgangswert erfahren hat, der vor dem Zeitpunkt bestimmt wurde, zu dem die Körperflüssigkeitsprobe erhalten wurde, (ii) eine Urinausscheidung von mindestens 0,5 ml/kg/Std. über die 2 Stunden vor dem Zeitpunkt hat, zu dem die Körperflüssigkeitsprobe erhalten wurde, und (iii) keinen Anstieg des Serumkreatinins von 0,3 mg/dl oder größer über einem Ausgangswert erfahren hat, der vor dem Zeitpunkt bestimmt wurde, zu dem die Körperflüssigkeitsprobe erhalten wurde,

(l) das Subjekt keinen 3-fachen oder größeren Anstieg des Serumkreatinins über einem Ausgangswert erfahren hat, der vor dem Zeitpunkt bestimmt wurde, zu dem die Körperflüssigkeitsprobe erhalten wurde,

(m) das Subjekt eine Urinausscheidung von mindestens 0,3 ml/kg/Std. über die 24 Stunden vor dem Zeitpunkt hat, zu dem die Körperflüssigkeitsprobe erhalten wurde, oder Anurie über die 12 Stunden vor dem Zeitpunkt hat, zu dem die Körperflüssigkeitsprobe erhalten wurde, oder

(n) das Subjekt (i) keinen 3-fachen oder größeren Anstieg des Serumkreatinins über einem Ausgangswert erfahren hat, der vor dem Zeitpunkt bestimmt wurde, zu dem die Körperflüssigkeitsprobe erhalten wurde, (ii) eine Urinausscheidung von mindestens 0,3 ml/kg/Std. über die 24 Stunden vor dem Zeitpunkt hat, zu dem die Körperflüssigkeitsprobe erhalten wurde, oder Anurie über die 12 Stunden vor dem Zeitpunkt hat, zu dem die Körperflüssigkeitsprobe erhalten wurde, und (iii) keinen Anstieg des Serumkreatinins von 0,3 mg/dl oder größer über einem Ausgangswert erfahren hat, der vor dem Zeitpunkt bestimmt wurde, zu dem die Körperflüssigkeitsprobe erhalten wurde.

11. Verfahren nach einem der Ansprüche 1-10, wobei die Körperflüssigkeitsprobe eine Urinprobe ist.

12. Verfahren nach einem der Ansprüche 1-11, wobei das Verfahren die Durchführung von Untersuchungen umfasst, die eines, zwei oder drei oder mehr von Fibroblasten-Wachstumsfaktor 21, Proheparin-bindendem EGF-ähnlichem Wachstumsfaktor, Tenascin C, Angiopoietin-verwandtem Protein 4, Fibroblasten-Wachstumsfaktor 19, Heparinbindendem Wachstumsfaktor 1, Angiopoietin-verwandtem Protein 6, Proepiregulin, Probetacellulin, Amphiregulin, Angiogenin, Thrombospondin-2 und Kollagen-alpha-1(XVIII)-Kette nachweisen.

13. Verwendung von Fibroblasten-Wachstumsfaktor 21 und optional einem oder mehreren Biomarkern, die aus der Gruppe ausgewählt sind bestehend aus Proheparin-bindendem EGF-ähnlichem Wachstumsfaktor, Tenascin C, Angiopoietinverwandtem Protein 4, Fibroblasten-Wachstumsfaktor 19, Heparin-bindendem Wachstumsfaktor 1, Angiopoietinverwandtem Protein 6, Proepiregulin, Probetacellulin, Amphiregulin, Angiogenin, Thrombospondin-2 und Kollagen-alpha-1(XVIII)-Kette, für die Bewertung von zukünftigem akutem Nierenversagen, wobei das zukünftige akute Nierenversagen wahrscheinlich innerhalb einer Zeitdauer von 24 Stunden von dem Zeitpunkt auftritt, zu dem die Körperflüssigkeitsprobe von dem Subjekt erhalten wurde.

14. Verwendung nach Anspruch 13, die die Verwendung eines Kits umfasst, umfassend:

Reagentien für die Durchführung von einer oder mehreren Untersuchungen, die zum Nachweis von Fibroblasten-Wachstumsfaktor 21 und optional einem oder mehreren Biomarkern ausgelegt ist, die aus der Gruppe ausgewählt sind, die aus Folgenden besteht: Proheparin-bindendem EGF-ähnlichem Wachstumsfaktor, Tenascin C, Angiopoietinverwandtem Protein 4, Fibroblasten-Wachstumsfaktor 19, Heparin-bindendem Wachstumsfaktor 1, Angiopoietin-verwandtem Protein 6, Proepiregulin, Probetacellulin, Amphiregulin, Angiogenin, Thrombospondin-2 und Kollagen-alpha-1 (XVIII)-Kette.

## Revendications

1. Procédé d'évaluation du statut rénal chez un sujet, comprenant :

réaliser un ou plusieurs dosages configurés pour détecter un ou plusieurs biomarqueurs sur un échantillon de fluide corporel obtenu auprès du sujet pour fournir un résultat de dosage ; et

mettre en corrélation le(s) résultat(s) de dosage avec le statut rénal du sujet,

dans lequel ladite étape de mise en corrélation comprend attribuer une probabilité d'un futur changement de statut rénal au sujet en fonction du/des résultat(s) de dosage,

dans lequel lesdits un ou plusieurs futurs changements de statut rénal comprennent une future insuffisance rénale aiguë (ARF), et

la probabilité du futur changement de statut rénal est qu'un événement pertinent est susceptible de se produire dans une période de 24 heures de l'heure à laquelle l'échantillon de fluide corporel est obtenu auprès du sujet, dans lequel le ou les biomarqueurs comprennent le facteur de croissance de fibroblastes 21, et en option un ou plusieurs éléments parmi le facteur de croissance de type EGF de liaison à la prohéparine, la ténascine C, la protéine 4 liée à l'angiopoïétine, le facteur de croissance de fibroblastes 19, le facteur de croissance de liaison à l'héparine 1, la protéine 6 liée à l'angiopoïétine, la proépiréguline, la probêtacelluline, l'amphiréguline, l'angiogénine, la thrombospondine-2 et la chaîne alpha-1(XVIII) de collagène.

2. Procédé selon la revendication 1, dans lequel ladite étape de mise en corrélation comprend

(a) attribuer un diagnostic de l'occurrence ou la non occurrence d'un ou plusieurs éléments parmi une lésion de la fonction rénale, une fonction rénale réduite ou une ARF au sujet en fonction du/des résultat(s) de dosage,
(b) évaluer si la fonction rénale s'améliore ou se dégrade ou non chez un sujet qui a souffert d'une lésion de la fonction rénale, d'une fonction rénale réduite ou d'une ARF en fonction du/des résultat(s) de dosage,
(c) attribuer un ou plusieurs éléments parmi : une probabilité que dans les 24 heures, le sujet (i) subira une augmentation de 1,5 fois ou supérieure de la créatinine sérique, (ii) aura un débit urinaire de moins de 0,5 ml/kg/h sur une période de 6 heures, ou (iii) subira une augmentation de 0,3 mg/dl ou supérieure de la créatinine sérique,
(d) attribuer un ou plusieurs éléments parmi : une probabilité que dans les 24 heures, le sujet (i) subira une augmentation de 2 fois ou supérieure de la créatinine sérique, (ii) aura un débit urinaire de moins de 0,5 ml/kg/h sur une période de 6 heures, ou (iii) subira une augmentation de 2 fois ou supérieure de la créatinine sérique, ou
(e) attribuer un ou plusieurs éléments parmi : une probabilité que dans les 24 heures, le sujet (i) subira une augmentation de 3 fois ou supérieure de la créatinine sérique, ou (ii) aura un débit urinaire de moins de 0,3 ml/kg/h sur une période de 24 heures ou une anurie sur une période de 12 heures.

3. Procédé selon une des revendications 1 et 2, dans lequel lesdits résultats de dosage comprennent au moins 2, 3 ou 4 éléments parmi :

une concentration mesurée de facteur de croissance de fibroblastes 21,
une concentration mesurée de facteur de croissance de type EGF de liaison à la prohéparine,
une concentration mesurée de ténascine C,
une concentration mesurée de protéine 4 liée à l'angiopoïétine,
une concentration mesurée de facteur de croissance de fibroblastes 19,
une concentration mesurée de facteur de croissance de liaison à l'héparine 1,
une concentration mesurée de protéine 6 liée à l'angiopoïétine,
une concentration mesurée de proépiréguline,
une concentration mesurée de probêtacelluline,
une concentration mesurée d'amphiréguline,
une concentration mesurée d'angiogénine,
une concentration mesurée de thrombospondine-2, et
une concentration mesurée de chaîne alpha-1(XVIII) de collagène.

4. Procédé selon une des revendications 1 à 3, dans lequel une pluralité de résultats de dosage est combinée en utilisant une fonction qui convertit la pluralité de résultats de dosage en un seul résultat composite.

5. Procédé selon la revendication 1, dans lequel ledit futur changement de statut rénal comprend un résultat clinique lié à une lésion rénale subie par le sujet.

6. Procédé selon une des revendications 1 à 3, dans lequel le sujet est sélectionné pour l'évaluation du statut rénal en fonction de

(a) la préexistence chez le sujet d'un ou plusieurs facteurs de risque connus pour une ARF prérénale, rénale intrinsèque ou post-rénale, ou
(b) un diagnostic existant d'un ou plusieurs éléments parmi une insuffisance cardiaque congestive, l'éclampsisme, l'éclampsie, le diabète sucré, l'hypertension, une maladie coronarienne, une protéinurie, une insuffisance rénale, une filtration glomérulaire en dessous de la plage normale, une cirrhose, une créatinine sérique au-dessus de la plage normale, une sepsie, une lésion de la fonction rénale, une fonction rénale réduite ou une ARF, ou en fonction du fait de subir ou d'avoir subi une chirurgie vasculaire majeure, un pontage coronarien ou une autre chirurgie cardiaque, ou en fonction de l'exposition à des AINS, à des cyclosporines, au tacrolimus, à des aminoglycosides, au foscarnet, à l'éthylèneglycol, à l'hémoglobine, à la myoglobine, à l'ifosfamide, à des métaux lourds, au méthotrexate, à des agents de contraste radio-opaques ou à la streptozotocine.

**7.** Procédé selon une des revendications 1 à 3, dans lequel ledit procédé est un procédé de diagnostic de l'occurrence ou la non occurrence d'insuffisance rénale aiguë chez ledit sujet,

un procédé de diagnostic de l'occurrence ou la non occurrence d'un besoin de thérapie de remplacement rénal chez ledit sujet,

un procédé de diagnostic de l'occurrence ou la non occurrence d'un besoin de transplantation rénale chez ledit sujet,

un procédé d'attribution d'un risque de l'occurrence ou la non occurrence future d'insuffisance rénale aiguë chez ledit sujet,

un procédé d'attribution d'un risque de l'occurrence ou la non occurrence future d'un besoin de thérapie de remplacement rénal chez ledit sujet, ou

un procédé d'attribution d'un risque de l'occurrence ou la non occurrence future d'un besoin de transplantation rénale chez ledit sujet.

**8.** Procédé selon une des revendications 1 à 3, dans lequel ledit futur changement de statut rénal comprend une ARF dans les 12 heures de l'heure à laquelle l'échantillon de fluide corporel est obtenu.

**9.** Procédé selon une des revendications 1 à 3, dans lequel

(A) le sujet est en RIFLE stade 0 ou R, dans lequel

(a) le sujet est en option en RIFLE stade 0, et ladite étape de mise en corrélation comprend attribuer une probabilité que le sujet atteindra RIFLE stade R, I ou F dans les 24 heures,

dans lequel le sujet est en option en RIFLE stade 0, et ladite étape de mise en corrélation comprend attribuer une probabilité que le sujet atteindra RIFLE stade I ou F dans les 24 heures,

(b) le sujet est en option en RIFLE stade 0 ou R, et ladite étape de mise en corrélation comprend attribuer une probabilité que le sujet atteindra RIFLE stade I ou F dans les 24 heures, ou

(c) le sujet est en option en RIFLE stade R, et ladite étape de mise en corrélation comprend attribuer une probabilité que le sujet atteindra RIFLE stade I ou F dans les 24 heures, ou

(B) dans lequel le sujet est en RIFLE stade 0, R ou I, et ladite étape de mise en corrélation comprend attribuer une probabilité que le sujet atteindra RIFLE stade F dans les 24 heures.

**10.** Procédé selon une des revendications 1 à 3, dans lequel

(a) le sujet n'est pas en insuffisance rénale aiguë,

(b) le sujet n'a pas subi une augmentation de 1,5 fois ou supérieure de la créatinine sérique sur une valeur en conditions basales déterminée avant l'heure à laquelle l'échantillon de fluide corporel est obtenu,

(c) le sujet a un débit urinaire d'au moins 0,5 ml/kg/h sur les 6 heures précédant l'heure à laquelle l'échantillon de fluide corporel est obtenu,

(d) le sujet n'a pas subi une augmentation de 0,3 mg/dl ou supérieure de la créatinine sérique sur une valeur en conditions basales déterminée avant l'heure à laquelle l'échantillon de fluide corporel est obtenu,

(e) le sujet (i) n'a pas subi une augmentation de 1,5 fois ou supérieure de la créatinine sérique sur une valeur en conditions basales déterminée avant l'heure à laquelle l'échantillon de fluide corporel est obtenu, (ii) a un débit urinaire d'au moins 0,5 ml/kg/h sur les 6 heures précédant l'heure à laquelle l'échantillon de fluide corporel est obtenu, et (iii) n'a pas subi une augmentation de 0,3 mg/dl ou supérieure de la créatinine sérique sur une valeur en conditions basales déterminée avant l'heure à laquelle l'échantillon de fluide corporel est obtenu,

(f) le sujet n'a pas subi une augmentation de 1,5 fois ou supérieure de la créatinine sérique sur une valeur en conditions basales déterminée avant l'heure à laquelle l'échantillon de fluide corporel est obtenu,

(g) le sujet a un débit urinaire d'au moins 0,5 ml/kg/h sur les 6 heures précédant l'heure à laquelle l'échantillon de fluide corporel est obtenu,

(h) le sujet (i) n'a pas subi une augmentation de 1,5 fois ou supérieure de la créatinine sérique sur une valeur en conditions basales déterminée avant l'heure à laquelle l'échantillon de fluide corporel est obtenu, (ii) a un débit urinaire d'au moins 0,5 ml/kg/h sur les 12 heures précédant l'heure à laquelle l'échantillon de fluide corporel est obtenu, et (iii) n'a pas subi une augmentation de 0,3 mg/dl ou supérieure de la créatinine sérique sur une valeur en conditions basales déterminée avant l'heure à laquelle l'échantillon de fluide corporel est obtenu,

(i) le sujet n'a pas subi une augmentation de 2 fois ou supérieure de la créatinine sérique sur une valeur en conditions basales déterminée avant l'heure à laquelle l'échantillon de fluide corporel est obtenu,

(j) le sujet a un débit urinaire d'au moins 0,5 ml/kg/h sur les 12 heures précédant l'heure à laquelle l'échantillon de fluide corporel est obtenu,

(k) le sujet (i) n'a pas subi une augmentation de 2 fois ou supérieure de la créatinine sérique sur une valeur en conditions basales déterminée avant l'heure à laquelle l'échantillon de fluide corporel est obtenu, (ii) a un débit urinaire d'au moins 0,5 ml/kg/h sur les 2 heures précédant l'heure à laquelle l'échantillon de fluide corporel est obtenu, et (iii) n'a pas subi une augmentation de 0,3 mg/dl ou supérieure de la créatinine sérique sur une valeur en conditions basales déterminée avant l'heure à laquelle l'échantillon de fluide corporel est obtenu,

(l) le sujet n'a pas subi une augmentation de 3 fois ou supérieure de la créatinine sérique sur une valeur en conditions basales déterminée avant l'heure à laquelle l'échantillon de fluide corporel est obtenu,

(m) le sujet a un débit urinaire d'au moins 0,3 ml/kg/h sur les 24 heures précédant l'heure à laquelle l'échantillon de fluide corporel est obtenu, ou une anurie sur les 12 heures précédant l'heure à laquelle l'échantillon de fluide corporel est obtenu, ou

(n) le sujet (i) n'a pas subi une augmentation de 3 fois ou supérieure de la créatinine sérique sur une valeur en conditions basales déterminée avant l'heure à laquelle l'échantillon de fluide corporel est obtenu, (ii) a un débit urinaire d'au moins 0,3 ml/kg/h sur les 24 heures précédant l'heure à laquelle l'échantillon de fluide corporel est obtenu, ou une anurie sur les 12 heures précédant l'heure à laquelle l'échantillon de fluide corporel est obtenu, et (iii) n'a pas subi une augmentation de 0,3 mg/dl ou supérieure de la créatinine sérique sur une valeur en conditions basales déterminée avant l'heure à laquelle l'échantillon de fluide corporel est obtenu.

11. Procédé selon une des revendications 1 à 10, dans lequel l'échantillon de fluide corporel est un échantillon d'urine.

12. Procédé selon une des revendications 1 à 11, dans lequel ledit procédé comprend réaliser des dosages qui détectent un, deux ou trois, ou plus, éléments parmi le facteur de croissance de fibroblastes 21, le facteur de croissance de type EGF de liaison à la prohéparine, la ténascine C, la protéine 4 liée à l'angiopoïétine, le facteur de croissance de fibroblastes 19, le facteur de croissance de liaison à l'héparine 1, la protéine 6 liée à l'angiopoïétine, la proépi-réguline, la probêtacelluline, l'amphiréguline, l'angiogénine, la thrombospondine-2 et la chaîne alpha-1(XVIII) de collagène.

13. Utilisation du facteur de croissance de fibroblastes 21, et en option d'un ou plusieurs biomarqueurs sélectionnés parmi le groupe constitué du facteur de croissance de type EGF de liaison à la prohéparine, de la ténascine C, de la protéine 4 liée à l'angiopoïétine, du facteur de croissance de fibroblastes 19, du facteur de croissance de liaison à l'héparine 1, de la protéine 6 liée à l'angiopoïétine, de la proépiréguline, de la probêtacelluline, de l'amphiréguline, de l'angiogénine, de la thrombospondine-2 et de la chaîne alpha-1(XVIII) de collagène pour l'évaluation d'une future insuffisance rénale aiguë, dans laquelle la future insuffisance rénale aiguë est susceptible de se produire dans une période de 24 heures de l'heure à laquelle l'échantillon de fluide corporel est obtenu auprès d'un sujet.

14. Utilisation selon la revendication 13 comprenant utiliser un kit, comprenant :

des réactifs pour réaliser un ou plusieurs dosages configurés pour détecter le facteur de croissance de fibroblastes 21, et en option un ou plusieurs biomarqueurs sélectionnés parmi le groupe constitué du facteur de croissance de type EGF de liaison à la prohéparine, de la ténascine C, de la protéine 4 liée à l'angiopoïétine, du facteur de croissance de fibroblastes 19, du facteur de croissance de liaison à l'héparine 1, de la protéine 6 liée à l'angiopoïétine, de la proépiréguline, de la probêtacelluline, de l'amphiréguline, de l'angiogénine, de la thrombospondine-2 et de la chaîne alpha-1(XVIII) de collagène.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010059996 A
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

**Non-patent literature cited in the description**

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med,* 2006, vol. 7 ((4)), 177-197
- **THAKAR et al.** *J. Am. Soc. Nephrol,* 2005, vol. 16, 162-68
- **MEHRAN et al.** *J. Am. Coll. Cardiol,* 2004, vol. 44, 1393-99
- **WIJEYSUNDERA et al.** *JAMA,* 2007, vol. 297, 1801-9
- **GOLDSTEIN ; CHAWLA.** *Clin. J. Am. Soc. Nephrol.,* 2010, vol. 5, 943-49
- **CHAWLA et al.** *Kidney Intl.,* 2005, vol. 68, 2274-80
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** *Nephrol. Dial. Transplant.,* 2008, vol. 23, 1203-1210
- Merck Manual of Diagnosis and Therapy. Merck Research Laboratories, 1999
- **HANLEY, J. A. ; MCNEIL, B.J.** The meaning and use of the area under a receiver operating characteristic (ROC) curve. *Radiology,* 1982, vol. 143, 29-36